# EUROPEAN PATENT APPLICATION

(11) **EP 2 336 104 A1**
(43) Date of publication of application: **22.06.2011**
(21) Application number: 09811514.0
(22) Date of filing: 02.09.2009
(51) Int. Cl.: C07D 205/08, A01N 43/36, A01N 43/40, A01N 43/44, A01P 13/02, C07D 207/27, C07D 211/72, C07D 211/76, C07D 213/64, C07D 221/20, C07D 223/10, C07D 239/06, C07D 239/10, C07D 243/04, C07D 491/044

(54) **ORTHO-SUBSTITUTED HALOALKYLSULFONANILIDE DERIVATIVE AND HERBICIDE**

(30) Priority: 02.09.2008 JP 2008225137; 07.10.2008 JP 2008260652; 13.02.2009 JP 2009031818; 18.03.2009 JP 2009066161; 18.03.2009 JP 2009066163; 24.06.2009 JP 2009149403
(71) Applicant: Nissan Chemical Industries, Ltd., Chiyoda-ku Tokyo 101-0054 (JP)
(72) Inventor: KUDOU, Takao, Funabashi-shi Chiba 274-8507 (JP); TANIMA, Daisuke, Funabashi-shi Chiba 274-8507 (JP); MASUZAWA, Yoshihide, Funabashi-shi Chiba 274-8507 (JP); YANO, Tetsuhiko, Minamisaitama-gun Saitama 349-0294 (JP)
(74) Representative: Blodig, Wolfgang
(86) International application number: PCT/JP2009/065343
(87) International publication number: WO 2010/026989

(57) **Abstract**

Novel herbicides are provided.

A haloalkylsulfonanilide derivative represented by the formula (1) or an agrochemically acceptable salt thereof:
the formula (1): 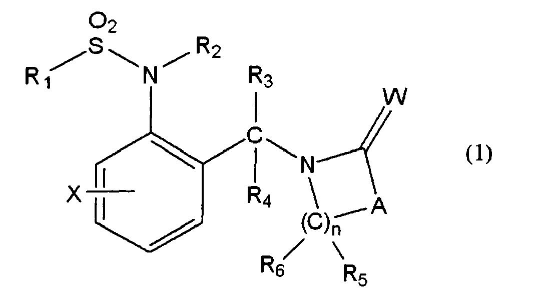 wherein A is -C(R₇)(R₈)- or -N(R₉)-,
W is an oxygen atom or a sulfur atom, n is an integer of from 1 to 4, R₁ is halo C₁ -C₆ alkyl, R₂ is a hydrogen atom, C₁ - C₆ alkyl or the like, each of R₃ and R₄ is independently a hydrogen atom, C₁ - C₆ alkyl or the like, each of R₅, R₆, R₇ and R₈ is independently a hydrogen atom, a halogen, C₁ - C₆ alkyl or the like, R₉ is a hydrogen atom, C₁ - C₆ alkyl or the like, and X is halogen, C₁ - C₆ alkyl or the like.

## Description

### TECHNICAL FIELD

The present invention relates to haloalkylsulfonanilide derivatives and agrochemicals, especially herbicides, containing them.

### BACKGROUND ART

Some haloalkylsulfonanilide derivatives are known to have herbicidal activities (Patent Documents 1 to 3), but nothing has been disclosed about the 4- to 7-membered lactam-N-alkyl substituted haloalkylsulfoanilide structure and the 4- to 7-membered urea-N-alkyl substituted haloalkylsulfoanilide structure of the present invention.

### PRIOR ART DOCUMENTS

### PATENT DOCUMENTS

Patent Document 1: WO 04/011429
Patent Document 2: JP-A-2008-74840
Patent Document 3: WO 08/059948

### DISCLOSURE OF INVENTION

### TECHNICAL PROBLEM

The object of the present invention is to provide haloalkylsulfonanilide derivatives and effective agrochemicals, especially herbicides, containing such a compound as an active ingredient.

### SOLUTION TO PROBLEM

The present inventors conducted extensive research to attain the above-mentioned object and, as a result, found novel haloalkylsulfonanilide derivatives and their herbicidal activities and crop specificities. The present invention was accomplished on the basis of the discovery.

Namely, the present invention provides the compound according to the following [1] to [8] (hereinafter referred to also as the compound of the present invention), the agrochemical according to the following [9], the herbicide according to the following [10] and the intermediates according to the following [11] and [12].
[1] A haloalkylsulfonanilide derivative represented by the formula (1) or an agrochemically acceptable salt thereof:
   the formula (1): 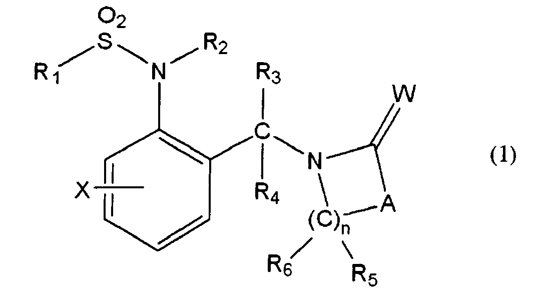 wherein A is -C(R₇)(R₈)- or -N(R₉)-,
      W is an oxygen atom or a sulfur atom,
      n is an integer of from 1 to 4,
      R₁ is halo C₁ - C₆ alkyl,
      R₂ is a hydrogen atom, C₁ - C₆ alkyl, halo C₁ - C₆ alkyl, C₂ - C₆ alkenyl, halo C₂ -C₆ alkenyl, C₂ - C₆ alkynyl, halo C₂ - C₆ alkynyl, C₁ - C₆ alkoxy C₁ - C₆ alkyl, halo C₁ -C₆ alkoxy C₁ - C₆ alkyl, C₁ - C₆ alkoxy C₁ - C₆ alkoxy C₁ - C₆ alkyl, tri C₁ - C₆ alkylsilyl C₁ - C₆ alkoxy C₁ - C₆ alkyl, phenyl C₁ - C₆ alkyl, substituted phenyl C₁ - C₆ alkyl having at least one substituent selected from Y, C₁- C₁₂ alkylcarbonyl, halo C₁ - C₁₂ alkylcarbonyl, cyclo C₃ - C₆ alkylcarbonyl, cyclo C₃ - C₆ alkyl C₁ - C₆ alkylcarbonyl, C₂ - C₆ alkenylcarbonyl, phenylcarbonyl, substituted phenylcarbonyl having at least one substituent selected from Y, heterocyclic carbonyl, substituted heterocyclic carbonyl having at least one substituent selected from Y, C₁ - C₁₂ alkoxycarbonyl, halo C₁ - C₁₂ alkoxycarbonyl, C₁ - C₆ alkoxy C₁ - C₆ alkoxycarbonyl, C₁ - C₆ alkylthio C₁ - C₆ alkoxycarbonyl, C₁- C₆ alkylsulfinyl C₁ - C₆ alkoxycarbonyl, C₁- C₆ alkylsulfonyl C₁- C₆ alkoxycarbonyl, C₂ - C₆ alkenyloxycarbonyl, halo C₂ - C₆ alkenyloxycarbonyl, C₂ - C₆ alkynyloxycarbonyl, phenoxycarbonyl, substituted phenoxycarbonyl having at least one substituent selected from Y, phenyl C₁ - C₆ alkoxycarbonyl, substituted phenyl C₁ - C₆ alkoxycarbonyl having at least one substituent selected from Y, phenoxy C₁ - C₆ alkylcarbonyl, substituted phenoxy C₁ - C₆ alkylcarbonyl having at least one substituent selected from Y, mono(C₁ - C₆ alkyl)aminocarbonyl, mono(halo C₁ - C₆ alkyl)aminocarbonyl, symmetric or asymmetric di(C₁ - C₆ alkyl)aminocarbonyl, symmetric or asymmetric halo di(C₁ - C₆ alkyl)aminocarbonyl, C₁ - C₆ alkylthiocarbonyl, halo C₁- C₆ alkylthiocarbonyl, C₁ - C₆ alkylsulfonyl, halo C₁- C₆ alkylsulfonyl, phenylsulfonyl, substituted phenylsulfonyl having at least one substituent selected from Y, C₁ - C₆ alkylthio C₁- C₆ alkyl, halo C₁- C₆ alkylthio C₁- C₆ alkyl, phenyl C₁- C₆ alkoxy C₁- C₆ alkyl, substituted phenyl C₁- C₆ alkoxy C₁- C₆ alkyl having at least one substituent selected from Y, phenylthio C₁- C₆ alkyl, substituted phenylthio C₁ - C₆ alkyl having at least one substituent selected from Y, phenyl C₁- C₆ alkylthio C₁- C₆ alkyl, substituted phenyl C₁- C₆ alkylthio C₁- C₆ alkyl having at least one substituent selected from Y, phenylsulfonyl C₁ - C₆ alkyl, substituted phenylsulfonyl C₁ - C₆ alkyl having at least one substituent selected from Y, phenyl C₁ - C₆ alkylsulfonyl C₁ - C₆ alkyl, substituted phenyl C₁ - C₆ alkylsulfonyl C₁ - C₆ alkyl having at least one substituent selected from Y, C₁- C₆ alkylcarbonyloxy C₁- C₆ alkyl, phenylcarbonyloxy C₁- C₆ alkyl, substituted phenylcarbonyloxy C₁ - C₆ alkyl having at least one substituent selected from Y, phenylcarbonyl C₁ - C₆ alkyl, substituted phenylcarbonyl C₁ - C₆ alkyl having at least one substituent selected from Y, C₁ - C₆ alkoxycarbonyloxy C₁ - C₆ alkyl, phenylcarbonyloxy C₁ - C₆ alkoxy C₁ - C₆ alkyl, substituted phenylcarbonyloxy C₁ - C₆ alkoxy C₁ - C₆ alkyl having at least one substituent selected from Y, mono C₁ - C₆ alkylaminocarbonyloxy C₁- C₆ alkyl, symmetric or asymmetric di(C₁- C₆ alkyl)aminocarbonyloxy C₁ - C₆ alkyl, phenylaminocarbonyloxy C₁ - C₆ alkyl, substituted phenylaminocarbonyloxy C₁ - C₆ alkyl having at least one substituent selected from Y, C₁- C₆ alkyl(phenyl)aminocarbonyloxy C₁- C₆ alkyl, substituted C₁- C₆ alkyl(phenyl)aminocarbonyloxy C₁ - C₆ alkyl having at least one substituent selected from Y, C₁ - C₆ alkylcarbonyl C₁ - C₆ alkyl, C₁ - C₆ alkoxycarbonyl C₁ - C₆ alkyl, halo C₁ - C₆ alkylthio, symmetric or asymmetric di(C₁ - C₆ alkyl)aminothio or cyano,
      each of R₃ and R₄ is independently a hydrogen atom, C₁- C₆ alkyl, halo C₁ - C₆ alkyl, cyclo C₃ - C₆ alkyl, halocyclo C₃ - C₆ alkyl, C₁ - C₆ alkoxy, halo C₁ - C₆ alkoxy, halogen or cyano, or R₃ and R₄ may form a 3- to 7-membered ring together with each other,
      each of R₅, R₆, R₇ and R₈ is independently a hydrogen atom, a halogen, C₁- C₆ alkyl, halo C₁- C₆ alkyl, C₂ - C₆ alkenyl, C₂ - C₆ alkynyl, cyclo C₃ - C₆ alkyl, halocyclo C₃ - C₆ alkyl, C₁ - C₆ alkoxy C₁ - C₆ alkyl, halo C₁ - C₆ alkoxy C₁ - C₆ alkyl, hydroxy C₁ - C₆ alkyl, C₁- C₆ alkylthio C₁- C₆ alkyl, halo C₁- C₆ alkylthio C₁- C₆ alkyl, mono(C₁ - C₆ alkyl)amino C₁ - C₆ alkyl, symmetric or asymmetric di(C₁ - C₆ alkyl)amino C₁ - C₆ alkyl, phenyl, substituted phenyl having at least one substituent selected from Y, heterocyclyl, substituted heterocyclyl having at least one substituent selected from Y, phenyl C₁ - C₆ alkyl, substituted phenyl C₁ - C₆ alkyl having at least one substituent selected from Y, phenoxy C₁- C₆ alkyl, substituted phenoxy C₁ - C₆ alkyl having at least one substituent selected from Y, C₁ - C₆ alkylcarbonyl, halo C₁ - C₆ alkylcarbonyl, phenylcarbonyl, substituted phenylcarbonyl having at least one substituent selected from Y, C₁ - C₆ alkoxycarbonyl, halo C₁ - C₆ alkoxycarbonyl, carboxyl, mono(C₁ - C₆ alkyl)aminocarbonyl, symmetric or asymmetric di(C₁ - C₆ alkyl)aminocarbonyl, phenylaminocarbonyl, substituted phenylaminocarbonyl having at least one substituent selected from Y on the ring, phenyl C₁ - C₆ alkylaminocarbonyl, substituted phenyl C₁ -C₆ alkylaminocarbonyl having at least one substituent selected from Y on the ring, C₁ - C₆ alkoxy, halo C₁ - C₆ alkoxy, phenoxy, substituted phenoxy having at least one substituent selected from Y on the ring, C₁ - C₆ alkylthio, halo C₁ - C₆ alkylthio, phenylthio, substituted phenylthio having at least one substituent selected from Y on the ring, C₁ - C₆ alkylsulfonyl, halo C₁ - C₆ alkylsulfonyl, mono(C₁ - C₆ alkyl)amino, di(C₁ - C₆ alkyl)amino, C₁ - C₆ alkylcarbonyloxy, hydroxy, amino, cyano or nitro,
      R₉ is a hydrogen atom, C₁ - C₆ alkyl, halo C₁ - C₆ alkyl, cyclo C₃ - C₆ alkyl, halocyclo C₃ - C₆ alkyl, C₂ - C₆ alkenyl, halo C₂ - C₆ alkenyl, C₂ - C₆ alkynyl, C₁ - C₆ alkoxy C₁ - C₆ alkyl, halo C₁ - C₆ alkoxy C₁ - C₆ alkyl, hydroxy C₁ - C₆ alkyl, cyclo C₃ - C₆ alkyl C₁ - C₆ alkyl, phenyl C₁ - C₆ alkyl, substituted phenyl C₁ - C₆ alkyl having at least one substituent selected from Y on the ring, phenyl, substituted phenyl having at least one substituent selected from Y on the ring, phenoxy C₁ - C₆ alkyl, substituted phenoxy C₁ - C₆ alkyl having at least one substituent selected from Y on the ring, C₁ - C₆ alkylcarbonyl C₁ - C₆ alkyl, halo C₁ - C₆ alkylcarbonyl C₁ - C₆ alkyl, C₁ - C₆ alkoxycarbonyl C₁ - C₆ alkyl, halo C₁ - C₆ alkoxycarbonyl C₁ - C₆ alkyl, C₁ - C₆ alkylcarbonyl, halo C₁ - C₆ alkylcarbonyl, cyclo C₃ - C₆ alkylcarbonyl, cyclo C₃ - C₆ alkyl C₁ - C₆ alkylcarbonyl, C₂ - C₆ alkenylcarbonyl, phenylcarbonyl, substituted phenylcarbonyl having at least one substituent selected from Y on the ring, heterocyclic carbonyl, substituted heterocyclic carbonyl having at least one substituent selected from Y, C₁ - C₆ alkoxycarbonyl, halo C₁ - C₆ alkoxycarbonyl, phenoxycarbonyl, substituted phenoxycarbonyl having at least one substituent selected from Y, aminocarbonyl, mono(C₁ - C₆ alkyl)aminocarbonyl, monohalo(C₁ - C₆ alkyl)aminocarbonyl, symmetric or asymmetric di(C₁ - C₆ alkyl)aminocarbonyl, symmetric or asymmetric halo di(C₁ - C₆ alkyl)aminocarbonyl, C₁ - C₆ alkylthiocarbonyl, halo C₁ - C₆ alkylthiocarbonyl, C₁ - C₆ alkylsulfonyl, halo C₁ - C₆ alkylsulfonyl, phenylsulfonyl, substituted phenylsulfonyl having at least one substituent selected from Y, C₁ - C₆ alkylthio C₁ - C₆ alkyl, halo C₁ - C₆ alkylthio C₁ - C₆ alkyl, phenylthio C₁ - C₆ alkyl, substituted phenylthio C₁ - C₆ alkyl having at least one substituent selected from Y, C₁ - C₆ alkylsulfinyl C₁ - C₆ alkyl, halo C₁ - C₆ alkylsulfinyl C₁ - C₆ alkyl, phenylsulfinyl C₁ - C₆ alkyl, substituted phenylsulfinyl C₁ - C₆ alkyl having at least one substituent selected from Y, C₁ - C₆ alkylsulfonyl C₁ - C₆ alkyl, halo C₁ - C₆ alkylsulfonyl C₁ - C₆ alkyl, phenylsulfonyl C₁ - C₆ alkyl, substituted phenylsulfonyl C₁ - C₆ alkyl having at least one substituent selected from Y, cyano, amino or hydroxy, or
      R₅, R₆, R₇ or R₈ may form, together with R₅, R₆, R₇ or R₈ on the same carbon, an optionally substituted 3- to 7-membered ring which may contain one or two hetero atoms selected from oxygen atoms, sulfur atoms and nitrogen atoms (the nitrogen atoms may be substituted with C₁ - C₆ alkyl, C₂ - C₆ alkenyl, C₂ - C₆ alkynyl or cyclo C₃ - C₆ alkyl), an olefin or carbonyl, or
      R₅, R₆, R₇ or R₈ may form, together with R₅, R₆, R₇ or R₈ on a different carbon, an optionally substituted 3- to 8-membered ring which may contain one or two hetero atoms selected from oxygen atoms, sulfur atoms and nitrogen atoms (the nitrogen atoms may be substituted with C₁ - C₆ alkyl, C₂ - C₆ alkenyl, C₂ - C₆ alkynyl or cyclo C₃ - C₆ alkyl), or
      R₅ or R₆ may form a bond together with R₅ or R₆ on an adjacent carbon,
      when n is from 2 to 4, R₅ or R₆ may be the same as or different from R₅ or R₆ on an adjacent carbon,
      X is halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, phenyl, substituted phenyl having at least one substituent selected from Y on the ring, heterocyclyl, substituted heterocyclyl having at least one substituent selected from Y on the ring, phenoxy, substituted phenoxy having at least one substituent selected from Y on the ring, phenylthio, substituted phenylthio having at least one substituent selected from Y on the ring, phenylsulfinyl, substituted phenylsulfinyl having at least one substituent selected from Y on the ring, phenylsulfonyl, substituted phenylsulfonyl having at least one substituent selected from Y on the ring, C₁ - C₆ alkylcarbonyl, halo C₁ - C₆ alkylcarbonyl, phenylcarbonyl, substituted phenylcarbonyl having at least one substituent selected from Y on the ring, C₁ - C₆ alkoxycarbonyl, halo C₁ - C₆ alkoxycarbonyl, carboxyl, mono(C₁ - C₆ alkyl)aminocarbonyl, monohalo(C₁ - C₆ alkyl)aminocarbonyl, symmetric or asymmetric di(C₁ - C₆ alkyl)aminocarbonyl, symmetric or asymmetric halo di(C₁ - C₆ alkyl)aminocarbonyl, phenylaminocarbonyl, substituted phenylaminocarbonyl having at least one substituent selected from Y on the ring, phenyl C₁ - C₆ alkylaminocarbonyl, substituted phenyl C₁ - C₆ alkylaminocarbonyl having at least one substituent selected from Y on the ring, hydroxy, amino, cyano or nitro,
      Y is a halogen, C₁- C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁- C₆ alkoxy, C₁- C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, phenyl, substituted phenyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁- C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, heterocyclyl, substituted heterocyclyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenoxy, substituted phenoxy having at least one substituent selected from the group consisting of halogen, C₁ - C₆ alkyl, C₂ - C₆ alkenyl, C₂ - C₆ alkynyl, cyclo C₃ - C₆ alkyl, halo C₁- C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxyl, amino, cyano and nitro, phenylthio, substituted phenylthio having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁- C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenylsulfinyl, substituted phenylsulfinyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁- C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenylsulfonyl, substituted phenylsulfonyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁- C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, C₁ - C₆ alkylcarbonyl, halo C₁- C₆ alkylcarbonyl, phenylcarbonyl, substituted phenylcarbonyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁- C₆ alkylthio, halo C₁-C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, C₁ - C₆ alkoxycarbonyl, carboxyl, mono(C₁ - C₆ alkyl)aminocarbonyl, symmetric or asymmetric di(C₁ - C₆ alkyl)aminocarbonyl, phenylaminocarbonyl, substituted phenylaminocarbonyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenyl C₁ - C₆ alkylaminocarbonyl, substituted phenyl C₁ - C₆ alkylaminocarbonyl having at least one substituent selected from the group consisting of halogen, C₁ - C₆ alkyl, C₂ - C₆ alkenyl, C₂ - C₆ alkynyl, cyclo C₃ - C₆ alkyl, halo C₁- C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁- C₆ alkoxycarbonyl, carboxyl, mono(C₁-C₆ alkyl)aminocarbonyl, symmetric or asymmetric di(C₁ - C₆ alkyl)aminocarbonyl, hydroxy, amino, cyano and nitro, hydroxy, amino, cyano or nitro, or
      Y may be C₁ - C₄ alkylene, halo C₁ - C₄ alkylene, C₂ - C₄ alkenylene or halo C₂ - C₄ alkenylene which may contain one or two hetero atoms selected from oxygen atoms, sulfur atoms and nitrogen atoms (the nitrogen atoms may be substituted with C₁ - C₆ alkyl, C₂ - C₆ alkenyl, C₂ - C₆ alkynyl or cyclo C₃ - C₆ alkyl) and form a 5- or 6-membered ring together with an adjacent carbon or nitrogen atom on a benzene ring or a heterocyclyl, and
      the heteroxyclyl is thienyl, furyl, pyrrolyl, oxazolyl, isoxazolyl, isoxazolinyl, thiazolyl, isothiazolyl, pyrazolyl, imidazolyl, 1,3,4-oxadiazolyl, 1,2,4-oxadiazolyl, 1,3,4-thiadiazolyl, 1,2,4-thiadiazolyl, 1,2,4-triazolyl, 1,2,3-thiadiazolyl, 1,2,3-triazolyl, 1,2,3,4-tetrazolyl, pyridyl, pyrimidinyl, pyrazinyl, pyridazinyl, 1,3,5-triazinyl, 1,2,4-triazinyl, benzothienyl, benzofuryl, indolyl, benzothiazolyl, benzoimidazolyl, benzoisoxazolyl, benzoisothiazolyl, indazolyl, benzoxazolyl, quinolyl, isoquinolyl, quinoxalinyl, phthalazinyl, cinnolinyl or quinazolinyl,
      provided that in a group having two or more substituents, the substituents may be the same or different.
[2] The haloalkylsulfonanilide derivative according to the above [1] wherein n is 3 or 4, or an agrochemically acceptable salt thereof.
[3] The haloalkylsulfonanilide derivative according to the above [1] wherein A is-C(R₇)(R₈)-, or an agrochemically acceptable salt thereof.
[4] The haloalkylsulfonanilide derivative according to the above [3] wherein n is 1, or an agrochemically acceptable salt thereof.
[5] The haloalkylsulfonanilide derivative according to the above [3] wherein n is 2, or an agrochemically acceptable salt thereof.
[6] The haloalkylsulfonanilide derivative according to the above [3] wherein n is 3, or an agrochemically acceptable salt thereof.
[7] The haloalkylsulfonanilide derivative according to the above [3] wherein n is 4, or an agrochemically acceptable salt thereof.
[8] The haloalkylsulfonanilide derivative according to any one of the above [1] to [7] wherein R₃ and R₄ are hydrogen atoms, or an agrochemically acceptable salt thereof.
[9] An agrochemical containing the haloalkylsulfonanilide derivative as defined in any one of the above [1] to [8] or an agrochemically acceptable salt thereof, as an active ingredient.
[10] A herbicide containing the haloalkylsulfonanilide derivative as defined in any one of the above [1] to [8] or an agrochemically acceptable salt thereof, as an active ingredient.
[11] An intermediate represented by the formula (2):
   the formula (2): 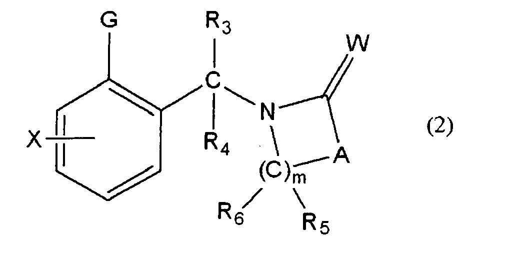 wherein A, W, R₃, R₄, R₅, R₆ and X are the same as defined above,
      m is an integer of 3 or 4, and
      G is amino or nitro,
      provided that wherein A is -C(R₇)(R₈)-, at least one of R₅, R₆, R₇ and R₈ is not a hydrogen atom.
[12] An intermediate represented by the formula (3):
   the formula (3): 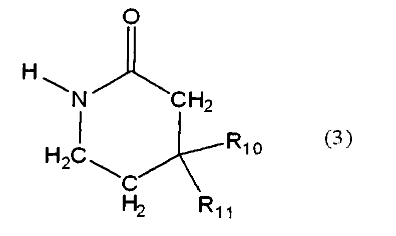 wherein R₁₀ and R₁₁ are C₁ - C₆ alkylene and form, together with each other, a 3-to 7-membered ring which may contain one or two hetero atoms selected from oxygen atoms, sulfur atoms and nitrogen atoms (the nitrogen atoms may be substituted with C₁ - C₆ alkyl, C₂ - C₆ alkenyl, C₂ - C₆ alkynyl or cyclo C₃ - C₆ alkyl).

The compounds of the present invention and agrochemically acceptable salts thereof show synergic herbicidal effect when used in the form of a mixture with a certain kind of herbicide.

### ADVANTAGEOUS EFFECTS OF INVENTION

The haloalkylsulfonanilide derivatives of the present invention have excellent herbicidal effect and are agriculturally and horticulturally useful in paddy fields, crop plant fields and orchards.

### DESCRIPTION OF EMBODIMENTS

When the compounds of the present invention have at least one asymmetric carbon atom, the present invention covers all the optical isomers, racemates and diastereomers thereof.

Now, the options for A, W, R₁, R₂, R₃, R₄, R₅, R₆, R₇, R₈, R₉, n and X will be illustrated.

As options for A, the following are, for example, mentioned:
Al: -C(R₇)(R₈)-, and
All: -N(R₉)-.

As options for W, the following are, for example, mentioned:
WI: an oxygen atom, and
WII: a sulfur atom.

As options for R₁, the following are, for example, mentioned.
R₁I: trifluoromethyl, and
R₁II: halo C₁-C₆ alkyl.

As options for R₂, the following are, for example, mentioned. R₂I: a hydrogen atom, C₁ - C₁₂ alkylcarbonyl, halo C₁ - C₁₂ alkylcarbonyl, C₂ - C₆ alkenylcarbonyl, phenylcarbonyl, substituted phenylcarbonyl having at least one substituent selected from Y, C₁ - C₁₂ alkoxycarbonyl, halo C₁ - C₁₂ alkoxycarbonyl, C₂ - C₆ alkenyloxycarbonyl, phenoxycarbonyl, C₁ - C₆ alkylthiocarbonyl or halo C₁- C₆ alkylthiocarbonyl,
R₂II: C₁ - C₆ alkyl, C₁ - C₆ alkoxy C₁ - C₆ alkyl, phenyl C₁ - C₆ alkyl, substituted phenyl C₁ - C₆ alkyl having at least one substituent selected from Y on the ring, C₁ - C₆ alkylthio C₁ - C₆ alkyl, halo C₁ - C₆ alkylthio C₁ - C₆ alkyl, phenoxy C₁ - C₆ alkyl or C₁ - C₆ alkylcarbonyloxy C₁ - C₆ alkyl, and
R₂III: C₁ - C₆ alkylsulfonyl, halo C₁ - C₆ alkylsulfonyl, phenylsulfonyl or substituted phenylsulfonyl having at least one substituent selected from Y.

As options for R₃, the following are, for example, mentioned:
R₃I: a hydrogen atom, and
R₃II: C₁ - C₆ alkyl, halo C₁ - C₆ alkyl, C₃ - C₆ cycloalkyl, halo C₃ - C₆ cycloalkyl, C₁ - C₆ alkoxy, halo C₁ - C₆ alkoxy, halogen or cyano.

As options for R₄, the following are, for example, mentioned:
R₄I: a hydrogen atom, and
R₄II: C₁ - C₆ alkyl, halo C₁- C₆ alkyl, C₃ - C₆ cycloalkyl, halo C₃ - C₆ cycloalkyl, C₁ - C₆ alkoxy, halo C₁ - C₆ alkoxy, halogen or cyano.

As options for R₅, R₆, R₇ and R₈, the following are, for example, mentioned:
R₅I, R₆I, R₇I or R₈I: a hydrogen atom, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy C₁ - C₆ alkyl, halo C₁ - C₆ alkoxy C₁ - C₆ alkyl, C₁ - C₆ alkylthio C₁ - C₆ alkyl or halo C₁ - C₆ alkylthio C₁ - C₆ alkyl,
R₅II, R₆II, R₇II or R₈II: phenyl or substituted phenyl having at least one substituent selected from Y on the ring,
R₅III, R₆III, R₇III or R₈III: halogen, C₁ - C₆ alkoxy, C₁ - C₆ alkylthio, hydroxy, amino, cyano or nitro, and
R₅IV, R₆IV, R₇IV or R₈IV: C₁ - C₆ alkylcarbonyl, halo C₁ - C₆ alkylcarbonyl, phenylcarbonyl, substituted phenylcarbonyl having at least one substituent selected from Y on the ring or C₁ - C₆ alkoxycarbonyl.

As options for R₉, the following are, for example, mentioned:
R₉I: a hydrogen atom, C₁ - C₆ alkyl, halo C₁ - C₆ alkyl, cyclo C₃ - C₆ alkyl, halocyclo C₃ -C₆ alkyl, C₁ - C₆ alkoxy C₁ - C₆ alkyl, halo C₁ - C₆ alkoxy C₁ - C₆ alkyl, phenyl C₁ - C₆ alkyl, substituted phenyl C₁ - C₆ alkyl having at least one substituent selected from Y on the ring, phenyl or substituted phenyl having at least one substituent selected from Y on the ring, and
R₉II: C₁ - C₆ alkylcarbonyl, halo C₁ - C₆ alkylcarbonyl, cyclo C₃ - C₆ alkylcarbonyl, cyclo C₃ - C₆ alkyl C₁ - C₆ alkylcarbonyl, phenylcarbonyl, substituted phenylcarbonyl having at least one substituent selected from Y, C₁ - C₆ alkoxycarbonyl, halo C₁ - C₆ alkoxycarbonyl, phenoxycarbonyl or substituted phenoxycarbonyl having at least one substituent selected from Y, and R₉III: C₁ - C₆ alkylsulfonyl, halo C₁ - C₆ alkylsulfonyl, phenylsulfonyl or substituted phenylsulfonyl having at least one substituent selected from Y.

As options for n, the following are, for example, mentioned:
nl: 3 or 4, and
nII: 1 or 2.

As options for X, the following are, for example, mentioned:
XI: a hydrogen atom, XII: a halogen, C₁ - C₆ alkoxy, halo C₁ - C₆ alkoxy, C₁ - C₆ alkylthio, halo C₁ - C₆ alkylthio, phenoxy, substituted phenoxy having at least one identical or different substituent selected from Y on the ring, phenylthio, substituted phenylthio having at least one identical or different substituent selected from Y, hydroxy, amino, cyano or nitro,
XIII: C₁ - C₆ alkyl, cyclo C₃ - C₆ alkyl, halo C₁ - C₆ alkyl, halocyclo C₃ - C₆ alkyl, C₁ - C₆ alkoxy C₁ - C₆ alkyl or halo C₁ - C₆ alkoxy C₁ - C₆ alkyl,
XIV: C₁ - C₆ alkylcarbonyl, halo C₁ - C₆ alkylcarbonyl, phenylcarbonyl, substituted phenylcarbonyl having at least one identical or different substituent selected from Y on the ring or C₁ - C₆ alkoxycarbonyl, and
XV: phenyl, substituted phenyl having at least one identical or different substituent selected from Y on the ring, heterocyclyl or substituted heterocyclyl having at least one identical or different substituent selected from Y on the ring.

These options for the respective groups may be combined arbitrarily to define the scope of the compounds of the present invention. Examples of combinations of these options for the respective groups are shown in Table 1. However, the combinations shown in Table 1 are mere examples, and the present invention is by no means restricted thereto.

**TABLE 1**

| R₃ = R₃ I, R₄ = R₄ I, R₆ = R₆ I, R₈ = R₈ I | | | | | | | | |
|---|---|---|---|---|---|---|---|---|
| W | A | R₁ | R₂ | R₅ | R₇ | R₉ | X | n |
| WI | AI | R₁ I | R ₂ I | R₅ I | R₇ I | - | XI | n I |
| WI | AI | R₁ I | R ₂ I | R₅ I | R₇ I | - | XII | n I |
| WI | AI | R₁ I | R ₂ I | R₅ I | R₇ I | - | XIII | n I |
| WI | AI | R₁ I | R ₂ I | R₅ I | R₇ I | - | XIV | n I |
| WI | AI | R₁ I | R ₂ I | R₅ I | R₇ I | - | XV | n I |
| WI | AI | R₁ I | R₂ I | R₅ I | R₇ I | - | XI | nII |
| WI | AI | R₁ I | R₂ I | R₅ I | R₇ II | - | XI | nI |
| WI | AI | R₁ I | R₂ I | R₅ I | R₇ III | - | XI | nI |
| WI | AI | R₁ I | R₂ I | R₅ I | R₇ IV | - | XI | nI |
| WI | AI | R₁ I | R₂ I | R₅ II | R₇ I | - | XI | nI |
| WI | AI | R₁ I | R₂ I | R₅ III | R₇ I | - | XI | nI |
| WI | AI | R₁ I | R ₂ I | R₅ IV | R₇ I | - | XI | nI |
| WI | AI | R₁ I | R₂ II | R₅ I | R₇ I | - | XI | nI |
| WI | AI | R₁ I | R ₂ III | R₅ I | R₇ I | - | XI | nI |
| WI | AI | R₁ II | R₂ I | R₅ I | R₇ I | - | XI | nI |
| WI | AII | R₁ I | R₂ I | R₅ I | - | R₉ I | XI | nI |
| WI | AII | R₁ I | R₂ I | R₅ I | - | R₉ I | XI | nII |
| WI | AII | R₁ I | R₂ I | R₅ I | - | R₉ II | XI | nI |
| WI | AII | R₁ I | R₂ I | R₅ I | - | R₉ III | XI | nI |
| WI | AII | R₁ I | R₂ II | R₅ I | - | R₉ I | XI | nI |
| WI | AII | R₁ I | R₂ III | R₅ I | - | R₉ I | XI | nI |
| WI | AII | R₁ II | R₂ I | R₅ I | - | R₉ I | XI | nI |
| WII | AI | R₁ I | R₂ I | R₅ I | R₇ I | - | XI | nI |
| WII | AII | R₁ 1 | R₂ I | R₅ I | - | R₉ I | XI | nI |

Now, examples of each atom or group in the definitions of R₁, R₂, R₃, R₄, R₅, R₆, R₇, R₈, R₉, R₁₀, R₁₁, X and Y will be given.

The following symbols herein have the following meanings.
i. iso, s: secondary, t: tertiary, c: cyclo, p: para.

Heterocyclyl means the following: namely, thienyl such as thiophen-2-yl or thiophen-3-yl, furyl such as furan-2-yl or furan-3-yl, pyrrolyl such as pyrrol-1-yl, pyrrol-2-yl or pyrrol-3-yl, oxazolyl such as oxazol-2-yl, oxazol-4-yl or oxazol-5-yl, isoxazoly such as isoxazol-3-yl, isoxazol-4-yl or isoxazol-5-yl, isoxazolinyl such as isoxazolin-3-yl, isoxazolin-4-yl or isoxazolin-5-yl, thiazolyl such as thiazol-2-yl, thiazol-4-yl or thiazol-5-yl, isothiazoly such as isothiazol-3-yl, isothiazol-4-yl or isothiazol-5-yl, pyrazoly such as pyrazol-1-yl, pyrazol-3-yl, pyrazol-4-yl or pyrazol-5-yl, imidazolyl such as imidazol-1-yl, imidazol-2-yl or imidazol-4-yl, 1,3,4-oxadiazolyl such as 1,3,4-oxadiazol-2-yl, 1,2,4-oxadiazoly such as 1,2,4-oxadiazol-3-yl or 1,2,4-oxadiazol-5-yl, 1,3,4-thiadiazolyl such as 1,3,4-thiadiazol-2-yl, 1,2,4-thiadiazolyl such as 1,2,4-thiadiazol-3-yl or 1,2,4-thiadiazol-5-yl, 1,2,4-triazolyl such as 1,2,4-triazol-1-yl, 1,2,4-triazol-3-yl or 1,2,4-triazol-5-yl, 1,2,3-thiadiazolyl such as 1,2,3-thiadiazol-4-yl or 1,2,3-thiadiazol-5-yl, 1,2,3-triazolyl such as 1,2,3-triazol-1-yl, 1,2,3-triazol-2-yl or 1,2,3-triazol-4-yl, 1,2,3,4-tetrazolyl such as 1,2,3,4-tetrazol-1-yl, 1,2,3,4-tetrazol-2-yl or 1,2,3,4-tetrazol-5-yl, pyridyl such as pyridin-2-yl, pyridin-3-yl or pyridin-4-yl, pyrimidinyl such as pyrimidin-2-yl, pyrimidin-4-yl or pyrimidin-5-yl, pyrazinyl such as pyrazin-2-yl, pyridazinyl such as pyridazin-3-yl or pyridazin-4-yl, 1,3,5-triazinyl such as 1,3,5-triazin-2-yl, 1,2,4-triazinyl such as 1,2,4-triazin-3-yl, 1,2,4-triazin-5-yl or 1,2,4-triazin-6-yl, such as benzothienyl such as benzothiophen-2-yl, benzothiophen-3-yl, benzothiophen-4-yl, benzothiophen-5-yl, benzothiophen-6-yl or benzothiophen-7-yl, benzofuryl such as benzofuran-2-yl, benzofuran-3-yl, benzofuran-4-yl, benzofuran-5-yl, benzofuran-6-yl or benzofuran-7-yl, indolyl such as indol-1-yl, indol-2-yl, indol-3-yl, indol-4-yl, indol-5-yl, indol-6-yl or indol-7-yl, benzothiazolyl such as benzothiazol-2-yl, benzothiazol-4-yl, benzothiazol-5-yl, benzothiazol-6-yl or benzothiazol-7-yl, benzimidazolyl such as benzimidazol-1-yl, benzimidazol-2-yl, benzimidazol-4-yl, benzimidazol-5-yl, benzimidazol-6-yl or benzimidazol-7-yl, benzoisoxazolyl such as benzisoxazol-3-yl, benzisoxazol-4-yl, benzisoxazol-5-yl, benzisoxazol-6-yl or benzisoxazol-7-yl, benzisothiazolyl such as benzisothiazol-3-yl, benzisothiazol-4-yl, benzisothiazol-5-yl, benzisothiazol-6-yl or benzisothiazol-7-yl, indazolyl such as indazol-1-yl, indazol-3-yl, indazol-4-yl, indazol-5-yl, indazol-6-yl or indazol-7-yl, benzoxazolyl such as benzoxazol-2-yl, benzoxazol-4-yl, benzoxazol-5-yl, benzoxazol-6-yl or benzoxazol-7-yl, quinolyl such as quinolin-2-yl, quinolin-3-yl, quinolin-4-yl, quinolin-5-yl, quinolin-6-yl, quinolin-7-yl or quinolin-8-yl, isoquinolyl such as isoquinolin-1-yl, isoquinolin-3-yl, isoquinolin-4-yl, isoquinolin-5-yl, isoquinolin-6-yl, isoquinolin-7-yl or isoquinolin-8-yl, quinoxalinyl such as quinoxalin-2-yl, quinoxalin-3-yl, quinoxalin-5-yl, quinoxalin-6-yl, quinoxalin-7-yl or quinoxalin-8-yl, phthalazinyl such as phthalazin-1-yl, phthalazin-4-yl, phthalazin-5-yl, phthalazin-6-yl, phthalazin-7-yl or phthalazin-8-yl, cinnolinyl such as cinnolin-3-yl, cinnolin-4-yl, cinnolin-5-yl, cinnolin-6-yl, cinnolin-7-yl or cinnolin-8-yl, and quinazolinyl such as quinazolin-2-yl, quinazolin-4-yl, quinazolin-5-yl, quinazolin-6-yl, quinazolin-7-yl or quinazolin-8-yl. In the present invention, "heterocyclylcarbonyl" means the above-mentioned groups attached to carbonyl.

As a halogen, a fluorine atom, a chlorine atom, a bromine atom and an iodine atom may be mentioned.

Alkyl may be methyl, ethyl, propyl, i-propyl, butyl, i-butyl, t-butyl, s-butyl, pentyl, 1-methylbutyl, 2-methylbutyl, 3-methylbutyl, 1-ethylpropyl, 1,1-dimethylpropyl, 1,2-dimethylpropyl, neo-pentyl, hexyl, 1-methylpentyl, 2-methylpentyl, 3-methylpentyl, 4-methylpentyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1-ethyl-1-methylpropyl, 1-ethyl-2-methylpropyl or the like and is selected within a given range of carbon atoms.

Haloalkyl may be fluoromethyl, chloromethyl, bromomethyl, 2-fluoroethyl, 2-chloroethyl, 2-bromoethyl, 3-fluoropropyl, 3-chloropropyl, difluoromethyl, chlorodifluoromethyl, trifluoromethyl, dichloromethyl, trichloromethyl, 2,2-difluoroethyl, 2,2,2-trifluoroethyl, 2,2,2-trichloroethyl, chlorodifluoromethyl, bromodifluoromethyl, pentafluoroethyl, heptafluoropropyl, heptafluoroisopropyl, 4-chlorobutyl, 4-fluorobutyl or the like and is selected within a given range of carbon atoms.

Cycloalkyl may be cyclopropyl, cyclobutyl, cyclopentyl, cyclohexyl or the like and is selected within a given range of carbon atoms.

Halocycloalkyl may be 2-fluorocyclopropyl, 1-chlorocyclopropyl, 2-bromo-1-methylcyclopropyl, 2,2-difluorocyclopropyl, 1,2-dichlorocyclopropyl or the like and is selected within a given range of carbon atoms.

Cycloalkylalkyl may be cyclopropylmethyl, 2-cyclopropylethyl, cyclopentylmethyl, cyclohexylmethyl or the like and is selected within a given range of carbon atoms.

Alkenyl may be ethenyl, 1-propenyl, 2-propenyl, 1-butenyl, 2-butenyl, 3-butenyl, 1-methyl-2-propenyl, 2-methyl-2-propenyl, 1-pentenyl, 2-pentenyl, 3-pentenyl, 4-pentenyl, 1,1-dimethyl-2-propenyl, 1,2-dimethyl-2-propenyl, 1-hexenyl, 1,1-dimethyl-2-butenyl, 1,2-dimethyl-2-butenyl, 1,3-dimethyl-2-butenyl, 2,3-dimethyl-2-butenyl, 1-ethyl-2-butenyl, 2-ethyl-2-butenyl, 1,1,2-trimethyl-2-propenyl, 1-ethyl-1-methyl-2-propenyl or the like and is selected within a given range of carbon atoms.

Haloalkenyl may be 1-chloroethenyl, 2-chloroethenyl, 2-fluoroethenyl, 2,2-dichloroethenyl, 3-chloro-2-propenyl, 3-fluoro-2-propenyl, 2-chloro-2-propenyl, 4-chloro-3-butenyl or the like and is selected within a given range of carbon atoms.

Alkynyl may be ethynyl, 1-propynyl, 2-propynyl, 1-butynyl, 2-butynyl, 3-butynyl, 1-methyl-2-propynyl, 1-pentynyl, 1-methyl-2-butynyl, 2-methyl-3-butynyl, 1-hexynyl, 1-methyl-3-pentynyl, 2-methyl-3-pentynyl, 3-methyl-4-pentynyl, 4-methyl-2-pentynyl, 1,1-dimethyl-2-butynyl, 1,2-dimethyl-3-butynyl, 2,2-dimethyl-3-butynyl, 1-ethyl-2-butynyl, 2-ethyl-3-butynyl or the like and is selected within a given range of carbon atoms.

Haloalkynyl may be chloroethynyl, fluoroethynyl, bromoethynyl, 3-chloro-2-propynyl, 4-chloro-2-butynyl or the like and is selected within a given range of carbon atoms.

Phenyl having at least one substituent selected from Y may be 2-fluorophenyl, 3-fluorophenyl, 4-fluorophenyl, 2-chlorophenyl, 3-bromophenyl, 4-iodophenyl, 2,3-difluorophenyl, 2,4-difluorophenyl, 2,6-difluorophenyl, 3,4-difluorophenyl, 3,5-difluorophenyl, 2,4-dichlorophenyl, 2-fluoro-4-chlorophenyl, 2,3,4,5,6-pentafluorophenyl, 2-methylphenyl, 2,5-dimethylphenyl, 4-ethenylphenyl, 2-ethynylphenyl, 3-cyclopropylphenyl, 2-cyclopentylphenyl, 4-cyclohexylphenyl, 2-methoxyphenyl, 3,4-dimethoxyphenyl, 3,4,5-trimethoxyphenyl, 4-methoxymethylphenyl, 2-aminophenyl, 4-dimethylaminophenyl, 2-aminocarbonylphenyl, 2-dimethylaminocarbonylphenyl, 4-trifluoromethoxyphenyl, 2-difluoromethoxyphenyl, 2-ethenyloxyphenyl, 3-cyclopropyloxyphenyl, 2-biphenyl, 4-acetylphenyl, 3-methoxycarbonylphenyl, 2-methylthiophenyl, 3-trifluoromethylthiophenyl, 4-difluoromethylthiophenyl, 2-methylsulfinylphenyl, 3-trifluoromethylsulfinylphenyl, 4-difluoromethylsulfinylphenyl, 2-methylsulfonylphenyl, 3-trifluoromethylsulfonylphenyl, 4-difluoromethylsulfonylphenyl, 2-cyanophenyl, 3-nitrophenyl, 4-trifluoromethylphenyl, 2-difluoromethylphenyl, 2,3-methylenedioxyphenyl, 3,4-methylenedioxyphenyl or the like.

Alkoxy may be methoxy, ethoxy, propyloxy, i-propyloxy, butyloxy, i-butyloxy, s-butyloxy, t-butyloxy, pentyloxy, 1-methylbutyloxy, 2-methylbutyloxy, 3-methylbutyloxy, 1,1-dimethylpropyloxy, 1,2-dimethylpropyloxy, 2,2-dimethylpropyloxy, 1-ethylpropyloxy, hexyloxy, 1-methylpentyloxy, 2-methylpentyloxy, 3-methylpentyloxy, 4-methylpentyloxy, 1,1-dimethylbutyloxy, 1,2-dimethylbutyloxy, 1,3-dimethylbutyloxy, 2,2-dimethylbutyloxy, 2,3-dimethylbutyloxy, 3,3-dimethylbutyloxy, 1-ethylbutyloxy, 2-ethylbutyloxy, 1,1,2-trimethylpropyloxy, 1,2,2-trimethylpropyloxy, 1-ethyl-1-methylpropyloxy, 1-ethyl-2-methylpropyloxy or the like and is selected within a given range of carbon atoms.

Haloalkoxy may be fluoromethoxy, difluoromethoxy, trifluoromethoxy, chlorodifluoromethoxy, bromodifluoromethoxy, dichlorofluoromethoxy, chloromethoxy, dichloromethoxy, trichloromethoxy, bromomethoxy, 1-fluoroethoxy, 2-fluoroethoxy, 2-chloroethoxy, 2-bromoethoxy, 2,2-difluoroethoxy, 2,2,2-trifluoroethoxy, 1,1,2,2-tetrafluoroethoxy, pentafluoroethoxy, 2,2,2-trichloroethoxy, 2,2,2-trifluoroethoxy, 1-fluoropropyloxy, 2-fluoropropyloxy, 3-fluoropropyloxy, 3-chloropropyloxy, 3-bromopropyloxy, 1-fluorobutyloxy, 2-fluorobutyloxy, 3-fluorobutyloxy, 4-fluorobutyloxy, 4-chlorobutyloxy or the like and is selected within a given range of carbon atoms.

Phenylalkyl and phenylalkyl having at least one substituent selected from Y on the ring may be benzyl, 2-fluorobenzyl, 3-fluorobenzyl, 4-fluorobenzyl, 2-chlorobenzyl, 3-bromobenzyl, 4-iodobenzyl, 2,4-difluorobenzyl, 2-methylbenzyl, 3-methylbenzyl, 4-methylbenzyl, 2-methoxybenzyl, 4-aminobenzyl, 4-trifluoromethoxybenzyl, 2-methylthiobenzyl, 3-trifluoromethylthiobenzyl, 2-cyanobenzyl, 4-nitrobenzyl, 2-trifluoromethylbenzyl, 2-phenethyl or the like and is selected within a given range of carbon atoms.

Phenylcarbonylalkyl and phenylcarbonylalkyl having at least one substituent selected from Y on the ring may be phenylcarbonylmethyl, 2-fluorophenylcarbonylmethyl, 2-chlorophenylcarbonylmethyl, 3-bromophenylcarbonylmethyl, 4-iodophenylcarbonylmethyl, 4-methylphenylcarbonylmethyl, 2-methoxyphenylcarbonylmethyl, 4-aminophenylcarbonylmethyl, 4-trifluoromethoxyphenylcarbonylmethyl, 2-methylthiophenylcarbonylmethyl, 3-trifluoromethylthiophenylcarbonylmethyl, 2-cyanophenylcarbonylmethyl, 4-nitrophenylcarbonylmethyl, 2-trifluoromethylphenylcarbonylmethyl, 2-phenylcarbonylethyl or the like and is selected within a given range of carbon atoms.

Phenoxy having at least one substituent selected from Y on the ring may be 2-fluorophenoxy, 2-chlorophenoxy, 4-bromophenoxy, 4-methylphenoxy, 2-methoxyphenoxy, 3-aminophenoxy, 4-trifluoromethoxyphenoxy, 2-methylthiophenoxy, 3-trifluoromethylthiophenoxy, 4-cyanophenoxy, 2-nitrophenoxy, 4-trifluoromethylphenoxy or the like.

Alkylthio may be methylthio, ethylthio, propylthio, i-propylthio, butylthio, i-butylthio, s-butylthio, t-butylthio or the like and is selected within a given range of carbon atoms.

Haloalkylthio may be fluoromethylthio, chloromethylthio, chlorodifluoromethylthio, bromodifluoromethylthio, trifluoromethylthio, trichloromethylthio, 2,2,2-trifluoroethylthio, 1,1,2,2-tetrafluoroethylthio, 2-fluoroethylthio, pentafluoroethylthio or the like and is selected within a given range of carbon atoms.

Phenylthio having at least one substituent selected from Y on the ring may be 2-fluorophenylthio, 2-chlorophenylthio, 4-bromophenylthio, 4-methylphenylthio, 2-methoxyphenylthio, 3-aminophenylthio, 4-trifluoromethoxyphenylthio, 2-methylthiophenylthio, 3-trifluoromethylthiophenylthio, 4-cyanophenylthio, 2-nitrophenylthio, 4-trifluoromethylphenylthio or the like.

Alkylsulfinyl may be methylsulfinyl, ethylsulfinyl, propylsulfinyl, i-propylsulfinyl, butylsulfinyl, i-butylsulfinyl, s-butylsulfinyl, t-butylsulfinyl or the like and is selected within a given range of carbon atoms.

Haloalkylsulfinyl may be fluoromethylsulfinyl, chloromethylsulfinyl, chlorodifluoromethylsulfinyl, bromodifluoromethylsulfinyl, trifluoromethylsulfinyl, trichloromethylsulfinyl, 2,2,2-trifluoroethylsulfinyl, 1,1,2,2-tetrafluoroethylsulfinyl, 2-fluoroethylsulfinyl, pentafluoroethylsulfinyl or the like and is selected within a given range of carbon atoms.

Phenylsulfinyl having at least one substituent selected from Y on the ring may be 2-fluorophenylsulfinyl, 2-chlorophenylsulfinyl, 4-bromophenylsulfinyl, 4-methylphenylsulfinyl, 2-methoxyphenylsulfinyl, 3-aminophenylsulfinyl, 4-trifluoromethoxyphenylsulfinyl, 2-methylthiophenylsulfinyl, 3-trifluoromethylthiophenylsulfinyl, 4-cyanophenylsulfinyl, 2-nitrophenylsulfinyl, 4-trifluoromethylphenylsulfinyl or the like.

Alkylsulfonyl may be methylsulfonyl, ethylsulfonyl, propylsulfonyl, i-propylsulfonyl, butylsulfonyl, i-butylsulfonyl, s-butylsulfonyl, t-butylsulfonyl or the like and is selected within a given range of carbon atoms.

Haloalkylsulfonyl may be fluoromethylsulfonyl, chloromethylsulfonyl, chlorodifluoromethylsulfonyl, bromodifluoromethylsulfonyl, trifluoromethylsulfonyl, trichloromethylsulfonyl, 2,2,2-trifluoroethylsulfonyl, 1,1,2,2-tetrafluoroethylsulfonyl, 2-fluoroethylsulfonyl, pentafluoroethylsulfonyl or the like and is selected within a given range of carbon atoms.

Phenylsulfonyl having at least one substituent selected from Y on the ring may be 2-fluorophenylsulfonyl, 2-chlorophenylsulfonyl, 4-bromophenylsulfonyl, 4-methylphenylsulfonyl, 2-methoxyphenylsulfonyl, 3-aminophenylsulfonyl, 4-trifluoromethoxyphenylsulfonyl, 2-methylthiophenylsulfonyl, 3-trifluoromethylthiophenylsulfonyl, 4-cyanophenylsulfonyl, 2-nitrophenylsulfonyl, 4-trifluoromethylphenylsulfonyl or the like.

Alkylcarbonyl may be acetyl, propionyl, butyryl, i-butyryl, valeroyl, i-valeroyl, 2-methylbutyryl, pivaloyl, hexanoyl, 2-ethylbutyryl, 2-methylvaleroyl, 4-methylvaleroyl, heptanoyl, 2,2-dimethylvaleroyl, 2-ethylhexanoyl, 2-propylvaleroyl, octanoyl, nonanoyl, 3,5,5-trimethylhexanoyl, decanoyl or the like and is selected within a given range of carbon atoms.

Haloalkylcarbonyl may be fluoroacetyl, chloroacetyl, difluoroacetyl, dichloroacetyl, trifluoroacetyl, chlorodifluoroacetyl, bromodifluoroacetyl, trichloroacetyl, pentafluoropropionyl, 4-chlorobutyryl, heptafluorobutyryl, 3-chloro-2,2-dimethylpropionyl or the like and is selected within a given range of carbon atoms.

Cycloalkylcarbony may be cyclopropanecarnonyl, cyclobutanecarbonyl, cyclopentanecarbonyl, cyclohexanecarbonyl or the like and is selected within a given range of carbon atoms.

Cycloalkylalkylcarbonyl may be cyclopropylmethylcarbonyl, 2-cyclopropylethylcarbonyl, cyclopentylmethylcarbonyl, cyclohexylmethylcarbonyl or the like and is selected within a given range of carbon atoms.

Alkenylcarbonyl may be ethenylcarbonyl, 1-propenylcarbonyl, 2-propenylcarbonyl, 1-butenylcarbonyl, 1-methyl-2-propenylcarbonyl, 2-methyl-2-propenylcarbonyl, 1-hexenylcarbonyl or the like and is selected within a given range of carbon atoms.

Phenylcarbonyl having at least one substituent selected from Y on the ring may be 2-fluorophenylcarbonyl, 2-chlorophenylcarbonyl, 4-bromophenylcarbonyl, 4-methylphenylcarbonyl, 2-methoxyphenylcarbonyl, 3-aminophenylcarbonyl, 4-trifluoromethoxyphenylcarbonyl, 2-methylthiophenylcarbonyl, 3-trifluoromethylthiophenylcarbonyl, 4-cyanophenylcarbonyl, 2-nitrophenylcarbonyl, 4-trifluoromethylphenylcarbonyl.

Alkoxycarbonyl may be methoxycarbonyl, ethoxycarbonyl, propyloxycarbonyl, i-propyloxycarbonyl, butyloxycarbonyl, s-butyloxycarbonyl, i-butyloxycarbonyl, t-butyloxycarbonyl, pentyloxycarbonyl, hexyloxycarbonyl or the like and is selected within a given range of carbon atoms.

Haloalkoxycarbonyl may be fluoromethoxycarbonyl, difluoromethoxycarbonyl, trifluoromethoxycarbonyl, 1-fluoroethoxycarbonyl, 2-fluoroethoxycarbonyl, 2-chloroethoxycarbonyl, 2-bromoethoxycarbonyl, 2,2-difluoroethoxycarbonyl, 2,2,2-trifluoroethoxycarbonyl, 1-fluoropropyloxycarbonyl, 2-fluoropropyloxycarbonyl, 3-fluoropropyloxycarbonyl, 3-chloropropyloxycarbonyl, 3-bromopropyloxycarbonyl, 4-fluorobutyloxycarbonyl, 4-chlorobutyloxycarbonyl or the like and is selected within a given range of carbon atoms.

Alkoxyalkoxycarbonyl may be methoxymethoxycarbonyl, ethoxymethoxycarbonyl, propyloxymethoxycarbonyl, i-propyloxymethoxycarbonyl, butyloxymethoxycarbonyl, 2-methoxyethoxycarbonyl, 1-methoxyethoxycarbonyl or the like and is selected within a given range of carbon atoms.

Alkylthioalkoxycarbonyl may be methylthiomethoxycarbonyl, ethylthiomethoxycarbonyl, propylthiomethoxycarbonyl, i-propylthiomethoxycarbonyl, butylthiomethoxycarbonyl, 2-methylthioethoxycarbonyl, 1-methylthioethoxycarbonyl or the like and is selected within a given range of carbon atoms.

Alkylsulfinylalkoxycarbonyl may be methylsulfinylmethoxycarbonyl, ethylsulfinylmethoxycarbonyl, propylsulfinylmethoxycarbonyl, i-propylsulfinylmethoxycarbonyl, butylsulfinylmethoxycarbonyl, 2-methylsulfinylethoxycarbonyl, 1-methylsulfinylethoxycarbonyl or the like and is selected within a given range of carbon atoms.

Alkylsulfonylalkoxycarbonyl may be methylsulfonylmethoxycarbonyl, ethylsulfonylmethoxycarbonyl, propylsulfonylmethoxycarbonyl, i-propylsulfonylmethoxycarbonyl, butylsulfonylmethoxycarbonyl, 2-methylsulfonylethoxycarbonyl, 1-methylsulfonylethoxycarbonyl or the like and is selected within a given range of carbon atoms.

Alkenyloxycarbonyl may be ethenyloxycarbonyl, 1-propenyloxycarbonyl, 2-propenyloxycarbonyl, 1-butenyloxycarbonyl, 1-methyl-2-propenyloxycarbonyl, 2-methyl-2-propenyloxycarbonyl, 1-hexenyloxycarbonyl or the like and is selected within a given range of carbon atoms.

Haloalkenyloxycarbonyl may be 1-chtoroethenyloxycarbonyl, 2-chloroethenyloxycarbonyl, 2-fluoroethenyloxycarbonyl, 2,2-dichloroethenyloxycarbonyl, 3-chloro-2-propenyloxycarbonyl, 3-fluoro-2-propenyloxycarbonyl, 2-chloro-2-propenyloxycarbonyl, 4-chloro-3-butenyloxycarbonyl or the like and is selected within a given range of carbon atoms.

Alkynyloxycarbonyl may be ethynyloxycarbonyl, 1-propynyloxycarbonyl, 2-propynyloxycarbonyl, 1-butynyloxycarbonyl, 1-methyl-2-propynyloxycarbonyl, 2-methyl-2-propynyloxycarbonyl, 1-hexynyloxycarbonyl or the like and is selected within a given range of carbon atoms.

Phenoxycarbonyl having at least one substituent selected from Y on the ring may be 2-fluorophenoxycarbonyl, 2-chlorophenoxycarbonyl, 4-bromophenoxycarbonyl, 4-methylphenoxycarbonyl, 2-methoxyphenoxycarbonyl, 3-aminophenoxycarbonyl, 4-trifluoromethoxyphenoxycarbonyl, 2-methylthiophenoxycarbonyl, 3-trifluoromethylthiophenoxycarbonyl, 4-cyanophenoxycarbonyl, 2-nitrophenoxycarbonyl, 4-trifluoromethylphenoxycarbonyl or the like.

Phenylalkoxycarbonyl and phenylalkoxycarbonyl having at least one substituent selected from Y on the ring may be benzyloxycarbonyl, 2-fluorobenzyloxycarbonyl, 2-chlorobenzyloxycarbonyl, 3-bromobenzyloxycarbonyl, 4-iodobenzyloxycarbonyl, 4-methylbenzyloxycarbonyl, 2-methoxybenzyloxycarbonyl, 4-aminobenzyloxycarbonyl, 4-trifluoromethoxybenzyloxycarbonyl, 2-methylthiobenzyloxycarbonyl, 3-trifluoromethylthiobenzyloxycarbonyl, 2-cyanobenzyloxycarbonyl, 4-nitrobenzyloxycarbonyl, 2-trifluoromethylbenzyloxycarbony, 2-phenethyloxycarbonyl or the like.

Phenoxyalkylcarbonyl and phenoxyalkylcarbonyl having at least one substituent selected from Y on the ring may be phenoxymethylcarbonyl, 2-phenoxyethylcarbonyl, 2-fluorophenoxymethylcarbonyl, 2-chlorophenoxymethylcarbonyl, 4-bromophenoxymethylcarbonyl, 4-methylphenoxymethylcarbonyl, 2-methoxyphenoxymethylcarbonyl, 3-aminophenoxymethylcarbonyl, 4-trifluoromethoxyphenoxymethylcarbonyl, 2-methylthiophenoxymethylcarbonyl, 3-trifluoromethylthiophenoxymethylcarbonyl, 4-cyanophenoxymethylcarbonyl, 2-nitrophenoxymethylcarbonyl, 4-trifluoromethylphenoxymethylcarbonyl or the like.

Alkylthiocarbonyl may be methylthiocarbonyl, ethylthiocarbonyl, propylthiocarbonyl, i-propylthiocarbonyl, butylthiocarbonyl, i-butylthiocarbonyl, s-butylthiocarbonyl, t-butylthiocarbonyl or the like and is selected within a given range of carbon atoms.

Haloalkylthiocarbonyl may be fluoromethylthiocarbonyl, chlorodifluoromethylthiocarbonyl, bromodifluoromethylthiocarbonyl, trifluoromethylthiocarbonyl, trichloromethylthiocarbonyl, 2,2,2-trifluoroethylthiocarbonyl, 1,1,2,2-tetrafluoroethylthiocarbonyl, 2-fluoroethylthiocarbonyl, pentafluoroethylthiocarbonyl or the like and is selected within a given range of carbon atoms.

Monoalkylaminocarbonyl may be methylaminocarbonyl, ethylaminocarbonyl, propylaminocarbonyl, i-propylaminocarbonyl, butylaminocarbonyl, s-butylaminocarbonyl, i-butylaminocarbonyl, t-butylaminocarbonyl, pentylaminocarbonyl, hexylaminocarbonyl or the like and is selected within a given range of carbon atoms.

Monohaloalkylaminocarbonyl may be fluoromethylaminocarbonyl, difluoromethylaminocarbonyl, trifluoromethylaminocarbonyl, chloromethylaminocarbonyl, bromomethylaminocarbonyl, iodomethylaminocarbonyl, 2-fluoroethylaminocarbonyl, 2-chloroethylaminocarbonyl, 3-fluoropropylaminocarbonyl, 2-fluoropropylaminocarbonyl, 1-fluoropropylaminocarbonyl or the like and is selected within a given range of carbon atoms.

Dialkylaminocarbonyl may be dimethylaminocarbonyl, diethylaminocarbonyl, dipropylaminocarbonyl, di(i-propyl)aminocarbonyl, dibutylaminocarbonyl, di(s-butyl)aminocarbonyl, di(i-butyl)aminocarbonyl, di(t-butyl)aminocarbonyl, dipentylaminocarbonyl, dihexylaminocarbonyl, ethyl(methyl)aminocarbonyl, methyl(propyl)aminocarbonyl or the like and is selected within a given range of carbon atoms.

Di(haloalkyl)aminocarbonyl may be di(fluoromethyl)aminocarbonyl, di(chloromethyl)aminocarbonyl, di(bromomethyl)aminocarbonyl, fluoromethyl(methyl)aminocarbonyl, chloromethyl(methyl)aminocarbonyl, chloromethyl(ethyl)aminocarbonyl, di(2-fluoroethyl)aminocarbonyl, (2-fluoroethyl)methylaminocarbonyl or the like and is selected within a given range of carbon atoms.

Phenylaminocarbonyl having at least one substituent selected from Y on the ring may be 2-fluorophenylaminocarbonyl, 2-chlorophenylaminocarbonyl, 4-bromophenylaminocarbonyl, 4-methylphenylaminocarbonyl, 2-methoxyphenylaminocarbonyl, 3-aminophenylaminocarbonyl, 4-trifluoromethoxyphenylaminocarbonyl, 2-methylthiophenylaminocarbonyl, 3-trifluoromethylthiophenylaminocarbonyl, 4-cyanophenylaminocarbonyl, 2-nitrophenylaminocarbonyl, 4-trifluoromethylphenylaminocarbonyl or the like.

Phenylalkylaminocarbonyl and phenylalkylaminocarbonyl having at least one substituent selected from Y on the ring may be benzylaminocarbonyl, 2-fluorobenzylaminocarbonyl, 2-chlorobenzylaminocarbonyl, 3-bromobenzylaminocarbonyl, 4-iodobenzylaminocarbonyl, 4-methylbenzylaminocarbonyl, 2-methoxybenzylaminocarbonyl, 4-aminobenzylaminocarbonyl, 4-trifluoromethoxybenzylaminocarbonyl, 2-methylthiobenzylaminocarbonyl, 3-trifluoromethylthiobenzylaminocarbonyl, 2-cyanobenzylaminocarbonyl, 4-nitrobenzylaminocarbonyl, 2-trifluoromethylbenzylaminocarbony, 2-phenethylaminocarbonyl or the like.

Alkoxyalkyl may be methoxymethyl, ethoxymethyl, propyloxymethyl, i-propyloxymethyl, butyloxymethyl, i-butyloxymethyl, s-butyloxymethyl, t-butyloxymethyl, pentyloxymethyl, 1-methoxyethyl, 2-methoxyethyl, 2-ethoxyethyl, 3-methoxypropyl or the like and is selected within a given range of carbon atoms.

Haloalkoxyalkyl may be fluoromethoxymethyl, chloromethoxymethyl, bromomethoxymethyl, 2,2,2-trifluoroethoxymethyl, 2-(chrolomethoxy)ethyl or the like and is selected within a given range of carbon atoms.

Alkoxyalkoxyalkyl may be methoxymethoxymethyl, ethoxymethoxymethyl, propyloxymethoxymethyl, i-propyloxymethoxymethyl, butyloxymethoxymethyl, 1-(methoxymethoxy)ethyl, 2-(methoxymethoxy)ethyl, 2-(ethoxymethoxy)ethyl, 2-(ethoxy)ethoxymethyl or the like and is selected within a given range of carbon atoms.

Trialkylsilylalkoxyalkyl may be trimethylsilylmethoxymethyl, 2-(trimethylsilyl)ethoxymethyl, 2-(trimethylsilylmethoxy)ethyl, 2-[2-(trimethylsilyl)ethoxy]ethyl or the like and is selected within a given range of carbon atoms.

Hydroxyalkyl may be hydroxymethyl, 1-hydroxyethyl, 2-hydroxyethyl, 1-hydroxypropyl, 2-hydroxypropyl, 3-hydroxypropyl, 1-hydroxy-1-methylethyl, 2-hydroxy-1-methylethyl, 2-hydroxy-1, 1-dimethylethyl or the like and is selected within a given range of carbon atoms.

Phenoxyalky and phenoxyalky having at least one substituent selected from Y on the ring may be phenoxymethyl, 2-fluorophenoxymethyl, 2-chlorophenoxymethyl, 3-bromophenoxymethyl, 4-iodophenoxymethyl, 4-methylphenoxymethyl, 2-methoxyphenoxymethyl, 4-aminophenoxymethyl, 4-trifluoromethoxyphenoxymethyl, 2-methylthiophenoxymethyl, 3-trifluoromethylthiophenoxymethyl, 2-cyanophenoxymethyl, 4-nitrophenoxymethyl, 2-trifluoromethylphenoxymethyl, 2-(phenoxy)ethyl or the like.

Phenylcarbonyloxyalkoxyalkyl and phenylcarbonyloxyalkoxyalkyl having at least one substituent selected from Y on the ring may be phenylcarbonyloxymethoxymethyl, 2-fluorophenylcarbonyloxymethoxymethyl, 2-chlorophenylcarbonyloxymethoxymethyl, 3-bromophenylcarbonyloxymethoxymethyl, 4-iodophenylcarbonyloxymethoxymethyl, 4-methylphenylcarbonyloxymethoxymethyl, 2-methoxyphenylcarbonyloxymethoxymethyl, 4-aminophenylcarbonyloxymethoxymethyl, 4-trifluoromethoxyphenylcarbonyloxymethoxymethyl, 2-methylthiophenylcarbonyloxymethoxymethyl, 3-trifluoromethylthiophenylcarbonyloxymethoxymethyl, 2-cyanophenylcarbonyloxymethoxymethyl, 4-nitrophenylcarbonyloxymethoxymethyl, 2-trifluoromethylphenylcarbonyloxymethoxymethyl, 2-(phenylcarbonyloxy)ethoxymethyl, 2-(phenylcarbonyloxymethoxy)ethyl or the like.

Alkylthioalkyl may be methylthiomethyl, ethylthiomethyl, propylthiomethyl, i-propylthiomethyl, butylthiomethyl, i-butylthiomethyl, s-butylthiomethyl, t-butylthiomethyl, pentylthiomethyl, 1-methylthioethyl, 2-methylthioethyl, 2-ethylthioethy, 3-methylthiopropyl or the like and is selected within a given range of carbon atoms.

Haloalkylthioalkyl may be fluoromethylthiomethyl, chloromethylthiomethyl, bromomethylthiomethyl, 2,2,2-trifluoroethylthiomethyl, 2-(chloromethylthio)ethyl or the like and is selected within a given range of carbon atoms.

Phenylthioalkyl and phenylthioalkyl having at least one substituent selected from Y on the ring may be phenylthiomethyl, 2-fluorophenylthiomethyl, 2-chlorophenylthiomethyl, 3-bromophenylthiomethyl, 4-iodophenylthiomethyl, 4-methylphenylthiomethyl, 2-methoxyphenylthiomethyl, 4-aminophenylthiomethyl, 4-trifluoromethoxyphenylthiomethyl, 2-methylthiophenylthiomethyl, 3-trifluoromethylthiophenylthiomethyl, 2-cyanophenylthiomethyl, 4-nitrophenylthiomethyl, 2-trifluoromethylphenylthiomethyl, 2-(phenylthio)ethyl or the like and is selected within a given range of carbon atoms.

Phenylalkylthioalkyl and phenylalkylthioalkyl having at least one substituent selected from Y on the ring may be benzylthiomethyl, 2-fluorobenzylthiomethyl, 2-chlorobenzylthiomethyl, 3-bromobenzylthiomethyl, 4-iodobenzylthiomethyl, 4-methylbenzylthiomethyl, 2-methoxybenzylthiomethyl, 4-aminobenzylthiomethyl, 4-trifluoromethoxybenzylthiomethyl, 2-methylthiobenzylthiomethyl, 3-trifluoromethylthiobenzylthiomethyl, 2-cyanobenzylthiomethyl, 4-nitrobenzylthiomethyl, 2-trifluoromethylbenzylthiomethyl, phenethylthiomethyl, 2-(benzylthio)ethyl or the like and is selected within a given range of carbon atoms.

Alkylcarbonylalkyl may be methylcarbonylmethyl, ethylcarbonylmethyl, propylcarbonylmethyl, i-propylcarbonylmethyl, butylcarbonylmethyl, i-butylcarbonylmethyl, s-butylcarbonylmethyl, t-butylcarbonylmethyl, pentylcarbonylmethyl, 1-(methylcarbonyl)ethyl, 2-(methylcarbonyl)ethyl, 2-(ethylcarbonyl)ethyl, 3-(methylcarbonyl)propyl or the like and is selected within a given range of carbon atoms.

Haloalkylcarbonylalkyl may be fluoromethylcarbonylmethyl, chloromethylcarbonylmethyl, bromomethylcarbonylmethyl, 2,2,2-trifluoroethylcarbonylmethyl, 2-(chloromethylcarbonyl)ethyl or the like and is selected within a given range of carbon atoms.

Alkoxycarbonylalkyl may be methoxycarbonylmethyl, ethoxycarbonylmethyl, propyloxycarbonylmethyl, i-propyloxycarbonylmethyl, butyloxycarbonylmethyl, 2-(methoxycarbonyl)ethyl, 2-(ethoxycarbonyl)ethyl, 3-(methoxycarbonyl)propyl or the like and is selected within a given range of carbon atoms.

Haloalkoxycarbonylalkyl may be fluoromethoxycarbonylmethyl, chloromethoxycarbonylmethyl, bromomethoxycarbonylmethyl, 2,2,2-trifluoroethoxycarbonylmethyl, 2-(chloromethoxycarbonyl)ethyl or the like and is selected within a given range of carbon atoms.

Alkoxycarbonyloxyalkyl may be methoxycarbonyloxymethyl, ethoxycarbonyloxymethyl, propyloxycarbonyloxymethyl, i-propyloxycarbonyloxymethyl, butyloxycarbonyloxymethyl, 2-methoxycarbonyloxyethyl, 2-(ethoxycarbonyloxy)ethyl, 3-(methoxycarbonyloxy)propyl or the like and is selected within a given range of carbon atoms.

Monoalkylaminocarbonyloxyalkyl may be methylaminocarbonyloxymethyl, ethylaminocarbonyloxymethyl, propylaminocarbonyloxymethyl, i-propylaminocarbonyloxymethyl, butylaminocarbonyloxymethyl, 2-(methylaminocarbonyloxy)ethyl, 2-(ethylaminocarbonyloxy)ethyl, 3-(methylaminocarbonyloxy)propyl or the like and is selected within a given range of carbon atoms.

Dialkylaminocarbonyloxyalkyl may be dimethylaminocarbonyloxymethyl, diethylaminocarbonyloxymethyl, ethyl(methyl)aminocarbonyloxymethyl, methyl(propyl)aminocarbonyloxymethyl, butyl(methyl)aminocarbonyloxymethyl, 2-(diethylaminocarbonyloxy)ethyl, 2-{ethyl(methyl)aminocarbonyloxy}propyl or the like and is selected within a given range of carbon atoms.

Phenylaminocarbonyloxyalkyl and phenylaminocarbonyloxyalkyl having at least one substituent selected from Y on the ring may be phenylaminocarbonyloxymethyl, 2-fluorophenylaminocarbonyloxymethyl, 2-chlorophenylaminocarbonyloxymethyl, 3-bromophenylaminocarbonyloxymethyl, 4-iodophenylaminocarbonyloxymethyl, 4-methylphenylaminocarbonyloxymethyl, 2-methoxyphenylaminocarbonyloxymethyl, 4-aminophenylaminocarbonyloxymethyl, 4-trifluoromethoxyphenylaminocarbonyloxymethyl, 2-methylthiophenylaminocarbonyloxymethyl, 3-trifluoromethylphenylaminocarbonyloxymethyl, 2-cyanophenylaminocarbonyloxymethyl, 4-nitrophenylaminocarbonyloxymethyl, 2-trifluoromethylphenylaminocarbonyloxymethyl, 2-(phenylaminocarbonyloxy)ethyl or the like and is selected within a given range of carbon atoms.

Alkyl(phenyl)aminocarbonyloxyalkyl and alkyl(phenyl)aminocarbonyloxyalkyl having at least one substituent selected from Y on the ring may be methyl(phenyl)aminocarbonyloxymethyl, methyl(2-fluorophenyl)aminocarbonyloxymethyl, methyl(2-chlorophenyl)aminocarbonyloxymethyl, methyl(3-bromophenyl)aminocarbonyloxymethyl, methyl(4-iodophenyl)aminocarbonyloxymethyl, methyl(4-methylphenyl)aminocarbonyloxymethyl, methyl(2-methoxyphenyl)aminocarbonyloxymethyl, methyl(4-aminophenyl)aminocarbonyloxymethyl, methyl(4-trifluoromethoxy)phenylaminocarbonyloxymethyl, methyl(2-methylthiophenyl)aminocarbonyloxymethyl, methyl(3-trifluoromethylphenyl)aminocarbonyloxymethyl, methyl(2-cyanophenyl)aminocarbonyloxymethyl, methyl(4-nitrophenyl)aminocarbonyloxymethyl, ethyl(phenyl)aminocarbonyloxymethyl, 2-{methyl(phenyl)aminocarbonyloxy}ethyl or the like and is selected within a given range of carbon atoms.

Alkylsulfinylalkyl may be methylsulfinylmethyl, ethylsulfinylmethyl, propylsulfinylmethyl, i-propylsulfinylmethyl, butylsulfinylmethyl, i-butylsulfinylmethyl, s-butylsulfinylmethyl, t-butylsulfinylmethyl, pentylsulfinylmethyl, 1-(methylsulfinyl)ethyl, 2-(methylsulfinyl)ethyl, 2-(ethylsulfinyl)ethyl, 3-(methylsulfinyl)propyl or the like and is selected within a given range of carbon atoms.

Haloalkylsulfinylalkyl may be fluoromethylsulfinylmethyl, chloromethylsulfinylmethyl, bromomethylsulfinylmethyl, 2,2,2-trifluoroethylsulfinylmethyl, 2-(chloromethylsulfinyl)ethyl or the like and is selected within a given range of carbon atoms.

Phenylsulfinylalkyl and phenylsulfinylalkyl having at least one substituent selected from Y on the ring may be phenylsulfinylmethyl, 2-fluorophenylsulfinylmethyl, 2-chlorophenylsulfinylmethyl, 3-bromophenylsulfinylmethyl, 4-iodophenylsulfinylmethyl, 4-methylphenylsulfinylmethyl, 2-methoxyphenylsulfinylmethyl, 4-aminophenylsulfinylmethyl, 4-trifluoromethoxyphenylsulfinylmethyl, 2-methylthiophenylsulfinylmethyl, 3-trifluoromethylthiophenylsulfinylmethyl, 2-cyanophenylsulfinylmethyl, 4-nitrophenylsulfinylmethyl, 2-trifluoromethylphenylsulfinylmethyl 2-(phenylsulfinyl)ethyl or the like and is selected within a given range of carbon atoms.

Alkylsulfonylalkyl may be methylsulfonylmethyl, ethylsulfonylmethyl, propylsulfonylmethyl, i-propylsulfonylmethyl, butylsulfonylmethyl, i-butylsulfonylmethyl, s-butylsulfonylmethyl, t-butylsulfonylmethyl, pentylsulfonylmethyl, 1-(methylsulfonyl)ethyl, 2-(methylsulfonyl)ethyl, 2-(ethylsulfonyl)ethyl, 3-(methylsulfonyl)propyl or the like and is selected within a given range of carbon atoms.

Haloalkylsulfonylalkyl may be fluoromethylsulfonylmethyl, chloromethylsulfonylmethyl, bromomethylsulfonylmethyl, 2,2,2-trifluoroethylsulfonylmethyl, 2-(chloromethylsulfonyl)ethyl or the like and is selected within a given range of carbon atoms.

Phenylsulfonylalkyl and phenylsulfonylalkyl having at least one substituent selected from Y on the ring may be phenylsulfonylmethyl, 2-fluorophenylsulfonylmethyl, 2-chlorophenylsulfonylmethyl, 3-bromophenylsulfonylmethyl, 4-iodophenylsulfonylmethyl, 4-methylphenylsulfonylmethyl, 2-methoxyphenylsulfonylmethyl, 4-aminophenylsulfonylmethyl, 4-trifluoromethoxyphenylsulfonylmethyl, 2-methylthiophenylsulfonylmethyl, 3-trifluoromethylthiophenylsulfonylmethyl, 2-cyanophenylsulfonylmethyl, 4-nitrophenylsulfonylmethyl, 2-trifluoromethylphenylsulfonylmethyl, 2-(phenylsulfonyl)ethyl or the like and is selected within a given range of carbon atoms.

Phenylalkylsulfonylalkyl and phenylalkylsulfonylalkyl having at least one substituent selected from Y on the ring may be benzylsulfonylmethyl, 2-fluorobenzylsulfonylmethyl, 2-chlorobenzylsulfonylmethyl, 3-bromobenzylsulfonylmethyl, 4-iodobenzylsulfonylmethyl, 4-methylbenzylsulfonylmethyl, 2-methoxybenzylsulfonylmethyl, 4-aminobenzylsulfonylmethyl, 4-trifluoromethoxybenzylsulfonylmethyl, 2-methylthiobenzylsulfonylmethyl, 3-trifluoromethylthiobenzylsulfonylmethyl, 2-cyanobenzylsulfonylmethyl, 4-nitrobenzylsulfonylmethyl, 2-trifluoromethylbenzylsulfonylmethyl, phenethylsulfonylmethyl, 2-(benzylsulfonyl)ethyl or the like and is selected within a given range of carbon atoms.

Phenylalkoxyalkyl and phenylalkoxyalkyl having at least one substituent selected from Y on the ring may be benzyloxymethyl, 2-fluorobenzyloxymethyl, 2-chlorobenzyloxymethyl, 3-bromobenzyloxymethyl, 4-iodobenzyloxymethyl, 4-methylbenzyloxymethyl, 2-methoxybenzyloxymethyl, 4-aminobenzyloxymethyl, 4-trifluoromethoxybenzyloxymethyl, 2-methylthiobenzyloxymethyl, 3-trifluoromethylthiobenzyloxymethyl, 2-cyanobenzyloxymethyl, 4-nitrobenzyloxymethyl, 2-trifluoromethylbenzyloxymethyl, 2-phenethyloxymethyl, 1-(benzyloxy)ethyl, 2-(benzyloxy)ethyl or the like and is selected within a given range of carbon atoms.

Alkylcarbonyloxyalkyl may be acetoxymethyl, propionyloxymethyl, butyryloxymethyl, i-butyryloxymethyl, valeroyloxymethyl, i-valeroyloxymethyl, 2-methylbutyryloxymethyl, pivaloyloxymethyl, heptanoyloxymethyl, 1-(acetoxy)ethyl, 2-(acetoxy)ethyl, 2-(propionyloxy)ethyl, 3-(acetoxy)propyl or the like and is selected within a given range of carbon atoms.

Phenylcarbonyloxyalkyl and phenylcarbonyloxyalkyl having at least one substituent selected from Y on the ring may be phenylcarbonyloxymethyl, 2-fluorophenylcarbonyloxymethyl, 2-chlorophenylcarbonyloxymethyl, 3-bromophenylcarbonyloxymethyl, 4-iodophenylcarbonyloxymethyl, 4-methylphenylcarbonyloxymethyl, 2-methoxyphenylcarbonyloxymethyl, 4-aminophenylcarbonyloxymethyl, 4-trifluoromethoxyphenylcarbonyloxymethyl, 2-methylthiophenylcarbonyloxymethyl, 3-trifluoromethylthiophenylcarbonyloxymethyl, 2-cyanophenylcarbonyloxymethyl, 4-nitrophenylcarbonyloxymethyl, 2-trifluoromethylphenylcarbonyloxymethyl, 2-(phenylcarbonyloxy)ethyl or the like and is selected within a given range of carbon atoms.

Dialkylaminothio may be dimethylaminothio, diethylaminothio, dipropylaminothio, di(i-propyl)aminothio, dibutylaminothio, di(s-butyl)aminothio, di(i-butyl)aminothio, di(t-butyl)aminothio, dipentylaminothio, dihexylaminothio, ethyl(methyl)aminothio, methyl(propyl)aminothio or the like and is selected within a given range of carbon atoms.

Monoalkylaminoalkyl may be methylaminomethyl, ethylaminomethyl, propylaminomethyl, i-propylaminomethyl, butylaminomethyl, 2-(methylamino)ethyl, 2-(ethylamino)ethyl, 3-(methylamino)propyl or the like and is selected within a given range of carbon atoms.

Dialkylaminoalkyl may be dimethylaminomethyl, diethylaminomethyl, ethyl(methyl)aminomethyl, methyl(propyl)aminomethyl, butyl(methyl)aminomethyl, 2-(diethylamino)ethyl, 2-{ethyl(methyl)amino}propyl or the like and is selected within a given range of carbon atoms.

Monoalkylamino may be methylamino, ethylamino, propylamino, i-propylamino, butylamino, s-butylamino, i-butylamino, t-butylamino, pentylamino, hexylamino or the like and is selected within a given range of carbon atoms.

Di(alkyl)amino may be dimethylamino, diethylamino, dipropylamino, di(i-propyl)amino, dibutylamino, di(s-butyl)amino, di(i-butyl)amino, di(t-butyl)amino, dipentylamino, dihexylamino, ethyl(methyl)amino, methyl(propyl)amino or the like and is selected within a given range of carbon atoms.

Alkylcarbonyloxy may be acetoxy, propionyloxy, butyryloxy, i-butyryloxy, valeroyloxy, i-valeroyloxy, 2-methylbutyryloxy, pivaloyloxy, hexanoyloxy, 2-ethylbutyryloxy, 2-methylvaleroyloxy, 4-methylvaleroyloxy, heptanoyloxy, 2,2-dimethylvaleroyloxy, 2-ethylhexanoyloxy, 2-propylvaleroyloxy, octanoyloxy, nonanoyloxy, 3,5,5-trimethylhexanoyloxy, decanoyloxy or the like and is selected within a given range of carbon atoms.

"R₃ and R₄ may form a 3- to 7-membered ring together with each other" means that R₃, R₄ may form cyclopropyl, cyclobutyl, cyclopentyl, cyclohexyl, cycloheptyl, epoxy, tetrahydrofuran , tetrahydropyran, tetrahydrothiophene, tetrahydrothiopyran, pyrrolidine, piperidine or the like containing the carbon attached thereto.

"R₅, R₆, R₇ or R₈ may form, together with R₅, R₆, R₇ or R₈ on the same carbon, an optionally substituted 3- to 7-membered ring which may contain one or two hetero atoms selected from oxygen atoms, sulfur atoms and nitrogen atoms (the nitrogen atoms may be substituted with C₁-C₆ alkyl, C₂-C₆ alkenyl, C₂-C₆ alkynyl or cyclo C₃₋C₆ alkyl)" means that R₅, R₆, R₇ or R₈ and R₅, R₆, R₇ or R₈ on the same carbon may form cyclopropyl, dichlorocyclopropyl, dimethylcyclopropyl, cyclobutyl, cyclopentyl, cyclohexyl, cycloheptyl, epoxy, tetrahydrofuran , tetrahydropyran, tetrahydrothiophene, tetrahydrothiopyran, pyrrolidine, piperidine or the like containing the carbon attached thereto.

"R₅, R₆, R₇ or R₈ may form, together with R₅, R₆, R₇ or R₈ on a different carbon, an optionally substituted 3- to 8-membered ring which may contain one or two hetero atoms selected from oxygen atoms, sulfur atoms and nitrogen atoms (the nitrogen atoms may be substituted with C₁-C₆ alkyl, C₂-C₆ alkenyl, C₂-C₆ alkynyl or cyclo C₃-C₆ alkyl)" means that R₅, R₆, R₇ or R₈ and R₅, R₆, R₇ or R₈ on a different carbon may form a linkage such as -CH₂-, -CH₂ -CH₂-, -CH₂ -CCl₂-, -O-, -O-CH₂-, -S-, -S-CH₂-, -NR₁₂- (R₁₂ is C₁-C6 alkyl, C₂-C₆ alkenyl, C₂-C₆ alkynyl or cycloC₃-C₆ alkyl) and -N R₁₂-CH₂- (R₁₂ is the same as defined previously).

"R₅ or R₆ may form a bond together with R₅ or R₆ on an adjacent carbon" means that a double bond may be formed between the carbon attached to R₅ or R₆ and the adjacent carbon attached to R₅ or R₆.

"Y may be C₁-C4 alkylene, halo C₁- C₄ alkylene, C₂-C₄ alkenylene or halo C₂-C₄ alkenylene which may contain one or two hetero atoms selected from oxygen atoms, sulfur atoms and nitrogen atoms (the nitrogen atoms may be substituted with C₁-C₆ alkyl, C₂-C₆ alkenyl, C₂-C₆ alkynyl or cyclo C₃-C₆ alkyl) and form a 5- or 6-membered ring together with an adjacent carbon or nitrogen atom on a benzene ring or a heterocyclyl" means that indene, dihydroindene, naphthalene, dihydronaphthalene, tetrahydronaphthalene, benzofuran, dihydrobenzofuran, chromene, chroman, benzothiophene, dihydrobenzothiophene, thiochromene, thiochroman, indole, indoline, quinoline, dihydroquinoline, tetrahydroquinoline, cyclopentapyridine, pyrrolopyridine, naphthylidine, pyrazolopyridine, triazolopyrimidine or the like containing the benzene ring or a heterocyclyl attached to Y.

The compounds of the present invention can be prepared , for example, by the processes represented by the following reaction schemes 1 to 7, but may be prepared by other processes. 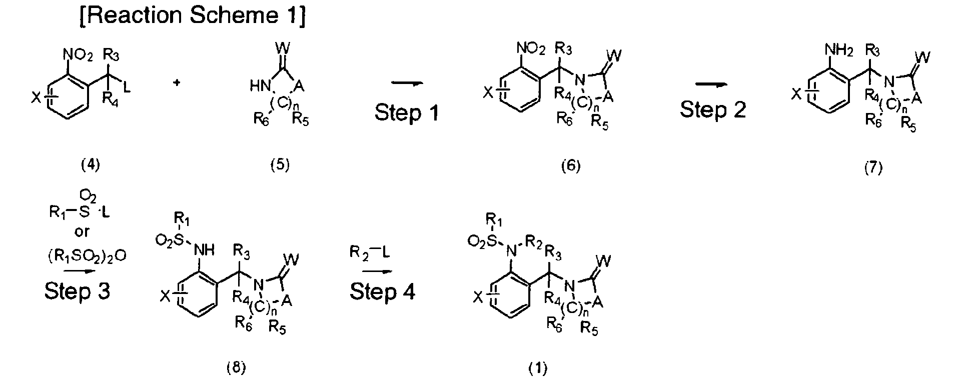 [wherein R₁, R₂, R₃, R₄, R₅, R₆, A, W, X and n are the same as defined previously, and L is a leaving group such as a halogen atom.]

As shown in Reaction Scheme 1, a compound represented by the formula (4) and a compound represented by the formula (5) are reacted to give a compound represented by the formula (6). After or without isolation, the compound represented by the formula (6) is converted to a compound represented by the formula (7) by reduction of the nitro group. After or without isolation, the compound represented by the formula (7) is reacted with a haloalkylsulfonyl derivative represented by the formula R₁SO₂L or (R₁SO₂)₂O to form a compound represented by the formula (8). Further, after or without isolation, the compound represented by the formula (8) is reacted with a compound represented by the formula R₂-L to give a compound of the present invention represented by the formula (1).

The reaction intermediate (4) may be commercially available or may be synthesized in accordance with Journal of Organic Chemistry 73(15),5989-5992(2008) or Bioorganic & Medicinal Chemistry 15(20),6574-6595(2007).

The reaction intermediate (5) may be commercially available or may be synthesized in accordance with Journal of the American Chemical Society 102(25),7578-7579(1980), Journal of Organic Chemistry 72(12), 4536-4538 (2007 Journal of the American Chemical Society 109(12),3789-3790(1987), Tetrahedron 43(10),2343-2348(1987), Tetrahedron Letters 32(34),4393-4396(1991), Tetrahedron 57,8647-8651(2001) or the Reference Examples herein. 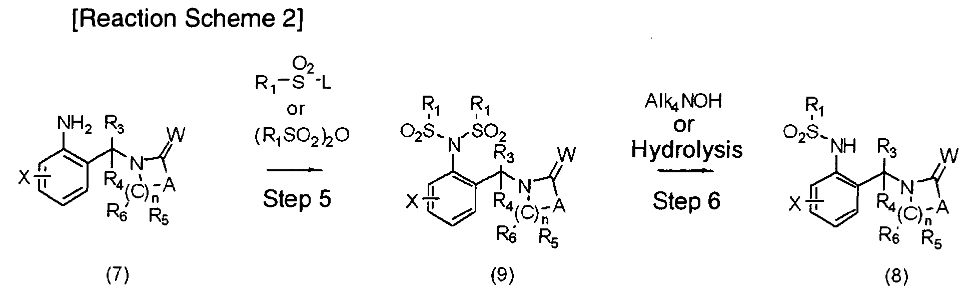 [wherein R₁, R₃, R₄, R₅, R₆, A, W, X and n are the same as defined previously, L is a leaving group such as a halogen atom, and Alk is an alkyl group.]

As shown in Reaction Scheme 2, a compound represented by the formula (7) is reacted with an excess of a haloalkylsulfonyl derivative represented by the formula R₁SO₂L or (R₁SO₂)₂O to give a compound of the present invention represented by the formula (9). After or without isolation, the compound represented by the formula (9) is reacted with a compound represented by the formula Alk₄NOH or hydrolyzed with sodium hydroxide, potassium hydroxide or the like to give a compound of the present invention represented by the formula (8). 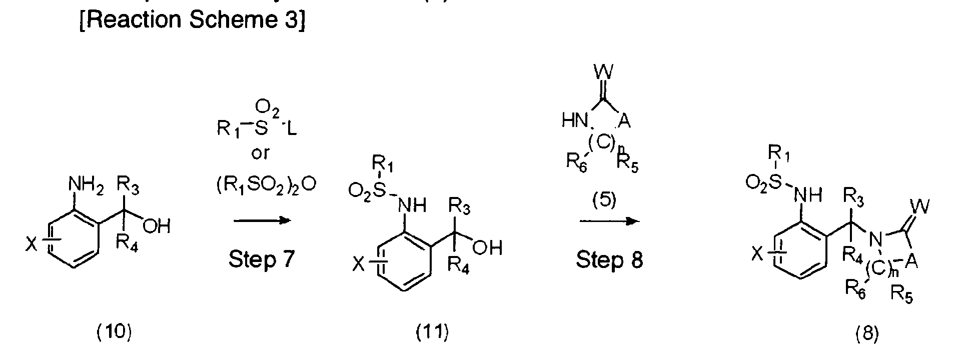 [wherein R₁, R₃, R₄, R₅, R₆, A, W, X and n are the same as defined previously, and L is a leaving group such as a halogen atom.]

As shown in Reaction Scheme 3, a compound represented by the formula (10) is reacted with a haloalkylsulfonyl derivative represented by the formula R₁SO₂L or (R₁SO₂)₂O to give a compound represented by the formula (11). Further, after or without isolation, the compound represented by the formula (11) is reacted with a compound represented by the formula (5) to give a compound of the present invention represented by the formula (8).

The reaction intermediate (10) may be commercially available or may be synthesized in accordance with Medicinal Chemistry 4(4),298-308(2008) or 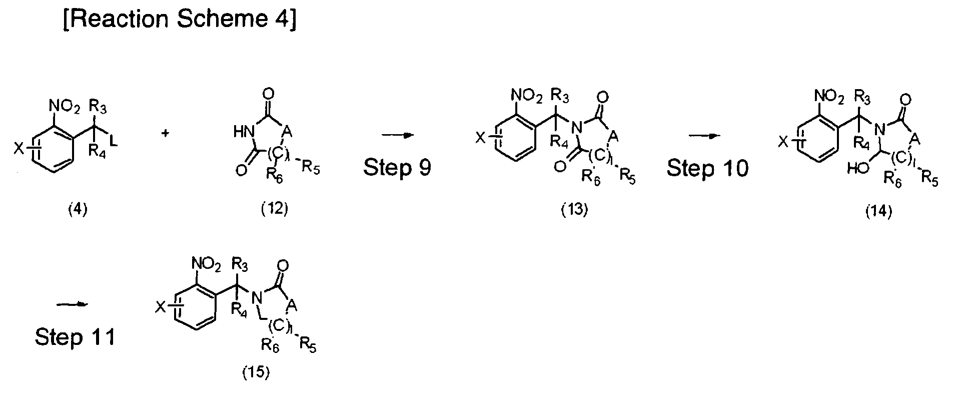 [wherein R₃, R₄, R₅, R₆, A and X are the same as defined previously, and I is an integer of from 1 to 3.]

As shown in Reaction Scheme 4, a compound represented by the formula (4) and a compound represented by the formula (12) are reacted to give a compound represented by the formula (13). After or without isolation, the compound represented by the formula (13) is converted to a compound represented by the formula (14) by reduction of the carbonyl group. Further, after or without isolation, the compound represented by the formula (14) is converted to a compound represented by the formula (15) by reduction of the hydroxyl group.

The reaction intermediate (12) may be commercially available or may be synthesized in accordance with Heterocycles 65(1),9-22(2005), Journal de Pharmacie de Belgique 17(1-2),3-13(1962) or Australian Journal of Chemistry 13,127-144(1960). 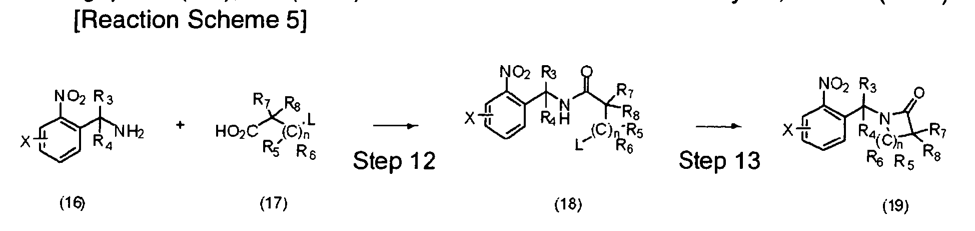 [wherein R₃, R₄, R₅, R₆, R₇, R₈, X and n are the same as defined previously, and L is a leaving group such as a halogen atom.]

As shown in Reaction Scheme 5, a compound represented by the formula (16) and a compound represented by the formula (17) are reacted with a condensation agent to give a compound represented by the formula (18). After or without isolation, the compound represented by the formula (18) is cyclized to give a compound represented by the formula (19).

The reaction intermediate (16) may be commercially available or may be synthesized in accordance with W02006/113432, Bioorganic & Medicinal Chemistry Letters 16(13),3463-3468(2006) or Chemical Communications (14),1557-1559(2006).

The reaction intermediate (17) may be commercially available or may be synthesized in accordance with Journal of the Chemical Society 4286-4288(1962), Tetrahedron Letters 46(41),7007-7009(2005), Canadian Journal of Chemistry 64(3),457-463(1986) or Synthesis(11),963-965(1982). 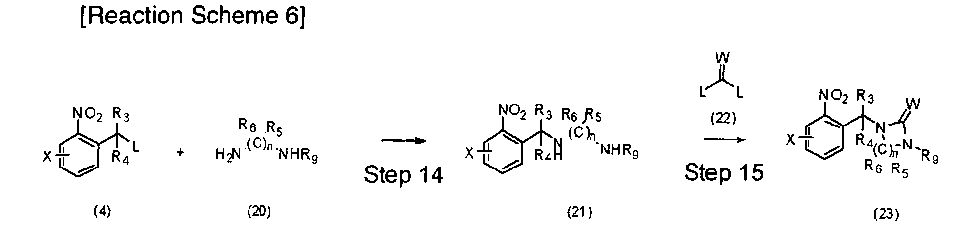 [wherein R₃, R₄, R₅, R₆, R₉, W, X and n are the same as defined previously, and L is a leaving group such as a halogen atom.]

As shown in Reaction Scheme 6, a compound represented by the formula (4) and a compound represented by the formula (20) are reacted to give a compound represented by the formula (21). After or without isolation, the compound represented by the formula (21) is cyclized with a compound represented by the formula (22) to give a compound represented by the formula (23).

The reaction intermediate (20) may be commercially available or may be synthesized in accordance with Journal of Organic Chemistry 74(6),2571-2574(2009) or Journal of Organic Chemistry 67(12),4086-4092(2002).

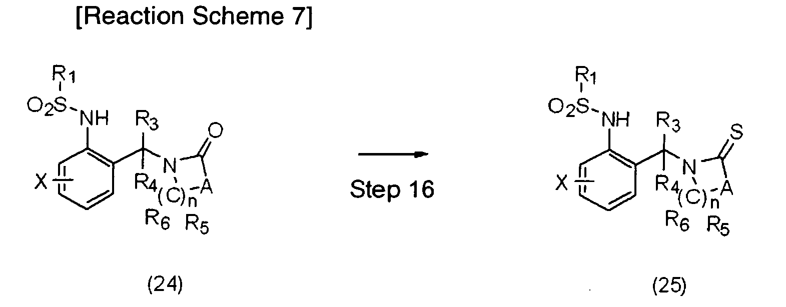

### [wherein R₁, R₃, R₄, R₅, R₆, A, X and n are the same as defined previously.]

As shown in Reaction Scheme 7, a compound represented by the formula (24) is thiolated to give a compound of the present invention represented by the formula (25).

The reactions of Steps 1, 3 to 5, 7, 9 and 12 to 15 in Reaction Schemes 1 to 7 may be carried out in the presence of a base, and as the base, an inorganic salt such as sodium hydroxide, potassium hydroxide, sodium carbonate, potassium carbonate, sodium hydrogen carbonate or sodium hydride, an organic base such as pyridine, 4-dimethylaminopyridine, triethylamine, tributylamine, N,N-dimethylaniline or 1,8-diazabicyclo[5.4.0]-7-undecene, an organic lithium such as butyllithium or s-butyllithium, an organic lithium amide such as lithiumdiisopropylamide or lithiumbis(trimethylsilyl)amide, or a metal alkoxide such as sodium methoxide, sodium ethoxide or potassium t-butoxide may be mentioned. The base is used usually in an amount of from 0 to 10 eq., preferably from 0 to 2 eq., in relation to 1 eq. of the compound as the reactant.

In the reactions in Reaction Schemes 1 to 7, a solvent may be used, in necessary. Any solvents inert to the reactions without any particular restrictions. For example, hydrocarbons such as hexane, cyclohexane, benzene and toluene, halogenated hydrocarbons such as carbon tetrachloride, chloroform and 1,2-dichloroethane, ethers such as diethyl ether, diisopropyl ether, 1,4-dioxane and tetrahydrofuran, ketones such as acetone, methyl ethyl ketone and methyl isobutyl ketone, nitriles such as acetonitrile and propionitrile, carboxylic acid esters such as ethyl acetate and ethyl propionate, nitrogen-containing aprotic polar solvents such as N,N-dimethylformamide, N,N-dimethylacetamide, N-methyl-2-pyrrolidone and 1,3-dimethyl-2-imidazolidinone, sulfur-containing aprotic polar solvents such as dimethyl sulfoxide and sulfolane, pyridines such as pyridine and picoline and the like may be mentioned. These solvent may be used singly or in combination of at least two.

The reactions in Reaction Schemes 1 to 7 may be carried out in a homogeneous system or a two-phase system. In the case of a two-phase system, use of a phase transfer catalyst can be effective. As the phase transfer catalyst to be used, a quaternary ammonium salt such as tetrabutylammonium chloride or tetrabutylammonium bromide or a crown ether such as 18-crown-6 may be mentioned.

In Reaction Schemes 1 to 7, the reaction temperatures are usually from -90 to 200°C, preferably 0 to 120°C.

In Reaction Schemes 1 to 7, the reaction times are usually from 0.05 to 100 hours, preferably from 0.5 to 10 hours.

The reaction of Step 8 in Reaction Scheme 3 may be carried out in the presence of an acid catalyst. As the acid catalyst to be used, an organic acid such as hydrochloric acid, sulfuric acid, trifluroacetic acid, methanesulfonic acid or p-toluenesulfonic acid, a Lewis acid such as zinc chloride, titanium tetrachloride or boron trifluoride etherate complex may be mentioned. The acid catalyst is used usually in an amount of from 0 to 10 eq., preferably from 0 to 2 eq., in relation to 1 eq. of the compound as the reactant.

In the reaction of Step 10 in Reaction Scheme 4, the carbonyl group may be reduced by any method without any particular restrictions, for example, with a metal such as iron, zinc chloride or tin chloride, by hydrogenation in the presence of a palladium catalyst, or with a metal hydride such as sodium boron hydride, lithium boron hydride or aluminum lithium hydride.

In the reaction of Step 11 in Reaction Scheme 4, the hydroxyl group may be reduced by any method without any particular restrictions, for example, with triethylsilane/trifluoroacetic acid or by dehydration with an acid catalyst followed by hydrogenation in the presence of a metal catalyst such as palladium.

The condensation agent used in the reaction of Step 12 in Reaction Scheme 5 is not particularly restricted and may be WSC (Water Soluble Carbodiiumide), N,N'-dicyclohexylcarbodiimide, oxalyl chloride, thionyl chloride or the like.

The thiolation agent used in Reaction Scheme 7 is not particularly restricted and may be Lawesson's reagent, Yokoyama's reagent, PSCl₃, P₂S₅ or the like.

The desired product obtained by the above-mentioned reactions may be isolated and purified by filtration, extraction, washing, column chromatography, recrystallization, distillation and other operations.

Next, examples of the haloalkylsulfonanilde compounds covered by the present invention and their intermediates are shown in Tables 2 to 4 and Tables 5 to 9, respectively, but the present invention should not be restricted thereto.

The symbols in the tables have the following meanings, and for example, Ph-2-Cl means 2-chlorophenyl.

Me: methyl group, Et: ethyl group, Pr: propyl group, Bu: butyl group, Pen: pentyl group, Hex: hexyl group, Ph: phenyl group.

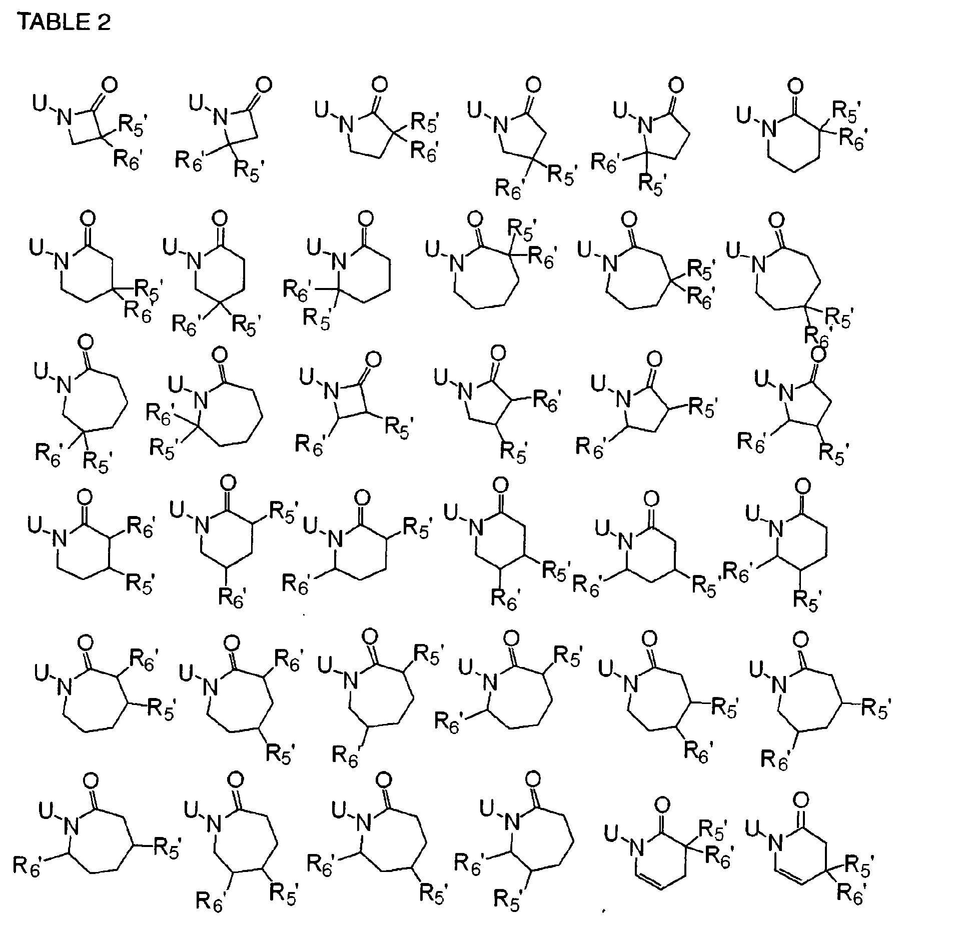

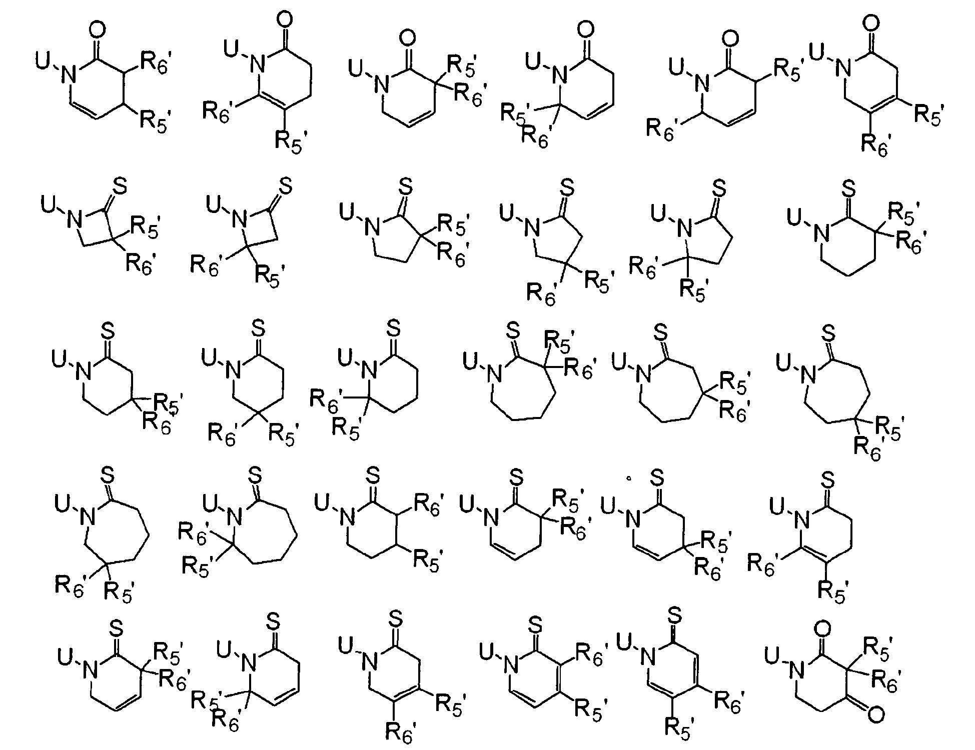

U is represented by the following formula (26).

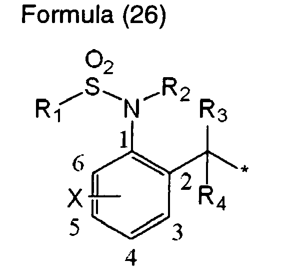

In the table, the position of a substituent X is represented by the numerals in the formula (26), and for example, 3-F means that the carbon marked with 3 on the benzene ring is substituted with F.

| R₃ = R₄ =H | | | | |
|---|---|---|---|---|
| R₁ | R₂ | R₅' | R₆' | X |
| CF₃ | H | H | H | H |
| CF₃ | H | Me | H | H |
| CF₃ | H | Me | Me | H |
| CF₃ | H | Me | Cl | H |
| CF₃ | H | Me | CO₂ Et | H |
| CF₃ | H | Et | H | H |
| CF₃ | H | Et | Me | H |
| CF₃ | H | Et | CO₂ Et | H |
| CF₃ | H | Pr - n | H | H |
| CF₃ | H | Pr - n | Me | H |
| CF₃ | H | Pr - i | H | H |
| CF₃ | H | Pr - i | Me | H |
| CF₃ | H | Pr - c | H | H |
| CF₃ | H | Pr - c | Me | H |
| CF₃ | H | Bu - n | H | H |
| CF₃ | H | Bu - n | Me | H |
| CF₃ | H | Bu - i | H | H |
| CF₃ | H | Bu - i | Me | H |
| CF₃ | H | Bu - s | H | H |
| CF₃ | H | Bu - s | Me | H |
| CF₃ | H | Bu - t | H | H |
| CF₃ | H | Bu - t | Me | H |
| CF₃ | H | Bu - c | H | H |
| CF₃ | H | Bu - c | Me | H |
| CF₃ | H | Pen - n | H | H |
| CF₃ | H | Pen - n | Me | H |
| CF₃ | H | Pen - c | H | H |
| CF₃ | H | Pen - c | Me | H |
| CF₃ | H | Hex - n | H | H |
| CF₃ | H | Hex - n | Me | H |
| CF₃ | H | Hex - c | H | H |
| CF₃ | H | Hex - c | Me | H |
| CF₃ | H | C l | H | H |
| CF₃ | H | C l | Cl | H |
| CF₃ | H | Br | H | H |
| CF₃ | H | Br | Br | H |
| CF₃ | H | I | H | H |
| CF₃ | H | F | H | H |
| CF₃ | H | CH=CH₂ | H | H |
| CF₃ | H | CH=CH₂ | Me | H |
| CF₃ | H | C≡CH | H | H |
| CF₃ | H | C≡CH | Me | H |
| CF₃ | H | CH₂ CH=CH₂ | H | H |
| CF₃ | H | CH₂CH=CH₂ | Me | H |
| CF₃ | H | CH₂C≡CH | H | H |
| CF₃ | H | CH₂C≡CH | Me | H |
| CF₃ | H | CH₂ C l | H | H |
| CF₃ | H | CH₂ C l | Me | H |
| CF₃ | H | CH₂ Cl | CH₂ C | l H |
| CF₃ | H | CHCl₂ | H | H |
| CF₃ | H | CC l₃ | H | H |
| CF₃ | H | CH₂F | H | H |
| CF₃ | H | CHF₂ | H | H |
| CF₃ | H | CF₃ | H | H |
| CF₃ | H | CF₃ | Me | H |
| CF₃ | H | CF₃ | Et | H |
| C F ₃ | H | CH₂CH₂Cl | H | H |
| CF₃ | H | CH₂OH | H | H |
| CF₃ | H | CH₂SMe | H | H |
| CF₃ | H | GH₂ SCH₂ C l | H | H |
| CF₃ | H | Ph | H | H |
| CF₃ | H | Ph | Me | H |
| CF₃ | H | Ph-2-Cl | H | H |
| CF₃ | H | Ph-2-Cl | Me | H |
| CF₃ | H | Ph-3-Cl | H | H |
| CF₃ | H | Ph-3-Cl | Me | H |
| CF₃ | H | Ph-4-Cl | H | H |
| CF₃ | H | Ph-4-Cl | Me | H |
| CF₃ | H | Ph-3-I | H | H |
| CF₃ | H | Ph-4-F | H | H |
| CF₃ | H | Ph-2-Me | H | H |
| CF₃ | H | Ph-3-OMe | H | H |
| CF₃ | H | Ph-4-SMe | H | H |
| CF₃ | H | Ph-3-CN | H | H |
| CF₃ | H | Ph-4-NO₂ | H | H |
| CF₃ | H | Ph-2-CO₂Me | H | H |
| CF₃ | H | Ph-3-NH₂ | H | H |
| CF₃ | H | Ph-4-Ph | H | H |
| CF₃ | H | Ph-2-OPh | H | H |
| CF₃ | H | Ph-3-SPh | H | H |
| CF₃ | H | Ph-4-CONMe₂ | H | H |
| CF₃ | H | Ph-2-OH | H | H |
| CF₃ | H | COMe | H | H |
| CF₃ | H | COEt | H | H |
| CF₃ | H | COCH₂ Cl | H | H |
| CF₃ | H | CO₂H | H | H |
| CF₃ | H | CO₂Me | H | H |
| CF₃ | H | CO₂Me | Me | H |
| CF₃ | H | CO₂ Et | H | H |
| CF₃ | H | CO₂ Bu- i | H | H |
| CF₃ | H | CONH₂ | H | H |
| CF₃ | H | CONHMe | H | H |
| CF₃ | H | CONMe₂ | H | H |
| CF₃ | H | CH₂Ph | H | H |
| CF₃ | H | CH₂Ph-2-Cl | H | H |
| CF₃ | H | CH₂ Ph-3-Cl | H | H |
| CF₃ | H | CH₂ Ph-4-Cl | H | H |
| CF₃ | H | COPh | H | H |
| CF₃ | H | COPh-2-Cl | H | H |
| CF₃ | H | COPh-3-Cl | H | H |
| CF₃ | H | COPh-4-Cl | H | H |
| CF₃ | H | OH | H | H |
| CF₃ | H | OMe | H | H |
| CF₃ | H | OMe | Me | H |
| CF₃ | H | OEt | H | H |
| CF₃ | H | OEt | Me | H |
| CF₃ | H | SMe | H | H |
| CF₃ | H | SMe | Me | H |
| CF₃ | H | SPh | H | H |
| CF₃ | H | CN | H | H |
| CF₃ | H | NH₂ | H | H |
| CF₃ | H | NHMe | H | H |
| CF₃ | H | NHMe | Me | H |
| CF₃ | H | NMe₂ | H | H |
| CF₃ | H | NMe₂ | Me | H |
| CF₃ | H | -CH₂-O-CH₂- | | H |
| CF₃ | H | - (CH₂)₂- | | H |
| CF₃ | H | -CH₂CCl₂- | | H |
| CF₃ | H | -CH₂CF₂- | | H |
| CF₃ | H | - (CH₂)₃- | | H |
| CF₃ | H | - (CH₂)₄- | | H |
| CF₃ | H | - (CH₂)₅ - | | H |
| CF₃ | H | H | H | 3-F |
| CF₃ | H | H | H | 4-F |
| CF₃ | H | H | H | 5-F |
| CF₃ | H | H | H | 6-F |
| CF₃ | H | H | H | 4-Cl |
| CF₃ | H | H | H | 5-Cl |
| CF₃ | H | H | H | 4-Br |
| CF₃ | H | H | H | 4- I |
| CF₃ | H | H | H | 4-Me |
| CF₃ | H | H | H | 5-Me |
| CF₃ | H | H | H | 4-OMe |
| CF₃ | H | H | H | 4-SMe |
| CF₃ | H | Me | H | 3-F |
| CF₃ | H | Me | H | 4-F |
| CF₃ | H | Me | H | 5-F |
| CF₃ | H | Me | H | 6-F |
| CF₃ | H | Me | H | 4-Cl |
| CF₃ | H | Me | H | 5-Cl |
| CF₃ | H | Me | H | 4-Br |
| CF₃ | H | Me | H | 4 - I |
| CF₃ | H | Me | H | 4-Me |
| CF₃ | H | Me | H | 5-Me |
| CF₃ | H | Me | H | 4-OMe |
| CF₃ | H | Me | H | 4-SMe |
| CF₃ | H | Me | Me | 3-F |
| CF₃ | H | Me | Me | 4-F |
| CF₃ | H | Me | Me | 5-F |
| CF₃ | H | Me | Me | 6-F |
| CF₃ | H | Me | Me | 4-Cl |
| CF₃ | H | Me | Me | 5-Cl |
| CF₃ | H | Me | Me | 4-Br |
| CF₃ | H | Me | Me | 4 - I |
| CF₃ | H | Me | Me | 4-Me |
| CF₃ | H | Me | Me | 5-Me |
| CF₃ | H | Me | Me | 4-OMe |
| CF₃ | H | Me | Me | 4-SMe |
| CF₃ | Me | H | H | H |
| CF₃ | Me | Me | H | H |
| CF₃ | Me | Me | Me | H |
| CF₃ | Et | H | H | H |
| CF₃ | Et | Me | H | H |
| CF₃ | Et | Me | Me | H |
| CF₃ | COMe | H | H | H |
| CF₃ | COMe | Me | H | H |
| CF₃ | COMe | Me | Me | H |
| CF₃ | COMe | Et | H | H |
| CF₃ | COMe | Et | Me | H |
| CF₃ | COMe | Pr-n | H | H |
| CF₃ | COMe | Pr-n | Me | H |
| CF₃ | COMe | C l | H | H |
| CF₃ | COMe | CH₂CH=CH₂ | H | H |
| CF₃ | COMe | CH₂C l | H | H |
| CF₃ | COMe | CF₃ | H | H |
| CF₃ | COMe | CF₃ | Me | H |
| CF₃ | COMe | Ph | H | H |
| CF₃ | COMe | Ph | Me | H |
| CF₃ | COMe | Ph-2-Cl | H | H |
| CF₃ | COMe | Ph-3-Cl | H | H |
| CF₃ | COMe | Ph-4-Cl | H | H |
| CF₃ | COMe | COMe | H | H |
| CF₃ | COMe | CO₂H | H | H |
| CF₃ | COMe | CO₂Me | H | H |
| CF₃ | COMe | CONH₂ | H | H |
| CF₃ | COMe | CONHMe | H | H |
| CF₃ | COMe | CONMe₂ | H | H |
| CF₃ | COMe | OH | H | H |
| CF₃ | COMe | OMe | H | H |
| CF₃ | COMe | OMe | Me | H |
| CF₃ | COMe | SMe | H | H |
| C F ₃ | COMe | SMe | Me | H |
| CF₃ | COMe | NHMe | H | H |
| CF₃ | COMe | NHMe | Me | H |
| CF₃ | COMe | CH₂ Ph | H | H |
| CF₃ | COMe | CH₂ Ph-2-Cl | H | H |
| CF₃ | COMe | CH₂ Ph-3-Cl | H | H |
| CF₃ | COMe | CH₂ Ph-4-Cl | H | H |
| CF₃ | COMe | COPh | H | H |
| CF₃ | COMe | COPh-2-Cl | H | H |
| CF₃ | COMe | COPh-3-Cl | H | H |
| CF₃ | COMe | COPh-4-Cl | H | H |
| CF₃ | COMe | -CH₂-O-CH₂- | | H |
| CF₃ | COMe | - (CH₂)₂- | | H |
| CF₃ | COMe | - (CH₂)₃- | | H |
| CF₃ | COMe | - (CH₂)₄- | | H |
| CF₃ | COMe | - (CH₂)₅ | | H |
| CF₃ | COEt | H | H | H |
| CF₃ | COEt | Me | H | H |
| CF₃ | COEt | Me | Me | H |
| CF₃ | COEt | Me | Cl | H |
| CF₃ | COEt | Me | CO₂ Et | H |
| CF₃ | COEt | Et | H | H |
| CF₃ | COEt | Et | Me | H |
| CF₃ | COEt | Et | CO₂ E t | H |
| CF₃ | COEt | Pr-n | H | H |
| CF₃ | COEt | Pr-n | Me | H |
| CF₃ | COEt | Pr-i | H | H |
| CF₃ | COEt | Pr-i | Me | H |
| CF₃ | COEt | Pr- c | H | H |
| CF₃ | COEt | Pr- c | Me | H |
| CF₃ | COEt | Bu-n | H | H |
| CF₃ | COEt | Bu-n | Me | H |
| C F ₃ | COEt | Bu-i | H | H |
| CF₃ | COEt | Bu-i | Me | H |
| CF₃ | COEt | Bu- s | H | H |
| CF₃ | COEt | Bu-s | Me | H |
| CF₃ | COEt | Bu-t | H | H |
| CF₃ | COEt | Bu-t | Me | H |
| CF₃ | COEt | Bu-c | H | H |
| CF₃ | COEt | Bu-c | Me | H |
| CF₃ | COEt | Pen-n | H | H |
| CF₃ | COEt | Pen-n | Me | H |
| CF₃ | COEt | Pen-c | H | H |
| CF₃ | COEt | Pen-c | Me | H |
| CF₃ | COEt | Hex-n | H | H |
| CF₃ | COEt | Hex-n | Me | H |
| CF₃ | COEt | Hex-c | H | H |
| CF₃ | COEt | Hex-c c | Me | H |
| CF₃ | COEt | Cl | H | H |
| CF₃ | COEt | Cl | C l | H |
| CF₃ | COEt | Cl | Me | H |
| CF₃ | COEt | Br | H | H |
| CF₃ | COEt | Br | Br | H |
| CF₃ | COEt | I | H | H |
| CF₃ | COEt | F | H | H |
| CF₃ | COEt | CH=CH₂ | H | H |
| CF₃ | COEt | CH=CH₂ | Me | H |
| CF₃ | COEt | C≡CH | H | H |
| CF₃ | COEt | C≡CH | Me | H |
| CF₃ | COEt | CH₂CH=CH₂ | H | H |
| CF₃ | COEt | CH₂ CH=CH₂ | Me | H |
| CF₃ | COEt | CH₂C≡CH | H | H |
| CF₃ | COEt | CH₂C≡CH | Me | H |
| CF₃ | COEt | CH₂ C I | H | H |
| CF₃ | COEt | CH₃ C l | Me | H |
| CF₃ | COEt | CH₂ C l | CH₂ C l | H |
| CF₃ | COEt | CHCl₂ | H | H |
| CF₃ | COEt | CCl₃ | H | H |
| CF₃ | COEt | CH₂F | H | H |
| CF₃ | COEt | CHF₂ | H | H |
| CF₃ | COEt | CF₃ | H | H |
| CF₃ | COEt | CF₃ | Me | H |
| CF₃ | COEt | CF₃ | Et | H |
| CF₃ | COEt | CH₂CH₂Cl | H | H |
| CF₃ | COEt | CH₂OH | H | H |
| CF₃ | COEt | CH₂SMe | H | H |
| CF₃ | COEt | CH₂SCH₂Cl | H | H |
| CF₃ | COEt | Ph | H | H |
| CF₃ | COEt | Ph | Me | H |
| CF₃ | COEt | Ph-2-Cl | H | H |
| CF₃ | COEt | Ph-2-Cl | Me | H |
| CF₃ | COEt | Ph-3-Cl | H | H |
| CF₃ | COEt | Ph-3-Cl | Me | H |
| CF₃ | COEt | Ph-4-Cl | H | H |
| CF₃ | COEt | Ph-4-Cl | Me | H |
| CF₃ | COEt | Ph-2-Br | H | H |
| CF₃ | COEt | Ph-3-I | H | H |
| CF₃ | COEt | Ph-4-F | H | H |
| CF₃ | COEt | Ph-2-Me | H | H |
| CF₃ | COEt | Ph-3-OMe | H | H |
| CF₃ | COEt | Ph-4-SMe | H | H |
| CF₃ | COEt | Ph-2-SO₂Me | H | H |
| CF₃ | COEt | Ph-3-CN | H | H |
| CF₃ | COEt | P h-4-NO₂ | H | H |
| CF₃ | COEt | Ph-2-CO₂Me | H | H |
| CF₃ | COEt | Ph-3-NH₂ | H | H |
| CF₃ | COEt | Ph-4-Ph | H | H |
| CF₃ | COEt | Ph-2-OPh | H | H |
| CF₃ | COEt | Ph-3-SPh | H | H |
| CF₃ | COEt | Ph-4-CONMe₂ | H | H |
| CF₃ | COEt | Ph-2-OH | H | H |
| CF₃ | COEt | COMe | H | H |
| CF₃ | COEt | COEt | H | H |
| CF₃ | COEt | COCH₂Cl | H | H |
| CF₃ | COEt | CO₂H | H | H |
| CF₃ | COEt | CO₂Me | H | H |
| CF₃ | COEt | CO₂Me | Me | H |
| CF₃ | COEt | CO₂Et | H | H |
| CF₃ | COEt | CO₂Bu-i | H | H |
| CF₃ | COEt | CONH₂ | H | H |
| CF₃ | COEt | CONHMe | H | H |
| CF₃ | COEt | CONMe₂ | H | H |
| CF₃ | COEt | CH₂Ph | H | H |
| CF₃ | COEt | CH₂Ph-2-Cl | H | H |
| CF₃ | COEt | CH₂Ph-3-Cl CH₂Ph-3-Cl | H | H |
| CF₃ | COEt | CH₂Ph-4-Cl | H | H |
| CF₃ | COEt | COPh | H | H |
| CF₃ | COEt | COPh-2-Cl | H | H |
| CF₃ | COEt | COPh-3-Cl | H | H |
| CF₃ | COEt | COPh-4-Cl | H | H |
| CF₃ | COEt | OH | H | H |
| CF₃ | COEt | OMe | H | H |
| CF₃ | COEt | OMe | Me | H |
| CF₃ | COEt | OEt | H | H |
| CF₃ | COEt | OEt | Me | H |
| CF₃ | COEt | SMe | H | H |
| CF₃ | COEt | SMe | Me | H |
| CF₃ | COEt | SPh | H | H |
| CF₃ | COEt | CN | H | H |
| CF₃ | COEt | NH₂ | H | H |
| CF₃ | COEt | NHMe | H | H |
| CF₃ | COEt | NHMe | Me | H |
| CF₃ | COEt | NMe₂ | H | H |
| CF₃ | COEt | NMe₂ | Me | H |
| CF₃ | COEt | -CH₂-O-CH₂- | | H |
| CF₃ | COEt | -(CH₂)₂- | | H |
| CF₃ | COEt | -CH₂CCl₂- | | H |
| CF₃ | COEt | -CH₂CF₂- | | H |
| CF₃ | COEt | -(CH₂)₃- | | H |
| CF₃ | COEt | -(CH₂)₄- | | H |
| CF₃ | COEt | -(CH₂)₅- | | H |
| CF₃ | COEt | H | H | 3-F |
| CF₃ | COEt | H | H | 4-F |
| CF₃ | COEt | H | H | 5-F |
| CF₃ | COEt | H | H | 6-F |
| CF₃ | COEt | H | H | 4-Cl |
| CF₃ | COEt | H | H | 5-Cl |
| CF₃ | COEt | H | H | 4-Br |
| CF₃ | COEt | H | H | 4-I |
| CF₃ | COEt | H | H | 4-Me |
| CF₃ | COEt | H | H | 5-Me |
| CF₃ | COEt | H | H | 4-OMe |
| CF₃ | COEt | H | H | 4-SMe |
| CF₃ | COEt | Me | H | 3-F |
| CF₃ | COEt | Me | H | 4-F |
| CF₃ | COEt | Me | H | 5-F |
| CF₃ | COEt | Me | H | 6-F |
| CF₃ | COEt | Me | H | 4-Cl |
| CF₃ | COEt | Me | H | 5-Cl |
| CF₃ | COEt | Me | H | 4-Br |
| CF₃ | COEt | Me | H | 4-I |
| CF₃ | COEt | Me | H | 4-Me |
| CF₃ | COEt | Me | H | 5-Me |
| CF₃ | COEt | Me | H | 4-OMe |
| CF₃ | COEt | Me | H | 4-SMe |
| CF₃ | COEt | Me | Me | 3-F |
| CF₃ | COEt | Me | Me | 4-F |
| CF₃ | COEt | Me | Me | 5-F |
| CF₃ | COEt | Me | Me | 6-F |
| CF₃ | COEt | Me | Me | 4-Cl |
| CF₃ | COEt | Me | Me | 5-Cl |
| CF₃ | COEt | Me | Me | 6-Cl |
| CF₃ | COEt | Me | Me | 3-Br |
| CF₃ | COEt | Me | Me | 4-Br |
| CF₃ | COEt | Me | Me | 5-Br |
| CF₃ | COEt | Me | Me | 4-I |
| CF₃ | COEt | Me | Me | 4-Me |
| CF₃ | COEt | Me | Me | 5-Me |
| CF₃ | COEt | Me | Me | 4-OMe |
| CF₃ | COEt | Me | Me | 4-SMe |
| CF₃ | COPr-n | H | H | H |
| CF₃ | COPr-n | Me | H | H |
| CF₃ | COPr-n | Me | Me | H |
| CF₃ | COPr-n | Et | H | H |
| CF₃ | COPr-n | Et | Me | H |
| CF₃ | COPr-n | Pr-n | H | H |
| CF₃ | COPr-n | Pr-n | Me | H |
| CF₃ | COPr-n | Cl | H | H |
| CF₃ | COPr-n | CH₂CH=CH₂ | H | H |
| CF₃ | COPr-n | CH₂Cl | H | H |
| CF₃ | COPr-n | CF₃ | H | H |
| CF₃ | COPr-n | CF₃ | Me | H |
| CF₃ | COPr-n | Ph | H | H |
| CF₃ | COPr-n | Ph | Me | H |
| CF₃ | COPr-n | Ph-2-Cl | H | H |
| CF₃ | COPr-n | Ph-3-Cl | H | H |
| CF₃ | COPr-n | Ph-4-Cl | H | H |
| CF₃ | COPr-n | COMe | H | H |
| CF₃ | COPr-n | CO₂H | H | H |
| CF₃ | COPr-n | CO₂Me | H | H |
| CF₃ | COPr-n | CONH₂ | H | H |
| CF₃ | COPr-n | CONHMe | H | H |
| CF₃ | COPr-n | CONMe₂ | H | H |
| CF₃ | COPr-n | OH | H | H |
| CF₃ | COPr-n | OMe | H | H |
| CF₃ | COPr-n | OMe | Me | H |
| CF₃ | COPr-n | SMe | H | H |
| CF₃ | COPr-n | SMe | Me | H |
| CF₃ | COPr-n | NHMe | H | H |
| CF₃ | COPr-n | NHMe | Me | H |
| CF₃ | COPr-n | CH₂Ph | H | H |
| CF₃ | COPr-n | CH₂Ph-2-Cl | H | H |
| CF₃ | COPr-n | CH₂Ph-3-Cl | H | H |
| CF₃ | COPr-n | CH₂Ph-4-Cl | H | H |
| CF₃ | COPr-n | COPh | H | H |
| CF₃ | COPr-n | COPh-2-Cl | H | H |
| CF₃ | COPr-n | COPh-3-Cl | H | H |
| CF₃ | COPr-n | COPh-4-Cl | H | H |
| CF₃ | COPr-n | -CH₂-O-CH₂- | | H |
| CF₃ | COPr-n | -(CH₂)₂- | | H |
| CF₃ | COPr-n | -(CH₂)₃- | | H |
| CF₃ | COPr-n | -(CH₂) | | H |
| CF₃ | COPr-n | -(CH₂)₅- | | H |
| CF₃ | COPr-i | H | H | H |
| CF₃ | COPr-i | Me | H | H |
| CF₃ | COPr-i | Me | Me | H |
| CF₃ | COPr-i | Et | H | H |
| CF₃ | COPr-i | Et | Me | H |
| CF₃ | COPr-i | Pr-n | H | H |
| CF₃ | COPr-i | Pr-n | Me | H |
| CF₃ | COPr-i | Cl | H | H |
| CF₃ | COPr-i | CH₂CH=CH₂ | H | H |
| CF₃ | COPr-i | CH₂Cl | H | H |
| CF₃ | COPr-i | CF₃ | H | H |
| CF₃ | COPr-i | CF₃ | Me | H |
| CF₃ | COPr-i | Ph | H | H |
| CF₃ | COPr-i | Ph | Me | H |
| CF₃ | COPr-i | Ph-2-Cl | H | H |
| CF₃ | COPr-i | Ph-3-Cl | H | H |
| CF₃ | COPr-i | Ph-4-Cl | H | H |
| CF₃ | COPr-i | COMe | H | H |
| CF₃ | COPr-i | CO₂H | H | H |
| CF₃ | COPr-i | CO₂Me | H | H |
| CF₃ | COPr-i | CONH₂ | H | H |
| CF₃ | COPr-i | CONHMe | H | H |
| CF₃ | COPr-i | CONMe₂ | H | H |
| CF₃ | COPr-i | OH | H | H |
| CF₃ | COPr-i | OMe | H | H |
| CF₃ | COPr-i | OMe | Me | H |
| CF₃ | COPr-i | SMe | H | H |
| CF₃ | COPr-i | SMe | Me | H |
| CF₃ | COPr-i | NHMe | H | H |
| CF₃ | COPr-i | NHMe | Me | H |
| CF₃ | COPr-i | CH₂Ph | H | H |
| CF₃ | COPr-i | CH₂Ph-2-Cl | H | H |
| CF₃ | COPr-i | CH₂Ph-3-Cl | H | H |
| CF₃ | COPr-i | CH₂Ph-4-Cl | H | H |
| CF₃ | COPr-i | COPh | H | H |
| CF₃ | COPr-i | COPh-2-Cl | H | H |
| CF₃ | COPr-i | COPh-3-Cl | H | H |
| CF₃ | COPr-i | COPh-4-Cl | H | H |
| CF₃ | COPr-i | -CH₂-O-CH₂- | | H |
| CF₃ | COPr-i | -(CH₂)₂- | | H |
| CF₃ | COPr-i | -(CH₂)₃- | | H |
| CF₃ | COPr-i | -(CH₂)₄- | | H |
| CF₃ | COPr-i | -(CH₂)₅- | | H |
| CF₃ | COPr-c | H | H | H |
| CF₃ | COPr-c | Me | H | H |
| CF₃ | COPr-c | Me | Me | H |
| CF₃ | COPr-c | Et | H | H |
| CF₃ | COPr-c | Et | Me | H |
| CF₃ | COPr-c | Pr-n | H | H |
| CF₃ | COPr-c | Pr-n | Me | H |
| CF₃ | COPr-c | Cl | H | H |
| CF₃ | COPr-c | CH₂CH=CH₂ | H | H |
| CF₃ | COPr-c | CH₂Cl | H | H |
| CF₃ | COPr-c | CF₃ | H | H |
| CF₃ | COPr-c | CF₃ | Me | H |
| CF₃ | COPr-c | Ph | H | H |
| CF₃ | COPr-c | Ph | Me | H |
| CF₃ | COPr-c | Ph-2-Cl | H | H |
| CF₃ | COPr-c | Ph-3-Cl | H | H |
| CF₃ | COPr-c | Ph-4-Cl | H | H |
| CF₃ | COPr-c | COMe | H | H |
| CF₃ | COPr-c | CO₂H | H | H |
| CF₃ | COPr-c | CO₂Me | H | H |
| CF₃ | COPr-c | CONH₂ | H | H |
| CF₃ | COPr-c | CONHMe | H | H |
| CF₃ | COPr-c | CONMe₂ | H | H |
| CF₃ | COPr-c | OH | H | H |
| CF₃ | COPr-c | OMe | H | H |
| CF₃ | COPr-c | OMe | Me | H |
| CF₃ | COPr-c | SMe | H | H |
| CF₃ | COPr-c | SMe | Me | H |
| CF₃ | COPr-c | NHMe | H | H |
| CF₃ | COPr-c | NHMe | Me | H |
| CF₃ | COPr-c | CH₂Ph | H | H |
| CF₃ | COPr-c | CH₂Ph-2-Cl | H | H |
| CF₃ | COPr-c | CH₂Ph-3-Cl | H | H |
| CF₃ | COPr-c | CH₂Ph-4-Cl | H | H |
| CF₃ | COPr-c | COPh | H | H |
| CF₃ | COPr-c | COPh-2-Cl | H | H |
| CF₃ | COPr-c | COPh-3-Cl | H | H |
| CF₃ | COPr-c | COPh-4-Cl | H | H |
| CF₃ | COPr-c | -CH₂-O-CH₂- | | H |
| CF₃ | COPr-c | -(CH₂)₂- | | H |
| CF₃ | COPr-c | -(CH₂)₃- | | H |
| CF₃ | COPr-c | -(CH₂)₄- | | H |
| CF₃ | COPr-c | -(CH₂)₅- | | H |
| CF₃ | COBu-n | H | H | H |
| CF₃ | COBu-n | Me | H | H |
| CF₃ | COBu-n | Me | Me | H |
| CF₃ | COBu-n | -(CH₂)₂- | | H |
| CF₃ | COBu-n | -(CH₂)₃- | | H |
| CF₃ | COBu-c | H | H | H |
| CF₃ | COBu-c | Me | H | H |
| CF₃ | COBu-c | Me | Me | H |
| CF₃ | COBu-c | -(CH₂)₂- | | H |
| CF₃ | COBu-c | -(CH₂)₃- | | H |
| CF₃ | COBu-t | H | H | H |
| CF₃ | COBu-t | Me | H | H |
| CF₃ | COBu-t | Me | Me | H |
| CF₃ | COBu-t | -CH₂-O-CH₂- | | H |
| CF₃ | COBu-t | -(CH₂)₂- | | H |
| CF₃ | COBu-t | -(CH₂)₃- | | H |
| CF₃ | COPen-n | H | H | H |
| CF₃ | COPen-n | Me | H | H |
| CF₃ | COPen-n | Me | Me | H |
| CF₃ | COPen-n | -(CH₂)₂- | | H |
| CF₃ | COPen-n | -(CH₂)₃- | | H |
| CF₃ | COHex-n | H | H | H |
| CF₃ | COHex-n | Me | H | H |
| CF₃ | COHex-n | Me | Me | H |
| CF₃ | COHex-n | -(CH₂)₂- | | H |
| CF₃ | COHex-n | -(CH₂)₃- | | H |
| CF₃ | COCH=CH₂ | H | H | H |
| CF₃ | COCH=CH₂ | Me | H | H |
| CF₃ | COCH=CH₂ | Me | Me | H |
| CF₃ | COCH=CH₂ | -(CH₂)₂- | | H |
| CF₃ | COCH=CH₂ | -(CH₂)₃- | | H |
| CF₃ | COPh | H | H | H |
| CF₃ | COPh | Me | H | H |
| CF₃ | COPh | Me | Me | H |
| CF₃ | COPh | Et | H | H |
| CF₃ | COPh | Et | Me | H |
| CF₃ | COPh | Pr-n | H | H |
| CF₃ | COPh | Pr-n | Me | H |
| CF₃ | COPh | Cl | H | H |
| CF₃ | COPh | CH₂CH=CH₂ | H | H |
| CF₃ | COPh | CH₂Cl | H | H |
| CF₃ | COPh | CF₃ | H | H |
| CF₃ | COPh | CF₃ | Me | H |
| CF₃ | COPh | Ph | H | H |
| CF₃ | COPh | Ph | Me | H |
| CF₃ | COPh | Ph-2-Cl | H | H |
| CF₃ | COPh | Ph-3-Cl | H | H |
| CF₃ | COPh | Ph-4-Cl | H | H |
| CF₃ | COPh | COMe | H | H |
| CF₃ | COPh | CO₂H | H | H |
| CF₃ | COPh | CO₂Me | H | H |
| CF₃ | COPh | CONH₂ | H | H |
| CF₃ | COPh | CONHMe | H | H |
| CF₃ | COPh | CONMe₂ | H | H |
| CF₃ | COPh | OH | H | H |
| CF₃ | COPh | OMe | H | H |
| CF₃ | COPh | OMe | Me | H |
| CF₃ | COPh | SMe | H | H |
| CF₃ | COPh | SMe | Me | H |
| CF₃ | COPh | NHMe | H | H |
| CF₃ | COPh | NHMe | Me | H |
| CF₃ | COPh | CH₂Ph | H | H |
| CF₃ | COPh | CH₂Ph-2-Cl | H | H |
| CF₃ | COPh | CH₂Ph-3-Cl | H | H |
| CF₃ | COPh | CH₂Ph-4-Cl | H | H |
| CF₃ | COPh | COPh | H | H |
| CF₃ | COPh | COPh-2-Cl | H | H |
| CF₃ | COPh | COPh-3-Cl | H | H |
| CF₃ | COPh | COPh-4-Cl | H | H |
| CF₃ | COPh | -CH₂-O-CH₂- | | H |
| CF₃ | COPh | -(CH₂)₂- | | H |
| CF₃ | COPh | -(CH₂)₃- | | H |
| CF₃ | COPh | -(CH₂)₄- | | H |
| CF₃ | COPh | -(CH₂)₅- | | H |
| CF₃ | CO₂Me | H | H | H |
| CF₃ | CO₂Me | Me | H | H |
| CF₃ | CO₂Me | Me | Me | H |
| CF₃ | CO₂Me | Et | H | H |
| CF₃ | CO₂Me | Et | Me | H |
| CF₃ | CO₂Me | Pr-n | H | H |
| CF₃ | CO₂Me | Pr-n | Me | H |
| CF₃ | CO₂Me | Cl | H | H |
| CF₃ | CO₂Me | CH₂ CH=CH₂ | H | H |
| CF₃ | CO₂Me CO₂Me | CH₂ Cl | H | H |
| CF₃ | CO₂Me | CF₃ | H | H |
| CF₃ | CO₂Me | CF₃ | Me | H |
| CF₃ | CO₂Me CO₂Me | Ph | H | H |
| CF₃ | CO₂Me | Ph | Me | H |
| CF₃ | CO₂Me | Ph-2-Cl | H | H |
| CF₃ | CO₂Me | Ph-3-Cl | H | H |
| CF₃ | CO₂Me | Ph-4-Cl | H | H |
| CF₃ | CO₂Me | COMe | H | H |
| CF₃ | CO₂Me | CO₂H | H | H |
| CF₃ | CO₂Me | CO₂Me | H | H |
| CF₃ | CO₂Me | CONH₂ | H | H |
| CF₃ | CO₂Me | CONHMe | H | H |
| CF₃ | CO₂Me | CONMe₂ | H | H |
| CF₃ | CO₂Me | OH | H | H |
| CF₃ | CO₂Me | OMe | H | H |
| CF₃ | CO₂Me | OMe | Me | H |
| CF₃ | CO₂Me | SMe | H | H |
| CF₃ | CO₂Me | SMe | Me | H |
| CF₃ | CO₂Me | NHMe | H | H |
| CF₃ | CO₂Me | NHMe | Me | H |
| CF₃ | CO₂Me | CH₂Ph | H | H |
| CF₃ | CO₂Me | CH₂Ph-2-Cl | H | H |
| CF₃ | CO₂Me | CH₂ Ph-3-Cl | H | H |
| CF₃ | CO₂Me | CH₂Ph-4-Cl | H | H |
| CF₃ | CO₂Me | COPh | H | H |
| CF₃ | CO₂Me | COPh-2-Cl | H | H |
| CF₃ | CO₂Me | COPh-3-Cl | H | H |
| CF₃ | CO₂Me | COPh-4-Cl | H | H |
| CF₃ | CO₂Me | -CH₂-O-CH₂- | | H |
| CF₃ | CO₂Me | -(CH₂)₂- | | H |
| CF₃ | CO₂Me | -(CH₂)₃- | | H |
| CF₃ | CO₂Me | -(CH₂)₄- | | H |
| CF₃ | CO₂Me | -(CH₂)₅- | | H |
| CF₃ | CO₂Et | H | H | H |
| CF₃ | CO₂Et | Me | H | H |
| CF₃ | CO₂Et | Me | Me | H |
| CF₃ | CO₂Et | Me | Cl | H |
| CF₃ | CO₂Et | Me | CO₂Et | H |
| CF₃ | CO₂Et | Et | H | H |
| CF₃ | CO₂Et | Et t | Me | H |
| CF₃ | CO₂Et | Et t | CO₂Et | H |
| CF₃ | CO₂Et | Pr-n | H | H |
| CF₃ | CO₂Et | Pr-n | Me | H |
| CF₃ | CO₂Et | Pr-i | H | H |
| CF₃ | CO₂Et | Pr-i | Me | H |
| CF₃ | CO₂Et | Pr-c | H | H |
| CF₃ | CO₂Et | Pr-c | Me | H |
| CF₃ | CO₂Et | Bu-n | H | H |
| CF₃ | CO₂Et | Bu-n | Me | H |
| CF₃ | CO₂Et | Bu-i | H | H |
| CF₃ | CO₂Et | Bu-i | Me | H |
| CF₃ | CO₂Et | Bu-s | H | H |
| CF₃ | CO₂Et | Bu-s | Me | H |
| CF₃ | CO₂Et | Bu-t | H | H |
| CF₃ | CO₂Et | Bu-t | Me | H |
| CF₃ | CO₂Et | Bu-c | H | H |
| CF₃ | CO₂Et | Bu-c | Me | H |
| CF₃ | CO₂Et | Pen-n | H | H |
| CF₃ | CO₂Et | Pen-n | Me | H |
| CF₃ | CO₂Et | Pen-c | H | H |
| CF₃ | CO₂Et | Pen-c | Me | H |
| CF₃ | CO₂Et | Hex-n | H | H |
| CF₃ | CO₂Et | Hex-n | Me | H |
| CF₃ | CO₂Et | Hex-c | H | H |
| CF₃ | CO₂Et | Hex-c | Me | H |
| CF₃ | CO₂Et | Cl | H | H |
| CF₃ | CO₂Et | Cl | Cl | H |
| CF₃ | CO₂Et | Br | H | H |
| CF₃ | CO₂Et | Br | Br | H |
| CF₃ | CO₂Et | I | H | H |
| CF₃ | CO₂Et | F | H | H |
| CF₃ | CO₂Et | CH=CH₂ | H | H |
| CF₃ | CO₂Et | CH=CH₂ | Me | H |
| CF₃ | CO₂Et | C≡CH | H | H |
| CF₃ | CO₂Et | C≡CH | Me | H |
| CF₃ | CO₂Et | CH₂CH=CH₂ | H | H |
| CF₃ | CO₂Et | CH₂CH=CH₂ | Me | H |
| CF₃ | CO₂Et | CH₂C≡CH | H | H |
| CF₃ | CO₂Et | CH₂C≡CH | Me | H |
| CF₃ | CO₂Et | CH₂Cl | H | H |
| CF₃ | CO₂Et | CH₂Cl | Me | H |
| CF₃ | CO₂Et | CH₂Cl | CH₂Cl | H |
| CF₃ | CO₂Et | CHCl₂ | H | H |
| CF₃ | CO₂Et | CCl₃ | H | H |
| CF₃ | CO₂Et | CH₂F | H | H |
| CF₃ | CO₂Et | CHF₂ | H | H |
| CF₃ | CO₂Et | CF₃ | H | H |
| CF₃ | CO₂Et | CF₃ | Me | H |
| CF₃ | CO₂Et | CF₃ | Et | H |
| CF₃ | CO₂Et | CH₂CH₂Cl | H | H |
| CF₃ | CO₂Et | CH₂OH | H | H |
| CF₃ | CO₂Et | CH₂SMe | H | H |
| CF₃ | CO₂Et | CH₂SCH₂Cl | H | H |
| CF₃ | CO₂Et | Ph | H | H |
| CF₃ | CO₂Et | Ph | Me | H |
| CF₃ | CO₂Et | Ph-2-Cl | H | H |
| CF₃ | CO₂Et | Ph-3-Cl | H | H |
| CF₃ | CO₂Et | Ph-4-Cl | H | H |
| CF₃ | CO₂Et | Ph-2-Br | H | H |
| CF₃ | CO₂Et | Ph-3-I | H | H |
| CF₃ | CO₂Et | Ph-4-F | H | H |
| CF₃ | CO₂Et | Ph-2-Me | H | H |
| CF₃ | CO₂Et | Ph-3-OMe | H | H |
| CF₃ | CO₂Et | Ph-4-SMe | H | H |
| CF₃ | CO₂Et | Ph-2-SO₂Me | H | H |
| CF₃ | CO₂Et | Ph-3-CN | H | H |
| CF₃ | CO₂Et | Ph-4-NO₂ | H | H |
| CF₃ | CO₂Et | Ph-2-CO₂Me | H | H |
| CF₃ | CO₂Et | Ph-3-NH₂ | H | H |
| CF₃ | CO₂Et | Ph-4-Ph | H | H |
| CF₃ | CO₂Et | Ph-2-OPh | H | H |
| CF₃ | CO₂Et | Ph-3-SPh | H | H |
| CF₃ | CO₂Et | Ph-4-CONMe₂ | H | H |
| CF₃ | CO₂Et | Ph-2-OH | H | H |
| CF₃ | CO₂Et | COMe | H | H |
| CF₃ | CO₂Et | COEt | H | H |
| CF₃ | CO₂Et | COCH₂Cl | H | H |
| CF₃ | CO₂Et | CO₂H | H | H |
| CF₃ | CO₂Et | CO₂Me | H | H |
| CF₃ | CO₂Et | CO₂Me | Me | H |
| CF₃ | CO₂Et | CO₂Et | H | H |
| CF₃ | CO₂Et | CO₂Bu-i | H | H |
| CF₃ | CO₂Et | CONH₂ | H | H |
| CF₃ | CO₂Et | CONHMe | H | H |
| CF₃ | CO₂Et | CONMe₂ | H | H |
| CF₃ | CO₂Et | CH₂Ph | H | H |
| CF₃ | CO₂Et | CH₂Ph-2-Cl | H | H |
| CF₃ | CO₂Et | CH₂Ph-3-Cl | H | H |
| CF₃ | CO₂Et | CH₂Ph-4-Cl | H | H |
| CF₃ | CO₂Et | COPh | H | H |
| CF₃ | CO₂Et | COPh-2-Cl | H | H |
| CF₃ | CO₂Et | COPh-3-Cl | H | H |
| CF₃ | CO₂Et | COPh-4-Cl | H | H |
| CF₃ | CO₂Et | OH | H | H |
| CF₃ | CO₂Et | OMe | H | H |
| CF₃ | CO₂Et | OMe | Me | H |
| CF₃ | CO₂Et | OEt | H | H |
| CF₃ | CO₂Et | OEt | Me | H |
| CF₃ | CO₂Et | SMe | H | H |
| CF₃ | CO₂Et | SMe | Me | H |
| CF₃ | CO₂Et | SPh | H | H |
| CF₃ | CO₂Et | CN | H | H |
| CF₃ | CO₂Et | NH₂ | H | H |
| CF₃ | CO₂Et | NHMe | H | H |
| CF₃ | CO₂Et | NHMe | Me | H |
| CF₃ | CO₂Et | NMe₂ | H | H |
| CF₃ | CO₂Et | NMe₂ | Me | H |
| CF₃ | CO₂Et | -CH₂-O-CH₂- | | H |
| CF₃ | CO₂Et | -(CH₂)₂- | | H |
| CF₃ | CO₂Et | -CH₂CCl₂- | | H |
| CF₃ | CO₂Et | -CH₂CF₂- | | H |
| CF₃ | CO₂Et | -(CH₂)₃- | | H |
| CF₃ | CO₂Et | -(CH₂)₄- | | H |
| CF₃ | CO₂Et | -(CH₂)₅- | | H |
| CF₃ | CO₂Et | H | H | 3-F |
| CF₃ | CO₂Et | H | H | 4-F |
| CF₃ | CO₂Et | H | H | 5-F |
| CF₃ | CO₂Et | H | H | 6-F |
| CF₃ | CO₂Et | H | H | 4-Cl |
| CF₃ | CO₂Et | H | H | 5-Cl |
| CF₃ | CO₂Et | H | H | 4-Br |
| CF₃ | CO₂Et | H | H | 4-I |
| CF₃ | CO₂Et | H | H | 4-Me |
| CF₃ | CO₂Et | H | H | 5-Me |
| CF₃ | CO₂Et | H | H | 4-OMe |
| CF₃ | CO₂Et | H | H | 4-SMe |
| CF₃ | CO₂Et | Me | H | 3-F |
| CF₃ | CO₂Et | Me | H | 4-F |
| CF₃ | CO₂Et | Me | H | 5-F |
| CF₃ | CO₂Et | Me | H | 6-F |
| CF₃ | CO₂Et | Me | H | 4-Cl |
| CF₃ | CO₂Et | Me | H | 5-Cl |
| CF₃ | CO₂Et | Me | H | 4-Br |
| CF₃ | CO₂Et | Me | H | 4- I |
| CF₃ | CO₂Et | Me | H | 4-Me |
| CF₃ | CO₂Et | Me | H | 5-Me |
| CF₃ | CO₂Et | Me | H | 4-OMe |
| CF₃ | CO₂Et | Me | H | 4-SMe |
| CF₃ | CO₂Et | Me | Me | 3-F |
| CF₃ | CO₂Et | Me | Me | 4-F |
| CF₃ | CO₂Et | Me | Me | 5-F |
| CF₃ | CO₂Et | Me | Me | 6-F |
| CF₃ | CO₂Et | Me | Me | 4-Cl |
| CF₃ | CO₂Et | Me | Me | 5-Cl |
| CF₃ | CO₂Et | Me | Me | 4-Br |
| CF₃ | CO₂Et | Me | Me | 4-I |
| CF₃ | CO₂Et | Me | Me | 4-Me |
| CF₃ | CO₂Et | Me | Me | 5-Me |
| CF₃ | CO₂Et | Me | Me | 4-OMe |
| CF₃ | CO₂Et | Me | Me | 4-SMe |
| CF₃ | CO₂Pr-n | H | H | H |
| CF₃ | CO₂Pr-n | Me | H | H |
| CF₃ | CO₂Pr-n | Me | Me | H |
| CF₃ | CO₂Pr-n | Et | H | H |
| CF₃ | CO₂Pr-n | Et | Me | H |
| CF₃ | CO₂Pr-n | Pr-n | H | H |
| CF₃ | CO₂Pr-n | Pr-n | Me | H |
| CF₃ | CO₂Pr-n | Cl | H | H |
| CF₃ | CO₂Pr-n | CH₂CH=CH₂ | | H |
| CF₃ | CO₂Pr-n | CH₂Cl | H | H |
| CF₃ | CO₂Pr-n | CF₃ | H | H |
| CF₃ | CO₂Pr-n | CF₃ | Me | H |
| CF₃ | CO₂Pr-n | Ph | H | H |
| CF₃ | CO₂Pr-n | Ph | Me | H |
| CF₃ | CO₂Pr-n | Ph-2-Cl | H | H |
| CF₃ | CO₂Pr-n | Ph-3-Cl | H | H |
| CF₃ | CO₂Pr-n | Ph-4-Cl | H | H |
| CF₃ | CO₂Pr-n | COMe | H | H |
| CF₃ | CO₂Pr-n | CO₂H | H | H |
| CF₃ | CO₂Pr-n | CO₂Me | H | H |
| CF₃ | CO₂Pr-n | CONH₂ | H | H |
| CF₃ | CO₂ P r - n | CONHMe | H | H |
| CF₃ | CO₂ P r - n | CONMe₂ | H | H |
| CF₃ | CO₂ Pr - n | OH | H | H |
| CF₃ | CO₂ Pr - n | OMe | H | H |
| CF₃ | CO₂ Pr - n | OMe | Me | H |
| CF₃ | CO₂ Pr - n | SMe | H | H |
| CF₃ | CO₂ Pr - n | SMe | Me | H |
| CF₃ | CO₂ Pr - n | NHMe | H | H |
| CF₃ | CO₂ Pr - n | NHMe | Me | H |
| CF₃ | CO₂ Pr - n | CH₂ Ph | H | H |
| CF₃ | CO₂ Pr - n | CH₂ Ph-2-Cl | H | H |
| CF₃ | CO₂ Pr - n | CH₂ Ph-3-Cl | H | H |
| CF₃ | CO₂ Pr - n | CH₂ Ph-4-Cl | H | H |
| CF₃ | CO₂ Pr - n | COPh | H | H |
| CF₃ | CO₂ Pr - n | COPh-2-Cl | H | H |
| CF₃ | CO₂ Pr - n | COPh-3-Cl | H | H |
| CF₃ | CO₂ Pr - n | COPh-4-Cl | H | H |
| CF₃ | CO₂ Pr - n | -CH₂ -O-CH₂ - | | H |
| CF₃ | CO₂ Pr - n | - (CH₂)₂ - | | H |
| CF₃ | CO₂ Pr - n | - (CH₂)₃ - | | H |
| CF₃ | CO₂ Pr - n | - (CH₂)₄ - | | H |
| CF₃ | CO₂ Pr - n | - (CH₂)₅ - | | H |
| CF₃ | CO₂ Pr - i | H | H | H |
| CF₃ | CO₂ Pr - i | Me | H | H |
| CF₃ | CO₂ Pr - i | Me | Me | H |
| CF₃ | CO₂ Pr - i | Et | H | H |
| CF₃ | CO₂ Pr - i | Et | Me | H |
| CF₃ | CO₂ Pr - i | Pr - n | H | H |
| CF₃ | CO₂ Pr - i | Pr - n | Me | H |
| CF₃ | CO₂ Pr - i | Cl | H | H |
| CF₃ | CO₂ Pr - i | CH₂CH=CH₂ | H | H |
| CF₃ | CO₂ Pr - i | CH₂ Cl | H | H |
| CF₃ | CO₂ Pr - i | CF₃ | H | H |
| CF₃ | CO₂ Pr - i | CF₃ | Me | H |
| CF₃ | CO₂ Pr - i | Ph | H | H |
| CF₃ | CO₂ Pr - i | Ph | Me | H |
| CF₃ | CO₂ Pr - i | Ph-2-Cl | H | H |
| CF₃ | CO₂ Pr - i | Ph-3-Cl | H | H |
| CF₃ | CO₂ Pr - i | Ph-4-Cl | H | H |
| CF₃ | CO₂ Pr - i | COMe | H | H |
| CF₃ | CO₂ Pr - i | CO₂H | H | H |
| CF₃ | CO₂ Pr - i | CO₂Me | H | H |
| CF₃ | CO₂ Pr - i | CONH₂ | H | H |
| CF₃ | CO₂ Pr - i | CONHMe | H | H |
| CF₃ | CO₂ Pr - i | CONMe₂ | H | H |
| CF₃ | CO₂ Pr - i | OH | H | H |
| CF₃ | CO₂ Pr - i | OMe | H | H |
| CF₃ | CO₂ Pr - i | OMe | Me | H |
| CF₃ | CO₂ Pr - i | SMe | H | H |
| C F ₃ | CO₂ Pr - i | SMe | Me | H |
| CF₃ | CO₂ Pr - i | NHMe | H | H |
| CF₃ | CO₂ Pr- i | NHMe | Me | H |
| CF₃ | CO₂ Pr - i | CH₂ Ph | H | H |
| CF₃ | CO₂ Pr - i | CH₂ Ph-2-Cl | H | H |
| CF₃ | CO₂ Pr - i | CH₂ Ph-3-Cl | H | H |
| CF₃ | CO₂ Pr - i | CH₂ Ph-4-Cl | H | H |
| CF₃ | CO₂ Pr - i | COPh | H | H |
| CF₃ | CO₂ Pr - i | COPh-2-Cl | H | H |
| CF₃ | CO₂ Pr - i | COPh-3-Cl | H | H |
| CF₃ | CO₂ Pr - i | COPh-4-Cl | H | H |
| CF₃ | CO₂ Pr - i | -CH₂ -O-CH₂ - | | H |
| CF₃ | CO₂ Pr - i | - (CH₂)₂ - | | H |
| CF₃ | CO₂ Pr - i | - (CH₂)₃ - | | H |
| CF₃ | CO₂ Pr - i | - (CH₂)₄ - | | H |
| CF₃ | CO₂ Pr - i | - (CH₂)₅ - | | H |
| CF₃ | CO₂ Bu-n | H | H | H |
| CF₃ | CO₂ Bu - n | Me | H | H |
| CF₃ | CO₂ Bu-n | Me | Me | H |
| CF₃ | CO₂ Bu-n | - (CH₂)₂ - | | H |
| CF₃ | CO₂ Bu - n | - (CH₂)₃ - | | H |
| CF₃ | CO₂ Bu- i | H | H | H |
| CF₃ | CO₂ Bu- i | Me | H | H |
| CF₃ | CO₂ Bu- i | Me | Me | H |
| CF₃ | CO₂ Bu- i | Et | H | H |
| CF₃ | CO₂ Bu- i | Et | Me | H |
| CF₃ | CO₂ Bu- i | Pr - n | H | H |
| CF₃ | CO₂ Bu- i | Pr - n | Me | H |
| CF₃ | CO₂ Bu- i | Cl | H | H |
| CF₃ | CO₂ Bu- i | CH₂CH=CH₂ | H | H |
| CF₃ | CO₂ Bu- i | CH₂ Cl | H | H |
| CF₃ | CO₂ Bu- i | CF₃ | H | H |
| CF₃ | CO₂ Bu- i | CF₃ | Me | H |
| CF₃ | CO₂ Bu - i | Ph | H | H |
| CF₃ | CO₂ Bu- i | Ph | Me | H |
| CF₃ | CO₂ Bu - i | Ph-2-Cl | H | H |
| CF₃ | CO₂ Bu - i | Ph-3-Cl | H | H |
| CF₃ | CO₂ Bu- i | Ph-4-Cl | H | H |
| CF₃ | CO₂ Bu- i | COMe | H | H |
| CF₃ | CO₂ Bu- i | CO₂H | H | H |
| CF₃ | CO₂ Bu- i | CO₂ Me | H | H |
| CF₃ | CO₂ Bu- i | CONH₂ | H | H |
| CF₃ | CO₂ Bu- i | CONHMe | H | H |
| CF₃ | CO₂ Bu - i | CONMe₂ | H | H |
| CF₃ | CO₂ Bu - i | OH | H | H |
| CF₃ | CO₂ Bu - i | OMe | H | H |
| CF₃ | CO₂ Bu - i | OMe | Me | H |
| CF₃ | CO₂ Bu - i | SMe | H | H |
| CF₃ | CO₂ Bu - i | SMe | Me | H |
| CF₃ | CO₂ Bu - i | NHMe | H | H |
| CF₃ | CO₂ Bu - i | NHMe | Me | H |
| CF₃ | CO₂ Bu - i | CH₂ Ph | H | H |
| CF₃ | CO₂ Bu - i | CH₂ Ph-2-Cl | H | H |
| CF₃ | CO₂ Bu - i | CH₂ Ph-3-Cl | H | H |
| CF₃ | CO₂ Bu - i | CH₂ Ph-4-Cl | H | H |
| CF₃ | CO₂ Bu - i | COPh | H | H |
| CF₃ | CO₂ Bu - i | COPh-2-Cl | H | H |
| CF₃ | CO₂ Bu - i | COPh-3-Cl | H | H |
| CF₃ | CO₂ Bu - i | COPh-4-Cl | H | H |
| CF₃ | CO₂ Bu - i | -CH₂-O-CH₂- | | H |
| CF₃ | CO₂ Bu - i | - (CH₂)₂ - | | H |
| CF₃ | CO₂ Bu - i | - (CH₂)₃ - | | H |
| CF₃ | CO₂ Bu - i | - (CH₂)₄ - | | H |
| CF₃ | CO₂ Bu- i | - (CH₂)₅ - | | H |
| CF₃ | CO₂ Bu - t | H | H | H |
| CF₃ | CO₂ Bu - t | Me | H | H |
| CF₃ | CO₂ Bu- t | Me | Me | H |
| CF₃ | CO₂ Bu - t | - (CH₂)₂ - | | H |
| CF₃ | CO₂ Bu - t | - (CH₂)₃ - | | H |
| CF₃ | CO₂ Pen-n | H | H | H |
| CF₃ | CO₂ Pen-n | Me | H | H |
| CF₃ | CO₂ Pen-n | Me | Me | H |
| CF₃ | CO₂ Pen-n | - (CH₂)₂ - | | H |
| CF₃ | CO₂ Pen-n | - (CH₂)₃ - | | H |
| CF₃ | E - 1 | H | H | H |
| CF₃ | E - 1 | Me | H | H |
| CF₃ | E - 1 | Me | Me | H |
| CF₃ | E - 1 | - (CH₂)₂ - | | H |
| CF₃ | E - 1 | - (CH₂)₃ - | | H |
| CF₃ | CO₂ Hex-n | H | H | H |
| CF₃ | CO₂ Hex-n | Me | H | H |
| CF₃ | CO₂ Hex-n | Me | Me | H |
| CF₃ | CO₂ Hex-n | - (CH₂)₂ - | | H |
| CF₃ | CO₂ Hex-n | - (CH₂)₃ - | | H |
| CF₃ | CO₂ Ph | H | H | H |
| CF₃ | CO₂ Ph | Me | H | H |
| CF₃ | CO₂ Ph | Me | Me | H |
| CF₃ | CO₂ Ph | - (CH₂)₂ - | | H |
| CF₃ | CO₂ Ph | - (CH₂)₃ - | | H |
| CF₃ | CO₂ CH₂ Ph | H | H | H |
| CF₃ | CO₂ CH₂ Ph | Me | H | H |
| CF₃ | CO₂ CH₂ Ph | Me | Me | H |
| CF₃ | CO₂ CH₂ Ph | - (CH₂)₂ - | | H |
| CF₃ | CO₂ CH₂ Ph | - (CH₂)₃ - | | H |
| CF₃ | E-2 | H | H | H |
| CF₃ | E-2 | Me | H | H |
| CF₃ | E-2 | Me | Me | H |
| CF₃ | E-2 | - (CH₂)₂ - | | H |
| CF₃ | E-2 | - (CH₂)₃ - | | H |
| CF₃ | E-3 | H | H | H |
| CF₃ | E-3 | Me | H | H |
| CF₃ | E-3 | Me | Me | H |
| CF₃ | E-3 | Et | H | H |
| CF₃ | E-3 | Et | Me | H |
| CF₃ | E-3 | Pr - n | H | H |
| CF₃ | E-3 | Pr - n | Me | H |
| CF₃ | E-3 | Cl | H | H |
| CF₃ | E-3 | CH₂ CH=CH₂ | H | H |
| CF₃ | E-3 | CH₂ Cl | H | H |
| CF₃ | E-3 | CF₃ | H | H |
| CF₃ | E-3 | CF₃ | Me | H |
| CF₃ | E-3 | Ph | H | H |
| CF₃ | E-3 | Ph | Me | H |
| CF₃ | E-3 | Ph-2-Cl | H | H |
| CF₃ | E-3 | Ph-3-Cl | H | H |
| CF₃ | E-3 | Ph-4-Cl | H | H |
| CF₃ | E-3 | COMe | H | H |
| CF₃ | E-3 | CO₂H | H | H |
| CF₃ | E-3 | CO₂ Me | H | H |
| CF₃ | E-3 | CONH₂ | H | H |
| CF₃ | E-3 | CONHMe | H | H |
| CF₃ | E-3 | CONMe₂ | H | H |
| CF₃ | E-3 | OH | H | H |
| CF₃ | E-3 | OMe | H | H |
| CF₃ | E-3 | OMe | Me | H |
| CF₃ | E-3 | SMe | H | H |
| CF₃ | E-3 | SMe | Me | H |
| CF₃ | E-3 | NHMe | H | H |
| CF₃ | E-3 | NHMe | Me | H |
| CF₃ | E-3 | CH₂ Ph | H | H |
| CF₃ | E-3 | CH₂ Ph-2-Cl | H | H |
| CF₃ | E-3 | CH₂ Ph-3-Cl | H | H |
| CF₃ | E-3 | CH₂ Ph-4-Cl | H | H |
| CF₃ | E-3 | COPh | H | H |
| CF₃ | E-3 | COPh-2-Cl | H | H |
| CF₃ | E-3 | COPh-3-Cl | H | H |
| CF₃ | E-3 | COPh-4-Cl | H | H |
| CF₃ | E-3 | -CH₂-O-CH₂- | | H |
| CF₃ | E-3 | - (CH₂)₂ - | | H |
| CF₃ | E-3 | - (CH₂)₃ - | | H |
| CF₃ | E-3 | -(CH₂)₄- | | H |
| CF₃ | E-3 | - (CH₂)₅ - | | H |
| CF₃ | CH₂ OMe | H | H | H |
| CF₃ | CH₂ OMe | Me | H | H |
| CF₃ | CH₂ OMe | Me | Me | H |
| CF₃ | CH₂ OMe | - (CH₂)₂ - | | H |
| CF₃ | CH₂ OMe | - (CH₂)₃ - | | H |
| CF₃ | COCH₂ Cl | H | H | H |
| CF₃ | COCH₂ Cl | Me | H | H |
| CF₃ | COCH₂ Cl | Me | Me | H |
| CF₃ | COCH₂ Cl | - (CH₂)₂ - | | H |
| CF₃ | COCH₂ Cl | - (CH₂)₃ - | | H |
| CF₃ | E-4 | H | H | H |
| CF₃ | E-4 | Me | H | H |
| CF₃ | E-4 | Me | Me | H |
| CF₃ | E-4 | - (CH₂)₂ - | | H |
| CF₃ | E-4 | - (CH₂)₃- | | H |
| CF₃ | E-5 | H | H | H |
| CF₃ | E-5 | Me | H | H |
| CF₃ | E-5 | Me | Me | H |
| CF₃ | E-5 | - (CH₂)₂- | | H |
| CF₃ | E-5 | - (CH₂)₃- | | H |
| CF₃ | E-6 | H | H | H |
| CF₃ | E-6 | Me | H | H |
| CF₃ | E-6 | Me | Me | H |
| CF₃ | E-6 | - (CH₂)₂- | | H |
| CF₃ | E-6 | - (CH₂)₃ - | | H |
| CF₃ | E-7 | H | H | H |
| CF₃ | E-7 | Me | H | H |
| CF₃ | E-7 | Me | Me | H |
| CF₃ | E-7 | - (CH₂)₂- | | H |
| CF₃ | E-7 | - (CH₂)₃ - | | H |
| CF₃ | E-8 | H | H | H |
| CF₃ | E-8 | Me | H | H |
| CF₃ | E-8 | Me | Me | H |
| CF₃ | E-8 | - (CH₂)₂ - | | H |
| CF₃ | E-8 | -(CH₂)₃- | | H |
| CF₃ | E-9 | H | H | H |
| CF₃ | E-9 | Me | H | H |
| CF₃ | E-9 | Me | Me | H |
| CF₃ | E-9 | - (CH₂)₂ - | | H |
| CF₃ | E-9 | -(CH₂)₃- | | H |
| CF₃ | E - 1 0 | H | H | H |
| CF₃ | E - 1 0 | Me | H | H |
| CF₃ | E - 1 0 | Me | Me | H |
| CF₃ | E - 1 0 | - (CH₂) ₂ - | | H |
| CF₃ | E-10 | - (CH₂)₃ - | | H |
| CF₃ | E-11 | H | H | H |
| CF₃ | E- 11 | Me | H | H |
| CF₃ | E-11 | Me | Me | H |
| CF₃ | E-11 | -(CH₂)₂- | | H |
| CF₃ | E-11 | - (CH₂)₃ | | H |
| CF₃ | E-12 | H | H | H |
| CF₃ | E-12 | Me | H | H |
| CF₃ | E-12 | Me | Me | H |
| CF₃ | E-12 | - (CH₂)₂- | | H |
| CF₃ | E-12 | - (CH₂)₃- | | H |
| CF₃ | CH₂ SMe | H | H | H |
| CF₃ | CH₂ SMe | Me | H | H |
| CF₃ | CH₂ SMe | Me | Me | H |
| CF₃ | CH₂ SMe | - (CH₂)₂- | | H |
| CF₃ | CH₂ SMe | - (CH₂)₃- | | H |
| CF₃ | CH₂ OCH₂ Ph | H | H | H |
| CF₃ | CH₂ OCH₂ Ph | Me | H | H |
| CF₃ | CH₂ OCH₂ Ph | Me | Me | H |
| CF₃ | CH₂ OCH₂ Ph | - (CH₂)₂ - | | H |
| CF₃ | CH₂ OCH₂ Ph | - (CH₂)₃- | | H |
| CF₃ | CH₂ OCOMe | H | H | H |
| CF₃ | CH₂ OCOMe | Me | H | H |
| CF₃ | CH₂ OCOMe | Me | Me | H |
| CF₃ | CH₂ OCOMe | - (CH₂)₂ - | | H |
| CF₃ | CH₂ OCOMe | - (CH₂)₃ - | | H |
| CF₃ | CH₂ OCOEt | H | H | H |
| CF₃ | CH₂ OCOEt | Me | H | H |
| CF₃ | CH_{z} OCOEt | Me | Me | H |
| CF₃ | CH₂ OCOEt | -(CH₂)₂ - | | H |
| CF₃ | CH₂ OCOEt | -(CH₂)₃- | | H |
| CF₃ | CO SMe | H | H | H |
| CF₃ | CO SMe | Me | H | H |
| CF₃ | CO SMe | Me | Me | H |
| CF₃ | CO SMe | - (CH₂)₂ - | | H |
| CF₃ | CO SMe | - (CH₂)₃ - | | H |
| CF₃ | CO SEt | H | H | H |
| CF₃ | CO SEt | Me | H | H |
| CF₃ | CO SEt | Me | Me | H |
| CF₃ | CO SEt | - (CH₂)₂ - | | H |
| CF₃ | CO SEt | - (CH₂)₃ - | | H |
| CF₃ | CH₂ SO₂ Me | H | H | H |
| CF₃ | CH₂ SO₂ Me | Me | H | H |
| CF₃ | CH₂ SO₂ Me | Me | Me | H |
| CF₃ | CH₂ SO₂ Me | - (CH₂)₂ - | | H |
| CF₃ | CH₂ SO₂ Me | - (CH₂)₃ - | | H |
| CF₃ | CH₂ SOMe | H | H | H |
| CF₃ | CH₂ SOMe | Me | H | H |
| CF₃ | CHO SOMe | Me | Me | H |
| CF₃ | CH₂ SOMe | - (CH₂)₂ | | H |
| CF₃ | CH₂ SOMe | - (CH₂)₃- | | H |
| CF₃ | CH₂ Ph | H | H | H |
| CF₃ | CH₂ Ph | Me | H | H |
| CF₃ | CH₂ Ph | Me | Me | H |
| CF₃ | CH₂ Ph | - (CH₂)₂ - | | H |
| CF₃ | CH₂ Ph | - (CH₂)₃ - | | H |
| CF₃ | SO₂ CF3 | H | H | H |
| CF₃ | SO₂ CF3 | Me | H | H |
| CF₃ | SO₂ CF₃ | Me | Me | H |
| CF₃ | SO₂ CF₃ | - (CH₂)₂ - | | H |
| CF₃ | SO₂ CF₃ | - (CH₂)₃ - | | H |
| CHF₂ | H | H | H | H |
| CHF₂ | H | Me | H | H |
| CHF₂ | H | Me | Me | H |
| CHF₂ | H | Et | H | H |
| CHF₂ | H | Et | Me | H |
| CHF₂ | H | Pr - n | H | H |
| CHF₂ | H | Cl | H | H |
| CHF₂ | H | CF₃ | H | H |
| CHF₂ | H | CF₃ | Me | H |
| CHF₂ | H | Ph | H | H |
| CHF₂ | H | Ph | Me | H |
| CHF₂ | H | Ph-2-Cl | H | H |
| CHF₂ | H | Ph-3-Cl | H | H |
| CHF₂ | H | Ph-4-Cl | H | H |
| CHF₂ | H | COMe | H | H |
| CHF₂ | H | CO₂ Me | H | H |
| CHF₂ | H | OMe | H | H |
| CHF₂ | H | OMe | Me | H |
| CHF₂ | H | SMe | H | H |
| CHF₂ | H | NHMe | H | H |
| CHF₂ | H | CH₂ Ph | H | H |
| CHF₂ | H | CH₂ Ph-2-Cl | H | H |
| CHF₂ | H | CH₂ Ph-3-Cl | H | H |
| CHF₂ | H | CH₂ Ph-4-Cl | H | H |
| CHF₂ | H | COPh | H | H |
| CHF₂ | H | COPh-2-Cl | H | H |
| CHF₂ | H | COPh-3-Cl | H | H |
| CHF₂ | H | COPh-4-Cl | H | H |
| CHF₂ | H | -CH₂-O-CH₂ - | | H |
| CHF₂ | H | - (CH₂)₂ - | | H |
| CHF₂ | H | - (CH₂)₃ - | | H |
| CHF₂ | H | - (CH₂)₄ - | | H |
| CHF₂ | H | - (CH₂)₅ - | | H |
| CHF₂ | COEt | H | H | H |
| CHF₂ | COEt | Me | H | H |
| CHF₂ | COEt | Me | Me | H |
| CHF₂ | COEt | Et | H | H |
| CHF₂ | COEt | Et | Me | H |
| CHF₂ | COEt | Pr - n | H | H |
| CHF₂ | COEt | Cl | H | H |
| CHF₂ | COEt | CF₃ | H | H |
| CHF₂ | COEt | CF₃ | Me | H |
| CHF₂ | COEt | Ph | H | H |
| CHF₂ | COEt | P h | Me | H |
| CHF₂ | COEt | P h - 2 - C l | H | H |
| CHF₂ | COEt | P h - 3 - Cl | H | H |
| CHF₂ | COEt | P h - 4 - Cl | H | H |
| CHF₂ | COEt | COMe | H | H |
| CHF₂ | COEt | CO₂Me | H | H |
| CHF₂ | COEt | OMe | H | H |
| CHF₂ | COEt | OMe | Me | H |
| CHF₂ | COEt | SMe | H | H |
| CHF₂ | COEt | NHMe | H | H |
| CHF₂ | COEt | CH₂ Ph | H | H |
| CHF₂ | COEt | CH₂ Ph-2-Cl | H | H |
| CHF₂ | COEt | CH₂ Ph-3-Cl | H | H |
| CHF₂ | COEt | CH₂ Ph-4-Cl | H | H |
| CHF₂ | COEt | COPh | H | H |
| CHF₂ | COEt | COPh-2-Cl | H | H |
| CHF₂ | COEt | COPh-3-Cl | H | H |
| CHF₂ | COEt | COPh-4-Cl | H | H |
| CHF₂ | COEt t | -CH₂ -O-CH₂ | | H |
| CHF₂ | COEt | - (CH₂)₂ - | | H |
| CHF₂ | COEt | - (CH₂)₃ - | | H |
| CHF₂ | COEt | - (CH₂)₄ - | | H |
| CHF₂ | COEt | - (CH₂)₅ - | | H |
| CHF₂ | CO₂Et | H | H | H |
| CHF₂ | CO₂Et | Me | H | H |
| CHF₂ | CO₂Et | Me | Me | H |
| CHF₂ | CO₂Et | Et | H | H |
| CHF₂ | CO₂Et | Et | Me | H |
| CHF₂ | CO₂Et | Pr - n | H | H |
| CHF₂ | CO₂Et | Cl | H | H |
| CHF₂ | CO₂Et | CF₃ | H | H |
| CHF₂ | CO₂Et | CF₃ | Me | H |
| CHF₂ | CO₂Et | Ph | H | H |
| CHF₂ | CO₂Et | Ph | Me | H |
| CHF₂ | CO₂Et | Ph-2-Cl | H | H |
| CHF₂ | CO₂Et | Ph - 3 - Cl | H | H |
| CHF₂ | CO₂Et | P h - 4 - Cl | H | H |
| CHF₂ | CO₂Et | COMe | H | H |
| CHF₂ | CO₂Et | CO₂Me | H | H |
| CHF₂ | CO₂Et | OMe | H | H |
| CHF₂ | CO₂Et | OMe | Me | H |
| CHF₂ | CO₂Et | SMe | H | H |
| CHF₂ | CO₂Et | NHMe | H | H |
| CHF₂ | CO₂Et | CH₂ Ph | H | H |
| CHF₂ | CO₂Et | CH₂ Ph-2-Cl | H | H |
| CHF₂ | CO₂Et | CH₂ Ph-3-Cl | H | H |
| CHF₂ | CO₂Et | CH₂ Ph-4-Cl | H | H |
| CHF₂ | CO₂Et | COPh | H | H |
| CHF₂ | CO₂Et | COPh-2-Cl | H | H |
| CHF₂ | CO₂Et | COPh-3-Cl | H | H |
| CHF₂ | CO₂Et | COPh-4-Cl | H | H |
| CHF₂ | CO₂Et | -CH₂-O-CH₂- | | H |
| CHF₂ | CO₂Et | - (CH₂)₂ - | | H |
| CHF₂ | CO₂Et | - (CH₂)₃ - | | H |
| CHF₂ | CO₂Et | - (CH₂)₄ - | | H |
| CHF₂ | CO₂Et | - (CH₂)₅ - | | H |
| CHF₂ | CO₂ Bu - i | H | H | H |
| CHF₂ | CO₂ Bu - i | Me | H | H |
| CHF₂ | CO₂ Bu - i | Me | Me | H |
| CHF₂ | CO₂ Bu - i | Et | H | H |
| CHF₂ | CO₂ Bu - i | Et | Me | H |
| CHF₂ | CO₂ Bu - i | Pr - n | H | H |
| CHF₂ | CO₂ Bu - i | Cl | H | H |
| CHF₂ | CO₂ Bu - i | CF₃ | H | H |
| CHF₂ | CO₂ Bu - i | CF₃ | Me | H |
| CHF₂ | CO₂ Bu - i | Ph | H | H |
| CHF₂ | CO₂ Bu - i | Ph | Me | H |
| CHF₂ | CO₂ Bu - i | Ph-2-Cl | H | H |
| CHF₂ | CO₂ Bu - i | Ph-3-Cl | H | H |
| CHF₂ | CO₂ Bu - i | Ph-4-Cl | H | H |
| CHF₂ | CO₂ Bu - i | COMe | H | H |
| CHF₂ | CO₂ Bu - i | CO₂Me | H | H |
| CHF₂ | CO₂ Bu - i | OMe | H | H |
| CHF₂ | CO₂ Bu - i | OMe | Me | H |
| CHF₂ | CO₂ Bu - i | SMe | H | H |
| CHF₂ | CO₂ Bu - i | NHMe | H | H |
| CHF₂ | CO₂ Bu - i | CH₂ Ph | H | H |
| CHF₂ | CO₂ Bu - i | CH₂ Ph - 2 - Cl | H | H |
| CHF₂ | CO₂ Bu - i | CH₂ Ph - 3 - Cl | H | H |
| CHF₂ | CO₂ Bu - i | CH₂ Ph - 4 - Cl | H | H |
| CHF₂ | CO₂ Bu - i | COPh | H | H |
| CHF₂ | CO₂ Bu - i | COPh - 2 - Cl | H | H |
| CHF₂ | CO₂ Bu - i | COPh - 3 - Cl | H | H |
| CHF₂ | CO₂ Bu- i | COPh - 4 - Cl | H | H |
| CHF₂ | CO₂ Bu - i | -CH₂-O-CH₂ - | | H |
| CHF₂ | CO₂ Bu - i | - (CH₂)₂ - | | H |
| CHF₂ | CO₂ Bu - i | - (CH₂)₃ - | | H |
| CHF₂ | CO₂ Bu - i | - (CH₂)₄ - | | H |
| CHF₂ | CO₂ Bu - i | - (CH₂)₅ - | | H |
| CClF₂ | H | H | H | H |
| CClF₂ | H | Me | H | H |
| CClF₂ | H | Me | Me | H |
| CClF₂ | H | Et | H | H |
| CClF₂ | H | Et | Me | H |
| CClF₂ | H | Pr - n | H | H |
| CClF₂ | H | Cl | H | H |
| CClF₂ | H | CF₃ | H | H |
| CClF₂ | H | CF₃ | Me | H |
| CClF₂ | H | Ph | H | H |
| CClF₂ | H | Ph | Me | H |
| CClF₂ | H | Ph - 2 - Cl | H | H |
| CClF₂ | H | Ph - 3 - Cl | H | H |
| CClF₂ | H | Ph - 4 - Cl | H | H |
| CClF₂ | H | COMe | H | H |
| CClF2 | H | CO₂ Me | H | H |
| CClF₂ | H | OMe | H | H |
| CClF₂ | H | OMe | Me | H |
| CClF₂ | H | SMe | H | H |
| CClF₂ | H | NHMe | H | H |
| CClF₂ | H | CH₂ Ph | H | H |
| CClF₂ | H | CH₂ Ph - 2 - Cl | H | H |
| CClF₂ | H | CH₂ Ph - 3 - Cl | H | H |
| CClF₂ | H | CH₂ Ph - 4 - Cl | H | H |
| CClF₂ | H | COPh | H | H |
| CClF₂ | H | COPh - 2 - Cl | H | H |
| CClF₂ | H | COPh - 3 - Cl | H | H |
| CClF₂ | H | COPh -4 - Cl | H | H |
| CClF₂ | H | -CH₂-O-CH₂ - | | H |
| CClF₂ | H | - (CH₂)₂ - | | H |
| CClF₂ | H | - (CH₂)₃ - | | H |
| CClF₂ | H | - (CH₂)₄ - | | H |
| CClF₂ | H | - (CH₂)₅ - | | H |
| CClF₂ | COEt | H | H | H |
| CClF₂ | COEt | Me | H | H |
| CClF₂ | COEt | Me | Me | H |
| CClF₂ | COEt | Et | H | H |
| CClF₂ | COEt | Et | Me | H |
| CClF₂ | COEt | Pr - n | H | H |
| CClF₂ | COEt | Cl | H | H |
| CClF₂ | COEt | CF₃ | H | H |
| CClF₂ | COEt | CF₃ | Me | H |
| CClF₂ | COE t | Ph | H | H |
| CClF₂ | COEt | P h | Me | H |
| CClF₂ | COEt | Ph-2-Cl | H | H |
| CClF₂ | COEt | Ph-3-Cl | H | H |
| CClF₂ | COEt | Ph-4-Cl | H | H |
| CClF₂ | COEt | COMe | H | H |
| CClF₂ | COEt | CO₂ Me | H | H |
| CClF₂ | COEt | OMe | H | H |
| CClF₂ | COEt | OMe | Me | H |
| CClF₂ | COEt | SMe | H | H |
| CClF₂ | COEt | NHMe | H | H |
| CClF₂ | COEt | CH₂ Ph | H | H |
| CClF₂ | COEt | CH₂ Ph-2-Cl | H | H |
| CClF₂ | COEt | CH₂ Ph-3-Cl | H | H |
| CClF₂ | COEt | CH₂ Ph-4-Cl | H | H |
| CClF₂ | COEt | COPh | H | H |
| CClF₂ | COEt | COPh-2-Cl | H | H |
| CClF₂ | COEt | COPh-3-Cl | H | H |
| CClF₂ | COEt | COPh-4-Cl | H | H |
| CClF₂ | COEt | -CH₂-O-CH₂- | | H |
| CClF₂ | COEt | - (CH₂)₂- | | H |
| CClF₂ | COEt | - (CH₂)₃- | | H |
| CClF₂ | COEt | - (CH₂)₄- | | H |
| CClF₂ | COEt | - (CH₂)₅- | | H |
| CClF₂ | CO₂ E t | H | H | H |
| CClF₂ | CO₂ E t | Me | H | H |
| CClF₂ | CO₂ E t | Me | Me | H |
| CClF₂ | CO₂ E t | E t | H | H |
| CClF₂ | CO₂ E t | E t | Me | H |
| CClF₂ | CO₂ E t | P r - n | H | H |
| CClF₂ | CO₂ E t | Cl | H | H |
| CClF₂ | CO₂ E t | CF₃ | H | H |
| CClF₂ | CO₂ E t | CF₃ | Me | H |
| CClF₂ | CO₂ E t | Ph | H | H |
| CClF₂ | CO₂ E t | Ph | Me | H |
| CClF₂ | CO₂ E t | Ph-2-Cl | H | H |
| CClF₂ | CO₂ E t | Ph-3-Cl | H | H |
| CClF₂ | CO₂ E t | Ph-4-Cl | H | H |
| CClF₂ | CO₂ E t | COMe | H | H |
| CClF₂ | CO₂ E t | CO₂ Me | H | H |
| CClF₂ | CO₂ E t | OMe | H | H |
| CClF₂ | CO₂ E t | OMe | Me | H |
| CClF₂ | CO₂ E t | SMe | H | H |
| CClF₂ | CO₂ E t | NHMe | H | H |
| CClF₂ | CO₂ E t | CH₂ Ph | H | H |
| CClF₂ | CO₂ E t | CH₂Ph-2-Cl | H | H |
| CClF₂ | CO₂ E t | CH₂ Ph-3-Cl | H | H |
| CClF₂ | CO₂ E t | CH₂ Ph-4-Cl | H | H |
| CClF₂ | CO₂ E t | COPh | H | H |
| CClF₂ | CO₂ E t | COPh-2-Cl | H | H |
| CClF₂ | CO₂ E t | COPh-3-Cl | H | H |
| CClF₂ | CO₂ E t | COPh-4-Cl | H | H |
| CClF₂ | CO₂ E t | -CH₂-O-CH₂- | | H |
| CClF₂ | CO₂ E t | - (CH₂)₂- | | H |
| CClF₂ | CO₂ E t | - (CH₂)₃- | | H |
| CClF₂ | CO₂ E t | - (CH₂)₄- | | H |
| CClF₂ | CO₂ E t | - (CH₂)₅- | | H |
| CClF₂ | CO₂ Bu-i | H | H | H |
| CClF₂ | CO₂ B u- i | Me | H | H |
| CClF₂ | CO₂ Bu-i | Me | Me | H |
| CClF₂ | CO₂ Bu- i | E t | H | H |
| CClF₂ | CO₂ Bu-i | E t | Me | H |
| CClF₂ | CO₂ Bu- i | P r - n | H | H |
| CClF₂ | CO₂ B u- i | Cl | H | H |
| CClF₂ | CO₂ Bu- i | CF₃ | H | H |
| CClF₂ | CO₂ Bu- i | CF₃ | Me | H |
| CClF₂ | CO₂ Bu- i | Ph | H | H |
| CClF₂ | CO₂ Bu- i | Ph | Me | H |
| CClF₂ | CO₂ Bu- i | Ph-2-Cl | H | H |
| CClF₂ | CO₂ Bu- i | Ph-3-Cl | H | H |
| CClF₂ | CO₂ Bu- i | Ph-4-Cl | H | H |
| CClF₂ | CO₂ Bu- i | COMe | H | H |
| CClF₂ | CO₂ Bu- i | CO₂ Me | H | H |
| CClF₂ | CO₂ Bu- i | OMe | H | H |
| CClF₂ | CO₂ Bu- i | OMe | Me | H |
| CClF₂ | CO₂ Bu- i | SMe | H | H |
| CClF₂ | CO₂ B u- i | NHMe | H | H |
| CClF₂ | CO₂ Bu- i | CH₂ Ph | H | H |
| CClF₂ | CO₂ Bu-i | CH₂ Ph-2-Cl | H | H |
| CClF₂ | CO₂ Bu- i | CH₂ Ph-3-Cl | H | H |
| CClF₂ | CO₂ Bu-i | CH₂ Ph-4-Cl | H | H |
| CClF₂ | CO₂ Bu-i | COPh | H | H |
| CClF₂ | CO₂ Bu-i | COPh-2-Cl | H | H |
| CClF₂ | CO₂ Bu-i | COPh-3-Cl | H | H |
| CClF₂ | CO₂ Bu-i | COPh-4-Cl | H | H |
| CClF₂ | CO₂ Bu-i | -CH₂-O-CH₂- | | H |
| CClF₂ | CO₂ Bu-i | - (CH₂)₂- | | H |
| CClF₂ | CO₂ Bu-i | - (CH₂)₃- | | H |
| CClF₂ | CO₂ Bu-i | - (CH₂)₄- | | H |
| CClF₂ | CO₂ Bu-i | - (CH₂)₅- | | H |
| CBrF₂ | H | H | H | H |
| CBrF₂ | H | Me | H | H |
| CBrF₂ | H | Me | Me | H |
| CBrF₂ | H | Cl | H | H |
| CBrF₂ | H | P h | H | H |
| CBrF₂ | H | Ph | Me | H |
| CBrF₂ | H | COMe | H | H |
| CBrF₂ | H | CO₂ Me | H | H |
| CBrF₂ | H | OMe | H | H |
| CBrF₂ | H | OMe | Me | H |
| CBrF₂ | H | CH₂ Ph | H | H |
| CBrF₂ | H | COPh | H | H |
| CBrF₂ | H | -CH₂-O-CH₂- | | H |
| CBrF₂ | H | - (CH₂)₂- | | H |
| CBrF₂ | H | -(CH₂)₃- | | H |
| CBrF₂ | COEt | H | H | H |
| CBrF₂ | COEt | Me | H | H |
| CBrF₂ | COEt | Me | Me | H |
| CBrF₂ | COEt | Cl | H | H |
| CBrF₂ | COEt | Ph | H | H |
| CBrF₂ | COEt | Ph | Me | H |
| CBrF₂ | COEt | COMe | H | H |
| CBrF₂ | COEt | COMe | H | H |
| CBrF₂ | COEt | OMe | H | H |
| CBrF₂ | COEt | OMe | Me | H |
| CBrF₂ | COEt | CH₂ Ph | H | H |
| CBrF₂ | COEt | COPh | H | H |
| CBrF₂ | COEt | -CH₁-O-CH₂- | | H |
| CBrF₂ | COEt | - (CH₂)₂- | | H |
| CBrF₂ | COEt | - (CH₂)₃- | | H |
| CBrF₂ | CO₂ E t | H | H | H |
| CBrF₂ | CO₂ E t | Me | H | H |
| CBrF₂ | CO₂ Et t | Me | Me | H |
| CBrF₂ | CO₂ E t | Cl | H | H |
| CBrF₂ | CO₂ Et | Ph | H | H |
| CBrF₂ | CO₂ Et | P h | Me | H |
| CBrF₂ | CO₂ Et | COMe | H | H |
| CBrF₂ | CO₂ Et | CO₂ Me | H | H |
| CBrF₂ | CO₂ Et | OMe | H | H |
| CBrF₂ | CO₂ Et | OMe | Me | H |
| CBrF₂ | CO₂ Et | CH₂ Ph | H | H |
| CBrF₂ | CO₂ Et | COPh | H | H |
| CBrF₂ | CO₂ Et | -CH₂-O-CH₂- | | H |
| CBrF₂ | CO₂ Et | - (CH₂)₂- | | H |
| CBrF₂ | CO₂ Et | - (CH₂)₃- | | H |
| CBrF₂ | CO₂ Bu-i | H | H | H |
| CBrF₂ | CO₂ Bu-i | Me | H | H |
| CBrF₂ | CO₂ Bu-i | Me | Me | H |
| CBrF₂ | CO₂ Bu-i | Cl | H | H |
| CBrF₂ | CO₂ Bu-i | Ph | H | H |
| CBrF₂ | CO₂ Bu-i | Ph | Me | H |
| CBrF₂ | CO₂ Bu-i | COMe | H | H |
| CBrF₂ | CO₂ Bu-i | CO₂ Me | H | H |
| CBrF₂ | CO₂ Bu-i | OMe | H | H |
| CBrF₂ | CO₂ Bu-i | OMe | Me | H |
| CBrF₂ | CO₂ Bu-i | CH₂ Ph | H | H |
| CBrF₂ | CO₂ Bu-i | COPh | H | H |
| CBrF₂ | CO₂ Bu-i | -CH₂-O-CH₂- | | H |
| CBrF₂ | CO₂ Bu-i | - (CH₂)₂- | | H |
| CBrF₂ | CO₂ Bu-i | - (CH₂)₃ - | | H |
| CCl₂F | H | H | H | H |
| CCl₂F | H | Me | H | H |
| CCl₂F | H | Me | Me | H |
| CCl₂F | H | Cl | H | H |
| CCl₂F | H | Ph | H | H |
| CCl₂F | H | Ph | Me | H |
| CCl₂F | H | COMe | H | H |
| CCl₂F | H | CO₂ Me | H | H |
| CCl₂F | H | OMe | H | H |
| CCl₂F | H | OMe | Me | H |
| CCl₂F | H | CH₂ Ph | H | H |
| CCl₂F | H | COPh | H | H |
| CCl₂F | H | -CH₂-O-CH₂- | | H |
| CCl₂F | H | - (CH₂)₂ - | | H |
| CCl₂F | H | - (CH₂)₃- | | H |
| CCl₂F | COEt | H | H | H |
| CCl₂F | COEt | Me | H | H |
| CCl₂F | COEt | Me | Me | H |
| CCl₂F | COEt | Cl | H | H |
| CCl₂F | COEt | Ph | H | H |
| CCl₂F | COEt | Ph | Me | H |
| CCl₂F | COEt | COMe | H | H |
| CCl₂F | COEt | CO₂ Me | H | H |
| CCl₂F | COEt | OMe | H | H |
| CCl₂F | COEt | OMe | Me | H |
| CCl₂F | COEt | CH₂ Ph | H | H |
| CCl₂F | COEt | COPh | H | H |
| CCl₂F | COEt | -CH₂-O-CH₂- | | H |
| CCl₂F | COEt | - (CH₂)₂- | | H |
| CCl₂F | COEt | - (CH₂)₃- | | H |
| CCl₂F | CO₂ Et | H | H | H |
| CCl₂F | CO₂ Et | Me | H | H |
| CCl₂F | CO₂ Et | Me | Me | H |
| CCl₂F | CO₂ Et | Cl | H | H |
| CCl₂F | CO₂ Et | Ph | H | H |
| CCl₂F | CO₂ Et | Ph | Me | H |
| CCl₂F | CO₂ Et | COMe | H | H |
| CCl₂F | CO₂ Et | CO₂ Me | H | H |
| CCl₂ F | CO₂ E t | OMe | H | H |
| CCl₂F | CO₂ Et | OMe | Me | H |
| CCl₂F | CO₂ E t | CH_{Z} Ph | H | H |
| CCl₂F | CO₂ Et | COPh | H | H |
| CCl₂F | CO₂ Et | -CH₂-O-CH₂- | | H |
| CCl₂F | CO₂ Et | - (CH₂)₂- | | H |
| CCl₂F | CO₂ Et | - (CH₂)₃- | | H |
| CCl₂F | CO₂ Bu- i | H | H | H |
| CCl₂F | CO₂ B u- i | Me | H | H |
| CCl₂F | CO₂ Bu- i | Me | Me | H |
| CCl₂F | CO₂ Bu-i | Cl | H | H |
| CCl₂F | CO₂ Bu- i | Ph | H | H |
| CCl₂F | CO₂ Bu- i | Ph | Me | H |
| CCl₂F | CO₂ Bu-i | COMe | H | H |
| CCl₂F | CO₂ Bu- i | CO₂Me | H | H |
| CCl₂F | CO₂ Bu-i | OMe | H | H |
| CCl₂F | CO₂ B u- i | OMe | Me | H |
| CCl₂F | CO₂ B u- i | CH₂ Ph | H | H |
| CCl₂F | CO₂ Bu-i | COPh | H | H |
| CCl₂F | CO₂ B u- i | -CH₂-O-CH₂- | | H |
| CCl₂F | CO₂ Bu-i | - (CH₂)₂- | | H |
| CCl₂F | CO₂ B u- i | - (CH₂)₃- | | H |
| CBr₂F | H | H | H | H |
| CBr₂F | H | Me | H | H |
| CBr₂F | H | Me | Me | H |
| CBr₂F | H | - (CH₂)₂- | | H |
| CBr₂F | H | - (CH₂)₃- | | H |
| CBr₂F | CO₂ E t | H | H | H |
| CBr₂F | CO₂ E t | Me | H | H |
| CBr₂F | CO₂ E t | Me | Me | H |
| CBr₂F | CO₂ Et | - (CH₂)₂- | | H |
| CBr₂F | CO₂ Et | - (CH₂)₃- | | H |
| CCl₃ | H | H | H | H |
| CCl₃ | H | Me | H | H |
| CCl₃ | H | Me | Me | H |
| CCl₃ | H | - (CH₂)₂- | | H |
| CCl₃ | H | - (CH₂)₃- | | H |
| CCl₃ | CO₂ E t | H | H | H |
| CCl₃ | CO₂ E t | Me | H | H |
| CCl₃ | CO₂ Et | Me | Me | H |
| CCl₃ | CO₂ Et | - (CH₂)₂- | | H |
| CCl₃ | CO₂ Et | - (CH₂)₃- | | H |

In Table 2, E-1 to E-12 represent the following structures, respectively.

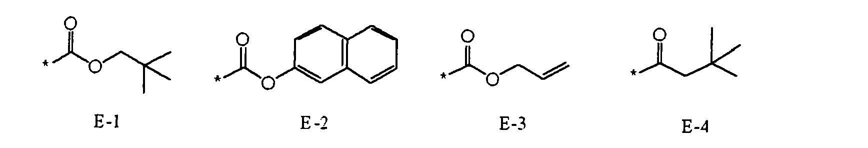

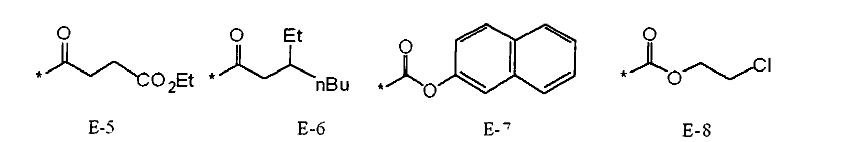

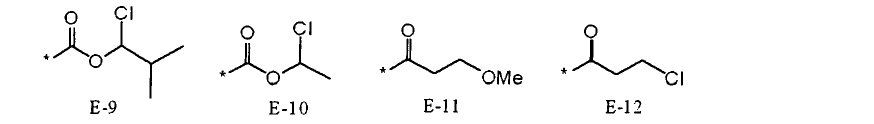

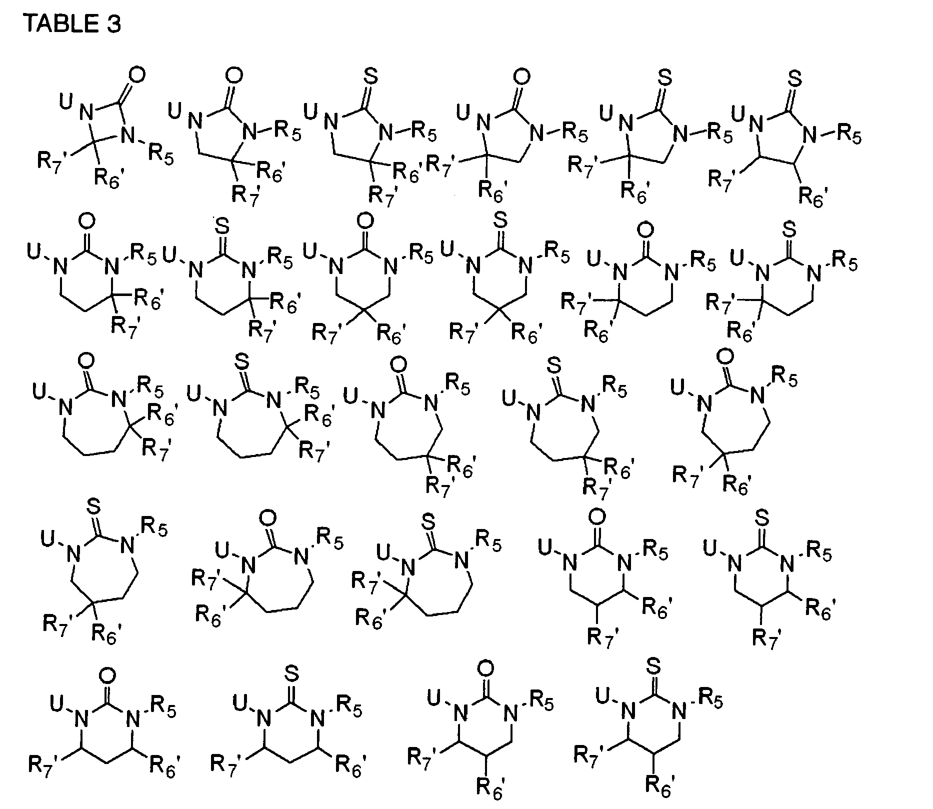

U is the same as the above-mentioned formula (26).

| R₃ =R₄ =H | | | | | |
|---|---|---|---|---|---|
| R₁ | R₂ | R₅ | R₆ ' | R₇' | X |
| CF₃ | H | H | H | H | H |
| CF₃ | H | Me | H | H | H |
| CF₃ | H | E t | H | H | H |
| CF₃ | H | Pr-c | H | H | H |
| CF₃ | H | CH₂ Pr-i | H | H | H |
| CF₃ | H | CH₂ Pr-c | H | H | H |
| CF₃ | H | CH₂ CH=CH₂ | H | H | H |
| CF₃ | H | CH₂ C=CH | H | H | H |
| CF₃ | H | CH₂ OMe | H | H | H |
| CF₃ | H | CH₂SMe | H | H | H |
| CF₃ | H | CH₂CO₂Me | H | H | H |
| CF₃ | H | CH₂ Ph | H | H | H |
| CF₃ | H | CH₂ Ph-2-Cl | H | H | H |
| CF₃ | H | CH₂ Ph-3-Cl | H | H | H |
| CF₃ | H | CH₂ Ph-4-Cl | H | H | H |
| CF₃ | H | P h | H | H | H |
| CF₃ | H | Ph-2-Cl | H | H | H |
| CF₃ | H | Ph-3-Cl | H | H | H |
| CF₃ | H | Ph-4-Cl | H | H | H |
| CF₃ | H | COMe | H | H | H |
| CF₃ | H | COPr-c | H | H | H |
| CF₃ | H | CONMe₂ | H | H | H |
| CF₃ | H | SO₂Me | H | H | H |
| CF₃ | H | SO₂ CF₃ | H | H | H |
| CF₃ | H | CO₂ Et | H | H | H |
| CF₃ | H | H | Me | H | H |
| CF₃ | H | Me | Me | H | H |
| CF₃ | H | Et | Me | H | H |
| CF₃ | H | Pr-c | Me | H | H |
| CF₃ | H | CH₂Pr-i | Me | H | H |
| CF₃ | H | CH₂Pr-c | Me | H | H |
| CF₃ | H | CH_{2C}H=CH₂ | Me | H | H |
| CF₃ | H | CH₂C=CH | Me | H | H |
| CF₃ | H | CH₂OMe | Me | H | H |
| CF₃ | H | CH₂SMe | Me | H | H |
| CF₃ | H | CH₂CO₂ Me | Me | H | H |
| CF₃ | H | CH₂Ph | Me | H | H |
| CF₃ | H | CH₂Ph-2-Cl | Me | H | H |
| CF₃ | H | CH₂ Ph-3-Cl | Me | H | H |
| CF₃ | H | CH₂Ph-4-Cl | Me | H | H |
| CF₃ | H | Ph | Me | H | H |
| CF₃ | H | Ph-2-Cl | Me | H | H |
| CF₃ | H | Ph-3-Cl | Me | H | H |
| CF₃ | H | Ph-4-Cl | Me | H | H |
| CF₃ | H | COMe | Me | H | H |
| CF₃ | H | COPr-c | Me | H | H |
| CF₃ | H | CONMe₂ | Me | H | H |
| CF₃ | H | SO₂Me | Me | H | H |
| CF₃ | H | SO₂CF₃ | Me | H | H |
| CF₃ | H | CO₂Et | Me | H | H |
| CF₃ | H | H | Me | Me | H |
| CF₃ | H | Me | Me | Me | H |
| CF₃ | H | Et | Me | Me | H |
| CF₃ | H | Pr-c | Me | Me | H |
| CF₃ | H | CH₂Pr-i | Me | Me | H |
| CF₃ | H | CH₂Pr-c | Me | Me | H |
| CF₃ | H | CH₂CH=CH₂ | Me | Me | H |
| CF₃ | H | CH₂C≡CH | Me | Me | H |
| CF₃ | H | CH₂OMe | Me | Me | H |
| CF₃ | H | CH₂SMe | Me | Me | H |
| CF₃ | H | CH₂CO₂Me | Me | Me | H |
| CF₃ | H | CH₂Ph | Me | Me | H |
| CF₃ | H | CH₂Ph-2-Cl | Me | Me | H |
| CF₃ | H | CH₂Ph-3-Cl | Me | Me | H |
| CF₃ | H | CH₂Ph-4-Cl | Me | Me | H |
| CF₃ | H | Ph | Me | Me | H |
| CF₃ | H | Ph-2-Cl | Me | Me | H |
| CF₃ | H | Ph-3-Cl | Me | Me | H |
| CF₃ | H | Ph-4-Cl | Me | Me | H |
| CF₃ | H | COMe | Me | Me | H |
| CF₃ | H | COPr-c | Me | Me | H |
| CF₃ | H | CONMe₂ | Me | Me | H |
| CF₃ | H | SO₂Me | Me | Me | H |
| CF₃ | H | SO₂CF₃ | Me | Me | H |
| CF₃ | H | CO₂ Et | Me | Me | H |
| CF₃ | H | H | Me | Et | H |
| CF₃ | H | Me | Me | Et | H |
| CF₃ | H | H | Me | Pr-n | H |
| CF₃ | H | Me | Me | Pr-n | H |
| CF₃ | H | H | Me | Pr-i | H |
| CF₃ | H | Me | Me | Pr-i | H |
| CF₃ | H | H | Me | Pr-c | H |
| CF₃ | H | Me | Me | Pr-c | H |
| CF₃ | H | H | Me | CF₃ | H |
| CF₃ | H | Me | Me | CF₃ | H |
| CF₃ | H | H | Me | Ph | H |
| CF₃ | H | Me | Me | Ph | H |
| CF₃ | H | H | Et | H | H |
| CF₃ | H | Me | Et | H | H |
| CF₃ | H | H | Et | Et | H |
| CF₃ | H | Me | Et | Et | H |
| CF₃ | H | H | Pr-n | H | H |
| CF₃ | H | Me | Pr-n | H | H |
| CF₃ | H | H | Pr-i | H | H |
| CF₃ | H | Me | Pr-i | H | H |
| CF₃ | H | H | Pr-c | H | H |
| CF₃ | H | Me | Pr-c | H | H |
| CF₃ | H | H | Bu-n | H | H |
| CF₃ | H | Me | Bu-n | H | H |
| CF₃ | H | H | Bu-i | H | H |
| CF₃ | H | Me | Bu-i | H | H |
| CF₃ | H | H | Bu-s | H | H |
| CF₃ | H | Me | Bu-s | H | H |
| CF₃ | H | H | Bu-t | H | H |
| CF₃ | H | Me | Bu-t | H | H |
| CF₃ | H | H | Bu-c | H | H |
| CF₃ | H | Me | Bu-c | H | H |
| CF₃ | H | H | Pen-n | H | H |
| CF₃ | H | Me | Pen-n | H | H |
| CF₃ | H | H | Pen-c | H | H |
| CF₃ | H | Me | Pen-c | H | H |
| CF₃ | H | H | Hex-n | H | H |
| CF₃ | H | Me | Hex-n | H | H |
| CF₃ | H | H | Hex-c | H | H |
| CF₃ | H | Me | Hex-c | H | H |
| CF₃ | H | H | Cl | H | H |
| CF₃ | H | Me | Cl | H | H |
| CF₃ | H | Et | Cl | H | H |
| CF₃ | H | H | Cl | Cl | H |
| CF₃ | H | Me | Cl | Cl | H |
| CF₃ | H | H | Br | H | H |
| CF₃ | H | Me | Br | H | H |
| CF₃ | H | H | I | H | H |
| CF₃ | H | Me | I | H | H |
| CF₃ | H | H | F | H | H |
| CF₃ | H | Me | F | H | H |
| CF₃ | H | H | F | F | H |
| CF₃ | H | Me | F | F | H |
| CF₃ | H | H | CH = CH₂ | H | H |
| CF₃ | H | Me | CH = CH₂ | H | H |
| CF₃ | H | H | CH = CH₂ | Me | H |
| CF₃ | H | Me | CH = CH₂ | Me | H |
| CF₃ | H | H | C ≡ CH | H | H |
| CF₃ | H | Me | C ≡ CH | H | H |
| CF₃ | H | H | C ≡ CH | Me | H |
| CF₃ | H | Me | C ≡ CH | Me | H |
| CF₃ | H | H | CH₂CH = CH₂ | H | H |
| CF₃ | H | Me | CH₂CH = CH₂ | H | H |
| CF₃ | H | H | CH₂CH = CH₂ | Me | H |
| CF₃ | H | Me | CH₂CH = CH₂ | Me | H |
| CF₃ | H | H | CH₂C ≡ CH | H | H |
| CF₃ | H | Me | CH₂C ≡ CH | H | H |
| CF₃ | H | H | CH₂C ≡ CH | Me | H |
| CF₃ | H | Me | CH₂C ≡ CH | Me | H |
| CF₃ | H | H | CH₂Cl | H | H |
| CF₃ | H | Me | CH₂Cl | H | H |
| CF₃ | H | H | CH₂Cl | Me | H |
| CF₃ | H | Me | CH₂Cl | Me | H |
| CF₃ | H | H | CHCl₂ | H | H |
| CF₃ | H | Me | CHCl₂ | H | H |
| CF₃ | H | H | CCl₃ | H | H |
| CF₃ | H | Me | CCl₃ | H | H |
| CF₃ | H | H | CH₂F | H | H |
| CF₃ | H | Me | CH₂F | H | H |
| CF₃ | H | H | CHF₂ | H | H |
| CF₃ | H | Me | CHF₂ | H | H |
| CF₃ | H | H | CF₃ | H | H |
| CF₃ | H | Me | CF₃ | H | H |
| CF₃ | H | H | CH₂CH₂Cl | H | H |
| CF₃ | H | Me | CH₂CH₂Cl | H | H |
| CF₃ | H | H | CH₂OH | H | H |
| CF₃ | H | Me | CH₂OH | H | H |
| CF₃ | H | H | CH₂SMe | H | H |
| CF₃ | H | Me | CH₂SMe | H | H |
| CF₃ | H | H | CH₂SCH₂Cl | H | H |
| CF₃ | H | Me | CH₂SCH₂Cl | H | H |
| CF₃ | H | H | Ph | H | H |
| CF₃ | H | Me | Ph | H | H |
| CF₃ | H | H | Ph-2-Cl | H | H |
| CF₃ | H | Me | Ph-2-Cl | H | H |
| CF₃ | H | H | Ph-3-Cl | H | H |
| CF₃ | H | Me | Ph-3-Cl | H | H |
| CF₃ | H | H | Ph-4-Cl | H | H |
| CF₃ | H | Me | Ph-4-Cl | H | H |
| CF₃ | H | Me | Ph-2-Br | H | H |
| CF₃ | H | Me | Ph-3-I | H | H |
| CF₃ | H | Me | Ph-4-F | H | H |
| CP₃ | H | Me | Ph-2-Me | H | H |
| CF₃ | H | Me | Ph-3-OMe | H | H |
| CF₃ | H | Me | Ph-4-SMe | H | H |
| CF₃ | H | Me | Ph-2-SO₂Me | H | H |
| CF₃ | H | Me | Ph-3-CN | H | H |
| CF₃ | H | Me | Ph-4-NO₂ | H | H |
| CF₃ | H | Me | Ph-2-CO₂Me | H | H |
| CF₃ | H | Me | Ph-3-NH₂ | H | H |
| CF₃ | H | Me | Ph-4-Ph | H | H |
| CF₃ | H | Me | Ph-2-OPh | H | H |
| CF₃ | H | Me | Ph-3-SPh | H | H |
| CF₃ | H | Me | Ph-4-OH | H | H |
| CF₃ | H | H | COMe | H | H |
| CF₃ | H | Me | COMe | H | H |
| CF₃ | H | H | COEt | H | H |
| CF₃ | H | Me | COEt | H | H |
| CF₃ | H | H | COCH₂ Cl | H | H |
| CF₃ | H | Me | COCH₂ Cl | H | H |
| CF₃ | H | H | CO₂H | H | H |
| CF₃ | H | Me | CO₂H | H | H |
| CF₃ | H | H | CO₂Me | H | H |
| CF₃ | H | Me | CO₂Me | H | H |
| CF₃ | H | H | CO₂Et | H | H |
| CF₃ | H | Me | CO₂Et | H | H |
| CF₃ | H | H | CONHMe | H | H |
| CF₃ | H | Me | CONHMe | H | H |
| CF₃ | H | H | CONMe₂ | H | H |
| CF₃ | H | Me | CONMe₂ | H | H |
| CF₃ | H | H | OH | H | H |
| CF₃ | H | Me | OH | H | H |
| CF₃ | H | H | OMe | H | H |
| CF₃ | H | Me | OMe | H | H |
| CF₃ | H | H | SMe | H | H |
| CF₃ | H | Me | SMe | H | H |
| CF₃ | H | H | CN | H | H |
| CF₃ | H | Me | CN | H | H |
| CF₃ | H | H | NH₂ | H | H |
| CF₃ | H | Me | NH₂ | H | H |
| CF₃ | H | H | NHMe | H | H |
| CF₃ | H | Me | NHMe | H | H |
| CF₃ | H | H | NMe₂ | H | H |
| CF₃ | H | Me | NMe₂ | H | H |
| CF₃ | H | H | -CH₂-O- | | H |
| CF₃ | H | Me | -CH₂-O- | | H |
| CF₃ | H | H | -CH₂-O- | | H |
| CF₃ | H | Me | -CH₂-O- | | H |
| CF₃ | H | H | -(CH₂)₂- | | H |
| CF₃ | H | Me | -(CH₂)₂- | | H |
| CF₃ | H | H | -(CH₂)₃- | | H |
| CF₃ | H | Me | -(CH₂)₃- | | H |
| CF₃ | H | Me | H | H | 3-F |
| CF₃ | H | Me | H | H | 4-F |
| CF₃ | H | Me | H | H | 5-F |
| CF₃ | H | Me | H | H | 6-F |
| CF₃ | H | Me | H | H | 4-Cl |
| CF₃ | H | Me | H | H | 5-Cl |
| CF₃ | H | Me | H | H | 4-Br |
| CF₃ | H | Me | H | H | 4-I |
| CF₃ | H | Me | H | H | 4-Me |
| CF₃ | H | Me | H | H | 5-Me |
| CF₃ | H | Me | H | H | 4-OMe |
| CF₃ | H | Me | H | H | 4-SMe |
| CF₃ | Me | H | H | H | H |
| CF₃ | Me | H | Me | H | H |
| CF₃ | Me | H | Me | Me | H |
| CF₃ | Me | Me | H | H | H |
| CF₃ | Me | Me | Me | H | H |
| CF₃ | Me | Me | Me | Me | H |
| CF₃ | COMe | H | H | H | H |
| CF₃ | COMe | H | Me | H | H |
| CF₃ | COMe | H | Me | Me | H |
| CF₃ | COMe | Me | H | H | H |
| CF₃ | COMe | Me | Me | H | H |
| CF₃ | COMe | Me | Me | Me | H |
| CF₃ | COMe | H | -CH₂-O- | | H |
| CF₃ | COMe | Me | -CH₂-O- | | H |
| CF₃ | COMe | H | - (CH₂)₂- | | H |
| CF₃ | COMe | Me | - (CH₂)₂- | | H |
| CF₃ | COMe | H | - (CH₂)₃- | | H |
| CF₃ | COMe | Me | - (CH₂)₃- | | H |
| CF₃ | COEt | H | H | H | H |
| CF₃ | COEt | H | Me | H | H |
| CF₃ | COEt | H | Me | Me | H |
| CF₃ | COEt | Me | H | H | H |
| CF₃ | COEt | Me | Me | H | H |
| CF₃ | COEt | Me | Me | Me | H |
| CF₃ | COEt | SO₂CF₃ | H | H | H |
| CF₃ | COEt | H | Et | H | H |
| CF₃ | COEt | Me | Et | H | H |
| CF₃ | COEt | H | Et | Me | H |
| CF₃ | COEt | Me | Et | Me | H |
| CF₃ | COEt | H | Pr-n | H | H |
| CF₃ | COEt | Me | Pr-n | H | H |
| CF₃ | COEt | H | Pr-n | Me | H |
| CF₃ | COEt | Me | Pr-n | Me | H |
| CF₃ | COEt | H | Cl | H | H |
| CF₃ | COEt | Me | Cl | H | H |
| CF₃ | COEt | H | CH₂CH=CH₂ | H | H |
| CF₃ | COEt | Me | CH₂CH=CH₂ | H | H |
| CF₃ | COEt | H | CH₂Cl | H | H |
| CF₃ | COEt | Me | CH₂Cl | H | H |
| CF₃ | COEt | H | CF₃ | H | H |
| CF₃ | COEt | Me | CF₃ | H | H |
| CF₃ | COEt | H | CF₃ | Me | H |
| CF₃ | COEt | Me | CF₃ | Me | H |
| CF₃ | COEt | H | Ph | H | H |
| CF₃ | COEt | Me | Ph | H | H |
| CF₃ | COEt | H | Ph | Me | H |
| CF₃ | COEt | Me | Ph | Me | H |
| CF₃ | COEt | H | Ph-2-Cl | H | H |
| CF₃ | COEt | Me | Ph-2-Cl | H | H |
| CF₃ | COEt | H | Ph-3-Cl | H | H |
| CF₃ | COEt | Me | Ph-3-Cl | H | H |
| CF₃ | COEt | H | Ph-4-Cl | H | H |
| CF₃ | COEt | Me | Ph-4-Cl | H | H |
| CF₃ | COEt | H | COMe | H | H |
| CF₃ | COEt | Me | COMe | H | H |
| CF₃ | COEt | H | CO₂H | H | H |
| CF₃ | COEt | Me | CO₂H | H | H |
| CF₃ | COEt | H | CO₂Me | H | H |
| CF₃ | COEt | Me | CO₂Me | H | H |
| CF₃ | COEt | H | CONH₂ | H | H |
| CF₃ | COEt | Me | CONH₂ | H | H |
| CF₃ | COEt | H | CONHMe | H | H |
| CF₃ | COEt | Me | CONHMe | H | H |
| CF₃ | COEt | H | CONMe₂ | H | H |
| CF₃ | COEt | Me | CONMe₂ | H | H |
| CF₃ | COEt | H | OH | H | H |
| CF₃ | COEt | Me | OH | H | H |
| CF₃ | COEt | H | OMe | H | H |
| CF₃ | COEt | Me | OMe | H | H |
| CF₃ | COEt | H | OMe | Me | H |
| CF₃ | COEt | Me | OMe | Me | H |
| CF₃ | COEt | H | SMe | H | H |
| CF₃ | COEt | Me | SMe | H | H |
| CF₃ | COEt | H | SMe | Me | H |
| CF₃ | COEt | Me | SMe | Me | H |
| CF₃ | COEt | H | NHMe | H | H |
| CF₃ | COEt | Me | NHMe | H | H |
| CF₃ | COEt | H | NHMe | Me | H |
| CF₃ | COEt | Me | NHMe | Me | H |
| CF₃ | COEt | H | CH₂ Ph | H | H |
| CF₃ | COEt | Me | CH₂ Ph | H | H |
| CF₃ | COEt | H | CH₂Ph-2-Cl | H | H |
| CF₃ | COEt | Me | CH₂Ph-2-Cl | H | H |
| CF₃ | COEt | H | CH₂Ph-3-Cl | H | H |
| CF₃ | COEt | Me | CH₂Ph-3-Cl | H | H |
| CF₃ | COEt | H | CH₂Ph-4-Cl | H | H |
| CF₃ | COEt | Me | CH₂Ph-4-C1 | H | H |
| CF₃ | COEtt | H | COPh | H | H |
| CF₃ | COEt | Me | COPh | H | H |
| CF₃ | COEt | H | COPh-2-Cl | H | H |
| CF₃ | COEt | Me | COPh-2-Cl | H | H |
| CF₃ | COEt | H | COPh-3-Cl | H | H |
| CF₃ | COEt | Me | COPh-3-Cl | H | H |
| CF₃ | COEt | H | COPh-4-Cl | H | H |
| CF₃ | COEt | Me | COPh-4-Cl | H | H |
| CF₃ | COEt | H | -CH₂-O- | | H |
| CF₃ | COEt | Me | -CH₂-O- | | H |
| CF₃ | COEt | H | -(CH₂)₂- | | H |
| CF₃ | COEt | Me | -(CH₂)₂- | | H |
| CF₃ | COEt | H | -(CH₂)₃- | | H |
| CF₃ | COEt | Me | -(CH₂)₃- | | H |
| CF₃ | COEt | Me | H | H | 3-F |
| CF₃ | COEt | Me | H | H | 4-F |
| CF₃ | COEt | Me | H | H | 5-F |
| CF₃ | COEt | Me | H | H | 6-F |
| CF₃ | COEt | Me | H | H | 4-C |
| CF₃ | COEt | Me | H | H | 5-C |
| CF₃ | COEt | Me | H | H | 4-Br |
| CF₃ | COEt | Me | H | H | 4-I |
| CF₃ | COEt | Me | H | H | 4-Me |
| CF₃ | COEt | Me | H | H | 5-Me |
| CF₃ | COEt | Me | H | H | 4-OMe |
| CF₃ | COEt | Me | H | H | 4-SMe |
| CF₃ | COPr-n | H | H | H | H |
| CF₃ | COPr-n | H | Me | H | H |
| CF₃ | COPr-n | H | Me | Me | H |
| CF₃ | COPr-n | Me | H | H | H |
| CF₃ | COPr-n | Me | Me | H | H |
| CF₃ | COPr-n | Me | Me | Me | H |
| CF₃ | COPr-n | H | -CH₂-O- | | H |
| CF₃ | COPr-n | Me | -CH₂-O- | | H |
| CF₃ | COPr-n | H | -(CH₂)₂- | | H |
| CF₃ | COPr-n | Me | -(CH₂)₂- | | H |
| CF₃ | COPr-n | H | -(CH₂)₃- | | H |
| CF₃ | COPr-n | Me | -(CH₂)₃- | | H |
| CF₃ | COPr-i | H | H | H | H |
| CF₃ | COPr-i | H | Me | H | H |
| CF₃ | COPr-i | H | Me | Me | H |
| CF₃ | COPr-i | Me | H | H | H |
| CF₃ | COPr-i | Me | Me | H | H |
| CF₃ | COPr-i | Me | Me | Me | H |
| CF₃ | COPr-i | H | -CH₂-O- | | H |
| CF₃ | COPr-i | Me | -CH₂-O- | | H |
| CF₃ | COPr -i | H | - (CH₂)₂ - | | H |
| CF₃ | COPr-i | Me | - (CH₂)₂- | | H |
| CF₃ | COPr-i | H | - (CH₂)₃- | | H |
| CF₃ | COPr-i | Me | - (CH₂)₃- | | H |
| CF₃ | COPr-c | H | H | H | H |
| CF₃ | COPr-c | H | Me | H | H |
| CF₃ | COPr-c | H | Me | Me | H |
| CF₃ | COPr-c | Me | H | H | H |
| CF₃ | COPr-c | Me | Me | H | H |
| CF₃ | COPr-c | Me | Me | Me | H |
| CF₃ | COPr-c | H | -CH₂-O- | | H |
| CF₃ | COPr-c | Me | -CH₂-O- | | H |
| CF₃ | COPr-c | H | - (CH₂)₂- | | H |
| CF₃ | COPr-c | Me | -(CH₂)₂- | | H |
| CF₃ | COPr-c | H | - (CH₂)₃- | | H |
| CF₃ | COPr-c | Me | - (CH₂)₃- | | H |
| CF₃ | COBu-n | H | H | H | H |
| CF₃ | COBu-n | H | Me | H | H |
| CF₃ | COBu-n | H | Me | Me | H |
| CF₃ | COBu-n | Me | H | H | H |
| CF₃ | COBu-n | Me | Me | H | H |
| CF₃ | COBu-n | Me | Me | Me | H |
| CF₃ | COBu-n | H | -CH₂-O- | | H |
| CF₃ | COBu-n | Me | -CH₂-O- | | H |
| CF₃ | COBu-n | H | - (CH₂)₂- | | H |
| CF₃ | COBu-n | Me | - (CH₂)₂- | | H |
| CF₃ | COBu-n | H | - (CH₂)₃- | | H |
| CF₃ | COBu-n | Me | -(CH₂)₃- | | H |
| CF₃ | COBu-c | H | H | H | H |
| CF₃ | COBu-c | H | Me | H | H |
| CF₃ | COBu-c | H | Me | Me | H |
| CF₃ | COBu-c | Me | H | H | H |
| CF₃ | COBu-c | Me | Me | H | H |
| CF₃ | COBu-c | Me | Me | Me | H |
| CF₃ | COBu-c | H | -CH₂-O- | | H |
| CF₃ | COBu-c | Me | -CH₂-O- | | H |
| CF₃ | COBu-c | H | - (CH₂)₂- | | H |
| CF₃ | COBu-c | Me | - (CH₂)₂- | | H |
| CF₃ | COBu-c | H | -(CH₂)₃- | | H |
| CF₃ | COBu-c | Me | - (CH₂)₃- | | H |
| CF₃ | COBu-t | H | H | H | H |
| CF₃ | COBu-t | H | Me | H | H |
| CF₃ | COBu-t | H | Me | Me | H |
| CF₃ | COBu-t | Me | H | H | H |
| CF₃ | COBu-t | Me | Me | H | H |
| CF₃ | COBu-t | Me | Me | Me | H |
| CF₃ | COBu-t | H | -CH₂-O- | | H |
| CF₃ | COBu-t | Me | -CH₂-O- | | H |
| CF₃ | COBu-t | H | - (CH₂)₂- | | H |
| CF₃ | COBu-t | Me | -(CH₂)₂- | | H |
| CF₃ | COBu-t | H | - (CH₂)₃- | | H |
| CF₃ | COBu-t | Me | - (CH₂)₃- | | H |
| CF₃ | COPen-n | H | H | H | H |
| CF₃ | COPen-n | H | Me | H | H |
| CF₃ | COPen-n | H | Me | Me | H |
| CF₃ | COPen-n | Me | H | H | H |
| CF₃ | COPen-n | Me | Me | H | H |
| CF₃ | COPen-n | Me | Me | Me | H |
| CF₃ | COPen-n | H | -CH₂-O- | | H |
| CF₃ | COPen-n | Me | -CH₂-O- | | H |
| CF₃ | COPen-n | H | - (CH₂)₂- | | H |
| CF₃ | COPen-n | Me | - (CH₂)₂- | | H |
| CF₃ | COPen-n | H | - (CH₂)₃- | | H |
| CF₃ | COPen-n | Me | - (CH₂)₃- | | H |
| CF₃ | COHex-n | H | H | H | H |
| CF₃ | COHeX-n | H | Me | H | H |
| CF₃ | COHex-n | H | Me | Me | H |
| CF₃ | COHex-n | Me | H | H | H |
| CF₃ | COHex-n | Me | Me | H | H |
| CF₃ | COHex-n | Me | Me | Me | H |
| CF₃ | COHex-n | H | -CH₂-O- | | H |
| CF₃ | COHex-n | Me | -CH₂-O- | | H |
| CF₃ | COHex-n | H | - (CH₂)₂- | | H |
| CF₃ | COHex-n | Me | - (CH₂)₂- | | H |
| CF₃ | COHex-n | H | - (CH₂)₃- | | H |
| CF₃ | COHex-n | Me | - (CH₂)₃- | | H |
| CF₃ | COPh | H | H | H | H |
| CF₃ | COPh | H | Me | H | H |
| CF₃ | COPh | H | Me | Me | H |
| CF₃ | COPh | Me | H | H | H |
| CF₃ | COPh | Me | Me | H | H |
| CF₃ | COPh | Me | Me | Me | H |
| CF₃ | COPh | H | -CH₂-O- | | H |
| CF₃ | COPh | Me | -CH₂-O- | | H |
| CF3 | COPh | H | -(CH₂)₂- | | H |
| CF₃ | COPh | Me | - (CH₂)₂- | | H |
| CF₃ | COPh | H | - (CH₂)₃- | | H |
| CF₃ | COPh | Me | -(CH₂)₃- | | H |
| CF₃ | CO₂ Me | H | H | H | H |
| CF₃ | CO₂ Me | H | Me | H | H |
| CF₃ | CO₂ Me | H | Me | Me | H |
| CF₃ | CO₂ Me | Me | H | H | H |
| CF₃ | CO₂ Me | Me | Me | H | H |
| CF₃ | CO₂ Me | Me | Me | Me | H |
| CF₃ | CO₂Me | H | -CH₂-O- | | H |
| CF₃ | CO₂Me | Me | -CH₂-O- | | H |
| CF₃ | CO₂Me | H | -(CH₂)2- | | H |
| CF₃ | CO₂Me | Me | -(CH₂)₂- | | H |
| CF₃ | CO₂Me | H | -(CH₂)₃- | | H |
| CF₃ | CO₂Me | Me | -(CH₂)₃- | | H |
| CF₃ | CO₂Et | H | H | H | H |
| CF₃ | CO₂Et | Me | H | H | H |
| CF₃ | CO₂Et | Et | H | H | H |
| CF₃ | CO₂Et | Pr-c | H | H | H |
| CF₃ | CO₂Et | CH₂Pr-i | H | H | H |
| CF₃ | CO₂Et | CH₂Pr-c | H | H | H |
| CF₃ | CO₂Et | CH₂CH=CH₂ | H | H | H |
| CF₃ | CO₂Et | CH₂C≡CH | H | H | H |
| CF₃ | CO₂Et | CH₂OMe | H | H | H |
| CF₃ | CO₂Et | CH₂SMe | H | H | H |
| CF₃ | CO₂Ett | CH₂CO₂Me | H | H | H |
| CF₃ | CO₂Et | CH₂Ph | H | H | H |
| CF₃ | CO₂Ett | CH₂Ph-2-Cl | H | H | H |
| CF₃ | CO₂Et | CH₂Ph-3-Cl | H | H | H |
| CF₃ | CO₂Et | CH₂Ph-4-Cl | H | H | H |
| CF₃ | CO2Et | Ph | H | H | H |
| CF₃ | CO₂Et | Ph-2-Cl | H | H | H |
| CF₃ | CO₂Et | Ph-3-Cl | H | H | H |
| CF₃ | CO₂Et | Ph-4-Cl | H | H | H |
| CF₃ | CO₂Et | COMe | H | H | H |
| CF₃ | CO₂Et | COPr-c | H | H | H |
| CF₃ | CO₂Et | CONMe₂ | H | H | H |
| CF₃ | CO₂Et | SO₂Me | H | H | H |
| CF₃ | CO₂Et | SO₂CF₃ | H | H | H |
| CF₃ | CO₂Et | CO₂Et | H | H | H |
| CF₃ | CO₂Et | H | Me | H | H |
| CF₃ | CO₂Et | Me | Me | H | H |
| CF₃ | CO₂Et | Et | Me | H | H |
| CF₃ | CO₂Et | Pr-c | Me | H | H |
| CF₃ | CO₂Et | CH₂Pr-i | Me | H | H |
| CF₃ | CO₂Et | CH₂Pr-c | Me | H | H |
| CF₃ | CO₂Et | CH₂CH=CH₂ | Me | H | H |
| CF₃ | CO₂Et | CH₂C=CH | Me | H | H |
| CF₃ | CO₂Et | CH₂OMe | Me | H | H |
| CF₃ | CO₂Et | CH₂SMe | Me | H | H |
| CF₃ | CO₂Et | CH₂CO₂Me | Me | H | H |
| CF₃ | CO₂Et | CH₂Ph | Me | H | H |
| CF₃ | CO₂Et | CH₂Ph-2-Cl | Me | H | H |
| CF₃ | CO₂Et | CH₂Ph-3-Cl | Me | H | H |
| CF₃ | CO₂Et | CH₂Ph-4-Cl | Me | H | H |
| CF₃ | CO₂Et | Ph | Me | H | H |
| CF₃ | CO₂Et | Ph-2-Cl | Me | H | H |
| CF₃ | CO₂Et | Ph-3-Cl | Me | H | H |
| CF₃ | CO₂Et | Ph-4-Cl | Me | H | H |
| CF₃ | CO₂E | COMe | Me | H | H |
| CF₃ | CO₂Et | COPr-c | Me | H | H |
| CF₃ | CO₂Et | CONMe₂ | Me | H | H |
| CF₃ | CO₂Et | SO₂Me | Me | H | H |
| CF₃ | CO₂Et | SO₂CF₃ | Me | H | H |
| CF₃ | CO₂Et | CO₂Et | Me | H | H |
| CF₃ | CO₂Et | H | Me | Me | H |
| CF₃ | CO₂Et | Me | Me | Me | H |
| CF₃ | CO₂Et | Et | Me | Me | H |
| CF₃ | CO₂Et | Pr-cc | Me | Me | H |
| CF₃ | CO₂Et | CH₂Pr-i | Me | Me | H |
| CF₃ | CO₂Et | CH₂Pr-c | Me | Me | H |
| CF₃ | CO₂Et | CH₂CH=CH₂ | Me | Me | H |
| CF₃ | CO₂Et | CH₂C=CH | Me | Me | H |
| CF₃ | CO₂Et | CH₂OMe | Me | Me | H |
| CF₃ | CO₂Et | CH₂SMe | Me | Me | H |
| CF₃ | CO₂Et | CH₂CO₂Me | Me | Me | H |
| CF₃ | CO₂Et | CH₂Ph | Me | Me | H |
| CF₃ | CO₂Et | CH₂Ph-2-Cl | Me | Me | H |
| CF₃ | CO₂Et | CH₂Ph-3-Cl | Me | Me | H |
| CF₃ | CO₂Et | CH₂Ph-4-Cl | Me | Me | H |
| CF₃ | CO₂Et | Ph | Me | Me | H |
| CF₃ | CO₂Et | Ph-2-C1 | Me | Me | H |
| CF₃ | CO₂Et | Ph-3-Cl | Me | Me | H |
| CF₃ | CO₂Et | Ph-4-Cl | Me | Me | H |
| CF₃ | CO₂Et | COMe | Me | Me | H |
| CF₃ | CO₂Et | COPr-c | Me | Me | H |
| CF₃ | CO₂Et | CONMe₂ | Me | Me | H |
| CF₃ | CO₂Et | SO₂Me | Me | Me | H |
| CF₃ | CO₂Et | SO₂CF₃ | Me | Me | H |
| CF₃ | CO₂Et | CO₂Et | Me | Me | H |
| CF₃ | CO₂Et | H | Me | Et | H |
| CF₃ | CO₂Et | Me | Me | Et | H |
| CF₃ | CO₂Et | H | Me | Pr-n | H |
| CF₃ | CO₂Et | Me | Me | Pr-n | H |
| CF₃ | CO₂Et | H | Me | Pr-i | H |
| CF₃ | CO₂Et | Me | Me | Pr-i | H |
| CF₃ | CO₂Et | H | Me | Pr-c | H |
| CF₃ | CO₂Et | Me | Me | Pr-c | H |
| CF₃ | CO₂Et | H | Me | CF₃ | H |
| CF₃ | CO₂Et | Me | Me | CF₃ | H |
| CF₃ | CO₂Et | H | Me | Ph | H |
| CF₃ | CO₂Et | Me | Me | Ph | H |
| CF₃ | CO₂Et | H | Et | H | H |
| CF₃ | CO₂ E t | Me | E t | H | H |
| CF₃ | CO₂ E t | H | E t | E t | H |
| CF₃ | CO₂ E t | Me | E t | E t | H |
| CF₃ | CO₂ Et | H | Pr-n | H | H |
| CF₃ | CO₂ Et | Me | Pr-n | H | H |
| CF₃ | CO₂ E t | H | Pr-i | H | H |
| CF₃ | CO₂ E t | Me | Pr-i | H | H |
| CF₃ | CO₂ E t | H | Pr-c | H | H |
| CF₃ | CO₂ E t | Me | Pr-c | H | H |
| CF₃ | CO₂ E t | H | Bu-n | H | H |
| CF₃ | CO₂ Et | Me | Bu-n | H | H |
| CF₃ | CO₂ E t | H | Bu-i | H | H |
| CF₃ | CO₂ E t | Me | Bu-i | H | H |
| CF₃ | CO₂ E t | H | Bu-s | H | H |
| CF₃ | CO₂ E t | Me | Bu- s | H | H |
| CF₃ | CO₂ E t | H | Bu- t | H | H |
| CF₃ | CO₂ E t | Me | Bu- t | H | H |
| CF₃ | CO₂ E t | H | Bu- c | H | H |
| CF₃ | CO₂ E t | Me | Bu-c | H | H |
| CF₃ | CO₂ E t | H | Pen-n | H | H |
| CF₃ | CO₂ E t | Me | Pen-n | H | H |
| CF₃ | CO₂ E t | H | Pen-c | H | H |
| CF₃ | CO₂ E t | Me | Pen- c | H | H |
| CF₃ | CO₂ E t | H | Hex-n | H | H |
| CF₃ | CO₂ E t | Me | Hex-n | H | H |
| CF₃ | CO₂ E t | H | Hex- c | H | H |
| CF₃ | CO₂ E t | Me | Hex-c | H | H |
| CF₃ | CO₂ E t | H | Cl | H | H |
| CF₃ | CO₂ E t | Me | Cl | H | H |
| CF₃ | CO₂ E t | E t | Cl | H | H |
| CF₃ | CO₂ E t | H | Cl | C l | H |
| CF₃ | CO₂ E t | Me | Cl | C l | H |
| CF₃ | CO₂ E t | H | Br | H | H |
| CF₃ | CO₂ E t | Me | Br | H | H |
| CF₃ | CO₂ E t | H | I | H | H |
| CF₃ | CO₂ E t | Me | I | H | H |
| CF₃ | CO₂ E t | H | F | H | H |
| CF₃ | CO₂ E t | Me | F | H | H |
| CF₃ | CO₂ E t | H | F | F | H |
| CF₃ | CO₂ E t | Me | F | F | H |
| CF₃ | CO₂ E t | H | CH=CH₂ | H | H |
| CF₃ | CO₂ E t | Me | CH=CH₂ | H | H |
| CF₃ | CO₂ E t | H | CH=CH₂ | Me | H |
| CF₃ | CO₂ E t | Me | CH=CH₂ | Me | H |
| CF₃ | CO₂ E t | H | C=CH | H | H |
| CF₃ | CO₂ E t | Me | C=CH | H | H |
| CF₃ | CO₂ E t | H | C=CH | Me | H |
| CF₃ | CO₂ Et | Me | C=CH | Me | H |
| CF₃ | CO₂ Et | H | CH₂CH=CH₂ | H | H |
| CF₃ | CO₂ Et | Me | CH₂CH=CH₂ | H | H |
| CF₃ | CO₂ Et | H | CH₂ CH=CH₂ | Me | H |
| CF₃ | CO₂ Et | Me | CH₂CH=CH₂ | Me | H |
| CF₃ | CO₂ Et | H | CH₂C=CH | H | H |
| CF₃ | CO₂ Et | Me | CH₂C=CH | H | H |
| CF₃ | CO₂ Et | H | CH₂C=CH | Me | H |
| CF₃ | CO₂ Et | Me | CH₂ C=CH | Me | H |
| CF₃ | CO₂ Et | H | CH₂ C l | H | H |
| CF₃ | CO₂ Et | Me | CH₂ C l | H | H |
| CF₃ | CO₂ Et | H | CH₂ C l | Me | H |
| CF₃ | CO₂ Et | Me | CH₂ C l | Me | H |
| CF₃ | CO₂ Et | H | CHCl₂ | H | H |
| CF₃ | CO₂ Et | Me | CHCl₂ | H | H |
| CF₃ | CO₂ Et | H | CC l₃ | H | H |
| CF₃ | CO₂ Et | Me | CC l₃ | H | H |
| C F ₃ | CO₂ Et | H | CH₂ F | H | H |
| CF₃ | CO₂ Et | Me | CH₂ F | H | H |
| CF₃ | CO₂ Et | H | CHF₂ | H | H |
| CF₃ | CO₂ Et | Me | CHF₂ | H | H |
| CF₃ | CO₂ Et | H | CF₃ | H | H |
| CF₃ | CO₂ Et | Me | CF₃ | H | H |
| CF₃ | CO₂ Et | H | CH₂ CH₂ C l | H | H |
| CF₃ | CO₂ Et | Me | CH₂ CH₂ C l | H | H |
| CF₃ | CO₂ Et | H | CH₂ OH | H | H |
| CF₃ | CO₂ Et | Me | CH₂ OH | H | H |
| CF₃ | CO₂ Et | H | CH₂ SMe | H | H |
| CF₃ | CO₂ Et | Me | CH₂SMe | H | H |
| CF₃ | CO₂ Et | H | CH₂ SCH₂ C l | H | H |
| CF₃ | CO₂ Et | Me | CH₂ SCH₂ C l | H | H |
| CF₃ | CO₂ Et | H | Ph | H | H |
| CF₃ | CO₂ Et | Me | Ph | H | H |
| CF₃ | CO₂ Et | H | Ph-2-Cl | H | H |
| CF₃ | CO₂ Et | Me | Ph-2-Cl | H | H |
| CF₃ | CO₂ Et | H | Ph-3-Cl | H | H |
| CF₃ | CO₂ Et | Me | Ph-3-Cl | H | H |
| CF₃ | CO₂ Et | H | Ph-4-Cl | H | H |
| CF₃ | CO₂ Et | Me | Ph-4-Cl | H | H |
| CF₃ | CO₂ Et | Me | Ph-2-Br | H | H |
| CF₃ | CO₂ Et | Me | Ph-3-I | H | H |
| CF₃ | CO₂ Et | Me | Ph-4-F | H | H |
| CF₃ | CO₂ Et | Me | Ph-2-Me | H | H |
| CF₃ | CO₂ Et | Me | Ph-3-OMe | H | H |
| CF₃ | CO₂ Et | Me | Ph-4-SMe | H | H |
| CF₃ | CO₂ Et | Me | Ph- 2-SO₂Me | H | H |
| CF₃ | CO₂ Et | Me | Ph-3-CN | H | H |
| CF₃ | CO₂ Et | Me | Ph-4-NO₂ | H | H |
| CF₃ | CO₂ E t | Me | Ph-2-CO₂Me | H | H |
| CF₃ | CO₂ E t | Me | Ph-3-NH₂ | H | H |
| CF₃ | CO₂ E t | Me | Ph-4-Ph | H | H |
| CF₃ | CO₂ E t | Me | Ph-2-OPh | H | H |
| CF₃ | CO₂ E t | Me | Ph-3-SPh | H | H |
| CF₃ | CO₂ E t | Me | Ph-4-OH | H | H |
| CF₃ | CO₂ E t | H | COMe | H | H |
| CF₃ | CO₂ E t | Me | COMe | H | H |
| CF₃ | CO₂ E t | H | COEt | H | H |
| CF₃ | CO₂ E t | Me | COEt | H | H |
| CF₃ | CO₂ E t | H | COCH₂Cl | H | H |
| CF₃ | CO₂ Et | Me | COCH₂Cl | H | H |
| CF₃ | CO₂ E t | H | CO₂ H | H | H |
| CF₃ | CO₂ E t | Me | CO₂ H | H | H |
| CF₃ | CO₂ E t | H | CO₂ Me | H | H |
| CF₃ | CO₂ E t | Me | CO₂ Me | H | H |
| CF₃ | CO₂ E t | H | CO₂ E t | H | H |
| CF₃ | CO₂ E t | Me | CO₂ E t | H | H |
| CF₃ | CO₂ Et | H | CONHMe | H | H |
| CF₃ | CO₂ E t | Me | CONHMe | H | H |
| CF₃ | CO₂ E t | H | CONMe₂ | H | H |
| CF₃ | CO₂ E t | Me | CONMe₂ | H | H |
| CF₃ | CO₂ E t | H | OH | H | H |
| CF₃ | CO₂ E t | Me | OH | H | H |
| CF₃ | CO₂ E t | H | OMe | H | H |
| CF₃ | CO₂ Et | Me | OMe | H | H |
| CF₃ | CO₂ E t | H | SMe | H | H |
| CF₃ | CO₂ E t | Me | SMe | H | H |
| CF₃ | CO₂ E t | H | CN | H | H |
| CF₃ | CO₂ E t | Me | CN | H | H |
| CF₃ | CO₂ E t | H | NH₂ | H | H |
| CF₃ | CO₂ E t | Me | NH₂ | H | H |
| CF₃ | CO₂ E t | H | NHMe | H | H |
| CF₃ | CO₂ Et | Me | NHMe | H | H |
| CF₃ | CO₂ E t | H | NMe₂ | H | H |
| CF₃ | CO₂ E t | Me | NMe₂ | H | H |
| CF₃ | CO₂ E t | H | -CH₂-O- | | H |
| CF₃ | CO₂ E t | Me | -CH₂-O- | | H |
| CF₃ | CO₂ E t | H | -CH₂-O- | | H |
| CF₃ | CO₂ Et | Me | -CH₂-O- | | H |
| CF₃ | CO₂ E t | H | - (CH₂)₂- | | H |
| CF₃ | CO₂ E t | Me | -(CH₂)₂- | | H |
| CF₃ | CO₂ E t | H | - (CH₂)₃- | | H |
| CF₃ | CO₂ E t | Me | - (CH₂)₃- | | H |
| CF₃ | CO₂ E t | Me | H | H | 3-F |
| CF₃ | CO₂ E t | Me | H | H | 4-F |
| CF₃ | CO₂ Et t | Me | H | H | 5-F |
| CF₃ | CO₂ E t | Me | H | H | 6-F |
| CF₃ | CO₂ E t | Me | H | H | 4-Cl |
| CF₃ | CO₂ Et | Me | H | H | 5-Cl |
| CF₃ | CO₂ E t | Me | H | H | 4-Br |
| CF₃ | CO₂ E t | Me | H | H | 4-I |
| CF₃ | CO₂ E t | Me | H | H | 4-Me |
| CF₃ | CO₂ E t | Me | H | H | 5-Me |
| CF₃ | CO₂ E t | Me | H | H | 4-OMe |
| CF₃ | CO₂ Et | Me | H | H | 4-SMe |
| CF₃ | CO₂ Pr-n | H | H | H | H |
| CF₃ | CO₂ Pr-n | H | Me | H | H |
| CF₃ | CO₂ Pr-n | H | Me | Me | H |
| CF₃ | CO₂ Pr-n | Me | H | H | H |
| CF₃ | CO₂ Pr-n | Me | Me | H | H |
| CF₃ | CO₂ Pr-n | Me | Me | Me | H |
| CF₃ | CO₂ Pr-n | H | -CH₂-O- | | H |
| CF₃ | CO₂ Pr-n | Me | -CH₂-O- | | H |
| CF₃ | CO₂ Pr-n | H | - (CH₂)₂- | | H |
| CF₃ | CO₂ Pr-n | Me | - (CH₂)₂- | | H |
| CF₃ | CO₂ P r -n | H | - (CH₂)₃- | | H |
| CF₃ | CO₂ Pr-n | Me | - (CH₂)₃- | | H |
| CF₃ | CO₂ Pr-i | H | H | H | H |
| CF₃ | CO₂ Pr-i | H | Me | H | H |
| CF₃ | CO₂ Pr-i | H | Me | Me | H |
| CF₃ | CO₂ Pr-i | Me | H | H | H |
| CF₃ | CO₂ Pr-i | Me | Me | H | H |
| CF₃ | CO₂ Pr-i | Me | Me | Me | H |
| CF₃ | CO₂ Pr-i | H | -CH₂-O- | | H |
| CF₃ | CO₂ Pr-i | Me | -CH₂-O- | | H |
| CF₃ | CO₂ P r - i | H | - (CH₂)₂- | | H |
| CF₃ | CO₂ Pr-i | Me | - (CH₂)₂- | | H |
| C F ₃ | CO₂ Pr-i | H | - (CH₂)₃- | | H |
| CF₃ | CO₂ Pr-i | Me | - (CH₂)₃- | | H |
| CF₃ | CO₂ Bu-n | H | H | H | H |
| CF₃ | CO₂ Bu-n | H | Me | H | H |
| CF₃ | CO₂ Bu-n | H | Me | Me | H |
| CF₃ | CO₂ Bu-n | Me | H | H | H |
| CF₃ | CO₂ Bu-n | Me | Me | H | H |
| CF₃ | CO₂ Bu-n | Me | Me | Me | H |
| CF₃ | CO₂ Bu-n | H | -CH₂-O- | | H |
| CF₃ | CO₂ Bu-n | Me | -CH₂-O- | | H |
| CF₃ | CO₂ Bu-n | H | - (CH₂)₂- | | H |
| CF₃ | CO₂ Bu-n | Me | - (CH₂)₂- | | H |
| CF₃ | CO₂ Bu-n | H | - (CH₂)₃- | | H |
| CF₃ | CO₂ Bu-n | Me | - (CH₂)₃- | | H |
| CF₃ | CO₂ Bu- i | H | H | H | H |
| CF₃ | CO₂ Bu- i | H | Me | H | H |
| CF₃ | CO₂ Bu- i | H | Me | Me | H |
| CF₃ | CO₂ Bu- i | Me | H | H | H |
| CF₃ | CO₂ Bu-i | Me | Me | H | H |
| CF₃ | CO₂ Bu- i | Me | Me | Me | H |
| CF₃ | CO₂ Bu-i | H | -CH₂-O- | | H |
| CF₃ | CO₂ Bu- i | Me | -CH₂-O- | | H |
| CF₃ | CO₂ Bu- i | H | - (CH₂) ₂ - | | H |
| CF₃ | CO₂ Bu- i | Me | - (CH₂)₂ - | | H |
| CF₃ | CO₂ Bu-i | H | - (CH₂)₃ - | | H |
| CF₃ | CO₂ Bu-i | Me | - (CH₂)₃ - | | H |
| CF₃ | CO₂ Bu-t | H | H | H | H |
| CF₃ | CO₂ Bu- t | H | Me | H | H |
| CF₃ | CO₂ Bu-t | H | Me | Me | H |
| CF₃ | CO₂ Bu-t | Me | H | H | H |
| CF₃ | CO₂ Bu- t | Me | Me | H | H |
| CF₃ | CO₂ Bu- t | Me | Me | Me | H |
| CF₃ | CO₂ Bu-t | H | -CH₂-O- | | H |
| CF₃ | CO₂ Bu- t | Me | -CH₂-O- | | H |
| C F ₃ | CO₂ Bu- t | H | - (CH₂)₂- | | H |
| CF₃ | CO₂ Bu- t | Me | - (CH₂)₂- | | H |
| CF₃ | CO₂ Bu- t | H | - (CH₂) | | H |
| CF₃ | CO₂ Bu- t | Me | - (CH₂)₃- | | H |
| CF₃ | CO₂ Pen-n | H | H | H | H |
| CF₃ | CO₂ Pen-n | H | Me | H | H |
| CF₃ | CO₂ Pen-n | H | Me | Me | H |
| CF₃ | CO₂ P e n-n | Me | H | H | H |
| CF₃ | CO₂ Pen-n | Me | Me | H | H |
| CF₃ | CO₂ Pen-n | Me | Me | Me | H |
| CF₃ | CO₂ Pen-n | H | -CH₂-O- | | H |
| CF₃ | CO₂ Pen-n | Me | -CH₂-O- | | H |
| CF₃ | CO₂ Pen-n | H | - (CH₂)₂- | | H |
| CF₃ | CO₂ Pen-n | Me | - (CH₂)₂- | | H |
| CF₃ | CO₂ Pen-n | H | - (CH₂)₃ - | | H |
| CF₃ | CO₂ Pen-n | Me | - (CH₂)₃- | | H |
| CF₃ | E - 1 | H | H | H | H |
| CF₃ | E - 1 | H | Me | H | H |
| CF₃ | E - 1 | H | Me | Me | H |
| CF₃ | E - 1 | Me | H | H | H |
| CF₃ | E - 1 | Me | Me | H | H |
| CF₃ | E - 1 | Me | Me | Me | H |
| CF₃ | E - 1 | H | -CH₂-O- | | H |
| CF₃ | E - 1 | Me | -CH₂-O- | | H |
| CF₃ | E - 1 | H | - (CH₂)₂ - | | H |
| CF₃ | E-1 | Me | - (CH₂)₂ - | | H |
| CF₃ | E-1 | H | - (CH₂)₃- | | H |
| CF₃ | E- 1 | Me | - (CH₂)₃ - | | H |
| CF₃ | CO₂Hex-n | H | H | H | H |
| CF₃ | CO₂Hex-n | H | Me | H | H |
| CF₃ | CO₂Hex-n | H | Me | Me | H |
| CF₃ | CO₂Hex-n | Me | H | H | H |
| CF₃ | CO₂Hex-n | Me | Me | H | H |
| CF₃ | CO₂Hex-n | Me | Me | Me | H |
| CF₃ | CO₂Hex-n | H | -CH₂ -O- | | H |
| CF₃ | CO₂Hex-n | Me | -CH₂ -O- | | H |
| CF₃ | CO₂Hex-n | H | - (CH₂)₂ - | | H |
| CF₃ | CO₂Hex-n | Me | - (CH₂)₂ - | | H |
| CF₃ | CO₂Hex-n | H | - (CH₂)₃ - | | H |
| CF₃ | CO₂Hex-n | Me | - (CH₂)₃ - | | H |
| CF₃ | CO₂Ph | H | H | H | H |
| CF₃ | CO₂Ph | H | Me | H | H |
| CF₃ | CO₂Ph | H | Me | Me | H |
| CF₃ | CO₂Ph | Me | H | H | H |
| CF₃ | CO₂Ph | Me | Me | H | H |
| CF₃ | CO₂Ph | Me | Me | Me | H |
| CF₃ | CO₂Ph | H | -CH₂-O- | | H |
| CF₃ | CO₂Ph | Me | -CH₂-O- | | H |
| CF₃ | CO₂Ph | H | -(CH₂)₂- | | H |
| CF₃ | CO₂Ph | Me | -(CH₂)₂- | | H |
| CF₃ | CO₂Ph | H | -(CH₂)₃- | | H |
| CF₃ | CO₂Ph | Me | -(CH₂)₃- | | H |
| CF₃ | CO₂ CH2 Ph | H | H | H | H |
| CF₃ | CO₂ CH₂ Ph | H | Me | H | H |
| CF₃ | CO₂ CH₂ Ph | H | Me | Me | H |
| CF₃ | CO₂ CH₂ Ph | Me | H | H | H |
| CF₃ | CO2 CH₂ Ph | Me | Me | H | H |
| CF₃ | CO₂ CH₂ Ph | Me | Me | Me | H |
| CF₃ | CO₂ CH₂ Ph | H | -CH₂-O- | | H |
| CF₃ | CO₂ CH₂ Ph | Me | -CH₂-O- | | H |
| CF₃ | CO₂ CH₂ Ph | H | -(CH₂)₂- | | H |
| CF₃ | CO₂ CH₂ Ph | Me | -(CH₂)₂- | | H |
| CF₃ | CO₂ CH₂ Ph | H | -(CH₂)₃- | | H |
| CF₃ | CO₂ CH₂ Ph | Me | -(CH₂)₃- | | H |
| CF₃ | E-2 | H | H | H | H |
| CF₃ | E-2 | H | Me | H | H |
| CF₃ | E-2 | H | Me | Me | H |
| CF₃ | E-2 | Me | H | H | H |
| CF₃ | E-2 | Me | Me | H | H |
| CF₃ | E-2 | Me | Me | Me | H |
| CF₃ | E-2 | H | -CH₂-O- | | H |
| CF₃ | E-2 | Me | -CH₂-O- | | H |
| CF₃ | E-2 | H | -(CH₂)₂- | | H |
| CF₃ | E-2 | Me | -(CH₂)₂- | | H |
| CF₃ | E-2 | H | -(CH₂)₃- | | H |
| C F ₃ | E-2 | Me | (CH₂ )₃ - | | H |
| C F ₃ | E-3 | H | H | H | H |
| C F ₃ | E-3 | H | Me | H | H |
| C F ₃ | E-3 | H | Me | Me | H |
| C F ₃ | E-3 | Me | H | H | H |
| C F ₃ | E-3 | Me | Me | H | H |
| C F ₃ | E-3 | Me | Me | Me | H |
| C F ₃ | E-3 | H | -CH₂ -O- | | H |
| C F ₃ | E-3 | Me | -CH₂-O- | | H |
| C F ₃ | E-3 | H | - (CH₂)₂- | | H |
| C F ₃ | E-3 | Me | - (CH₂) ₂ - | | H |
| C F ₃ | E-3 | H | - (CH₂)₃ - | | H |
| C F ₃ | E-3 | Me | - (CH₂) ₃ - | | H |
| C F ₃ | CH₂ OMe | H | H | H | H |
| C F ₃ | CHO₂ OME | H | Me | H | H |
| C F ₃ | CH₂ OMe | H | Me | Me | H |
| C F ₃ | CH₂ OMe | Me | H | H | H |
| C F ₃ | CH₂ OMe | Me | Me | H | H |
| C F ₃ | CH₂ OMe | Me | Me | Me | H |
| C F ₃ | GH₂ OMe | H | -CH₂-O- | | H |
| C F ₃ | CH₂ OMe | Me | - CH₂-O- | | H |
| C F ₃ | CH₂ OMe | H | - (CH₂)- | | H |
| C F ₃ | CH₂ OMe | Me | - (CH₂)₂ - | | H |
| C F ₃ | CH₂ OMe | H | - (CH₂) ₃ - | | H |
| C F ₃ | CH₂ OMe | Me | - (CH₂) ₃ - | | H |
| C F ₃ | COCH₂ Cl | H | H | H | H |
| C F ₃ | COCH₂ Cl | H | Me | H | H |
| C F ₃ | COCH₂ Cl | H | Me | Me | H |
| C F ₃ | COCH₂ Cl | Me | H | H | H |
| C F ₃ | COCH₂ Cl | Me | Me | H | H |
| C F ₃ | COCH₂ Cl | Me | Me | Me | H |
| C F ₃ | COCH₂ Cl | H | -CH₂-O- | | H |
| C F ₃ | COCH₂ Cl | Me | -CH₂-O- | | H |
| C F ₃ | COCH₂ Cl | H | - (CH₂)₂ - | | H |
| C F ₃ | COCH₂ Cl | Me | - (CH₂)₂ - | | H |
| C F ₃ | COCH₂ Cl | H | - (CH₂)₃ - | | H |
| C F ₃ | COCH₂ Cl | Me | - (CH₂)₃ - | | H |
| C F ₃ | E-4 | H | H | H | H |
| C F ₃ | E-4 | H | Me | H | H |
| C F ₃ | E-4 | H | Me | Me | H |
| C F ₃ | E-4 | Me | H | H | H |
| C F ₃ | E-4 | Me | Me | H | H |
| C F ₃ | E-4 | Me | Me | Me | H |
| C F ₃ | E-4 | H | -CH₂ -O- | | H |
| C F ₃ | E-4 | Me | -CH₂-O- | | H |
| C F ₃ | E-4 | H | - (CH₂)₂ - | | H |
| C F ₃ | E-4 | Me | - (GH₂)₂ - | | H |
| CF₃ | E-4 | H | -(CH₂)₃- | | H |
| CF₃ | E-4 | Me | -(CH₂)₃- | | H |
| CF₃ | E-5 | H | H | H | H |
| CF₃ | E-5 | H | Me | H | H |
| CF₃ | E-5 | H | Me | Me | H |
| CF₃ | E-5 | Me | H | H | H |
| CF₃ | E-5 | Me | Me | H | H |
| CF₃ | E-5 | Me | Me | Me | H |
| CF₃ | E-5 | H | -CH-O- | | H |
| CF₃ | E-5 | Me | -CH₂-O- | | H |
| CF₃ | E-5 | H | -(CH₂)₂- | | H |
| CF₃ | E-5 | Me | -(CH₂)₂- | | H |
| CF₃ | E-5 | H | -(CH₂)₃- | | H |
| CF₃ | E-5 | Me | -(CH₂)₃- | | H |
| CF₃ | E-6 | H | H | H | H |
| CF₃ | E-6 | H | Me | H | H |
| CF₃ | E-6 | H | Me | Me | H |
| CF₃ | E-6 | Me | H | H | H |
| CF₃ | E-6 | Me | Me | H | H |
| CF₃ | E-6 | Me | Me | Me | H |
| CF₃ | E-6 | H | -CH₂-O- | | H |
| CF₃ | E-6 | Me | -CH₂-O- | | H |
| CF₃ | E-6 | H | -(CH₂)₂- | | H |
| CF₃ | E-6 | Me | -(CH₂)₂- | | H |
| CF₃ | E-6 | H | -(CH₂)₃- | | H |
| CF₃ | E-6 | Me | -(CH₂)₃- | | H |
| CF₃ | E-7 | H | H | H | H |
| CF₃ | E-7 | H | Me | H | H |
| CF₃ | E-7 | H | Me | Me | H |
| CF₃ | E-7 | Me | H | H | H |
| CF₃ | E-7 | Me | Me | H | H |
| CF₃ | E-7 | Me | Me | Me | H |
| CF₃ | E-7 | H | -CH₂-O- | | H |
| CF₃ | E - 7 | Me | -CH₂-O- | | H |
| CF₃ | E-7 | H | -(CH₂)₂- | | H |
| CF₃ | E-7 | Me | -(CH₂)₂- | | H |
| CF₃ | E-7 | H | -(CH₂)₃- | | H |
| CF₃ | E-7 | Me | -(CH₂)₃- | | H |
| CF₃ | E-8 | H | H | H | H |
| CF₃ | E-8 | H | Me | H | H |
| CF₃ | E-8 | H | Me | Me | H |
| CF₃ | E-8 | Me | H | H | H |
| CF₃ | E-8 | Me | Me | H | H |
| CF₃ | E-8 | Me | Me | Me | H |
| CF₃ | E-8 | H | -CH₂-O- | | H |
| CF₃ | E-8 | Me | -CH₂-O- | | H |
| CF₃ | E-8 | H | -(CH₂)₂- | | H |
| CF₃ | E-8 | Me | -(CH₂)₂- | | H |
| CF₃ | E-8 | H | -(CH₂) ₃- | | H |
| CF₃ | E-8 | Me | -(CH₂)₃- | | H |
| CF₃ | E-9 | H | H | H | H |
| CF₃ | E-9 | H | Me | H | H |
| CF₃ | E-9 | H | Me | Me | H |
| CF₃ | E-9 | Me | H | H | H |
| CF₃ | E-9 | Me | Me | H | H |
| CF₃ | E-9 | Me | Me | Me | H |
| CF₃ | E-9 | H | -CH₂-O- | | H |
| CF₃ | E-9 | Me | -CH₂-O- | | H |
| CF₃ | E-9 | H | -(CH₂)₂- | | H |
| CF₃ | E-9 | Me | -(CH₂)₂- | | H |
| CF₃ | E-9 | H | -(CH₂)₃- | | H |
| CF₃ | E-9 | Me | -(CH₂)₃- | | H |
| CF₃ | E-10 | H | H | H | H |
| CF₃ | E-10 | H | Me | H | H |
| CF₃ | E-10 | H | Me | Me | H |
| CF₃ | E-10 | Me | H | H | H |
| CF₃ | E-10 | Me | Me | H | H |
| CF₃ | E-10 | Me | Me | Me | H |
| CF₃ | E-10 | H | -CH₂-O- | | H |
| CF₃ | E-10 | Me | -CH₂-O- | | H |
| CF₃ | E-10 | H | -(CH₂)₂- | | H |
| CF₃ | E-10 | Me | -(CH₂)₂- | | H |
| CF₃ | E-10 | H | -(CH₂)₃- | | H |
| CF₃ | E-10 | Me | -(CH₂)₃- | | H |
| CF₃ | E-11 | H | H | H | H |
| CF₃ | E-11 | H | Me | H | H |
| CF₃ | E-11 | H | Me | Me | H |
| CF₃ | E-11 | Me | H | H | H |
| CF₃ | E-11 | Me | Me | H | H |
| CF₃ | E-11 | Me | Me | Me | H |
| CF₃ | E-11 | H | -CH-O- | | H |
| CF₃ | E-11 | Me | -CH₂-O- | | H |
| CF₃ | E-11 | H | -(CH₂)₂- | | H |
| CF₃ | E-11 | Me | -(CH₂)₂- | | H |
| CF₃ | E-11 | H | -(CH₂)₃- | | H |
| CF₃ | E-11 | Me | -(CH₂)₃- | | H |
| CF₃ | E-12 | H | H | H | H |
| CF₃ | E-12 | H | Me | H | H |
| CF₃ | E-12 | H | Me | Me | H |
| CF₃ | E-12 | Me | H | H | H |
| CF₃ | E-12 | Me | Me | H | H |
| CF₃ | E-12 | Me | Me | Me | H |
| CF₃ | E-12 | H | -CH₂-O- | | H |
| CF₃ | E-12 | Me | -CH₂-O- | | H |
| CF₃ | E-12 | H | -(CH₂)₂- | | H |
| CF₃ | E-12 | Me | -(CH₂)₂- | | H |
| CF₃ | E-12 | H | -(CH₂)₃- | | H |
| CF₃ | E-12 | Me | -(CH₂)₃- | | H |
| CF₃ | CH₂SMe | H | H | H | H |
| CF₃ | CH₂SMe | H | Me | H | H |
| CF₃ | CH₂SMe | H | Me | Me | H |
| CF₃ | CH₂SMe | Me | H | H | H |
| CF₃ | CH₂SMe | Me | Me | H | H |
| CF₃ | CH₂SMe | Me | Me | Me | H |
| CF₃ | CH₂SMe | H | -CH₂-O- | | H |
| CF₃ | GH₂SMe | Me | -CH₂-O- | | H |
| CF₃ | CH₂SMe | H | -(CH₂)₂- | | H |
| CF₃ | CH₂SMe | Me | -(CH₂)₂- | | H |
| CF₃ | CH₂SMe | H | -(CH₂)₃- | | H |
| CF₃ | CH₂SMe | Me | -(CH₂)₃- | | H |
| CF₃ | CH₂OCH₂Ph | H | H | H | H |
| CF₃ | CH₂OCH₂Ph | H | Me | H | H |
| CF₃ | CH₂OCH₂Ph | H | Me | Me | H |
| CF₃ | CH₂OCH₂Ph | Me | H | H | H |
| CF₃ | CH₂OCH₂Ph | Me | Me | H | H |
| CF₃ | CH₂OCH₂Ph | Me | Me | Me | H |
| CF₃ | CH₂OCH₂Ph | H | -CH₂-O- | | H |
| CF₃ | CH₂OCH₂Ph | Me | -CH₂-O- | | H |
| CF₃ | CH₂OCH₂Ph | H | -(CH₂)₂- | | H |
| CF₃ | CH₂OCH₂Ph | Me | -(CH₂)₂- | | H |
| CF₃ | CH₂OCH₂Ph | H | -(CH₂)₃- | | H |
| CF₃ | CH₂OCH₂Ph | Me | -(CH₂)₃ | | H |
| CF₃ | CH₂OCOMe | H | H | H | H |
| CF₃ | CH₂OCOMe | H | Me | H | H |
| CF₃ | CH₂OCOMe | H | Me | Me | H |
| CF₃ | CH₂OCOMe | Me | H | H | H |
| CF₃ | CH₂OCOMe | Me | Me | H | H |
| CF₃ | CH₂OCOME | Me | Me | Me | H |
| CF₃ | CH₂OCOMe | H | -CH₂-O- | | H |
| CF₃ | CH₂OCOME | Me | -CH₂-O- | | H |
| CF₃ | CH₂OCOME | H | -(CH₂)₂- | | H |
| CF₃ | CH₂OCOMe | Me | -(CH₂)₂- | | H |
| CF₃ | CH₂OCOMe | H | -(CH₂)₃- | | H |
| CF₃ | CH₂OCOMe | Me | -(CH₂)₃- | | H |
| CF₃ | CH₂OCOEt | H | H | H | H |
| CF₃ | CH₂OCOEt | H | Me | H | H |
| CF₃ | CH₂OCOEt | H | Me | Me | H |
| CF₃ | CH₂OCOEt | Me | H | H | H |
| CF₃ | CH₂OCOEt | Me | Me | H | H |
| CF₃ | CH₂OCOEt | Me | Me | Me | H |
| CF₃ | CH₂OCOEt | H | -CH₂-O- | | H |
| CF₃ | CH₂OCOEt | Me | -CH₂-O- | | H |
| CF₃ | CH₂OCOEt | H | -(CH₂)₂- | | H |
| CF₃ | CH₂OCOEt | Me | -(CH₂)₂- | | H |
| CF₃ | CH₂OCOEt | H | -(CH₂)₃- | | H |
| CF₃ | CH₂OCOEt | Me | -(CH₂)₃- | | H |
| CF₃ | COSMe | H | H | H | H |
| CF₃ | COSMe | H | Me | H | H |
| CF₃ | COSMe | H | Me | Me | H |
| CF₃ | COSMe | Me | H | H | H |
| CF₃ | COSMe | Me | Me | H | H |
| CF₃ | COSMe | Me | Me | Me | H |
| CF₃ | COSMe | H | -CH₂-O- | | H |
| CF₃ | COSMe | Me | -CH₂-O- | | H |
| CF₃ | COSMe | H | -(CH₂)₂- | | H |
| CF₃ | COSMe | Me | -(CH₂)₂- | | H |
| CF₃ | COSMe | H | -(CH₂)₃- | | H |
| CF₃ | COSMe | Me | -(CH₂)₃- | | H |
| CF₃ | COSEt | H | H | H | H |
| CF₃ | COSEt | H | Me | H | H |
| CF₃ | COSEt | H | Me | Me | H |
| CF₃ | COSEt | Me | H | H | H |
| CF₃ | COSEt | Me | Me | H | H |
| CF₃ | COSEt | Me | Me | Me | H |
| CF₃ | COSEt | H | -CH₂-O- | | H |
| CF₃ | COSEt | Me | -CH₂-O- | | H |
| CF₃ | COSEt | H | -(CH₂)₂- | | H |
| CF₃ | COSEt | Me | -(CH₂)₂- | | H |
| CF₃ | COSEt | H | -(CH₂)₃- | | H |
| CF₃ | COSEt | Me | -(CH₂)₃- | | H |
| CF₃ | CH₂SO₂Me | H | H | H | H |
| CF₃ | CH₂SO₂Me | H | Me | H | H |
| CF₃ | CH₂SO₂Me | H | Me | Me | H |
| CF₃ | CH₃SO₂Me | Me | H | H | H |
| CF₃ | CH₂SO₂Me | Me | Me | H | H |
| CF₃ | CH₂SO₂Me | Me | Me | Me | H |
| CF₃ | CH₂SO₂Me | H | -CH₂-O- | | H |
| CF₃ | GH₂SO₂Me | Me | -CH₂-O- | | H |
| CF₃ | CH₂SO₂Me | H | -(CH₂)₂- | | H |
| CF₃ | CH₂SO₂Me | Me | -(CH₂)₂- | | H |
| CF₃ | CH₂SO₂Me | H | -(CH₂)₃- | | H |
| CF₃ | CH₂SO₂Me | Me | -(CH₂)₃- | | H |
| CF₃ | CH₂Ph | H | H | H | H |
| CF₃ | CH₂Ph | H | Me | H | H |
| CF₃ | CH₂Ph | H | Me | Me | H |
| CF₃ | CH₂Ph | Me | H | H | H |
| CF₃ | CH₂Ph | Me | Me | H | H |
| CF₃ | CH₂Ph | Me | Me | Me | H |
| CF₃ | CH₂ Ph | H | -CH₂-O- | | H |
| CF₃ | CH₂ Ph | Me | -CH₂-O- | | H |
| CF₃ | CH₂ Ph | H | - (CH₂)₂- | | H |
| CF₃ | CH₂ Ph | Me | - (CH₂)₂- | | H |
| CF₃ | CH₂ Ph | H | - (CH₂)₃- | | H |
| CF₃ | CH₂ Ph | Me | - (CH₂)₃- | | H |
| C F | SO₂ CF₃ | H | H | H | H |
| CF₃ | SO₂ CF₃ | H | Me | H | H |
| CF₃ | SO₂ CF₃ | H | Me | Me | H |
| CF₃ | SO₂ CF₃ | Me | H | H | H |
| CF₃ | SO₂ CF₃ | Me | Me | H | H |
| CF₃ | SO₂ CF₃ | Me | Me | Me | H |
| CF₃ | SO₂ CF₃ | SO₂ CF₃ | H | H | H |
| CF₃ | SO₂ CF₃ | H | Et | H | H |
| CF₃ | SO₂ CF₃ | Me | Et | H | H |
| CF₃ | SO₂ CF₃ | H | Et | Me | H |
| CF₃ | SO₂ CF₃ | Me | Et | Me | H |
| CF₃ | SO₂ CF₃ | H | Pr-n | H | H |
| CF₃ | SO₂ CF₃ | Me | Pr-n | H | H |
| CF₃ | SO₂ CF₃ | H | Cl | H | H |
| CF₃ | SO₂ CF₃ | Me | Cl | H | H |
| CF₃ | SO₂ CF₃ | H | CF₃ | H | H |
| CF₃ | SO₂ CF₃ | Me | CF₃ | H | H |
| CF₃ | SO₂ CF₃ | H | Ph | H | H |
| CF₃ | SO₂ CF₃ | Me | Ph | H | H |
| CF₃ | SO₂ CF₃ | H | Ph-2-Cl | H | H |
| CF₃ | SO₂ CF₃ | Me | Ph-2-Cl | H | H |
| CF₃ | SO₂ CF₃ | H | Ph-3-Cl | H | H |
| CF₃ | SO₂ CF₃ | Me | Ph-3-Cl | H | H |
| CF₃ | SO₂ CF₃ | H | Ph-4-Cl | H | H |
| CF₃ | SO₂ CF₃ | Me | Ph-4-Cl | H | H |
| CF₃ | SO₂ CF₃ | H | COMe | H | H |
| CF₃ | SO₂ CF₃ | Me | COMe | H | H |
| CF₃ | SO₂ CF₃ | H | CO₂Me | H | H |
| CF₃ | SO₂ CF₃ | Me | CO₂Me | H | H |
| CF₃ | SO₂ CF₃ | H | OMe | H | H |
| CF₃ | SO₂ CF₃ | Me | OMe | H | H |
| CF₃ | SO₂ CF₃ | H | OMe | Me | H |
| CF₃ | SO₂ CF₃ | Me | OMe | Me | H |
| CF₃ | SO₂ CF₃ | H | SMe | H | H |
| CF₃ | SO₂ CF₃ | Me | SMe | H | H |
| CF₃ | SO₂ CF₃ | H | NHMe | H | H |
| CF₃ | SO₂ CF₃ | Me | NHMe | H | H |
| CF₃ | SO₂ CF₃ | H | CH₂ Ph | H | H |
| CF₃ | SO₂ CF₃ | Me | CH₂ Ph | H | H |
| CF₃ | SO₂ CF₃ | H | CH₂ Ph-2-Cl | H | H |
| CF₃ | SO₂ CF₃ | Me | CH₂ Ph-2-Cl | H | H |
| CF₃ | SO₂ CF₃ | H | CH₂Ph-3-Cl | H | H |
| CF₃ | SO₂ CF₃ | Me | CH₂Ph-3-Cl | H | H |
| CF₃ | SO₂ CF₃ | H | CH₂Ph-4-Cl | H | H |
| CF₃ | SO₂ CF₃ | Me | CH₂Ph-4-Cl | H | H |
| CF₃ | SO₂ CF₃ | H | COPh | H | H |
| CF₃ | SO₂ CF₃ | Me | COPh | H | H |
| CF₃ | SO₂ CF₃ | H | COPh-2-Cl | H | H |
| CF₃ | SO₂ CF₃ | Me | COPh-2-Cl | H | H |
| CF₃ | SO₂ CF₃ | H | COPh-3-Cl | H | H |
| CF₃ | SO₂ CF₃ | Me | COPh-3-Cl | H | H |
| CF₃ | SO₂ CF₃ | H | COPh-4-Cl | H | H |
| CF₃ | SO₂ CF₃ | Me | COPh-4-Cl | H | H |
| CF₃ | SO₂ CF₃ | H | -CH₂-O- | | H |
| CF₃ | SO₂ CF₃ | Me | -CH₂-O- | | H |
| CF₃ | SO₂ CF₃ | H | - (CH₂)₂- | | H |
| C F ₃ | SO₂ CF₃ | Me | - (CH₂)₂- | | H |
| CF₃ | SO₂ CF₃ | H | - (CH₂)₃- | | H |
| CF₃ | SO₂ CF₃ | Me | - (CH₂)₃- | | H |
| CHF₂ | H | H | H | H | H |
| CHF₂ | H | H | Me | H | H |
| CHF₂ | H | H | Me | Me | H |
| CHF₂ | H | Me | H | H | H |
| CHF₂ | H | Me | Me | H | H |
| CHF₂ | H | Me | Me | Me | H |
| CHF₂ | H | H | - (CH₂)₂- | | H |
| CHF₂ | H | Me | - (CH₂)₂- | | H |
| CHF₂ | H | H | - (CH₂)₃- | | H |
| CHF₂ | H | Me | - (CH₂)₃- | | H |
| CHF₂ | CO Et | H | H | H | H |
| CHF₂ | CO Et | H | Me | H | H |
| CHF₂ | CO Et | H | Me | Me | H |
| CHF₂ | CO Et | Me | H | H | H |
| CHF₂ | COEt | Me | Me | H | H |
| CHF₂ | CO Et | Me | Me | Me | H |
| CHF₂ | CO Et | H | - (CH₂)₂- | | H |
| CHF₂ | CO Et | Me | - (CH₂)₂- | | H |
| CHF₂ | CO Et | H | - (CH₂)₃- | | H |
| CHF₂ | CO Et | Me | - (CH₂)₃- | | H |
| CHF₂ | CO₂ Et | H | H | H | H |
| CHF₂ | CO₂ Et | H | Me | H | H |
| CHF₂ | CO₂ Et | H | Me | Me | H |
| CHF₂ | CO₂ Et | Me | H | H | H |
| CHF₂ | CO₂ Et | Me | Me | H | H |
| CHF₂ | CO₂ Et | Me | Me | Me | H |
| CHF₂ | CO₂ Et | H | - (CH₂)₂- | | H |
| CHF₂ | CO₂ Et | Me | - (CH₂)₂- | | H |
| CHF₂ | CO₂ Et | H | - (CH₂)₃- | | H |
| CHF₂ | CO₂ Et | Me | - (CH₂)₃- | | H |
| CHF₂ | CO₂ Bu-i | H | H | H | H |
| CHF₂ | CO₂ Bu-i | H | Me | H | H |
| CHF₂ | CO₂ Bu-i | H | Me | Me | H |
| CHF₂ | CO₂ Bu-i | Me | H | H | H |
| CHF₂ | CO₂ Bu-i | Me | Me | H | H |
| CHF₂ | CO₂ Bu-i | Me | Me | Me | H |
| CHF₂ | CO₂ Bu-i | H | - (CH₂)₂- | | H |
| CHF₂ | CO₂ Bu-i | Me | - (CH₂)₂- | | H |
| CHF₂ | CO₂ Bu-i | H | - (CH₂)₃- | | H |
| CHF₂ | CO₂ Bu-i | Me | - (CH₂)₃- | | H |
| CClF₂ | H | H | H | H | H |
| CClF₂ | H | H | Me | H | H |
| CClF₂ | H | H | Me | Me | H |
| CClF₂ | H | Me | H | H | H |
| CClF₂ | H | Me | Me | H | H |
| CClF₂ | H | Me | Me | Me | H |
| CClF₂ | H | H | - (CH₂)₂ - | | H |
| CClF₂ | H | Me | - (CH₂)₂ - | | H |
| CClF₂ | H | H | - (CH₂)₃ - | | H |
| CClF₂ | H | Me | - (CH₂)₃ - | | H |
| CClF₂ | CO Et | H | H | H | H |
| CClF₂ | CO Et | H | Me | H | H |
| CClF₂ | CO Et | H | Me | Me | H |
| CClF₂ | CO Et | Me | H | H | H |
| CClF₂ | CO Et | Me | Me | H | H |
| CClF₂ | CO Et | Me | Me | Me | H |
| CClF₂ | CO Et | H | -(CH₂)₂- | | H |
| CClF₂ | CO Et | Me | -(CH₂)₂- | | H |
| CClF₂ | CO Et | H | -(CH₂)₃- | | H |
| CClF₂ | CO Et | Me | -(CH₂)₃- | | H |
| CClF₂ | CO₂ Et | H | H | H | H |
| CClF₂ | CO₂ Et | H | Me | H | H |
| CClF₂ | CO₂ Et | H | Me | Me | H |
| CClF₂ | CO₂ Et | Me | H | H | H |
| CClF₂ | CO₂ Et | Me | Me | H | H |
| CClF₂ | CO₂ Et | Me | Me | Me | H |
| CClF₂ | CO₂ Et | H | - (CH₂)₂- | | H |
| CClF₂ | CO₂ Et | Me | - (CH₂)₂- | | H |
| CClF₂ | CO₂ Et | H | - (CH₂)₃- | | H |
| CClF₂ | CO₂ Et | Me | - (CH₂)₃- | | H |
| CClF₂ | CO₂ Bu-i | H | H | H | H |
| CClF₂ | CO₂ Bu-i | H | Me | H | H |
| CClF₂ | CO₂ Bu-i | H | Me | Me | H |
| CClF₂ | CO₂ Bu-i | Me | H | H | H |
| CClF₂ | CO₂ Bu-i | Me | Me | H | H |
| CClF₂ | CO₂ Bu-i | Me | Me | Me | H |
| CClF₂ | CO₂Bu-i | H | - (CH₂)₂- | | H |
| CClF₂ | CO₂ Bu-i | Me | - (CH₂)₂- | | H |
| CClF₂ | CO₂Bu-i | H | - (CH₂)₃- | | H |
| CClF₂ | CO₂ Bu-i | Me | - (CH₂)₃- | | H |
| CBrF₂ | H | H | H | H | H |
| CBrF₂ | H | H | Me | H | H |
| CBrF₂ | H | H | Me | Me | H |
| CBrF₂ | H | Me | H | H | H |
| CBrF₂ | H | Me | Me | H | H |
| CBrF₂ | H | Me | Me | Me | H |
| CBrF₂ | H | H | - (CH₂)₂ - | | H |
| CBrF₂ | H | Me | - (CH₂)₂ - | | H |
| CBrF₂ | H | H | - (CH₂)₃ - | | H |
| CBrF₂ | H | Me | - (CH₂)₃ - | | H |
| CBrF₂ | CO Et | H | H | H | H |
| CBrF₂ | CO Et | H | Me | H | H |
| CBrF₂ | CO Et | H | Me | Me | H |
| CBrF₂ | CO Et | Me | H | H | H |
| CBrF₂ | CO Et | Me | Me | H | H |
| CBrF₂ | CO Et | Me | Me | Me | H |
| CBrF₂ | CO Et | H | - (CH₂)₂ - | | H |
| CBrF₂ | CO Et | Me | - (CH₂)₂ - | | H |
| CBrF₂ | CO Et | H | - (CH₂)₃ - | | H |
| CBrF₂ | CO Et | Me | - (CH₂)₃ - | | H |
| CBrF₂ | CO₂ Et | H | H | H | H |
| CBrF₂ | CO₂ Et | H | Me | H | H |
| CBrF₂ | CO₂ Et | H | Me | Me | H |
| CBrF₂ | CO₂ Et | Me | H | H | H |
| CBrF₂ | CO₂ Et | Me | Me | H | H |
| CBrF₂ | CO₂ Et | Me | Me | Me | H |
| CBrF₂ | CO₂ Et | H | - (CH₂)₂ - | | H |
| CBrF₂ | CO₂ Et | Me | - (CH₂)₂ - | | H |
| CBrF₂ | CO₂ Et | H | - (CH₂)₃ - | | H |
| CBrF₂ | CO₂ Et | Me | - (CH₂)₃ - | | H |
| CBrF₂ | CO₂ Bu-i | H | H | H | H |
| CBrF₂ | CO₂ Bu-i | H | Me | H | H |
| CBrF₂ | CO₂ Bu-i | H | Me | Me | H |
| CBrF₂ | CO₂ Bu-i | Me | H | H | H |
| CBrF₂ | CO₂ Bu-i | Me | Me | H | H |
| CBrF₂ | CO₂ Bu-i | Me | Me | Me | H |
| CBrF₂ | CO₂ Bu-i | H | - (CH₂)₂ - | | H |
| CBrF₂ | CO₂ Bu-i | Me | - (CH₂)₂ - | | H |
| CBrF₂ | CO₂ Bu-i | H | - (CH₂)₃ - | | H |
| CBrF₂ | CO₂ Bu-i | Me | - (CH₂)₃ - | | H |
| CCl₂F | H | H | H | H | H |
| CCl₂F | H | H | Me | H | H |
| CCl₂F | H | H | Me | Me | H |
| CCl₂F | H | Me | H | H | H |
| CCl₂F | H | Me | Me | H | H |
| CCl₂F | H | Me | Me | Me | H |
| CCl₂F | H | H | - (CH₂)₂ - | | H |
| CCl₂F | H | Me | - (CH₂)₂ - | | H |
| CCl₂F | H | H | - (CH₂)₃ - | | H |
| CCl₂F | H | Me | - (CH₂)₃ - | | H |
| CCl₂F | COE t | H | H | H | H |
| CCl₂F | COEt | H | Me | H | H |
| CCl₂F | COE t | H | Me | Me | H |
| CCl₂F | COEt | Me | H | H | H |
| CCl₂F | COE t | Me | Me | H | H |
| CCl₂F | COE t | Me | Me | Me | H |
| CCl₂F | CO Et | H | - (CH₂)₂ - | | H |
| CCl₂F | CO Et | Me | - (CH₂)₂ - | | H |
| CCl₂F | CO Et | H | - (CH₂)₃ - | | H |
| CCl₂F | CO Et | Me | - (CH₂)₃ - | | H |
| CCl₂F | CO₂ Et | H | H | H | H |
| CCl₂F | CO₂ Et | H | Me | H | H |
| CCl₂F | CO₂ Et | H | Me | Me | H |
| CCl₂F | CO₂ Et | Me | H | H | H |
| CCl₂F | CO₂ Et | Me | Me | H | H |
| CCl₂F | CO₂ Et | Me | Me | Me | H |
| CCl₂F | CO₂ Et | H | - (CH₂)₂ - | | H |
| CCl₂F | CO₂ Et | Me | - (CH₂)₂ - | | H |
| CCl₂F | CO₂ Et | H | - (CH₂)₃ - | | H |
| CCl₂F | CO₂ Et | Me | - (CH₂)₃ - | | H |
| CCl₂F | CO₂ Bu-i | H | H | H | H |
| CCl₂F | CO₂ Bu-i | H | Me | H | H |
| CCl₂F | CO₂ Bu-i | H | Me | Me | H |
| CCl₂F | CO₂ Bu-i | Me | H | H | H |
| CCl₂F | CO₂ Bu-i | Me | Me | H | H |
| CCl₂F | CO₂ Bu-i | Me | Me | Me | H |
| CCl₂F | CO₂ Bu-i | H | - (CH₂)₂ - | | H |
| CCl₂F | CO₂ Bu-i | Me | - (CH₂)₂ - | | H |
| CCl₂F | CO₂ Bu-i | H | - (CH₂)₃ - | | H |
| CCl₂F | CO₂ Bu-i | Me | - (CH₂)₃ - | | H |
| CBr₂F | H | H | H | H | H |
| CBr₂F | H | H | Me | H | H |
| CBr₂F | H | H | Me | Me | H |
| CBr₂F | H | Me | H | H | H |
| CBr₂F | H | Me | Me | H | H |
| CBr₂F | H | Me | Me | Me | H |
| CBr₂F | H | H | - (CH₂)₂- | | H |
| CBr₂F | H | Me | - (CH₂)₂- | | H |
| CBr₂F | H | H | - (CH₂)₂- | | H |
| CBr₂F | H | Me | - (CH₂)₃- | | H |
| CBr₂F | CO₂Et | H | H | H | H |
| CBr₂F | CO₂Et | H | Me | H | H |
| CBr₂F | CO₂Et | H | Me | Me | H |
| CBr₂F | CO₂Et | Me | H | H | H |
| CBr₂F | CO₂Et | Me | Me | H | H |
| CBr₂F | CO₂Et | Me | Me | Me | H |
| CBr₂F | CO₂Et | H | -(CH₂)₂- | | H |
| CBr₂F | CO₂Et | Me | -(CH₂)₂- | | H |
| CBr₂F | CO₂Et | H | -(CH₂)₃- | | H |
| CBr₂F | CO₂Et | Me | -(CH₂)₃- | | H |
| CCl₃ | H | H | H | H | H |
| CCl₃ | H | H | Me | H | H |
| CCl₃ | H | H | Me | Me | H |
| CCl₃ | H | Me | H | H | H |
| CCl₃ | H | Me | Me | H | H |
| CCl₃ | H | Me | Me | Me | H |
| CCl₃ | H | H | -(CH₂)₂- | | H |
| CCl₃ | H | Me | -(CH₂)₂- | | H |
| CCl₃ | H | H | -(CH₂)₃- | | H |
| CCl₃ | H | Me | -(CH₂)₃- | | H |
| CCl₃ | CO₂Et | H | H | H | H |
| CCl₃ | CO₂Et | H | Me | H | H |
| CCl₃ | CO₂Et | H | Me | Me | H |
| CCl₃ | CO₂Et | Me | H | H | H |
| CCl₃ | CO₂Et | Me | Me | H | H |
| CCl₃ | CO₂Et | Me | Me | Me | H |
| CCl₃ | CO₂Et | H | -(CH₂)₂- | | H |
| CCl₃ | CO₂Et | Me | -(CH₂)₂- | | H |
| CCl₃ | CO₂Et | H | -(CH₂)₃- | | H |
| CCl₃ | CO₂Et | Me | -(CH₂)₃- | | H |

In Table 3, E-1 to E-12 are the same as defined above.

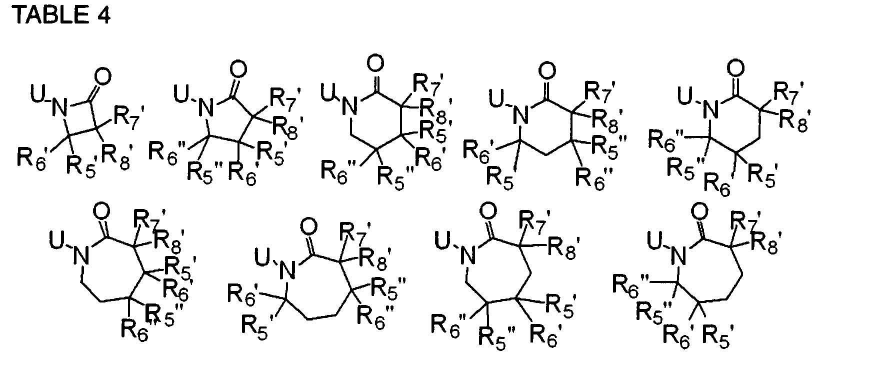

U is the same as the above-mentioned formula (26).

| R₁ =C F ₃ , R ₃ =R ₄ =H, X=H | | | | | | |
|---|---|---|---|---|---|---|
| R ₂ | R₅' | R₆' | R₅'' | R₆" | R₇' | R₈' |
| H | Me | H | H | H | Me | Me |
| H | Me | Me | H | H | Me | H |
| H | Me | H | Me | H | Me | H |
| H | Me | Me | Me | H | H | H |
| H | Me | Me | H | H | Me | Me |
| H | Me | H | Me | H | Me | Me |
| H | Me | Me | Me | H | H | Me |
| H | Me | Me | Me | Me | H | H |
| H | Me | H | H | H | Me | Et |
| H | Me | Et | H | H | Me | H |
| H | Et | H | H | H | Me | Me |
| H | Me | Me | H | H | Et | H |
| H | Me | Me | OH | H | H | H |
| H | H | bond | | H | H | H |
| H | H | -CH₂- | | H | H | H |
| H | H | -(CH₂)₂- | | H | H | H |
| H | H | -O- | | H | H | H |
| H | Me | bond | | H | H | H |
| H | Me | -CH₂- | | H | H | H |
| H | Me | -(CH₂)₂- | | H | H | H |
| H | Me | -O- | | H | H | H |
| COMe | Me | H | H | H | Me | Me |
| COMe | Me | Me | H | H | Me | H |
| COMe | Me | H | Me | H | Me | H |
| COMe | Me | Me | Me | H | H | H |
| COMe | Me | Me | H | H | Me | Me |
| COMe | Me | H | Me | H | Me | Me |
| COMe | Me | Me | Me | H | H | Me |
| COMe | Me | Me | Me | Me | H | H |
| COMe | H | bond | | H | H | H |
| COMe | H | -CH₂- | | H | H | H |
| COMe | H | -(CH₂)₂- | | H | H | H |
| COMe | H | -O- | | H | H | H |
| COEt | Me | H | H | H | Me | Me |
| COEt | Me | Me | H | H | Me | H |
| COEt | Me | H | Me | H | Me | H |
| COEt | Me | Me | Me | H | H | H |
| COEt | Me | Me | H | H | Me | Me |
| COEt | Me | H | Me | H | Me | Me |
| COEt | Me | Me | Me | H | H | Me |
| COEt | Me | Me | Me | Me | H | H |
| COEt | Me | H | H | H | Me | Et |
| COEt | Me | Et | H | H | Me | H |
| COEt | Et | H | H | H | Me | Me |
| COEt | Me | Me | H | H | Et | H |
| COEt | Me | Me | OH | H | H | H |
| COEt | H | bond | | H | H | H |
| COEt | H | -CH₂- | | H | H | H |
| COEt | H | -(CH₂)₂- | | H | H | H |
| COEt | H | -O- | | H | H | H |
| COEt | Me | bond | | H | H | H |
| COEt | Me | -CH₂- | | H | H | H |
| COEt | Me | -(CH₂)₂- | | H | H | H |
| COEt | Me | -O- | | H | H | H |
| COPr-n | Me | H | H | H | Me | Me |
| COPr-n | Me | Me | H | H | Me | H |
| COPr-n | Me | Me | Me | H | H | H |
| COPr-n | Me | H | Me | H | Me | H |
| COPr-n | Me | Me | H | H | Me | Me |
| COPr-n | Me | H | Me | H | Me | Me |
| COPr-n | Me | Me | Me | H | H | Me |
| COPr-n | Me | Me | Me | Me | H | H |
| COPr-n | H | bond | | H | H | H |
| COPr-n | H | -CH₂- | | H | H | H |
| COPr-n | H | -(CH₂)₂- | | H | H | H |
| COPr-n | H | -O- | | H | H | H |
| COPr-i | Me | H | H | H | Me | Me |
| COPr-i | Me | Me | H | H | Me | H |
| COPr-i | Me | Me | Me | H | H | H |
| COPr-i | Me | H | Me | H | Me | H |
| COPr-i | Me | Me | H | H | Me | Me |
| COPr-i | Me | H | Me | H | Me | Me |
| COPr-i | Me | Me | Me | H | H | Me |
| COPr-i | Me | Me | Me | Me | H | H |
| COPr-i | H | bond | | H | H | H |
| COPr-i | H | -CH₂- | | H | H | H |
| COPr-i | H | -(CH₂)₂- | | H | H | H |
| COPr-i | H | -O- | | H | H | H |
| COPr-c | Me | H | H | H | Me | Me |
| COPr-c | Me | Me | H | H | Me | H |
| COPr-c | Me | Me | Me | H | H | H |
| COPr-c | Me | H | Me | H | Me | H |
| COPr-c | Me | Me | H | H | Me | Me |
| COPr-c | Me | H | Me | H | Me | Me |
| COPr-c | Me | Me | Me | H | H | Me |
| COPr-c | Me | Me | Me | Me | H | H |
| COPr-c | H | bond | | H | H | H |
| COPr-c | H | -CH₂- | | H | H | H |
| COPr-c | H | -(CH₂)₂- | | H | H | H |
| COPr-c | H | -O- | | H | H | H |
| COBu-t | Me | H | H | H | Me | Me |
| COBu-t | Me | Me | H | H | Me | H |
| COBu-t | Me | Me | Me | H | H | H |
| COBu-t | Me | H | Me | H | Me | H |
| COBu-t | Me | Me | H | H | Me | Me |
| COBu-t | Me | H | Me | H | Me | Me |
| COBu-t | Me | Me | Me | H | H | Me |
| COBu-t | Me | Me | Me | Me | H | H |
| COBu-t | H | bond | | H | H | H |
| COBu-t | H | -CH₂- | | H | H | H |
| COBu-t | H | -(CH₂)₂- | | H | H | H |
| COBu-t | H | -O- | | H | H | H |
| COPr-c | H | -(CH₂)₂- | | H | H | H |
| COPr-c | H | -O- | | H | H | H |
| COPh | Me | H | H | H | Me | Me |
| COPh | Me | Me | H | H | Me | H |
| COPh | Me | Me | Me | H | H | H |
| COPh | Me | H | Me | H | Me | H |
| COPh | Me | Me | H | H | Me | Me |
| COPh | Me | H | Me | H | Me | Me |
| COPh | Me | Me | Me | H | H | Me |
| COPh | Me | Me | Me | Me | H | H |
| COPh | H | bond | | H | H | H |
| COPh | H | -CH₂- | | H | H | H |
| COPh | H | -(CH₂)₂- | | H | H | H |
| COPh | H | -O- | | H | H | H |
| CO₂Me | Me | H | H | H | Me | Me |
| CO₂Me | Me | Me | H | H | Me | H |
| CO₂Me | Me | Me | Me | H | H | H |
| CO₂Me | Me | H | Me | H | Me | H |
| CO₂Me | Me | Me | H | H | Me | Me |
| CO₂Me | Me | H | Me | H | Me | Me |
| CO₂Me | Me | Me | Me | H | H | Me |
| CO₂Me | Me | Me | Me | Me | H | H |
| CO₂Me | H | bond | | H | H | H |
| CO₂Me | H | -CH₂- | | H | H | H |
| CO₂Me | H | -(CH₂)₂- | | H | H | H |
| CO₂Me | H | -O- | | H | H | H |
| CO₂Et | Me | H | H | H | Me | Me |
| CO₂Et | Me | Me | H | H | Me | H |
| CO₂Et | Me | H | Me | H | Me | H |
| CO₂Et | Me | Me | Me | H | H | H |
| CO₂Et | Me | Me | H | H | Me | Me |
| CO₂Et | Me | H | Me | H | Me | Me |
| CO₂Et | Me | Me | Me | H | H | Me |
| CO₂Et | Me | Me | Me | Me | H | H |
| CO₂Et | Me | H | H | H | Me | E t |
| CO₂Et | Me | E t | H | H | Me | H |
| CO₂Et | E t | H | H | H | Me | Me |
| CO₂Et | Me | Me | H | H | E t | H |
| CO₂Et | Me | Me | O H | H | H | H |
| CO₂Et | H | bond | | H | H | H |
| CO₂Et | H | -CH₂- | | H | H | H |
| CO₂Et | H | -(CH₂)₂- | | H | H | H |
| CO₂Et | H | -O- | | H | H | H |
| CO₂Et | Me | bond | | H | H | H |
| CO₂Et | Me | -CH₂- | | H | H | H |
| CO₂Et | Me | -(CH₂)₂- | | H | H | H |
| CO₂Et | Me | -O- | | H | H | H |
| CO₂Pr-n | Me | H | H | H | Me | Me |
| CO₂Pr-n | Me | Me | H | H | Me | H |
| CO₂Pr-n | Me | Me | Me | H | H | H |
| CO₂Pr-n | Me | H | Me | H | Me | H |
| CO₂Pr-n | Me | Me | H | H | Me | Me |
| CO₂Pr-n | Me | H | Me | H | Me | Me |
| CO₂Pr-n | Me | Me | Me | H | H | Me |
| CO₂Pr-n | Me | Me | Me | Me | H | H |
| CO₂Pr-n | H | bond | | H | H | H |
| CO₂Pr-n | H | -CH₂- | | H | H | H |
| CO₂Pr-n | H | -(CH₂)₂- | | H | H | H |
| CO₂Pr-n | H | -O- | | H | H | H |
| CO₂Pr-i | Me | H | H | H | Me | Me |
| CO₂Pr-i | Me | Me | H | H | Me | H |
| CO₂Pr-i | Me | Me | Me | H | H | H |
| CO₂Pr-i | Me | H | Me | H | Me | H |
| CO₂Pr-i | Me | Me | H | H | Me | Me |
| CO₂Pr-i | Me | H | Me | H | Me | Me |
| CO₂Pr-i | Me | Me | Me | H | H | Me |
| CO₂Pr-i | Me | Me | Me | Me | H | H |
| CO₂Pr-i | H | bond | | H | H | H |
| CO₂Pr-i | H | -CH₂- | | H | H | H |
| CO₂Pr-i | H | -(CH₂)₂- | | H | H | H |
| CO₂Pr-i | H | -O- | | H | H | H |
| CO₂Bu-i | Me | H | H | H | Me | Me |
| CO₂Bu-i | Me | Me | H | H | Me | H |
| CO₂Bu-i | Me | Me | Me | H | H | H |
| CO₂Bu-i | Me | H | Me | H | Me | H |
| CO₂Bu-i | Me | Me | H | H | Me | Me |
| CO₂Bu-i | Me | H | Me | H | Me | Me |
| CO₂Bu-i | Me | Me | Me | H | H | Me |
| CO₂Bu-i | Me | Me | Me | Me | H | H |
| CO₂Bu-i | H | bond | | H | H | H |
| CO₂Bu-i | H | -CH₂- | | H | H | H |
| CO₂Bu-i | H | -(CH₂)₂- | | H | H | H |
| CO₂Bu-i | H | | -0- | H | H | H |
| E-1 | Me | H | H | H | Me | Me |
| E-1 | Me | Me | H | H | Me | H |
| E-1 | Me | Me | Me | H | H | H |
| E-1 | Me | H | Me | H | Me | H |
| E-1 | Me | Me | H | H | Me | Me |
| E-1 | Me | H | Me | H | Me | Me |
| E-1 | Me | Me | Me | H | H | Me |
| E-1 | Me | Me | Me | Me | H | H |
| E-1 | H | bond | | H | H | H |
| E-1 | H | -CH₂- | | H | H | H |
| E-1 | H | -(CH₂)₂- | | H | H | H |
| E-1 | H | -O- | | H | H | H |
| CO₂Bu-t | Me | H | H | H | Me | Me |
| CO₂Bu-t | Me | Me | H | H | Me | H |
| CO₂Bu-t | Me | Me | Me | H | H | H |
| CO₂Bu-t | Me | H | Me | H | Me | H |
| CO₂Bu-t | Me | Me | H | H | Me | Me |
| CO₂Bu-t | Me | H | Me | H | Me | Me |
| CO₂Bu-t | Me | Me | Me | H | H | Me |
| CO₂Bu-t | Me | Me | Me | Me | H | H |
| CO₂Bu-t | H | bond | | H | H | H |
| CO₂Bu-t | H | -CH₂- | | H | H | H |
| CO₂Bu-t | H | -(CH₂)₂- | | H | H | H |
| CO₂Bu-t | H | -O- | | H | H | H |
| CO₂Ph | Me | H | H | H | Me | Me |
| CO₂Ph | Me | Me | H | H | Me | H |
| CO₂Ph | Me | Me | Me | H | H | H |
| CO₂Ph | Me | H | Me | H | Me | H |
| CO₂Ph | Me | Me | H | H | Me | Me |
| CO₂Ph | Me | H | Me | H | Me | Me |
| CO₂Ph | Me | Me | Me | H | H | Me |
| CO₂Ph | Me | Me | Me | Me | H | H |
| CO₂Ph | H | bond | | H | H | H |
| CO₂Ph | H | -CH₂- | | H | H | H |
| CO₂Ph | H | -(CH₂)₂- | | H | H | H |
| CO₂Ph | H | -O- | | H | H | H |
| E-2 | Me | H | H | H | Me | Me |
| E-2 | Me | Me | H | H | Me | H |
| E-2 | Me | Me | Me | H | H | H |
| E-2 | Me | H | Me | H | Me | H |
| E-2 | Me | Me | H | H | Me | Me |
| E-2 | Me | H | Me | H | Me | Me |
| E-2 | Me | Me | Me | H | H | Me |
| E-2 | Me | Me | Me | Me | H | H |
| E-2 | H | bond | | H | H | H |
| E-2 | H | -CH₂- | | H | H | H |
| E-2 | H | -(CH₂)₂- | | H | H | H |
| E-2 | H | -0- | | H | H | H |
| E-3 | Me | H | H | H | Me | Me |
| E-3 | Me | Me | H | H | Me | H |
| E-3 | Me | Me | Me | H | H | H |
| E-3 | Me | H | Me | H | Me | H |
| E-3 | Me | Me | H | H | Me | Me |
| E-3 | Me | H | Me | H | Me | Me |
| E-3 | Me | Me | Me | H | H | Me |
| E-3 | Me | Me | Me | Me | H | H |
| E-3 | H | bond | | H | H | H |
| E-3 | H | -CH₂- | | H | H | H |
| E-3 | H | -(CH₂)₂- | | H | H | H |
| E-3 | H | -O- | | H | H | H |
| CH₂OMe | Me | H | H | H | Me | Me |
| CH₂OMe | Me | Me | H | H | Me | H |
| CH₂OMe | Me | Me | Me | H | H | H |
| CH₂OMe | Me | H | Me | H | Me | H |
| CH₂OMe | Me | Me | H | H | Me | Me |
| CH₂OMe | Me | H | Me | H | Me | Me |
| CH₂OMe | Me | Me | Me | H | H | Me |
| CH₂OMe | Me | Me | Me | Me | H | H |
| CH₂OMe | H | bond | | H | H | H |
| CH₂OMe | H | -CH₂- | | H | H | H |
| CH₂OMe | H | -(CH₂)₂- | | H | H | H |
| CH₂OMe | H | -O- | | H | H | H |
| COCH₂Cl | Me | H | H | H | Me | Me |
| COCH₂Cl | Me | Me | H | H | Me | H |
| COCH₂Cl | Me | Me | Me | H | H | H |
| COCH₂Cl | Me | H | Me | H | Me | H |
| COCH₂Cl | Me | Me | H | H | Me | Me |
| COCH₂Cl | Me | H | Me | H | Me | Me |
| COCH₂Cl | Me | Me | Me | H | H | Me |
| COCH₂Cl | Me | Me | Me | Me | H | H |
| COCH₂Cl | H | bond | | H | H | H |
| COCH₂Cl | H | -CH₂- | | H | H | H |
| COCH₂Cl | H | -(CH₂)₂- | | H | H | H |
| COCH₂Cl | H | -O- | | H | H | H |
| E-4 | Me | H | H | H | Me | Me |
| E-4 | Me | Me | H | H | Me | H |
| E-4 | Me | Me | Me | H | H | H |
| E-4 | Me | H | Me | H | Me | H |
| E-4 | Me | Me | H | H | Me | Me |
| E-4 | Me | H | Me | H | Me | Me |
| E-4 | Me | Me | Me | H | H | Me |
| E-4 | Me | Me | Me | Me | H | H |
| E-4 | H | bond | | H | H | H |
| E - 4 | H | -CH₂- | | H | H | H |
| E - 4 | H | -(CH₂)₂- | | H | H | H |
| E - 4 | H | -O- | | H | H | H |
| E - 5 | Me | H | H | H | Me | Me |
| E - 5 | Me | Me | H | H | Me | H |
| E - 5 | Me | Me | Me | H | H | H |
| E - 5 | Me | H | Me | H | Me | H |
| E - 5 | Me | Me | H | H | Me | Me |
| E - 5 | Me | H | Me | H | Me | Me |
| E - 5 | Me | Me | Me | H | H | Me |
| E - 5 | Me | Me | Me | Me | H | H |
| E - 5 | H | bond | | H | H | H |
| E - 5 | H | -CH₂- | | H | H | H |
| E - 5 | H | -(CH₂)₂- | | H | H | H |
| E - 5 | H | -O- | | H | H | H |
| E - 6 | Me | H | H | H | Me | Me |
| E - 6 | Me | Me | H | H | Me | H |
| E - 6 | Me | Me | Me | H | H | H |
| E - 6 | Me | H | Me | H | Me | H |
| E - 6 | Me | Me | H | H | Me | Me |
| E - 6 | Me | H | Me | H | Me | Me |
| E - 6 | Me | Me | Me | H | H | Me |
| E - 6 | Me | Me | Me | Me | H | H |
| E - 6 | H | bond | | H | H | H |
| E - 6 | H | -CH₂- | | H | H | H |
| E - 6 | H | -(CH₂)₂- | | H | H | H |
| E - 6 | H | -O- | | H | H | H |
| E - 7 | Me | H | H | H | Me | Me |
| E - 7 | Me | Me | H | H | Me | H |
| E - 7 | Me | Me | Me | H | H | H |
| E - 7 | Me | H | Me | H | Me | H |
| E - 7 | Me | Me | H | H | Me | Me |
| E - 7 | Me | H | Me | H | Me | Me |
| E - 7 | Me | Me | Me | H | H | Me |
| E - 7 | Me | Me | Me | Me | H | H |
| E - 7 | H | bond | | H | H | H |
| E - 7 | H | -CH₂- | | H | H | H |
| E - 7 | H | -(CH₂)₂- | | H | H | H |
| E - 7 | H | -O- | | H | H | H |
| E - 8 | Me | H | H | H | Me | Me |
| E - 8 | Me | Me | H | H | Me | H |
| E - 8 | Me | Me | Me | H | H | H |
| E - 8 | Me | H | Me | H | Me | H |
| E - 8 | Me | Me | H | H | Me | Me |
| E - 8 | Me | H | Me | H | Me | Me |
| E - 8 | Me | Me | Me | H | H | Me |
| E - 8 | Me | Me | Me | Me | H | H |
| E-8 | H | bond | | H | H | H |
| E-8 | H | -CH₂- | | H | H | H |
| E-8 | H | -(CH₂)₂- | | H | H | H |
| E-8 | H | -O- | | H | H | H |
| E-9 | Me | H | H | H | Me | Me |
| E-9 | Me | Me | H | H | Me | H |
| E-9 | Me | Me | Me | H | H | H |
| E-9 | Me | H | Me | H | Me | H |
| E-9 | Me | Me | H | H | Me | Me |
| E-9 | Me | H | Me | H | Me | Me |
| E-9 | Me | Me | Me | H | H | Me |
| E-9 | Me | Me | Me | Me | H | H |
| E-9 | H | bond | | H | H | H |
| E-9 | H | -CH₂- | | H | H | H |
| E-9 | H | -(CH₂)₂- | | H | H | H |
| E-9 | H | -O- | | H | H | H |
| E-10 | Me | H | H | H | Me | Me |
| E-10 | Me | Me | H | H | Me | H |
| E-10 | Me | Me | Me | H | H | H |
| E-10 | Me | H | Me | H | Me | H |
| E-10 | Me | Me | H | H | Me | Me |
| E-10 | Me | H | Me | H | Me | Me |
| E-10 | Me | Me | Me | H | H | Me |
| E-10 | Me | Me | Me | Me | H | H |
| E-10 | H | bond | | H | H | H |
| E-10 | H | -CH₂- | | H | H | H |
| E-10 | H | -(CH₂)₂- | | H | H | H |
| E-10 | H | -O- | | H | H | H |
| E-11 | Me | H | H | H | Me | Me |
| E-11 | Me | Me | H | H | Me | H |
| E-11 | Me | Me | Me | H | H | H |
| E-11 | Me | H | Me | H | Me | H |
| E-11 | Me | Me | H | H | Me | Me |
| E-11 | Me | H | Me | H | Me | Me |
| E-11 | Me | Me | Me | H | H | Me |
| E-11 | Me | Me | Me | Me | H | H |
| E-11 | H | bond | | H | H | H |
| E-11 | H | -CH₂- | | H | H | H |
| E-11 | H | -(CH₂)₂- | | H | H | H |
| E-11 | H | -O- | | H | H | H |
| E-12 | Me | H | H | H | Me | Me |
| E-12 | Me | Me | H | H | Me | H |
| E-12 | Me | Me | Me | H | H | H |
| E-12 | Me | H | M e | H | Me | H |
| E-12 | Me | Me | H | H | Me | Me |
| E-12 | Me | H | Me | H | Me | Me |
| E-12 | Me | Me | Me | H | H | Me |
| E-12 | Me | Me | Me | Me | H | H |
| E-12 | H | bond | | H | H | H |
| E-12 | H | -CH₂- | | H | H | H |
| E-12 | H | -(CH₂)₂- | | H | H | H |
| E-12 | H | -O- | | H | H | H |
| CH₂SMe | Me | H | H | H | Me | Me |
| CH₂SMe | Me | Me | H | H | Me | H |
| CH₂SMe | Me | Me | Me | H | H | H |
| CH₂SMe | Me | H | Me | H | Me | H |
| CH₂SMe | Me | Me | H | H | Me | Me |
| CH₂SMe | Me | H | Me | H | Me | Me |
| CH₂SMe | Me | Me | Me | H | H | Me |
| CH₂SMe | Me | Me | Me | Me | H | H |
| CH₂SMe | H | bond | | H | H | H |
| CH₂SMe | H | -CH₂- | | H | H | H |
| CH₂SMe | H | -(CH₂)₂- | | H | H | H |
| CH₂SMe | H | -O- | | H | H | H |
| CH₂OPh | Me | H | H | H | Me | Me |
| CH₂OPh | Me | Me | H | H | Me | H |
| CH₂OPh | Me | Me | Me | H | H | H |
| CH₂OPh | Me | H | Me | H | Me | H |
| CH₂OPh | Me | Me | H | H | Me | Me |
| CH₂OPh | Me | H | Me | H | Me | Me |
| CH₂OPh | Me | Me | Me | H | H | Me |
| CH₂OPh | Me | Me | Me | Me | H | H |
| CH₂OPh | H | bond | | H | H | H |
| CH₂OPh | H | -CH₂- | | H | H | H |
| CH₂OPh | H | -(CH₂)₂- | | H | H | H |
| CH₂OPh | H | -O- | | H | H | H |
| CH₂OCOMe | Me | H | H | H | Me | Me |
| CH₂OCOMe | Me | Me | H | H | Me | H |
| CH₂OCOMe | Me | Me | Me | H | H | H |
| CH₂OCOMe | Me | H | Me | H | Me | H |
| CH₂OCOMe | Me | Me | H | H | Me | Me |
| CH₂OCOMe | Me | H | Me | H | Me | Me |
| CH₂OCOMe | Me | Me | Me | H | H | Me |
| CH₂OCOMe | Me | Me | Me | Me | H | H |
| CH₂OCOMe | H | bond | | H | H | H |
| CH₂OCOMe | H | -CH₂- | | H | H | H |
| CH₂OCOMe | H | -(CH₂)₂- | | H | H | H |
| CH₂OCOMe | H | -O- | | H | H | H |
| CH₂OCOEt | Me | H | H | H | Me | Me |
| CH₂OCOEt | Me | Me | H | H | Me | H |
| CH₂OCOEt | Me | Me | Me | H | H | H |
| CH₂OCOEt | Me | H | Me | H | Me | H |
| CH₂OCOEt | Me | Me | H | H | Me | Me |
| CH₂OCOEt | Me | H | Me | H | Me | Me |
| CH₂OCO Et | Me | Me | Me | H | H | Me |
| CH₂OCO Et | Me | Me | Me | Me | H | H |
| CH₂OCO Et | H | bond | | H | H | H |
| CH₂OCO Et | H | -CH₂- | | H | H | H |
| CH₂OCO Et | H | -(CH₂)₂- | | H | H | H |
| CH₂OCO Et | H | -O- | | H | H | H |
| COSMe | Me | H | H | H | Me | Me |
| COSMe | Me | Me | H | H | Me | H |
| COSMe | Me | Me | Me | H | H | H |
| COSMe | Me | H | Me | H | Me | H |
| COSMe | Me | Me | H | H | Me | Me |
| COSMe | Me | H | Me | H | Me | Me |
| COSMe | Me | Me | Me | H | H | Me |
| COSMe | Me | Me | Me | Me | H | H |
| COSMe | H | bond | | H | H | H |
| COSMe | H | -CH₂- | | H | H | H |
| COSMe | H | -(CH₂)₂- | | H | H | H |
| COSMe | H | -O- | | H | H | H |
| COSEt | Me | H | H | H | Me | Me |
| COSEt | Me | Me | H | H | Me | H |
| COSEt | Me | Me | Me | H | H | H |
| COSEt | Me | H | Me | H | Me | H |
| COSEt | Me | Me | H | H | Me | Me |
| COSEt | Me | H | Me | H | Me | Me |
| COSEt | Me | Me | Me | H | H | Me |
| COSEt | Me | Me | Me | Me | H | H |
| COSEt | H | bond | | H | H | H |
| COSEt | H | -CH₂- | | H | H | H |
| COSEt | H | -(CH₂)₂- | | H | H | H |
| COSEt | H | -O- | | H | H | H |
| CH₂SO₂Me | Me | H | H | H | Me | Me |
| CH₂SO₂Me | Me | Me | H | H | Me | H |
| CH₂SO₂Me | Me | Me | Me | H | H | H |
| CH₂SO₂Me | Me | H | Me | H | Me | H |
| CH₂SO₂Me | Me | Me | H | H | Me | Me |
| CH₂SO₂Me | Me | H | Me | H | Me | Me |
| CH₂SO₂Me | Me | Me | Me | H | H | Me |
| CH₂SO₂Me | Me | Me | Me | Me | H | H |
| CH₂SO₂Me | H | bond | | H | H | H |
| CH₂SO₂Me | H | -CH₂- | | H | H | H |
| CH₂SO₂Me | H | -(CH₂)₂- | | H | H | H |
| CH₂SO₂Me | H | -O- | | H | H | H |
| CH₂SOMe | Me | H | H | H | Me | Me |
| CH₂SOMe | Me | Me | H | H | Me | H |
| CH₂SOMe | Me | Me | Me | H | H | H |
| CH₂SOMe | Me | H | Me | H | Me | H |
| CH₂SOMe | Me | Me | H | H | Me | Me |
| CH₂SOMe | Me | H | Me | H | Me | Me |
| CH₂SOMe | Me | Me | Me | H | H | Me |
| CH₂SOMe | Me | Me | Me | Me | H | H |
| CH₂SOMe | H | bond | | H | H | H |
| CH₂SOMe | H | -CH₂- | | H | H | H |
| CH₂SOMe | H | -(CH₂)₂- | | H | H | H |
| CH₂SOMe | H | -O- | | H | H | H |
| CH₂Ph | Me | H | H | H | Me | Me |
| CH₂Ph | Me | Me | H | H | Me | H |
| CH₂Ph | Me | Me | Me | H | H | H |
| CH₂Ph | Me | H | Me | H | Me | H |
| CH₂Ph | Me | Me | H | H | Me | Me |
| CH₂Ph | Me | H | Me | H | Me | Me |
| CH₂Ph | Me | Me | Me | H | H | Me |
| CH₂Ph | Me | Me | Me | Me | H | H |
| CH₂Ph | H | bond | | H | H | H |
| CH₂Ph | H | -CH₂- | | H | H | H |
| CH₂Ph | H | -(CH₂)₂- | | H | H | H |
| CH₂Ph | H | -O- | | H | H | H |
| SO₂CF₃ | Me | H | H | H | Me | Me |
| SO₂CF₃ | Me | Me | H | H | Me | H |
| SO₂CF₃ | Me | Me | Me | H | H | H |
| SO₂CF₃ | Me | H | Me | H | Me | H |
| SO₂CF₃ | Me | Me | H | H | Me | Me |
| SO₂CF₃ | Me | H | Me | H | Me | Me |
| SO₂CF₃ | Me | Me | Me | H | H | Me |
| SO₂CF₃ | Me | Me | Me | Me | H | H |

In Table 4, E-1 to E-12 are the same as defined above, and "bond" means that the indicated substituents form a bond between the two carbons attached to them, and when the two carbons lie next to each other, the bond is a double bond, and when the two carbons are separated, the bond is a single bond.

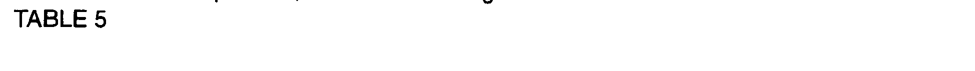

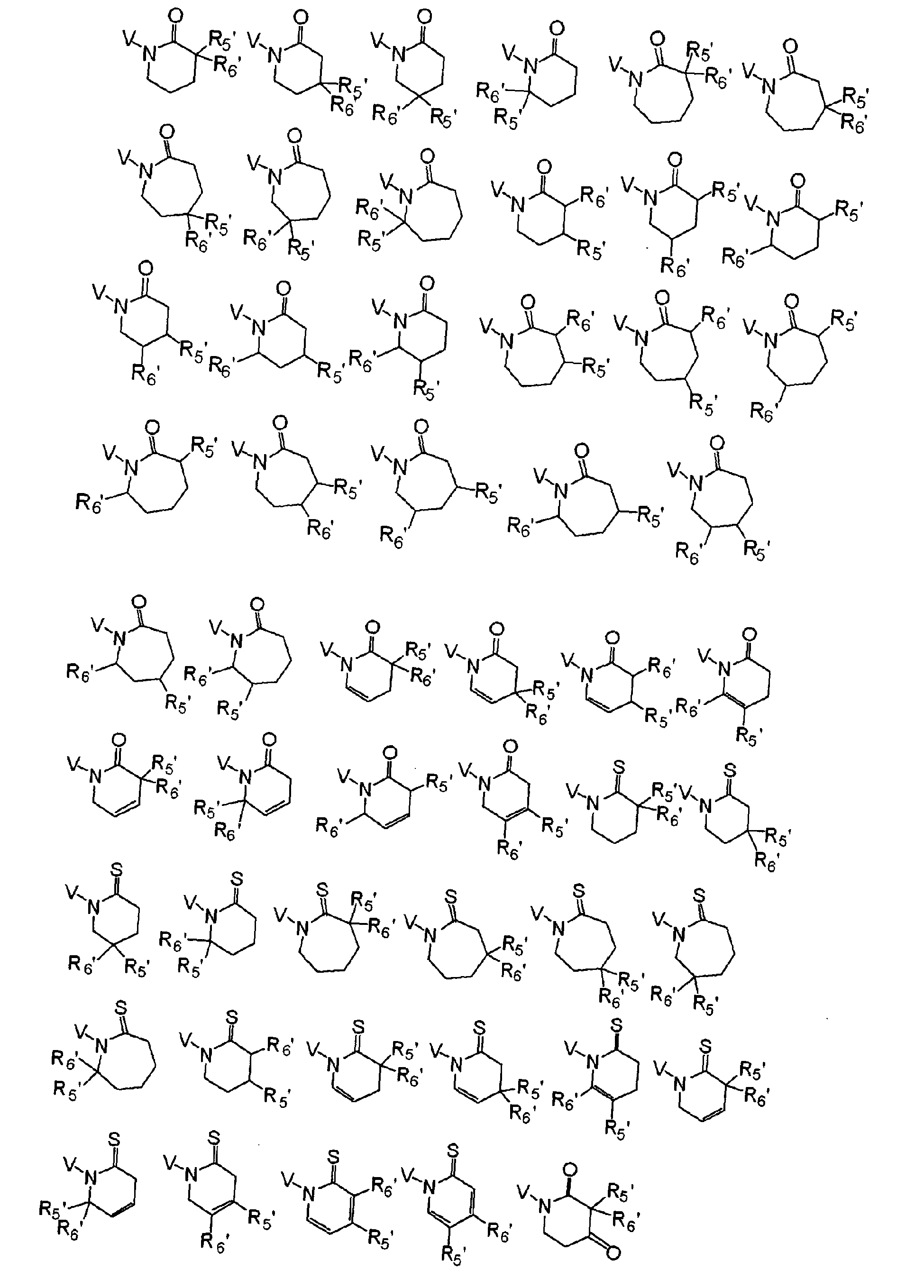

V is represented by the following formula (27).

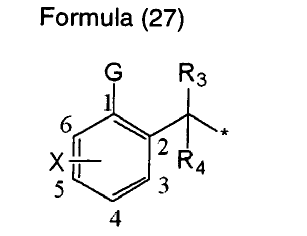

In the table, the position of a substituent X is represented by the numerals in the formula (27), and for example, 3-F means that the carbon marked with 3 on the benzene ring is substituted with F.

| R₃=R₄=H | | | |
|---|---|---|---|
| G | R₅ ' | R₆ ' | X |
| NO₂ | Me | H | H |
| NO₂ | Me | Me | H |
| NO₂ | Et | H | H |
| NO₂ | Et | Me | H |
| NO₂ | Pr-n | H | H |
| NO₂ | Pr-n | Me | H |
| NO₂ | Pr-i | H | H |
| NO₂ | Pr-i | Me | H |
| NO₂ | Pr-c | H | H |
| NO₂ | Pr-c | Me | H |
| NO₂ | Bu-n | H | H |
| NO₂ | Bu-n | Me | H |
| NO₂ | Bu-i | H | H |
| NO₂ | Bu-i | Me | H |
| NO₂ | Bu-s | H | H |
| NO₂ | Bu-s | Me | H |
| NO₂ | Bu-t | H | H |
| NO₂ | Bu-t | Me | H |
| NO₂ | Bu-c | H | H |
| NO₂ | Bu-c | Me | H |
| NO₂ | Pen-n | H | H |
| NO₂ | Pen-n | Me | H |
| NO₂ | Pen-c | H | H |
| NO₂ | Pen-c | Me | H |
| NO₂ | Hex-n | H | H |
| NO₂ | Hex-n | Me | H |
| NO₂ | Hex-c | H | H |
| NO₂ | Hex-c | Me | H |
| NO₂ | Cl | H | H |
| NO₂ | Cl | Cl | H |
| NO₂ | Br | H | H |
| NO₂ | I | H | H |
| NO₂ | F | H | H |
| NO₂ | CH=CH₂ | H | H |
| NO₂ | CH=CH₂ | Me | H |
| NO₂ | C≡CH | H | H |
| NO₂ | C≡CH | Me | H |
| NO₂ | CH₂CH=CH₂ | H | H |
| NO₂ | CH₂CH=CH₂ | Me | H |
| NO₂ | CH₂C≡CH | H | H |
| NO₂ | CH₂C≡CH | Me | H |
| NO₂ | CH₂Cl | H | H |
| NO₂ | CH₂Cl | Me | H |
| NO₂ | CH₂Cl | CH₂Cl | H |
| NO₂ | CHCl₂ | H | H |
| NO₂ | CCl₃ | H | H |
| NO₂ | CH₂F | H | H |
| NO₂ | CHF₂ | H | H |
| NO₂ | CF₃ | H | H |
| NO₂ | CF₃ | Me | H |
| NO₂ | CF₃ | Et | H |
| NO₂ | CH₂CH₂Cl | H | H |
| NO₂ | CH₂OH | H | H |
| NO₂ | CH₂SMe | H | H |
| NO₂ | CH₂SCH₂Cl | H | H |
| NO₂ | Ph | H | H |
| NO₂ | Ph | Me | H |
| NO₂ | Ph-2-Cl | H | H |
| NO₂ | Ph-2-Cl | Me | H |
| NO₂ | Ph-3-Cl | H | H |
| NO₂ | Ph-3-Cl | Me | H |
| NO₂ | Ph-4-Cl | H | H |
| NO₂ | Ph-4-Cl | Me | H |
| NO₂ | Ph-3-I | H | H |
| NO₂ | Ph-4-F | H | H |
| NO₂ | Ph-2-Me | H | H |
| NO₂ | Ph-3-OMe | H | H |
| NO₂ | Ph-4-SMe | H | H |
| NO₂ | Ph-3-CN | H | H |
| NO₂ | Ph-4-NO₂ | H | H |
| NO₂ | Ph-2-CO₂Me | H | H |
| NO₂ | Ph-3-NH₂ | H | H |
| NO₂ | Ph-4-Ph | H | H |
| NO₂ | Ph-2-OPh | H | H |
| NO₂ | Ph-3-SPh | H | H |
| NO₂ | Ph-4-CONMe₂ | H | H |
| NO₂ | COMe | H | H |
| NO₂ | COEt | H | H |
| NO₂ | COCH₂Cl | H | H |
| NO₂ | CO₂H | H | H |
| NO₂ | CO₂Me | H | H |
| NO₂ | CO₂Me | Me | H |
| NO₂ | CO₂Et | H | H |
| NO₂ | CO₂Bu-i | H | H |
| NO₂ | CONH₂ | H | H |
| NO₂ | CONHMe | H | H |
| NO₂ | CONMe₂ | H | H |
| NO₂ | CH₂Ph | H | H |
| NO₂ | CH₂Ph-2-Cl | H | H |
| NO₂ | CH₂Ph-3-Cl | H | H |
| NO₂ | CH₂Ph-4-Cl | H | H |
| NO₂ | COPh | H | H |
| NO₂ | COPh-2-Cl | H | H |
| NO₂ | COPh-3-Cl | H | H |
| NO₂ | COPh-4-Cl | H | H |
| NO₂ | OH | H | H |
| NO₂ | OMe | H | H |
| NO₂ | OMe | Me | H |
| NO₂ | SMe | H | H |
| NO₂ | SMe | Me | H |
| NO₂ | SPh | H | H |
| NO₂ | CN | H | H |
| NO₂ | NH₂ | H | H |
| NO₂ | NHMe | H | H |
| NO₂ | NHMe | Me | H |
| NO₂ | NMe₂ | H | H |
| NO₂ | NMe₂ | Me | H |
| NO₂ | -CH₂-O-CH₂- | | H |
| NO₂ | -(CH₂)₂- | | H |
| NO₂ | -(CH₂)₃- | | H |
| NO₂ | Me | H | 3-F |
| NO₂ | Me | H | 4-F |
| NO₂ | Me | H | 5-F |
| NO₂ | Me | H | 6-F |
| NO₂ | Me | H | 4-Cl |
| NO₂ | Me | H | 5-Cl |
| NO₂ | Me | H | 4-Br |
| NO₂ | Me | H | 4-I |
| NO₂ | Me | H | 4-Me |
| NO₂ | Me | H | 5-Me |
| NO₂ | Me | H | 4-OMe |
| NO₂ | Me | H | 4-SMe |
| NO₂ | Me | Me | 3-F |
| NO₂ | Me | Me | 4-F |
| NO₂ | Me | Me | 5-F |
| NO₂ | Me | Me | 6-F |
| NO₂ | Me | Me | 4-Cl |
| NO₂ | Me | Me | 5-Cl |
| NO₂ | Me | Me | 4-Br |
| NO₂ | Me | Me | 4-I |
| NO₂ | Me | Me | 4-Me |
| NO₂ | Me | Me | 5-Me |
| NO₂ | Me | Me | 4-OMe |
| NO₂ | Me | Me | 4-SMe |
| NH₂ | Me | H | H |
| NH₂ | Me | Me | H |
| NH₂ | Et | H | H |
| NH₂ | Et | Me | H |
| NH₂ | Pr-n | H | H |
| NH₂ | Pr-n | Me | H |
| NH₂ | Pr-i | H | H |
| NH₂ | Pr-i | Me | H |
| NH₂ | Pr-c | H | H |
| NH₂ | Pr-c | Me | H |
| NH₂ | Bu-n | H | H |
| NH₂ | Bu-n | Me | H |
| NH₂ | Bu-i | H | H |
| NH₂ | Bu-i | Me | H |
| NH₂ | Bu-s | H | H |
| NH₂ | Bu-s | Me | H |
| NH₂ | Bu-t | H | H |
| NH₂ | Bu-t | Me | H |
| NH₂ | Bu-c | H | H |
| NH₂ | Bu-c | Me | H |
| NH₂ | Pen-n | H | H |
| NH₂ | Pen-n | Me | H |
| NH₂ | Pen-c | H | H |
| NH₂ | Pen-c | Me | H |
| NH₂ | Hex-n | H | H |
| NH₂ | Hex-n | Me | H |
| NH₂ | Hex-c | H | H |
| NH₂ | Hex-c | Me | H |
| NH₂ | Cl | H | H |
| NH₂ | Cl | Cl | H |
| NH₂ | Br | H | H |
| NH₂ | I | H | H |
| NH₂ | F | H | H |
| NH₂ | CH=CH₂ | H | H |
| NH₂ | CH=CH₂ | Me | H |
| NH₂ | C≡CH | H | H |
| NH₂ | C≡CH | Me | H |
| NH₂ | CH2CH=CH₂ | H | H |
| NH₂ | CH₂CH=CH₂ | Me | H |
| NH₂ | CH₂C≡CH | H | H |
| NH₂ | CH₂C≡CH | Me | H |
| NH₂ | CH₂Cl | H | H |
| NH₂ | CH₂Cl | Me | H |
| NH₂ | CH₂Cl | CH₂Cl | H |
| NH₂ | CHCl₂ | H | H |
| NH₂ | CCl₃ | H | H |
| NH₂ | CH₂F | H | H |
| NH₂ | CHF₂ | H | H |
| NH₂ | CF₃ | H | H |
| NH₂ | CF₃ | Me | H |
| NH₂ | CF₃ | Et | H |
| NH₂ | CH₂CH₂Cl | H | H |
| NH₂ | CH₂OH | H | H |
| NH₂ | CH₂SMe | H | H |
| NH₂ | CH₂SCH₂Cl | H | H |
| NH₂ | Ph | H | H |
| NH₂ | Ph | Me | H |
| NH₂ | Ph-2-Cl | H | H |
| NH₂ | Ph-2-Cl | Me | H |
| NH₂ | Ph-3-Cl | H | H |
| NH₂ | Ph-3-Cl | Me | H |
| NH₂ | Ph-4-Cl | H | H |
| NH₂ | Ph-4-Cl | Me | H |
| NH₂ | Ph-3-I | H | H |
| NH₂ | Ph-4-F | H | H |
| NH₂ | Ph-2-Me | H | H |
| NH₂ | Ph-3-OMe | H | H |
| NH₂ | Ph-4-SMe | H | H |
| NH₂ | Ph-3-CN | H | H |
| NH₂ | Ph-4NO₂ | H | H |
| NH₂ | Ph-2-CO₂Me | H | H |
| NH₂ | Ph-3-NH₂ | H | H |
| NH₂ | Ph-4-Ph | H | H |
| NH₂ | Ph-2-OPh | H | H |
| NH₂ | Ph-3-SPh | H | H |
| NH₂ | Ph-4-CONMe₂ | H | H |
| NH₂ | COMe | H | H |
| NH₂ | COEt | H | H |
| NH₂ | COCH₂Cl | H | H |
| NH₂ | CO₂H | H | H |
| NH₂ | CO₂Me | H | H |
| NH₂ | CO₂Me | Me | H |
| NH₂ | CO₂Et | H | H |
| NH₂ | CO₂Bu-i | H | H |
| NH₂ | CONH₂ | H | H |
| NH₂ | CONHMe | H | H |
| NH₂ | CONMe₂ | H | H |
| NH₂ | CH₂Ph | H | H |
| NH₂ | CH₂Ph-2-Cl | H | H |
| NH₂ | CH₂Ph-3-Cl | H | H |
| NH₂ | CH₂Ph-4-Cl | H | H |
| NH₂ | COPh | H | H |
| NH₂ | COPh-2-Cl | H | H |
| NH₂ | COPh-3-Cl | H | H |
| NH₂ | COPh-4-Cl | H | H |
| NH₂ | OH | H | H |
| NH₂ | OMe | H | H |
| NH₂ | OMe | Me | H |
| NH₂ | SMe | H | H |
| NH₂ | SMe | Me | H |
| NH₂ | SPh | H | H |
| NH₂ | CN | H | H |
| NH₂ | NH₂ | H | H |
| NH₂ | NHMe | H | H |
| NH₂ | NHMe | Me | H |
| NH₂ | NMe₂ | H | H |
| NH₂ | NMe₂ | Me | H |
| NH₂ | -CH₂-O-CH₂- | | H |
| NH₂ | -(CH₂)₂- | | H |
| NH₂ | -(CH₂)₃- | | H |
| NH₂ | Me | H | 3-F |
| NH₂ | Me | H | 4-F |
| NH₂ | Me | H | 5-F |
| NH₂ | Me | H | 6-F |
| NH₂ | Me | H | 4-Cl |
| NH₂ | Me | H | 5-Cl |
| NH₂ | Me | H | 4-Br |
| NH₂ | Me | H | 4-I |
| NH₂ | Me | H | 4-Me |
| NH₂ | Me | H | 5-Me |
| NH₂ | Me | H | 4-OMe |
| NH₂ | Me | H | 4-SMe |
| NH₂ | Me | Me | 3-F |
| NH₂ | Me | Me | 4-F |
| NH₂ | Me | Me | 5-F |
| NH₂ | Me | Me | 6-F |
| NH₂ | Me | Me | 4-Cl |
| NH₂ | Me | Me | 5-Cl |
| NH₂ | Me | Me | 4-Br |
| NH₂ | Me | Me | 4-I |
| NH₂ | Me | Me | 4-Me |
| NH₂ | Me | Me | 5-Me |
| NH₂ | Me | Me | 4-OMe |
| NH₂ | Me | Me | 4-SMe |

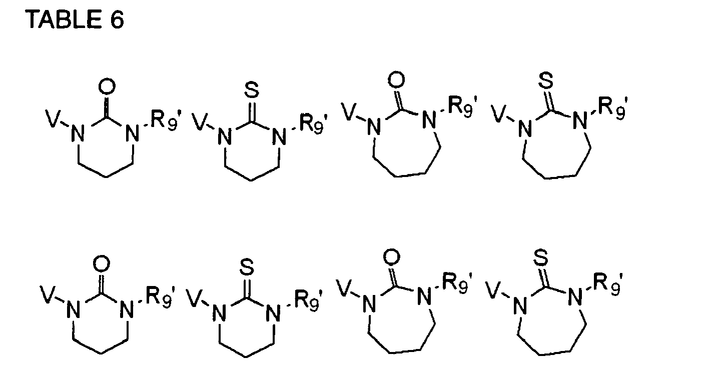

Visthesameastheabove-mentionedformula(27).

| R₃=R₄=H | |
|---|---|
| G | R₉' |
| NO₂ | H |
| NO₂ | Me |
| NO₂ | Et |
| NO₂ | Pr-c |
| NO₂ | CH₂Pr-i |
| NO₂ | CH₂Pr-c |
| NO₂ | CH₂CH=CH₂ |
| NO₂ | CH₂C≡CH |
| NO₂ | CH₂OMe |
| NO₂ | CH₂SMe |
| NO₂ | CH₂CO₂Me |
| NO₂ | CH₂CO₂Me |
| NO₂ | CH₂Ph |
| NO₂ | CH₂Ph-2-Cl |
| NO₂ | CH₂Ph-3-Cl |
| NO₂ | CH₂Ph-4-Cl |
| NO₂ | Ph |
| NO₂ | Ph-2-Cl |
| NO₂ | Ph-3-Cl |
| NO₂ | Ph-4-Cl |
| NO₂ | COMe |
| NO₂ | COPr-c |
| NO₂ | CONMe₂ |
| NO₂ | SO₂Me |
| NO₂ | SO₂CF₃ |
| NO₂ | CO₂Et |
| NH₂ | H |
| NH₂ | Me |
| NH₂ | Et |
| NH₂ | Pr-c |
| NH₂ | CH₂Pr-i |
| NH₂ | CH₂Pr-c |
| NH₂ | CH₂CH=CH₂ |
| NH₂ | CH₂C≡CH |
| NH₂ | CH₂OMe |
| NH₂ | CH₂SMe |
| NH₂ | CH₂CO₂Me |
| NH₂ | CH₂CO₂Me |
| NH₂ | CH₂Ph |
| NH₂ | CH₃Ph-2-Cl |
| NH₂ | CH₂Ph-3-Cl |
| NH₂ | CH₂Ph-4-Cl |
| NH₂ | Ph |
| NH₂ | Ph-2-Cl |
| NH₂ | Ph-3-Cl |
| NH₂ | Ph-4-Cl |
| NH₂ | COMe |
| NH₂ | COPr-c |
| NH₂ | CONMe₂ |
| NH₂ | SO₂Me |
| NH₂ | SO₂CF₃ |
| NH₂ | CO₂Et |

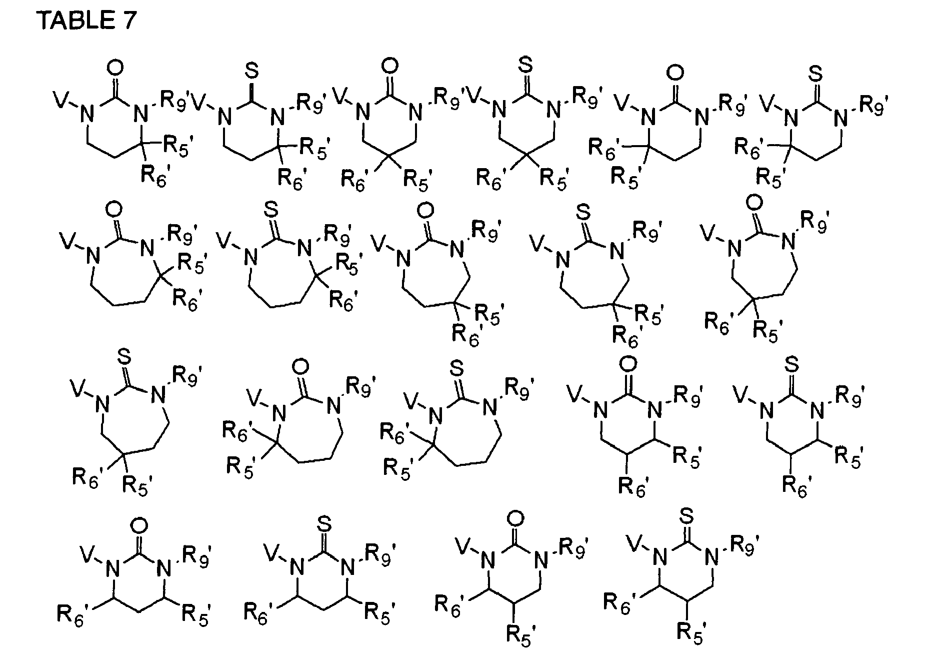

V is the same as the above-mentioned formula (27).

| R₃ =R₄=H | | | | |
|---|---|---|---|---|
| G | R₉' | R₅' | R₆' | X |
| NO₂ | H | Me | H | H |
| NO₂ | Me | Me | H | H |
| NO₂ | Et | Me | H | H |
| NO₂ | Pr-c | Me | H | H |
| NO₂ | CH₂Pr-i | Me | H | H |
| NO₂ | CH₂Pr-c | Me | H | H |
| NO₂ | CH₂CH=CH₂ | Me | H | H |
| NO₂ | CH₂C≡CH | Me | H | H |
| NO₂ | CH₂OMe | Me | H | H |
| NO₂ | CH₂SMe | Me | H | H |
| NO₂ | CH₂CO₂Me | Me | H | H |
| NO₂ | CH₂CO₂Me | Me | H | H |
| NO₂ | CH₂Ph | Me | H | H |
| NO₂ | CH₂Ph-2-Cl | Me | H | H |
| NO₂ | CH₂Ph-3-Cl | Me | H | H |
| NO₂ | CH₂Ph-4-Cl | Me | H | H |
| NO₂ | Ph | Me | H | H |
| NO₂ | Ph-2-Cl | Me | H | H |
| NO₂ | Ph-3-Cl | Me | H | H |
| NO₂ | Ph-4-Cl | Me | H | H |
| NO₂ | COMe | Me | H | H |
| NO₂ | COPr-c | Me | H | H |
| NO₂ | CONMe₂ | Me | H | H |
| NO₂ | SO₂Me | Me | H | H |
| NO₂ | SO₂CF₃ | Me | H | H |
| NO₂ | CO₂Et | Me | H | H |
| NO₂ | H | Me | Me | H |
| NO₂ | Me | Me | Me | H |
| NO₂ | Et | Me | Me | H |
| NO₂ | Pr-c | Me | Me | H |
| NO₂ | CH₂Pr-i | Me | Me | H |
| NO₂ | CH₂Pr-c | Me | Me | H |
| NO₂ | CH₂CH=CH₂ | Me | Me | H |
| NO₂ | CH₂C≡CH | Me | Me | H |
| NO₂ | CH₂OMe | Me | Me | H |
| NO₂ | CH₂SMe | Me | Me | H |
| NO₂ | CH₂CO₂Me | Me | Me | H |
| NO₂ | CH₂CO₂Me | Me | Me | H |
| NO₂ | CH₂Ph | Me | Me | H |
| NO₂ | CH₂Ph-2-Cl | Me | Me | H |
| NO₂ | CH₂Ph-3-Cl | Me | Me | H |
| NO₂ | CH₂Ph-4-Cl | Me | Me | H |
| NO₂ | Ph | Me | Me | H |
| NO₂ | Ph-2-Cl | Me | Me | H |
| NO₂ | Ph-3-Cl | Me | Me | H |
| NO₂ | Ph-4-Cl | Me | Me | H |
| NO₂ | COMe | Me | Me | H |
| NO₂ | COPr-c | Me | Me | H |
| NO₂ | CONMe₂ | Me | Me | H |
| NO₂ | SO₂Me | Me | Me | H |
| NO₂ | SO₂CF₃ | Me | Me | H |
| NO₂ | CO₂Et | Me | Me | H |
| NO₂ | H | Me | Et | H |
| NO₂ | Me | Me | Et | H |
| NO₂ | H | Et | H | H |
| NO₂ | Me | Et | H | H |
| NO₂ | H | Pr-n | H | H |
| NO₂ | Me | Pr-n | H | H |
| NO₂ | H | Pr-i | H | H |
| NO₂ | Me | Pr-i | H | H |
| NO₂ | H | Pr-c | H | H |
| NO₂ | Me | Pr-c | H | H |
| NO₂ | H | Bu-n | H | H |
| NO₂ | Me | Bu-n | H | H |
| NO₂ | H | Bu-i | H | H |
| NO₂ | Me | Bu-i | H | H |
| NO₂ | H | Bu-s | H | H |
| NO₂ | Me | Bu-s | H | H |
| NO₂ | H | Bu-t | H | H |
| NO₂ | Me | Bu-t | H | H |
| NO₂ | H | Bu-c | H | H |
| NO₂ | Me | Bu-c | H | H |
| NO₂ | H | Pen-n | H | H |
| NO₂ | Me | Pen-n | H | H |
| NO₂ | H | Pen-c | H | H |
| NO₂ | Me | Pen-c | H | H |
| NO₂ | H | Hex-n | H | H |
| NO₂ | Me | Hex-n | H | H |
| NO₂ | H | Hex-c | H | H |
| NO₂ | Me | Hex-c | H | H |
| NO₂ | H | Cl | H | H |
| NO₂ | Me | Cl | H | H |
| NO₂ | Et | Cl | H | H |
| NO₂ | H | Cl | Cl | H |
| NO₂ | Me | Cl | Cl | H |
| NO₂ | H | Br | H | H |
| NO₂ | Me | Br | H | H |
| NO₂ | H | I | H | H |
| NO₂ | Me | I | H | H |
| NO₂ | H | F | H | H |
| NO₂ | Me | F | H | H |
| NO₂ | H | F | F | H |
| NO₂ | Me | F | F | H |
| NO₂ | H | CH=CH₂ | H | H |
| NO₂ | Me | CH=CH₂ | H | H |
| NO₂ | H | C≡CH | H | H |
| NO₂ | Me | C≡CH | H | H |
| NO₂ | H | CH₂CH=CH₂ | H | H |
| NO₂ | Me | CH₂CH=CH₂ | H | H |
| NO₂ | H | CH₂C≡CH | H | H |
| NO₂ | Me | CH₂C≡CH | H | H |
| NO₂ | H | CH₂Cl | H | H |
| NO₂ | Me | CH₂Cl | H | H |
| NO₂ | H | CHCl₂ | H | H |
| NO₂ | Me | CHCl₂ | H | H |
| NO₂ | H | CCl₃ | H | H |
| NO₂ | Me | CCl₃ | H | H |
| NO₂ | H | CH₂F | H | H |
| NO₂ | Me | CH₂F | H | H |
| NO₂ | H | CHF₂ | H | H |
| NO₂ | Me | CHF2 | H | H |
| NO₂ | H | CF₃ | H | H |
| NO₂ | Me | CF₃ | H | H |
| NO₂ | H | CH₂CH₂Cl | H | H |
| NO₂ | Me | CH₂CH₂Cl | H | H |
| NO₂ | H | CH₂OH | H | H |
| NO₂ | Me | CH₂OH | H | H |
| NO₂ | H | CH₂SMe | H | H |
| NO₂ | Me | CH₂SMe | H | H |
| NO₂ | H | CH₂SCH₂Cl | H | H |
| NO₂ | Me | CH₂SCH₂Cl | H | H |
| NO₂ | H | Ph | H | H |
| NO₂ | Me | Ph | H | H |
| NO₂ | H | Ph-2-Cl | H | H |
| NO₂ | Me | Ph-2-Cl | H | H |
| NO₂ | H | Ph-3-Cl | H | H |
| NO₂ | Me | Ph-3-Cl | H | H |
| NO₂ | H | Ph-4-Cl | H | H |
| NO₂ | Me | Ph-4-Cl | H | H |
| NO₂ | Me | Ph-2-Br | H | H |
| NO₂ | Me | Ph-3-I | H | H |
| NO₂ | Me | Ph-4-F | H | H |
| NO₂ | Me | Ph-2-Me | H | H |
| NO₂ | Me | Ph-3-OMe | H | H |
| NO₂ | Me | Ph-4-SMe | H | H |
| NO₂ | Me | Ph-2-SO₂Me | H | H |
| NO₂ | Me | Ph-3-CN | H | H |
| NO₂ | Me | Ph-4-NO₂ | H | H |
| NO₂ | Me | Ph-2-CO₂Me | H | H |
| NO₂ | Me | Ph-3-NH₂ | H | H |
| NO₂ | Me | Ph-4-Ph | H | H |
| NO₂ | Me | Ph-2-OPh | H | H |
| NO₂ | Me | Ph-3-SPh | H | H |
| NO₂ | Me | Ph-4-OH | H | H |
| NO₂ | H | COMe | H | H |
| NO₂ | Me | COMe | H | H |
| NO₂ | H | COEt | H | H |
| NO₂ | Me | COEt | H | H |
| NO₂ | H | COCH₂Cl | H | H |
| NO₂ | Me | COCH₂Cl | H | H |
| NO₂ | H | CO₂H | H | H |
| NO₂ | Me | CO₂H | H | H |
| NO₂ | H | CO₂Me | H | H |
| NO₂ | Me | CO₂Me | H | H |
| NO₂ | H | COEt | H | H |
| NO₂ | Me | COEt | H | H |
| NO₂ | H | CONHMe | H | H |
| NO₂ | Me | CONHMe | H | H |
| NO₂ | H | CONMe₂ | H | H |
| NO₂ | Me | CONMe₂ | H | H |
| NO₂ | H | OH | H | H |
| NO₂ | Me | OH | H | H |
| NO₂ | H | OMe | H | H |
| NO₂ | Me | OMe | H | H |
| NO₂ | H | SMe | H | H |
| NO₂ | Me | SMe | H | H |
| NO₂ | H | CN | H | H |
| NO₂ | Me | CN | H | H |
| NO₂ | H | NH₂ | H | H |
| NO₂ | Me | NH₂ | H | H |
| NO₂ | H | NHMe | H | H |
| NO₂ | Me | NHMe | H | H |
| NO₂ | H | NMe₂ | H | H |
| NO₂ | Me | NMe₂ | H | H |
| NO₂ | H | -CH₂-O- | | H |
| NO₂ | Me | -CH₂-O- | | H |
| NO₂ | H | -(CH₂)₂- | | H |
| NO₂ | Me | -(CH₂)₂- | | H |
| NO₂ | H | -(CH₂)₃- | | H |
| NO₂ | Me | -(CH₂)₃- | | H |
| NO₂ | Me | H | H | 3-F |
| NO₂ | Me | H | H | 4-F |
| NO₂ | Me | H | H | 5-F |
| NO₂ | Me | H | H | 6-F |
| NO₂ | Me | H | H | 4-Cl |
| NO₂ | Me | H | H | 5-Cl |
| NO₂ | Me | H | H | 4-Br |
| NO₂ | Me | H | H | 4-I |
| NO₂ | Me | H | H | 4-Me |
| NO₂ | Me | H | H | 5-Me |
| NO₂ | Me | H | H | 4-OMe |
| NO₂ | Me | H | H | 4-SMe |
| NH₂ | H | Me | H | H |
| NH₂ | Me | Me | H | H |
| NH₂ | Et | Me | H | H |
| NH₂ | Pr-c | Me | H | H |
| NH₂ | CH₂Pr-i | Me | H | H |
| NH₂ | CH₂Pr-c | Me | H | H |
| NH₂ | CH₂CH=CH₂ | Me | H | H |
| NH₃ | CH₂C≡CH | Me | H | H |
| NH₂ | CH₂OMe | Me | H | H |
| NH₂ | CH₂SMe | Me | H | H |
| NH₂ | CH₂CO₂Me | Me | H | H |
| NH₂ | CH₂CO₂Me | Me | H | H |
| NH₂ | CH₂Ph | Me | H | H |
| NH₂ | CH₂Ph-2-Cl | Me | H | H |
| NH₂ | CH₂Ph-3-Cl | Me | H | H |
| NH₂ | CH₂Ph-4-Cl | Me | H | H |
| NH₂ | Ph | Me | H | H |
| NH₂ | Ph-2-Cl | Me | H | H |
| NH₂ | Ph-3-Cl | Me | H | H |
| NH₂ | Ph-4-Cl | Me | H | H |
| NH₂ | COMe | Me | H | H |
| NH₂ | COPr-c | Me | H | H |
| NH₂ | CONMe₂ | Me | H | H |
| NH₂ | SO₂Me | Me | H | H |
| NH₂ | SO₂CF3 | Me | H | H |
| NH₂ | CO₂Et | Me | H | H |
| NH₂ | H | Me | Me | H |
| NH₂ | Me | Me | Me | H |
| NH₂ | Et | Me | Me | H |
| NH₂ | Pr-c | Me | Me | H |
| NH₂ | CH₂Pr-i | Me | Me | H |
| NH₂ | CH₂Pr-c | Me | Me | H |
| NH₂ | CH₂CH=CH₂ | Me | Me | H |
| NH₂ | CH₂C≡CH | Me | Me | H |
| NH₂ | CH₂OMe | Me | Me | H |
| NH₂ | CH₂SMe | Me | Me | H |
| NH₂ | CH₂CO₂Me | Me | Me | H |
| NH₂ | CH₂CO₂Me | Me | Me | H |
| NH₂ | CH₂Ph | Me | Me | H |
| NH₂ | CH₃Ph-2-Cl | Me | Me | H |
| NH₂ | CH₂Ph-3-Cl | Me | Me | H |
| NH₂ | CH₂Ph-4-Cl | Me | Me | H |
| NH₂ | Ph | Me | Me | H |
| NH₂ | Ph-2-Cl | Me | Me | H |
| NH₂ | Ph-3-Cl | Me | Me | H |
| NH₂ | Ph-4-Cl | Me | Me | H |
| NH₂ | COMe | Me | Me | H |
| NH₂ | COPr-c | Me | Me | H |
| NH₂ | CONMe₂ | Me | Me | H |
| NH₂ | SO₂Me | Me | Me | H |
| NH₂ | SO₂CF₃ | Me | Me | H |
| NH₂ | CO₂Et | Me | Me | H |
| NH₂ | H | Me | Et | H |
| NH₂ | Me | Me | Et | H |
| NH₂ | H | Et | H | H |
| NH₂ | Me | Et | H | H |
| NH₂ | H | Pr-n | H | H |
| NH₂ | Me | Pr-n | H | H |
| NH₂ | H | Pr-i | H | H |
| NH₂ | Me | Pr-i | H | H |
| NH₂ | H | Pr-c | H | H |
| NH₂ | Me | Pr-c | H | H |
| NH₂ | H | Bu-n | H | H |
| NH₂ | Me | Bu-n | H | H |
| NH₂ | H | Bu-i | H | H |
| NH₂ | Me | Bu-i | H | H |
| NH₂ | H | Bu-s | H | H |
| NH₂ | Me | Bu-s | H | H |
| NH₂ | H | Bu-t | H | H |
| NH₂ | Me | Bu-t | H | H |
| NH₂ | H | Bu-c | H | H |
| NH₂ | Me | Bu-c | H | H |
| NH₂ | H | Pen-n | H | H |
| NH₂ | Me | Pen-n | H | H |
| NH₂ | H | Pen-c | H | H |
| NH₂ | Me | Pen-c | H | H |
| NH₂ | H | Hex-n | H | H |
| NH₂ | Me | Hex-n | H | H |
| NH₂ | H | Hex-c | H | H |
| NH₂ | Me | Hex-c | H | H |
| NH₂ | H | Cl | H | H |
| NH₂ | Me | Cl | H | H |
| NH₂ | Et | Cl | H | H |
| NH₂ | H | Cl | Cl | H |
| NH₂ | Me | Cl | Cl | H |
| NH₂ | H | Br | H | H |
| NH₂ | Me | Br | H | H |
| NH₂ | H | I | H | H |
| NH₂ | Me | I | H | H |
| NH₂ | H | F | H | H |
| NH₂ | Me | F | H | H |
| NH₂ | H | F | F | H |
| NH₂ | Me | F | F | H |
| NH₂ | H | CH=CH₂ | H | H |
| NH₂ | Me | CH=CH₂ | H | H |
| NH₂ | H | C≡CH | H | H |
| NH₂ | Me | C≡CH | H | H |
| NH₂ | H | CH₂CH=CH₂ | H | H |
| NH₂ | Me | CH₂CH=CH₂ | H | H |
| NH₂ | H | CH₂C=CH | H | H |
| NH₂ | Me | CH₂C≡CH | H | H |
| NH₂ | H | CH₂Cl | H | H |
| NH₂ | Me | CH₂Cl | H | H |
| NH₂ | H | CHCl₂ | H | H |
| NH₂ | Me | CHCl₂ | H | H |
| NH₂ | H | CCl₃ | H | H |
| NH₂ | Me | CCl₃ | H | H |
| NH₂ | H | CH₂F | H | H |
| NH₂ | Me | CH₂F | H | H |
| NH₂ | H | CHF₂ | H | H |
| NH₂ | Me | CHF₂ | H | H |
| NH₂ | H | CF₃ | H | H |
| NH₂ | Me | CF₃ | H | H |
| NH₂ | H | CH₂CH₂Cl | H | H |
| NH₂ | Me | CH₂CH₂Cl | H | H |
| NH₂ | H | CH₂OH | H | H |
| NH₂ | Me | CH₂OH | H | H |
| NH₂ | H | CH₂SMe | H | H |
| NH₂ | Me | CH₂SMe | H | H |
| NH₂ | H | CH₂SCH₂Cl | H | H |
| NH₂ | Me | CH₂SCH₂Cl | H | H |
| NH₂ | H | Ph | H | H |
| NH₂ | Me | Ph | H | H |
| NH₂ | H | Ph-2-Cl | H | H |
| NH₂ | Me | Ph-2-Cl | H | H |
| NH₂ | H | Ph-3-Cl | H | H |
| NH₂ | Me | Ph-3-Cl | H | H |
| NH₂ | H | Ph-4-Cl | H | H |
| NH₂ | Me | Ph-4-Cl | H | H |
| NH₂ | Me | Ph-2-Br | H | H |
| NH₂ | Me | Ph-3-I | H | H |
| NH₂ | Me | Ph-4-F | H | H |
| NH₂ | Me | Ph-2-Me | H | H |
| NH₂ | Me | Ph-3-OMe | H | H |
| NH₂ | Me | Ph-4-SMe | H | H |
| NH₂ | Me | Ph-2-SO₂Me | H | H |
| NH₂ | Me | Ph-3-CN | H | H |
| NH₂ | Me | Ph-4-NO₂ | H | H |
| NH₂ | Me | Ph-2-CO₂Me | H | H |
| NH₂ | Me | Ph-3-NH₂ | H | H |
| NH₂ | Me | Ph-4-Ph | H | H |
| NH₂ | Me | Ph-2-OPh | H | H |
| NH₂ | Me | Ph-3-SPh | H | H |
| NH₂ | Me | Ph-4-OH | H | H |
| NH₂ | H | COMe | H | H |
| NH₂ | Me | COMe | H | H |
| NH₂ | H | COEt | H | H |
| NH₂ | Me | COEt | H | H |
| NH₂ | H | COCH₂Cl | H | H |
| NH₂ | Me | COCH₂Cl | H | H |
| NH₂ | H | CO₂H | H | H |
| NH₂ | Me | CO₂H | H | H |
| NH₂ | H | CO₂Me | H | H |
| NH₂ | Me | CO₂Me | H | H |
| NH₂ | H | CO₂Et | H | H |
| NH₂ | Me | CO₂Et | H | H |
| NH₂ | H | CONHMe | H | H |
| NH₂ | Me | CONHMe | H | H |
| NH₂ | H | CONMe₂ | H | H |
| NH₂ | Me | CONMe₂ | H | H |
| NH₂ | H | OH | H | H |
| NH₂ | Me | OH | H | H |
| NH₂ | H | OMe | H | H |
| NH₂ | Me | OMe | H | H |
| NH₂ | H | SMe | H | H |
| NH₂ | Me | SMe | H | H |
| NH₂ | H | CN | H | H |
| NH₂ | Me | CN | H | H |
| NH₂ | H | NH₂ | H | H |
| NH₂ | Me | NH₂ | H | H |
| NH₂ | H | NHMe | H | H |
| NH₂ | Me | NHMe | H | H |
| NH₂ | H | NMe₂ | H | H |
| NH₂ | Me | NMe₂ | H | H |
| NH₂ | H | -CH₂-O- | | H |
| NH₂ | Me | -CH₂-O- | | H |
| NH₂ | H | -(CH₂)₂- | | H |
| NH₂ | Me | -(CH₂)₂- | | H |
| NH₂ | H | -(CH₂)₃- | | H |
| NH₂ | Me | -(CH₂)₃- | | H |
| NH₂ | Me | H | H | 3-F |
| NH₂ | Me | H | H | 4-F |
| NH₂ | Me | H | H | 5-F |
| NH₂ | Me | H | H | 6-F |
| NH₂ | Me | H | H | 4-Cl |
| NH₂ | Me | H | H | 5-Cl |
| NH₂ | Me | H | H | 4-Br |
| NH₂ | Me | H | H | 4-I |
| NH₂ | Me | H | H | 4-Me |
| NH₂ | Me | H | H | 5-Me |
| NH₂ | Me | H | H | 4-OMe |
| NH₂ | Me | H | H | 4-SMe |

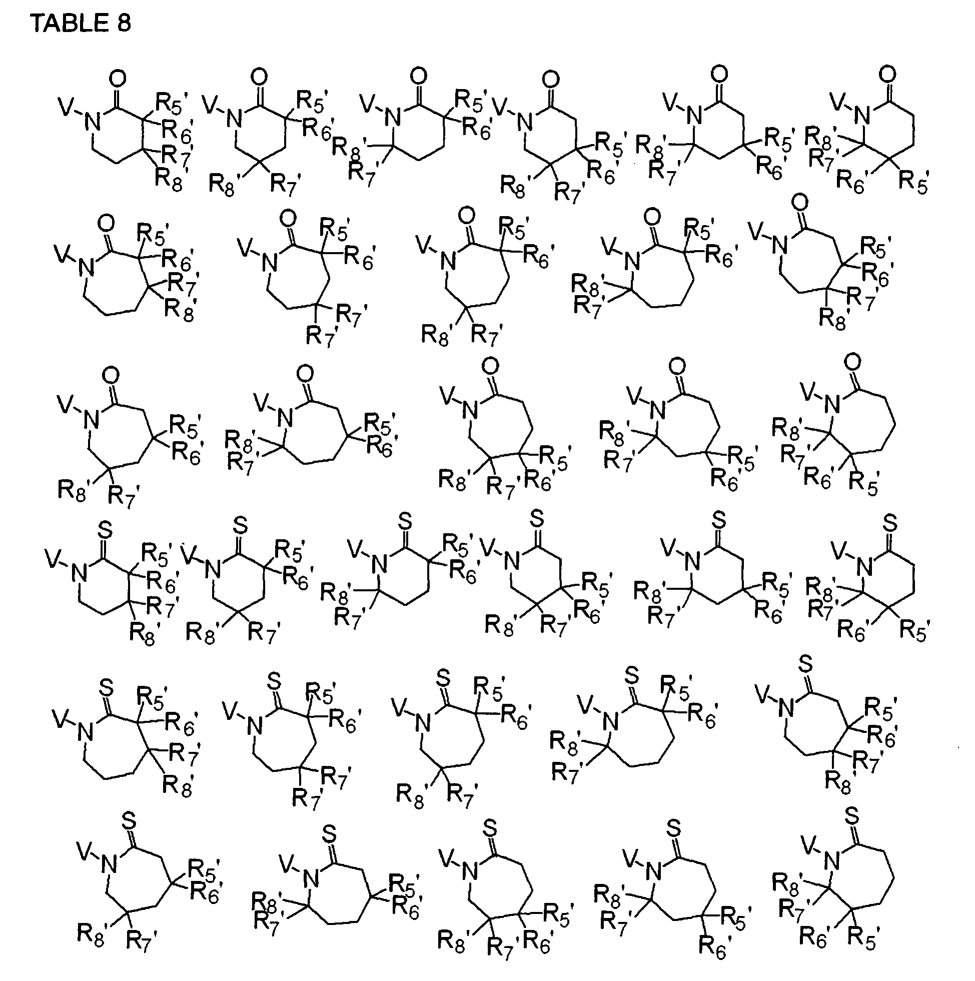

V is the same as the above-mentioned formula (27).

| R₁ =CF₃ R₃ =R₄ =H, X=H | | | | |
|---|---|---|---|---|
| G | R₅' | R₆' | R₇' | R₈' |
| NO₂ | Me | Me | Me | H |
| NO₂ | Me | H | Me | Me |
| NO₂ | Me | Me | Me | Me |
| NO₂ | Me | Et | Me | H |
| NO₂ | Me | Me | E t | H |
| NO₂ | Me | H | Me | Et |
| NO₂ | Et | H | Me | Me |
| NO₂ | Me | Et | Me | Me |
| NO₂ | Me | Me | Me | Et |
| NH₂ | Me | Me | Me | H |
| NH₂ | Me | H | Me | Me |
| NH₂ | Me | Me | Me | Me |
| NH₂ | Me | Et | Me | H |
| NH₂ | Me | Me | Et | H |
| NH₂ | Me | H | Me | Et |
| NH₂ | Et | H | Me | Me |
| NH₂ | Me | Et | Me | Me |
| NH₂ | Me | Me | Me | Et |

**TABLE 9**

| | |
|---|---|
| 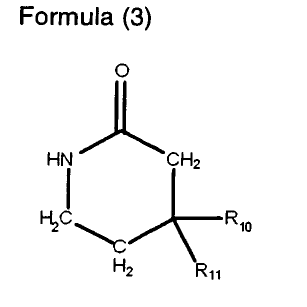 | |

| R₁₀ | R₁₁ |
|---|---|
| -(CH₂)₂- | |
| -CH₂CCl₂- | |
| -CH₂CF2 | |
| -(CH₂)₃- | |
| -(CH₂)₄- | |
| -(CH₂)₅- | |
| -(CH₂)₆- | |
| -CH₂-O- | |
| -(CH₂)₂-O- | |
| -(CH₂)₃-O- | |
| -(CH₂)₄-O- | |
| -(CH₂)₅-O- | |
| -CH₂-S- | |
| -(CH₂)₂-S- | |
| -(CH₂)₃-S- | |
| -(CH₂)₄-S- | |
| -(CH₂)₅-S- | |
| -CH₂-NH- | |
| -(CH₂)₂-NH- | |
| -(CH₂)₃-NH- | |
| -(CH₂)₄-NH- | |
| -(CH₂)₅-NH- | |
| -CH₂-O-CH₂- | |
| -(CH₂)₂-O-CH₂- | |
| -(CH₂)₃-O-CH₂- | |
| -(CH₂)₄-O-CH₂- | |
| -(CH₂)₂-O-(CH₂)₂- | |
| -(CH₂)₃-O-(CH₂)₂- | |
| -CH₂-S-CH₂- | |
| -(CH₂)₂-S-CH₂- | |
| -(CH₂)₃-S-CH₂- | |
| -(CH₂)₄-S-CH₂- | |
| (CH₂)₂-S-(CH₂)₂- | |
| -(CH₂)₃-S-(CH₂)₂- | |
| -CH₂-NH-CH₂- | |
| -(CH₂)₂-NH-CH₂- | |
| -(CH₂)₃-NH-CH₂- | |
| -(CH₂)₄-NH-CH₂- | |
| -(CH₂)₂-NH-(CH₂)₂- | |
| -(CH₂)₃-NH-(CH₂)₂- | |

The dose of the compounds of the present invention as a herbicide varies depending upon the application site, the season for application, the manner of application, the target weeds, the type of crop plants and the like. However, it is usually within a range of about from 0.001 to 50 kg, preferably about from 0.01 to 10 kg, per hectare (ha) as the amount of the active ingredient.

The compounds of the present invention may be used as a herbicide for paddy fields in submerged soil treatment or foliage treatment.

Paddy fields weeds include, for example, Potamogetonaceae weeds such as roundleaf pondweed (Potamogeton distinctus), Alismataceae weeds such as narrowleaf waterplantain (Alisma canaliculatum), japanese ribbon wapato (Sagittaria pygmaea) and arrowhead (Sagittaria trifolia), Gramineae weeds such as sprangletop (Leptochloa chinensis),barnyardgrass (Echinochloa crus-galli), barnyardgrass (Echinochloa oryzicola), leersia japonica Makino (Homalocenchrus japonocus) and Knotgrass (Paspalum distichum), Cyperaceae weeds such as water chestnut (Eleocharis kuroguwai), japanese bulrush (Scirpus juncoides), Shizui (Scirpus nipponicus), water nutgrass (Cyperus serotinus), umbrellaplant (Cyperus difformis) and Hinagavatsuri (Cyperus hakonensis), Lemnaceae weeds such as giant duckweed (Spirodela polyrhiza) and duckmeat (Lemna paucicostata), Commelinaceae weeds such as march dayflower (Murdannia keisak), Pontederiaceae weeds such as monochoria species (Monochoria korsakowii) and monochoria (Monochoria vaginalis), Elatinaceae weeds such as waterwort (Elatine triandra), Lythraceae weeds such as red stem (Ammannia multiflora) and toothcup (Rotala indica), Oenotheraceae weeds such as fireweed ludwigia (Lidwigia epilobioides), Scrophulariaceae weeds such as dopatorium (Dopatrium junceum), Ooabunome (Gratiola japonica), limnophila (Limnophila sessilifolia), false pimpernel (Lindernia pyxidaria) and low falsepimpemel (Lindernia dubia), Leguminosae weeds such as Indian jointvetch (Aeschynomene indica) and Compositae weeds such as devils beggarticks (Bidens frondosa) and bur beggarticks (Bidens tripartite).

The compounds of the present invention may be used as a herbicide for upland fields in soil treatment, soil incorporation treatment and foliage treatment.

Cropland weeds include, for example, broad-leaved weeds such as Solanaceous weeds (Solanaceae) represented by black nightshade (Solanum nigrum) and jimsonweed (Datura stramonium), Malvaceous weeds (Malvaceae) represented by velvetleaf (Abutilon theophrasti) and prickly sida (Sida spinosa), Convolvulaceous weeds (Convolvulaceae) represented by morningglories (Ipomoea spps.) including common morningglory (Ipomoea purpurea) and bindweeds (Calystegia spps.), Amaranthaceous weeds (Amaranthaceae) represented by livid amaranth (Amaranthus lividus) and redroot pigweed (Amaranthus retroflexus), Composite weeds (Compositae) represented by common cocklebur (xanthium pensylvanicum), common ragweed (Ambrosia artemisiaefolia), sunflower (Helianthus annuus), hairy galinsoga (Galinsoga ciliate), Creeping thistle (Cirsium arvense), common groundsel (Senecio vulgaris) and annual fleabane (Erigeron annus), Cruciferous weeds (Cruciferae) represented by India field cress (Rorippa indica), kedlock (Sinapis arvensis) and shepherd's purse (Capsella Bursapastoris), Polygonaceous weeds (Polygonaceae) represented by posumbu knotweed (Polygonum Blumei) and wild buckwheat (Polygonum convolvulus), Portulacaceous weeds (Portulacaceae) represented by common purslane (Portulaca oleracea), Chenopodiaceous weeds (Chenopodiaceae) represented by common lambsquater (Chenopodium album), figleaved goosefoot (Chenopodium ficifolium) and kochia (Kochia scoparia), Caryophyllaceous weeds (Caryophyllaceae) represented by common chickweed (Stellaria media), Scrophulariaceous weeds (Scrophulariaceae) represented by persian speedwell (Veronica persica), Commelinaceous weeds (Commelinaceae) represented by asiatic dayflower (Commelina communis), Labiate weeds (Labiatae) represented by deadnettle (Lamium amplexicaule) and red deadnettle (Lamium purpureum), Euphorbiaceous weeds (Euphorbiaceae) represented by prostrate spurge (Euphorbia supine) and spotted spurge (Euphorbia maculate), Rubiaceous weeds (Rubiaceae) represented by beadstraw (Galium spurium) and Indian madder (Rubia akane), Violaceous weeds (Violaceae) represented by violet (Viola mandshurica), Leguminous weeds (Leguminosae) represented by hempsesbania (Sesbania exaltata) and sicklepod (Cassia obtusifolia), Oxsaldaseous weeds (Oxsaldaseae) represented by creeping woodsorrel (Oxsalis courniculata), and Graminaceous weeds represented by shatternace (Sorgham bicolor), fall panicum (Panicum dichotomiflorum), johnsongrass (Sorghum halepense), barnyardgrass (Echinochloa crus-galli var. crus-galli), barnyardgrass (Echinochloa crus-galli var. praticola), barnyardgrass (crop) (Echinochloa utilis), large crabgrass (Digitaria ciliaris), wild oat (Avena fatua), blackgrass (Alopecurus myosuroides), goosegrass (Eleusine indica), green foxtail (Setaria viridis), giant foxtail (Setaria faberi) and water foxtail (Alopecurus aegualis), and Cyperaceous weeds represented by purple nutsedge (Cyperus rotundus,Cyperus esculentus).

The compounds of the present invention may be used not only agriculturally and horticulturally for paddy fields, upland fields and orchards but also for non-agricultural fields such as lawns, playgrounds, vacant lots, road shoulders and railroad shoulders in soil treatment, soil incorporation treatment and foliage treatment. The target weeds include, in addition to the cropland and orchard weeds mentioned above, annual bluegrass (Poa annua), dandelion (Taraxacum officinale), tall fleabane (Conyza sumatrensis), flexuous bittercress (Cardamine flexuosa), white clover (Trifolium repens), lawn pennywort (Hydrocotyle sibthorpioides), Asiatic plantain (Plantago asiatica), himekugu (Cyperus brevifolius, Kyllinga brevifolia), field horsetail (Equisetum arvense) and the like.

The compounds of the present invention are commonly applied before and after rice transplanting and may also be applied during rice transplanting.

The compounds of the present invention may be combined with other herbicides, various insecticides, fungicides, plant growth regulators or synergists at the time of the preparation of the formulations or at the time of the application, as the case requires.

Particularly, combined use of the compound of the present invention with another herbicide can be expected to result in lower cost attributable to reduction in the dose, a broader spectrum and a higher herbicidal effect attributable to synergistic action of the combined chemical. In such as case, the compound of the present invention can be combined with plural known herbicides simultaneously.

Herbicides preferably combined with the compounds of the present invention include, for example, pyrazosulfuron-ethyl (general name), bensulfuron-methyl (general name), cinosulfuron (general name), imazosulfuron (general name), azimsulfuron (general name), halosulfuron-methyl (general name), pretilachlor (general name), esprocarb (general name), pyrazolinate (general name), pyrazoxyfen (general name), benzofenap (general name), daimuron (general name), bromobutide (general name), naproanilide (general name), clomeprop (general name), CNP (general name), chlomethoxynil (general name), bifenox (general name), oxadiazon (general name), oxadiargyl (general name), Carfentrazone-ethyl (general name), cafenstrole (general name), oxaziclomefone (general name), indanofan (general name), pentoxazone (general name), pyriminobac-methyl (general name), cyhalofopbutyl (general name), fentrazamide (general name), mefenacet (general name), butachlor (general name), butenachlor (general name), dithiopyl (general name), benfuresate (general name), pyributicarb (general name), benthiocarb (general name), dimepiperate (general name), molinate (general name), butamifos (general name), quinclorac (general name), cinmethylin (general name), simetryn (general name), bensulide (general name), dimethametryn (general name), 2,4-D (general name), MCPA, MCPB, etobenzanid, cumyluron (general name), thenylchlor (general name), ethoxysulfuron (general name), quinoclamine (general name), Bentazone (general name), benzobicyclon (general name), pyriftalid (general name), bispyribac, pyraclonil (general name), anilofos (general name), OK-701 (trial name), cyclosulfamuron (general name), penoxsulam (general name), tefuryltrione (general name), pyrimisulfan (general name), propyrisulfuron (general name), NC-620 (trial name), mesotrion (general name), flucetosulfuron (general name), orthosulfamuron (general name), propanil (general name), metamifop (general name), etobenzanide (general name), KUH-071 (trial name) and ipfencarbazone (general name).

When the compounds of the present invention are used as a herbicide, they are usually mixed with a suitable solid carrier or a liquid carrier. Further, if desired, a surfactant, a penetrating agent, a spreader, a thickner, an antifreezing agent, a binder, an anticaking agent or a stabilizing agent to prepare an optional formulation such as a liquid formulation, an emulsifiable concentrate, a wettable powder, a dry flowable, a flowable, a dust or a granule.

Further, such formulations as mentioned above may be encapsulated in a watersoluble material to save labor and improve safety.

As solid carriers, for example, natural minerals such as kaolinite, pyrophyllite, sericite, talc, bentonite, acid clay, attapulgite, zeolite and diatomaceous earth, inorganic salts such as calcium carbonate, ammonium sulfate, sodium sulfate and potassium chloride, synthetic silica and synthetic silicates may be mentioned.

As liquid carriers, for example, water, alcohols (such as ethylene glycol, propylene glycol, isopropanol), aromatic hydrocarbons (such as xylene, alkylbenzene, alkylnaphthalene), ethers (such as butyl cellosolve), ketones (scuh as cyclohexanone), esters (such as γ-butyrolactone), acid amides (such as N-methylpyrrolidone, N-octylpyrrolidone) and vegetable oils (scuh as soybean soil, rapeseed oil, cottonseed oil and castor oil).

These solid and liquid carriers may be used singly or in combination of at least two.

As surfactants, for example, nonionic surfactants such as polyoxyethylene alkyl aryl ether, polyoxyethylene styryl phenyl ether, polyoxyethylene polyoxypropylene block copolymers, polyoxyethylene fatty acid esters, sorbitan fatty acid esters and polyoxyethylene sorbitan fatty acid esters, and ionic surfactants such as alkylbenzenesulfonate salts, lignin sulfonate salts, alkylsulfosuccinates, naphthalenesulfonate salts, alkylnaphthalenesulfonate salts, naphthalenesulfonic acid formalin condensate salts, alkylnaphthalenesulfonic acid formalin condensate salts, polyoxyethlene alkyl aryl ether sulfate or phosphate, polyoxyethylene styryl phenyl ether sulfate or phosphate and alkylamine salts.

The amounts of these surfactants are not particularly limited, but usually, they are preferably from 0.05 to 20 parts by weight in relation to 100 parts by weight of the formulation of the present invention. These surfactants may be used singly or in combination of at least two.

Now, examples of formulations of the compounds of the present invention will be given. However, it should be understood that the present invention is by no means restricted to such specific examples. In the following Formulation Examples, "parts" means parts by weight.

| Wettable powder | |
|---|---|
| Compound of the present invention | 0.1-80 parts |
| Solid carrier | 5-98.9 parts |
| Surfactant | 1-10 parts |
| Others | 0-5 parts |

As the others, for example, an anticaking agent and a stabilizing agent may be mentioned.

| Emulsifiable concentrate | |
|---|---|
| Compound of the present invention | 0.1-30 parts |
| Liquid carrier | 55-995 parts |
| Surfactant | 4.9-15 parts |

| Flowable | |
|---|---|
| Compound of the present invention | 0.1-70 parts |
| Liquid carrier | 15-98.89 parts |
| Surfactant | 1-12 parts |
| Others | 0.01-30 parts |

As the others, for example, an antifreezing agent and a thickener may be mentioned.

| Dry flowable | |
|---|---|
| Compound of the present invention | 0.1-90 parts |
| Solid carrier | 0-98.9 parts |
| Surfactant | 1-20 parts |
| Others | 0-10 parts |

As the others, for example, a binder and a stabilizing agent may be mentioned.

| Liquid formulation | |
|---|---|
| Compound of the present invention | 0.01-30 parts |
| Liquid carrier | 0.1-50 parts |
| Water | 50-99.89 parts |
| Others | 0-10 parts |

As the others, for example, an antifreezing agent and a spreader may be mentioned.

| Granule | |
|---|---|
| Compound of the present invention | 0.01-10 parts |
| Solid carrier | 90-99.99 parts |
| Others | 0-10 parts |

As the others, for example, a binder and a stabilizing agent may be mentioned.

The above-mentioned formulations are applied directly or after diluted with water by a factor of 1 to 10000.

### PREPARATION EXAMPLES

Now, specific examples of agrochemical preparations containing compounds of the present invention will be given. However, it should be understood that the present invention is by no means restricted to such specific examples. In the following formulation examples, "parts" means parts by weight.

| [Formulation Example 1] Wettable powder | |
|---|---|
| Compound 1-1 of the present invention | 20 parts |
| Pyrophyllite | 76 parts |
| Sorpol 5039 | 2 parts |
| (trade name for a mixture of a nonionic surfactant and an anionic surfactant manufactured by Toho Chemical Industry Co., Ltd.) | |
| Carplex #80 | 2 parts |
| (trade name for a synthetic hydrous silicate manufactured by Shionogi Pharmaceutical Co., Ltd.) | |

The above ingredients are homogenously pulverized and mixed to form a wettable powder.

| [Formulation Example 2] Emulsifiable concentrate | |
|---|---|
| Compound 1-1 of the present invention | 5 parts |
| Xylene | 75 parts |
| N-Methylpyrrolidone | 15 parts |
| Sorpol 2680 | 5 parts |
| (trade name for a mixture of a nonionic surfactant and an anionic surfactant manufactured by Toho Chemical Industry Co., Ltd.) | |

The above ingredients are mixed to from an emulsifiable concentrate.

| [Formulation Example 3] Flowable | |
|---|---|
| Compound 1-1 of the present invention | 25 parts |
| Agrizole S-710 | 10 parts |
| (trade name for a nonionic surfactant manufactured Kao Corporation) | |
| Lunox 1000C | 0.5 part |
| (trade name for an anionic surfactant manufactured by Toho Chemical Industry Co., Ltd.) | |
| Xanthan gum | 0.02 part |
| Water | 64.48 parts |

The above ingredients are homogeneously mixed and wet-pulverized to form a flowable.

| [Formulation Example 4] Dry flowable | |
|---|---|
| Compound 1-1 of the present invention | 75 parts |
| HITENOL NE-15 | 5 parts |
| (trade name for an anionic surfactant manufactured by Dai-Ichi Kogyo Seiyaku Co., Ltd.) | |
| Vanilex N | 10 parts |
| (trade name for an anionif surfactant manufactured by Nippon Paper Industries Co., Ltd.) | |
| Carplex #80 | 10 parts |
| (trade name for a synthetic hydrous silicate manufactured by Shionogi Parmaceutical Co., Ltd.) | |

The above ingredients are homogeneously mixed and pulverized, then kneaded with a small amount of water by stirring and granulated through an extrusion granulator and dried to form a dry flowable.

| [Formulation Example 5] Granule | |
|---|---|
| Compound 1-1 of the present invention | 1 part |
| Bentonite | 55 parts |
| Talc | 44 parts |

The above ingredients are homogenously mixed and pulverized then kneaded with a small amount of water by stirring and granulated through an extrusion granulator and dried to form a granule.

| [Formulation Example 6] Granule | |
|---|---|
| Compound 1-1 of the present invention | 1 part |
| Compound (A) | 0.07 part |
| DBSN | 3 parts |
| Epoxylated soybean oil | 1 part |
| Bentonite | 30 parts |
| Talc | 64.93 parts |

DBSN means sodium dodecylbenzenesulfonate.

Now, the syntheses of the compounds of the present invention will be described with reference to Examples. However, it should be understood that the present invention is by no means restricted to such specific Examples.

### EXAMPLES

### [EXAMPLE 1]

### Synthesis of 1-[2-(N-ethoxycarbonyl-N-trifluoromethanesulfonyl)aminobenzyl]piperidin-2-one (Compound III-9 of the present invention)

### Step 1;

### Synthesis of 1,1,1-trifluoro-N-[2-(hydroxymethyl)phenyl]methanesulfonamide

2-Aminobenzyl alcohol (4.0 g, 32.5 mmol) in 60 ml chloroform was cooled to 0°C and mixed with triethylamine (3.94 g, 39.0 mmol), and trifluoromethanesulfonic anhydride (11.0 g, 39.0 mmol) was added dropwise with stirring. The reaction solution was stirred for 4 hours while it was gradually warmed to room temperature. After the reaction, chloroform and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 1/1) (volume ratio, the same applies hereafter) to obtain 5.13 g of the desired product.

### Step 2;

### Synthesis of 1-(2-trifluoromethanesulfonylaminobenzyl)piperidin-2-one (Compound III-1 of the present invention)

1,1,1-trifluoro-N-[2-(hydroxymethyl)phenyl]methanesulfonamide(500 mg, 1.96 mmol) in 10 ml of toluene was mixed with 2-piperidinone (232 mg, 2.35 mmol) and p-toluenesulfonic acid monohydrate (169 mg, 0.98 mmol) and refluxed under heating with stirring for 6 hours with azeotropic water separation with a dean-stark apparatus. After the reaction, chloroform and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 1/1) to obtain 70 mg of the desired product (Compound III-1 of the present invention) as a pale yellow solid.

### Step 3;

### Synthesis of 1-[2-(N-ethoxycarbonyl-N-trifluoromethanesulfonyl)aminobenzyl]piperidin-2-one (Compound III-9 of the present invention)

A suspension of 63 wt% sodium hydride (dispersed in mineral oil, 7 mg, 0.18 mmol) in 5 ml of tetrahydrofuran was mixed with 1-(2-trifluoromethanesulfonylaminobenzyl)piperidin-2-one (40 mg, 0.12 mmol) with cooling with ice and stirring and stirred at room temperature for 5 minutes. Ethyl chloroformate (20 mg, 0.18 mmol) was gradually added dropwise with cooling with ice, and the reaction solution was stirred for another 16 hours while it was gradually warmed to room temperature. After the reaction, chloroform and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 4/1) to obtain 35 mg of the desired product (Compound III-9 of the present invention) as a white solid.

### [EXAMPLE 2]

### Synthesis of 1-[2-(N-ethoxycarbonyl-N-trifluoromethanesulfonyl)aminobenzyl]-4,4-dimethylpiperidin-2-one (Compound III-58 of the present invention)

### Step 1;

### Synthesis of 4,4-dimethyl-1-(2-nitrobenzyl)piperidine-2,6-dione

3,3-Dimethylglutarimide (3.0 g, 21.3 mmol) in 20 ml of dimethylformamide were mixed with potassium carbonate (4.41 g, 31.9 mmol) and 2-nitrobenzyl bromide (4.59 g, 21.3 mmol) and stirred at 70°C for 2 hours. After the reaction, ethyl acetate and saturated aqueous ammonium chloride were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/1) to obtain 4.80 g of the desired product as a pale yellow solid.

### Step 2;

### Synthesis of 6-hydroxy-4,4-dimethyl-1-(2-nitrobenzyl)piperidin-2-one

A suspension of sodium boron hydride(13.7g, 362 mmol) in 150m1 of tetrahydrofuran was mixed with 4,4-dimethyl-1-(2-nitrobenzyl)piperidine-2,6-dione(5.0g, 18.1 mmol) in 50 ml of tetrahydrofuran at 0°C with stirring and further mixed with 15 ml of water. The reaction solution was warmed to room temperature and stirred for 1 hour. After the reaction, 100 ml of saturated aqueous ammonium chloride was added, while the reaction solution was stirred at 0°C. After the reaction solution was concentrated under reduced pressure, and ethyl acetate was added. The organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/2) to obtain 2.31 g of the desired product as a pale yellow solid.

### Step 3;

### Synthesis of 4,4-dimethyl-1-(2-nitrobenzyl)piperidin-2-one (Intermediate XI-9)

6-Hydroxy-4,4-dimethyl-1-(2-nitrobenzyl)piperidin-2-one (1.3 g, 4.67 mmol) in 12 ml of trifluoroacetic acid was mixed with triethylsilane (815 mg, 7.01 mmol) at 0°C with stirring and stirred at room temperature for 2 hours. After the reaction, the reaction solution was concentrated under reduced pressure, and ethyl acetate and saturated aqueous sodium hydrogen carbonate were added. The organic layer was separated, washed with water, then dried with saturated aqueous sodium chloride and over anhydrous sodium sulfate and concentrated under reduced pressure to obtain 1.20 g of the desired product (Intermediate XI-9) as a yellow solid.

### Step 4;

### Synthesis of 1-(2-aminobenzyl)-4,4-dimethylpiperidin-2-one (Intermediate XI-10)

4,4-Dimethyl-1-(2-nitrobenzyl)piperidin-2-one (1.2 g, 4.57 mmol) in a solvent mixture of 9 ml of ethanol and 3 ml of water was mixed with reduced iron(1.02g, 18.3 mmol) and ammonium chloride(122mg, 2.29 mmol), and refluxed under heating for 1 hour with stirring. After the reaction, the reaction solution was allowed to cool to room temperature, filtered through Celite, and the Celite was washed with ethyl acetate. The organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/2) to obtain 900 mg of the desired product (Intermediate XI-10).

### Step 5;

### Synthesis of 4,4-dimethyl-1-(2-trifluoromethanesulfonylaminobenzyl)piperidin-2-one (Compound III-46 of the present invention)

1-(2-Aminobenzyl)-4,4-dimethylpiperidin-2-one (650 mg, 2.80 mmol) in 10 ml of dichloromethane was cooled to 0°C and mixed with triethylamine (850 mg, 8.40 mmol). Trifluoromethanesulfonic anhydride (1.58 g, 5.60 mmol) in 5ml dichloromethane was added dropwise, and the reaction solution was stirred for 1 hour while it was gradually warmed to room temperature. After the reaction, 1 N hydrochloric acid and chloroform were added, and the organic layer was separated, washed with water, then dried with saturated aqueous sodium chloride and over anhydrous sodium sulfate and concentrated under reduced pressure. The resulting residue was mixed with 10 ml of dioxane and aqueous tetrabutylammonium hydroxide (40%, 3.63g, 5.60 mmol) and stirred at room temperature for 2 hours. After the reaction, ethyl acetate and 1 N hydrochloric acid were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 4/1 to 1/2) to obtain 650 mg of the desired product (Compound III-46 of the present invention) as a pale yellow solid.

### Step 6;

### Synthesis of 1-[2-(N-ethoxycarbonyl-N-trifluoromethanesulfonyl)aminobenzyl]-4,4-dimethylpiperidin-2-one (Compound III-58 of the present invention)

4,4-Dimethyl-1-(2-trifluoromethanesulfonylaminobenzyl)piperidin-2-one (100 mg, 0.27 mmol) in 5 ml of acetonitrile was mixed with sodium hydrogen carbonate(120mg, 1.43 mmol) and ethyl chloroformate (0.14 g, 1.29 mmol) and refluxed under heating for 2 hours with stirring. After the reaction, ethyl acetate and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 1/1) to obtain 90 mg of the desired product (Compound III-58 of the present invention) as a white solid.

### [EXAMPLE 3]

### Synthesis of 1-[2-(N-cyclopropylcarbonyl-N-trifluoromethanesulfonyl)aminobenzyl]-4,4-dimethylpiperidin-2-one (Compound III-51 of the present invention)

A suspension of sodium hydride (63%)(40 mg, 1.05 mmol) in 3 ml of tetrahydrofuran was mixed with 4,4-dimethyl-1-(2-trifluoromethanesulfonylaminobenzyl)piperidin-2-one (160 mg, 0.44 mmol) with cooling with ice and stirred at 0°C for 5 minutes. Then, cyclopropanecarbonyl chloride (70mg, 0.67 mmol) was gradually added dropwise, and the reaction solution was stirred overnight while it was gradually warmed to room temperature. After the reaction, ethyl acetate and saturated aqueous ammonium chloride were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 3/1) to obtain 140 mg of the desired product (Compound III-51 of the present invention) as a white solid.

### [EXAMPLE 4]

### Synthesis of 1-[2-(N-ethoxycarbonyl-N-trifluoromethanesulfonyl)aminobenzyl]-4-methylpiperidin-2-one (Compound III-22 of the present invention)

### Step 1;

### Synthesis of 4-methyl-1-(2-nitrobenzyl)piperidine-2,6-dione

3-Methylglutaric anhydride (2.0 g, 15.6 mmol) in 20 ml of tetrahydrofuran was mixed with 2-nitrobenzylamine hydrochloride (2.94 g, 15.6 mmol), and triethylamine (3.32 g, 32.8 mmol) was added dropwise while the reaction solution was stirred with cooling with ice. The reaction solution was stirred at room temperature for 2 hours. After the reaction, the reaction solution was concentrated under reduced pressure and acidified with 3 N hydrochloric acid carefully while it was stirred with cooling with ice. Ethyl acetate was added, and the organic layer was separated, washed with water, then dried with saturated aqueous sodium chloride and over anhydrous sodium sulfate and concentrated under reduced pressure. The resulting residue was mixed with 10 ml of acetic anhydride and 2 ml of triethylamine and stirred at 80°C for 2 hours. After the reaction, the reaction solution was concentrated under reduced pressure and acidified with 3 N hydrochloric acid carefully while it was stirred with cooling with ice, and ethyl acetate was added. The organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/1) to obtain 3.42 g of the desired product as a pale yellow solid.

### Step 2;

### Synthesis of 6-hydroxy-4-methyl-1-(2-nitrobenzyl)piperidin-2-one

A suspension of sodium boron hydride (1.2 g, 31.7 mmol) in 20ml of tetrahydrofuran was mixed with 4-methyl-1-(2-nitrobenzyl)piperidine-2,6-dione(1.66 g, 6.33 mmol) in 10 ml of tetrahydrofuran at 0°C with stirring and further mixed with 3 ml of water. The reaction solution was at 0°C for 6 hours. After the reaction, the reaction solution was gradually added to 30 ml of 1 N hydrochloric acid at 0°C with stirring. The reaction solution was concentrated under reduced pressure, and ethyl acetate was added. The organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/2) to obtain 0.82 g of the desired product as a pale yellow solid.

### Step 3;

### Synthesis of 4-methyl-1-(2-nitrobenzyl)piperidin-2-one (Intermediate XI-1)

6-Hydroxy-4-methyl-1-(2-nitrobenzyl)piperidin-2-one (0.52 g, 1.97 mmol) in 5 ml of trifluoroacetic acid was mixed with triethylsilane (0.35 g, 3.01 mmol) at 0°C with stirring and stirred at room temperature for 1 hour. After the reaction, the reaction solution was concentrated under reduced pressure, and ethyl acetate and saturated aqueous sodium hydrogen carbonate were added. The organic layer was separated, washed with water, then dried with saturated aqueous sodium chloride and over anhydrous sodium sulfate and concentrated under reduced pressure to obtain 0.40 g of the desired product (Intermediate XI-1) as a yellow solid.

### Step 4;

### Synthesis of 1-(2-aminobenzyl)-4-methylpiperidin-2-one (Intermediate XI-2)

4-Methyl-1-(2-nitrobenzyl)piperidin-2-one (0.4g, 1.61 mmol) in a solvent mixture of 5 ml of ethanol and 2 ml of water, then and mixed with reduced iron (0.4g, 7.16 mmol) and ammonium chloride (40mg, 0.75 mmol) and refluxed under heating for 2 hours with stirring. After the reaction, the reaction solution was cooled to room temperature, filtered through Celite, and the Celite was washed with ethyl acetate. The organic layer was separated, washed with water, then dried with saturated aqueous sodium chloride and over anhydrous sodium sulfate and concentrated under reduced pressure to obtain 350 mg of the desired product (Intermediate XI-2).

### Step 5;

### Synthesis of 4-methyl-1-(2-trifluoromethanesulfonylaminobenzyl)piperidin-2-one (Compound III-19 of the present invention)

1-(2-Aminobenzyl)-4-methylpiperidin-2-one (350 mg, 1.60 mmol) in 8 ml of dichloromethane was cooled to 0°C and mixed with triethylamine (490 mg, 4.84 mmol), and trifluoromethanesulfonic anhydride (0.90 g, 3.19 mmol) in 3ml dichloromethane was added dropwise with stirring. The reaction solution was stirred for 1 hour while it was gradually warmed to room temperature. After the reaction, 1 N hydrochloric acid and chloroform were added, and the organic layer was separated, washed with water, then dried with saturated aqueous sodium chloride and over anhydrous sodium sulfate and concentrated under reduced pressure. The resulting residue was mixed with 8 ml of 1,4-dioxane and aqueous tetrabutylammonium hydroxide (40%, 2.08 g, 3.21 mmol) and stirred at room temperature for 2 hours. After the reaction, ethyl acetate and 1 N hydrochloric acid were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 4/1 to 1/2) to obtain 250 mg of the desired product (Compound III-19 of the present invention).

### Step 6;

### Synthesis of 1-[2-(N-ethoxycarbonyl-N-trifluoromethanesulfonyl)aminobenzyl]-4-methylpiperidin-2-one (Compound III-22 of the present invention)

4-Methyl-1-(2-trifluoromethanesulfonylaminobenzyl)piperidin-2-one (50 mg, 0.14 mmol) in 5 ml of acetonitrile was mixed with sodium hydrogen carbonate (40 mg, 0.48 mmol) and ethyl chloroformate (30 mg, 0.28 mmol) and refluxed under heating for 2 hours with stirring. After the reaction, ethyl acetate and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 1/1) to obtain 40 mg of the desired product (Compound III-22 of the present invention) as a white solid.

### [EXAMPLE 5]

### Synthesis of 1-[2-(N-ethoxycarbonyl-N-trifluoromethanesulfonyl)aminobenzyl]-3-ethylpiperidin-2-one (Compound IV-9 of the present invention)

### Step 1;

### Synthesis of 3-ethylpiperidin-2-one

n-Butyllithium-hexane solution (1.54mol/L)(30 ml, 46 mmol) was added dropwise to 2-piperidinone(2.25 g, 22.7 mmol) in 50ml of tetrahydrofuran at -78°C in a nitrogen atmosphere, and the resulting reaction solution was stirred at 0°C for 1 hour. Then, ethyl iodide (5.3 g, 34.1 mmol) was added dropwise with cooling with ice, and the reaction solution was stirred for 1 hour. After the reaction, saturated aqueous ammonium chloride and chloroform were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/9) to obtain 2.5 g of the desired product.

### Step 2;

### Synthesis of 3-ethyl-1-(2-trifluoromethanesulfonylaminobenzyl)piperidin-2-one (Compound IV-6 of the present invention)

1,1,1-Trifluoro-N-[2-(hydroxymethyl)phenyl]methanesulfonamide(2.5 g, 9.83 mmol) in 50ml of toluene was mixed with 3-ethylpiperidin-2-one (2.5 g, 19.7 mmol) and p-toluenesulfonic acid monohydrate (1.87g, 9.83 mmol) and refluxed under heating with stirring for 14 hours with azeotropic water separation with a dean-stark apparatus. After the reaction, the reaction solution was concentrated under reduced pressure, and the resulting residue was purified by silica gel column chromatography (n-hexane/ethyl acetate = 9/1 to 1/1) to obtain 0.82 g of the desired product (Compound IV-6 of the present invention).

### Step 3;

### Synthesis of 1-[2-(N-ethoxycarbonyl-N-trifluoromethanesulfonyl)aminobenzyl]-3-ethylpiperidin-2-one (Compound IV-9 of the present invention)

3-Ethyl-1-(2-trifluoromethanesulfonylaminobenzyl)piperidin-2-one (200 mg, 0.55 mmol) in 5 ml of acetonitrile was mixed with sodium hydrogen carbonate (130 mg, 1.64 mmol) and ethyl chloroformate (180 mg, 1.64 mmol) and refluxed under heating for 2 hours with stirring. After the reaction, ethyl acetate and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 1/1) to obtain 113 mg of the desired product (Compound IV-9 of the present invention).

### [EXAMPLE 6]

### Synthesis of 1-[2-(N-ethoxycarbonyl-N-trifluoromethanesulfonyl)aminobenzyl]-3-trifluoromethanesulfonyltetrahydropyrimidin-2(1 H)-one (Compound VI-4 of the present invention)

### Step 1;

### Synthesis of 1-(2-nitrobenzyl)tetrahydropyrimidin-2(1H)-one (Intermediate VIII-1)

1,3-Diaminopropane (4.1 g, 55.5 mmol) in 30ml of dichloromethane was mixed with 2-nitrobenzylbromide (3.0 g, 14.0 mmol) with cooling with ice and stirred at room temperature overnight. After the reaction, chloroform and water were added, and the organic layer was separated, washed with water, then dried with saturated aqueous sodium chloride and over anhydrous sodium sulfate and concentrated under reduced pressure. The resulting residue was dissolved in 80 ml of dichloromethane and stirred with 4-dimethylaminopyridine (0.17 g, 1.40 mmol) and 1,1'-carbonylbis-1 H-imidazole (2.9 g, 18.1 mmol) at room temperature for 7 hours. After the reaction, chloroform and saturated aqueous ammonium chloride were added, and the organic layer was separated, washed with water, then dried with saturated aqueous sodium chloride and over anhydrous sodium sulfate and concentrated under reduced pressure. The resulting solid was washed with ethyl acetate and isopropyl alcohol to obtain 1.57 g of the desired product (Intermediate VIII-1) as a pale yellow solid.

### Step 2;

### Synthesis of 1-(2-aminobenzyl)tetrahydropyrimidin-2(1 H)-one (Intermediate VIII-2)

1-(2-Nitrobenzyl)tetrahydropyrimidin-2(1 H)-one (1.54g, 6.55 mmol) in 30 ml of ethanol was mixed with 15 ml of water, reduced iron (1.8g, 32.7 mmol) and ammonium chloride (0.18 mg, 3.27 mmol) and stirred at 80°C for 45 minutes. After the reaction, the reaction solution was cooled to room temperature, and the insolubles were removed by filtration. The filtrate was concentrated under reduced pressure, and ethyl acetate and water were added to the resulting residue. The organic layer was separated, washed with water, then dried with saturated aqueous sodium chloride and over anhydrous sodium sulfate and concentrated under reduced pressure. The resulting solid was washed with isopropyl alcohol to obtain 0.91 g of the desired product (Intermediate VIII-2) as a pale yellow solid.

### Step 3;

### Synthesis of 1,1,1-trifluoro-N-{2-[(3-trifluoromethanesulfonyltetrahydro-2-pyrimidinon-1-yl)methyl]phenyl}-N-(trifluoromethanesulfonyl)methanesulfonamide (Compound VI-5 of the present invention)

1-(2-Aminobenzyl)tetrahydropyrimidin-2(1 H)-one (200 mg, 0.974 mmol) in 5ml of dichloromethane was mixed with triethylamine (395 mg, 3.90 mmol) and cooled to 0°C. Then, trifluoromethanesulfonic anhydride (825 mg, 2.92 mmol) in 1 ml of dichloromethane was added dropwise with stirring, and the reaction solution was gradually warmed to room temperature and stirred for 3 hours. After the reaction, chloroform and water were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 15/1 to 1/3) to obtain 173 mg of the desired product (Compound VI-5 of the present invention).

### Step 4;

### Synthesis of 1-(2-trifluoromethanesulfonyl)aminobenzyl]-3-trifluoromethanesulfonyltetrahydropyrimidin-2(1 H)-one (Compound VI-3 of the present invention)

1,1,1-Trifluoro-N-{2-[(3-trifluoromethanesulfonyltetrahydro-2-pyrimidinon-1-yl)methyl]phenyl}-N(trifluoromethanesulfonyl)methanesulfonamide (173 mg, 0.288 mmol) in 4 ml of 1,4-dioxane was mixed with aqueous tetrabutylammonium hydroxide (40%, 373 mg, 0.575 mmol) and stirred at room temperature for 3.5 hours. After the reaction, ethyl acetate and 1 N hydrochloric acid were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/2) to obtain 108 mg of the desired product (Compound VI-3 of the present invention).

### Step 5;

### Synthesis of 1-[2-(N-ethoxycarbonyl-N-trifluoromethanesulfonyl)aminobenzyl]-3-trifluoromethanesulfonyltetrahydropyrimidin-2(1H)-one (Compound VI-4 of the present invention)

[1-(2-Trifluoromethanesulfonyl)aminobenzyl]-3-trifluoromethanesulfonyltetrahydropyrimidin-2(1 H)-one (120 mg, 0.256 mmol) in 6 ml of acetonitrile was mixed with sodium hydrogen carbonate (43 mg, 0.511 mmol) and ethyl chloroformate (55 mg, 0.511 mmol) and refluxed under heating for 6 hours with stirring. After the reaction, ethyl acetate and saturated aqueous ammonium chloride were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/3) to obtain 128 mg of the desired product (Compound VI-4 of the present invention) as a white solid.

### [EXAMPLE 7]

### Synthesis of 1-[2-(N-ethoxycarbonyl-N-trifluoromethanesulfonyl)aminobenzyl]-3-ethoxycarbonyl-tetrahydropyrimidin-2(1 H)-one (Compound VI-2 of the present invention)

[1-(2-Trifluoromethanesulfonyl)aminobenzyl]tetrahydropyrimidin-2(1H)-one (30 mg, 0.089 mmol) in 5 ml of acetonitrile was mixed with sodium hydrogen carbonate (19 mg, 0.27 mmol) and ethyl chloroformate (29 mg, 0.27 mmol) and refluxed under heating for 5 hours with stirring. After the reaction, chloroform and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 4/1) to obtain 30 mg of the desired product (Compound VI-2 of the present invention).

### [EXAMPLE 8]

### Synthesis of 1-(2-trifluoromethanesulfonylaminobenzyl)piperidine-2-thione (Compound III-116 of the present invention)

1-(2-Trifluoromethanesulfonylaminobenzyl)piperidin-2-one (47.6 mg, 0.142 mmol) in 6 ml of toluene was mixed with Lawesson's reagent (65 mg, 0.16 mmol) and refluxed under heating for 5.5 hours with stirring. After the reaction, the reaction solution was concentrated under reduced pressure, and the resulting residue was purified by silica gel column chromatography (n-hexane/ethyl acetate = 15/1 to 1/4) to obtain 31 mg of the desired product (Compound III-116 of the present invention) as a white solid.

### [REFERENCE EXAMPLE 1]

### Synthesis of 6-methylpiperidin-2-one

### Step 1;

### Synthesis of 5-hydroxy-N-(4-methoxybenzyl)hexanamide

δ-Hexanolactone (2.0 g, 17.5 mmol) in 20 ml of toluene was mixed with 4-methoxybenzylamine (2.5 g, 18.2 mmol) and refluxed under heating for 3 hours with stirring. After the reaction, the reaction solution was concentrated under reduced pressure. The resulting crystals were collected by filtration and washed with n-hexane to obtain 4.07 g of the desired product as a pale yellow solid.

### Step 2;

### Synthesis of 5-chloro-N-(4-methoxybenzyl)hexanamide

5-Hydroxy-N-(4-methoxybenzyl)hexanamide(1.0 g, 3.77 mmol) in 10 ml of chloroform was mixed with thionyl chloride (0.9 g, 7.56 mmol) at 0°C with stirring, and the reaction solution was stirred at room temperature for 2 days. After the reaction, the reaction solution was concentrated under reduced pressure, and chloroform and saturated aqueous sodium hydrogen carbonate were added. The organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/1) to obtain 0.70 g of the desired product as a pale yellow solid.

### Step 3;

### Synthesis of 1-(4-methoxybenzyl)-6-methylpiperidin-2-one

5-Chloro-N-(4-methoxybenzyl)hexanamide(0.63 g, 2.34 mmol) in 10 ml of tetrahydrofuran was mixed with t-butoxypotassium (280 mg, 2.50 mmol)at 0°C with stirring and refluxed under heating for 15 hours with stirring. After the reaction, ethyl acetate and water were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/2) to obtain0.39 g of the desired product.

### Step 4;

### Synthesis of 6-methylpiperidin-2-one

1-(4-Methoxybenzyl)-6-methylpiperidin-2-one (0.39 g, 1.67 mmol) in 2 ml of trifluoroacetic acid was stirred at 80°C for 1 hour with a microwave reactor (Discover manufactured by CEM Corporation: Max 150 W). After the reaction, the reaction solution was concentrated under reduced pressure, and chloroform and water were added. The organic layer was separated, washed with water, then dried with saturated aqueous sodium chloride and over anhydrous sodium sulfate and concentrated under reduced pressure to obtain 150 mg of the desired product as a pale yellow solid.

### [REFERENCE EXAMPLE 2]

### Synthesis of 3,4,4-trimethyl-1-(2-nitrobenzyl)piperidin-2-one (Intermediate XI-13) and 4,4,5-trimethyl-1-(2-nitrobenzyl)piperidin-2-one (Intermediate XI-11)

### Step 1;

### Synthesis of 3,4,4-trimethyl-1-(2-nitrobenzyl)piperidine-2,6-dione

Lithium diisopropylamide solution in hexane and tetrahydrofuran (1.08 mol/l) (13.2 ml, 14.2 mmol) was added dropwise to 4,4-dimethylpiperidine-2,6-dione(2.0 g, 14.2 mmol) in 30 ml of tetrahydrofuran at -78°C in a nitrogen atmosphere, and the resulting reaction solution was stirred at -78°C for 10 minutes. After carbon dioxide was blown at 0°C for 2 minutes, the reaction solution was cooled again to -78°C, and lithium diisopropylamide solution in hexane and tetrahydrofuran (1.08 mol/l) (13.2ml, 14.2 mmol) was added dropwise. Then, methyl iodide (1.10 g, 7.80 mmol) and hexamethylphosphoric triamide (2.54 g, 14.2 mmol) were added dropwise, and the reaction solution was stirred at -20°C for 30 minutes. After the reaction, saturated aqueous ammonium chloride was added dropwise, the reaction solution was concentrated under reduced pressure the resulting residue was dissolved in 20 ml of dimethylformamide, mixed with 2-nitrobenzylbromide (3.06 g, 14.2 mmol) and potassium carbonate (1.96 g, 14.2 mmol) and stirred at 80°C for 30 minutes. After the reaction, ethyl acetate and water were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/1) to obtain 1.3 g of the desired product.

### Step 2;

### Synthesis of 6-hydroxy-3,4,4-trimethyl-1-(2-nitrobenzyl)piperidin-2-one and 6-hydroxy-4,4,5-trimethyl-1-(2-nitrobenzyl)piperidin-2-one

3,4,4-Trimethyl-1-(2-nitrobenzyl)piperidine-2,6-dione (2.81 g, 9.68 mmol) in 120 ml of tetrahydrofuran was mixed with sodium boron hydride (4.0 g, 96.8 mmol), and 6 ml of water was added dropwise. The reaction solution was stirred at room temperature overnight. After the reaction, the reaction solution was gradually added to saturated aqueous ammonium chloride at 0°C with stirring. Ethyl acetate was added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/9) to obtain 2.0.g of the desired product.

### Step 3;

### Synthesis of 3,4,4-trimethyl-1-(2-nitrobenzyl)piperidin-2-one (Intermediate XI -13) and 4,4,5-trimethyl-1-(2-nitrobenzyl)piperidin-2-one (Intermediate XI-11)

A mixture of 6-hydroxy-3,4,4-trimethyl-1-(2-nitrobenzyl)piperidin-2-one and 6-hydroxy-4,4,5-trimethyl-1-(2-nitrobenzyl)piperidin-2-one (2.0 g, 6.84 mmol) was dissolved in 20 ml of trifluoroacetic acid at 0°C. Tiethylsilane (1.19 g, 10.3 mmol) was added dropwise with stirring, and the reaction solution was stirred at room temperature for 1 hour. After the reaction, the reaction solution was concentrated under reduced pressure, and ethyl acetate and saturated aqueous sodium hydrogen carbonate were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/9) to obtain 0.87 g of 3,4,4-trimethyl-1-(2-nitrobenzyl)piperidin-2-one (Intermediate XI-13) and 0.3 g of 4,4,5-trimethyl-1-(2-nitrobenzyl)piperidin-2-one (Intermediate XI-11).

### [REFERENCE EXAMPLE 3]

### Synthesis of 5,5-dimethylazepan-2-one

4,4-Dimethylcyclohexanone (1.0 g, 7.92 mmol) in 10 ml of formic acid was mixed with hydroxylamine-O-sulfonic acid (1.34 g, 11.85 mmol) and refluxed under heating for 4 hours with stirring. After the reaction, the reaction solution was concentrated under reduced pressure and adjusted to pH 6 to pH 8 with 2 N aqueous sodium hydroxide. Chloroform was added, and the organic layer was separated, washed with water, then dried with saturated aqueous sodium chloride and over anhydrous sodium sulfate and concentrated under reduced pressure to obtain 1.05 g of the desired product as a pale yellow solid.

### [REFERENCE EXAMPLE 4]

### Synthesis of 1-methyltetrahydropyrimidin-2(1H)-one

N-Methyl-1,3-propanediamine (2.3 g, 26.1 mmol) and urea (1.57 g, 26.1 mmol) were dissolved in 10 ml of pyridine and refluxed under heating for 8 hours with stirring. After the reaction, the reaction solution was concentrated under reduced pressure, and chloroform and saturated aqueous ammonium chloride were added to the resulting residue. The organic layer was separated, washed with water, then dried with saturated aqueous sodium chloride and over anhydrous sodium sulfate and concentrated under reduced pressure the resulting solid was washed with hexane and diisopropyl ether and collected by filtration to obtain 155 mg of the desired product as a pale yellow solid.

### [REFERENCE EXAMPLE 5]

### Synthesis of 4,4-dimethylenepiperidin-2-one (Intermediate XIV-1)

### Step 1;

### Synthesis of 1,1-bis(methanesulfonyloxymethyl)cyclopropane

1,1-Bishydroxymethylcyclopropane(3.0 g, 29.4 mmol) in 40 ml of dichloromethane was mixed with triethylamine (11.9 g, 118 mmol), and methanesulfonyl chloride (10.1 g, 88.2 mmol) was added dropwise with cooling with ice. The reaction solution was stirred for 2 hours while it was gradually warmed to room temperature. After the reaction, chloroform and water were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/1) to obtain 2.85 g of the desired product as a white solid.

### Step 2;

### Synthesis of 1,1-bis(cyanomethyl)cyclopropane

1,1-Bis(methanesulfonyloxymethyl)cyclopropane(1.4 g, 5.42 mmol) in 15 ml of dimethylformamide was mixed with potassium cyanide (1.06 g, 16.3 mmol) and stirred at room temperature for 2 hours and then at 100°C for 3 hours. After the reaction, ethyl acetate and water were added, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/1) to obtain 450 mg of the desired product.

### Step 3;

### Synthesis of 3,3-dimethyleneglutaric acid

1,1-Bis(cyanomethyl)cyclopropane (2.2 g, 18.3 mmol) in 50 ml of methanol was mixed with 35 wt% aqueous sodium hydroxide (10.5 g, 91.7 mmol) and refluxed under heating for 4 hours with stirring. After the reaction, ethyl acetate and water were added, and the aqueous layer was separated and acidified with 1 N hydrochloric acid. Ethyl acetate was added to the aqueous layer, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 9/1 to 1/1) to obtain 460 mg of the desired product.

### Step 4;

### Synthesis of 3,3-dimethyleneglutarimide

3,3-Dimethyleneglutaric acid (460 mg, 2.91 mmol) and urea (210 mg, 3.50 mmol) were mixed and stirred at 175°C for 1 hour. After the reaction, the reaction solution was cooled to room temperature and filtered through Celite, and the celite was washed with methanol. The filtrate was concentrated under reduced pressure, and the resulting residue was purified by silica gel column chromatography (n-hexane/ethyl acetate = 1/1 to 1/10) to obtain 178 mg of the desired product.

### Step 5;

### Synthesis of 4,4-dimethylenepiperidin-2-one (Intermediate XIV-1)

3,3-Dimethyleneglutarimide (590 mg, 4.24 mmol) in 10 ml of ethanol was mixed with sodium boron hydride (480 mg, 12.7 mmol) with cooling with ice and stirring, and the reaction solution was stirred at 0°C for 40 minutes. After the reaction, hydrochloric acid-methanol (5-10 wt%) was added dropwise, and the reaction solution was neutralized, stirred at room temperature for 30 minutes and concentrated under reduced pressure. The resulting residue was dissolved in 8 ml of trifluoroacetic acid, mixed with triethylsilane (740 mg, 6.36 mmol) and stirred at room temperature overnight. After the reaction, the reaction solution was concentrated under reduced pressure. Ethyl acetate and saturated aqueous sodium hydrogen carbonate were added to the residue, and the organic layer was separated, washed with water, then dried with saturated aqueous sodium chloride and over anhydrous sodium sulfate and concentrated under reduced pressure. The resulting residue was purified by silica gel chromatography (n-hexane/ethyl acetate = 1/1 to 0/1) to obtain 110 mg of the desired product (Intermediate XIV-1) as a white solid (m.p.: 94 to 95°C).

### [REFERENCE EXAMPLE 6]

### Synthesis of 1,1,1-trifluoro-N-[2-(hydroxymethyl)phenyl]methanesulfonamide

2-Aminobenzyl alcohol (200 mg, 1.62 mmol) in 10 ml of dichloromethane was cooled to 0°C and mixed with N,N-diisopropylethylamine (250 mg, 1.93 mmol), and trifluoromethanesulfonyl chloride (326 mg, 1.93 mmol) was further added dropwise with stirring. The reaction solution was stirred for another 4 hours while it was gradually warmed to room temperature. After the reaction, chloroform and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate = 9/1 to 1/1) to obtain 200 mg of the desired product as a reddish brown solid.

### [REFERENCE EXAMPLE 7]

### Synthesis of 1,1-dichloro-6-azaspiro[2.5]octan-5-one (Intermediate XIV-2)

### Step 1;

### Synthesis of tert-butyl 1,1-dichloro-6-azaspiro[2.5]octan-6-carboxylate

1-N-Tertiary-butoxycarbonyl-4-methylenepiperidine (1.5g, 7.60 mmol) in 20 ml of chloroform was mixed with benzyltriethylammonium chloride (0.17g, 0.76 mmol), and the reaction solution was heated to 50°C with stirring. Sodium hydroxide (3.0g, 76.0 mmol) in 5 ml of water was added dropwise to the reaction solution. The reaction solution was stirred at 50°C for 3 hours and then at room temperature overnight. After the reaction, chloroform and water were added, and the organic layer was separated, washed with water, then dried with saturated aqueous sodium chloride and over anhydrous sodium sulfate and concentrated under reduced pressure. The resulting solid was washed with hexane and isopropyl ether and collected by filtration to obtain 1.8 g of the desired product as a white solid (m.p.: 75 to 76°C).

### Step 2;

### Synthesis of tert-butyl 1,1--dichloro-5-oxo-6-azaspiro[2.5]octan-6-carboxylate

tert-Butyl 1,1-dichloro-6-azaspiro[2.5]octane-6-carboxylate (300 mg, 1.07 mmol) in 18 ml of acetonitrile, 18 ml of carbon tetrachloride and 27 ml of water was mixed with sodium periodide (1.8 g, 8.57 mmol) and ruthenium chloride hydrate (4.5 mg, 0.0214 mmol) and stirred at room temperature overnight. After the reaction, chloroform and water were added, and the organic layer was separated and washed with aqueous sodium thiosulfate, then dried with saturated aqueous sodium chloride and over anhydrous sodium sulfate and concentrated under reduced pressure. The resulting solid was washed with hexane and diisopropyl ether and collected by filtration to obtain 231 mg of the desired product as a pale yellow solid (m.p.:134 to 135°C). Step 3;

### Synthesis of 1,1-dichloro-6-azaspiro[2.5]octan-5-one (Intermediate XIV-2)

Tertiary-butyl 1,1-dichloro-5-oxo-6-azaspiro[2.5]octane-6-carboxylate (220 mg, 0.748 mmol) in 2 ml of dichloromethane was mixed with 1 ml of trifluoroacetic acid and stirred at room temperature overnight. After the reaction, the reaction solution was concentrated under reduced pressure, and the residue was adjusted to pH 7 with saturated aqueous sodium hydrogen carbonate. Chloroform was added, and the organic layer was separated, washed with water, then dried with saturated aqueous sodium chloride and over anhydrous sodium sulfate and concentrated under reduced pressure. The resulting solid was washed with hexane and diisopropyl ether and collected by filtration to obtain 143 mg of the desired product (Intermediate XIV-2) as a white solid (m.p.: 185 to 186°C).

The compounds of the present invention can be synthesized in the same manners as in the above examples. The structural formulae of synthesized compounds, including those synthesized in the preceding Examples, are shown in Tables 10 to 19, the structural formulae of the synthesized intermediates are shown in Tables 20 to 23, and their physical properties are shown in Table 24.

The present invention is by no means restricted to these compounds only.

The symbols in Tables 10 to 23 have the following meanings.

Me: methyl group, Et: ethyl group, Pr: propyl group, Bu: butyl group, Pen: pentyl group, Hex: hexyl group, Ph: phenyl group.

The symbols in Table 24 have the following meanings.
s: singlet, d: doublet, t: triplet, m: multiplet.

**TABLE 10**

| | | | | | |
|---|---|---|---|---|---|
| 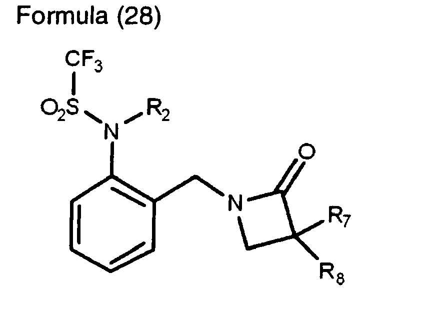 | | | | | |

| No. | R₂ | R₇ | R₈ | Physical properties | [m.p.(°C)] |
|---|---|---|---|---|---|
| I-1 | H | Me | Me | 114-116 | |
| I-2 | COEt | Me | Me | 91-93 | |
| I-3 | COPr-i | Me | Me | 67-68 | |
| I-4 | CO₂Et | Me | Me | 50-51 | |
| I-5 | CO₂Bu-i | Me | Me | resinoid | |

**TABLE 11**

| | | | | | |
|---|---|---|---|---|---|
| 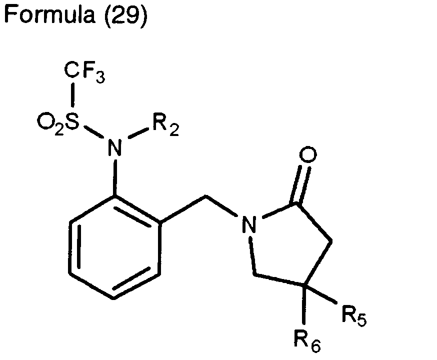 | | | | | |

| No. | R₂ | R₅ | R₆ | Physical properties | [m.p.(°C))] |
|---|---|---|---|---|---|
| II-1 | H | H | H | resinoid | |
| II-2 | CO₂Et | H | H | resinoid | |
| II-3 | CO₂Bu-i | H | H | resinoid | |

**TABLE 12**

| 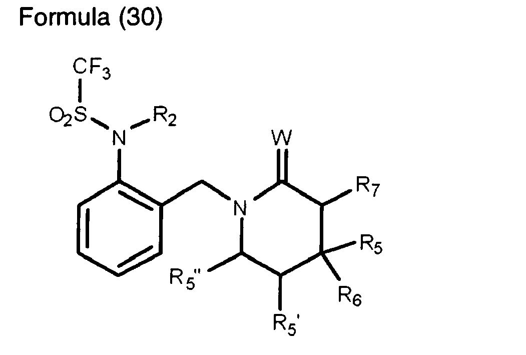 | | | | | | | | |
|---|---|---|---|---|---|---|---|---|
| No. | R₂ | R₇ | R₅ | R₆ | R₅' | R₅" | W | Physical properties [m.p.(°C)] |
| III-1 | H | H | H | H | H | H | O | 57-59 |
| III-2 | COEt | H | H | H | H | H | O | resinoid |
| III-3 | COPr-n | H | H | H | H | H | O | 110-110.5 |
| III-4 | COPr-i | H | H | H | H | H | O | resinoid |
| III-5 | COBu-n | H | H | H | H | H | O | 70-71 |
| III-6 | E-4 | H | H | H | H | H | O | 99-100 |
| III-7 | COCH=CH₂ | H | H | H | H | H | O | resinoid |
| III-8 | E-5 | H | H | H | H | H | O | resinoid |
| III-9 | CO₂Et | H | H | H | H | H | O | 86-87 |
| III-10 | CO₂Pr-i | H | H | H | H | H | O | resinoid |
| III-11 | CO₂Bu-i | H | H | H | H | H | O | resinoid |
| III-12 | E-1 | H | H | H | H | H | O | resinoid |
| III-13 | E-3 | H | H | H | H | H | O | resinoid |
| III-14 | CH₂OMe | H | H | H | H | H | O | resinoid |
| III-15 | CH₂OCH₂Ph | H | H | H | H | H | O | resinoid |
| III-16 | CH₂OCOEt | H | H | H | H | H | O | resinoid |
| III-17 | E-6 | H | H | H | H | H | O | 56-58 |
| III-18 | CH₂Ph | H | H | H | H | H | O | resinoid |
| III-19 | H | H | Me | H | H | H | O | resinoid |
| III-20 | COPr-c | H | Me | H | H | H | O | resinoid |
| III-21 | COPh | H | Me | H | H | H | | 106-108 |
| III-22 | CO₂Et | H | Me | H | H | H | O | 73-74 |
| III-23 | CO₂Bu-i | H | Me | H | H | H | O | resinoid |
| III-24 | CO₂Et | H | Et | H | H | H | O | resinoid |
| III-25 | H | H | Pr-n | H | H | H | O | resinoid |
| III-26 | COEt | H | Pr-n | H | H | H | O | resinoid |
| III-27 | COPr-c | H | Pr-n | H | H | H | O | resinoid |
| III-28 | CO₂Et | H | Pr-n | H | H | H | O | 61-62 |
| III-29 | CO₂Bu-i | H | Pr-n | H | H | H | O | 89-90 |
| III-30 | H | H | Pr-i | H | H | H | O | resinoid |
| III-31 | CO₂Et | H | Pr- | i H | H | H | O | 102-105 |
| III-32 | COEt | H | Pr-i | H | H | H | O | resinoid |
| III-33 | COPr-c | H | Pr-i | H | H | H | O | resinoid |
| III-34 | H | H | Pr-c | H | H | H | O | resinoid |
| III-35 | COEt | H | Pr-c | H | H | H | O | resinoid |
| III-36 | COPr-c | H | Pr-c | H | H | H | O | resinoid |
| III-37 | CO₂Et | H | Pr-c | H | H | H | O | resinoid |
| III-38 | CO₂Et | H | CF₃ | H | H | H | O | 124-125 |
| III-39 | H | H | OMe | H | H | H | O | resinoid |
| III-40 | CO₂Et | H | OMe | H | H | H | O | resinoid |
| III-41 | H | H | OEt | H | H | H | O | resinoid |
| III-42 | COEt | H | OEt | H | H | H | O | resinoid |
| III-43 | CO₂Et | H | OEt | H | H | H | O | resinoid |
| III-44 | H | H | SMe | H | H | H | O | resinoid |
| III-45 | CO₂Et | H | SMe | H | H | H | O | 79-81 |
| III-46 | H | H | Me | Me | H | H | O | 52-53 |
| III-47 | COME | H | Me | Me | H | H | O | 130-132 |
| III-48 | COEt | H | Me | Me | H | H | O | 106-107 |
| III-49 | COPr-n | H | Me | Me | H | H | O | 110-111 |
| III-50 | COPr-i | H | Me | Me | H | H | O | 56-58 |
| III-51 | COPr-c | H | Me | Me | H | H | O | 89-90 |
| III-52 | COBu-n | H | Me | Me | H | H | O | resinoid |
| III-53 | COBu-c | H | Me | Me | H | H | O | resinoid |
| III-54 | COPen-n | H | Me | Me | H | H | O | resinoid |
| III-55 | E-4 | H | Me | Me | H | H | O | 124-125 |
| III-56 | COPh | H | Me | Me | H | H | O | resinoid |
| III-57 | CO₂Me | H | Me | Me | H | H | O | resinoid |
| III-58 | CO₂Et | H | Me | Me | H | H | O | 86-87 |
| III-59 | CO₂Pr-n | H | Me | Me | H | H | O | 106-107 |
| III-60 | CO₂Bu-n | H | Me | Me | H | H | O | 63-65 |
| III-61 | E-1 | H | Me | Me | H | H | O | resinoid |
| III-62 | CO₂Bu-i | H | Me | Me | H | H | O | resinoid |
| III-63 | CO₂Hex-n | H | Me | Me | H | H | O | resinoid |
| III-64 | E-8 | H | Me | Me | H | H | O | resinoid |
| III-65 | E-9 | H | Me | Me | H | H | O | resinoid |
| III-66 | E-10 | H | Me | Me | H | H | O | resinoid |
| III-67 | E-3 | H | Me | Me | H | H | O | 89-90 |
| III-68 | E-11 | H | Me | Me | H | H | O | resinoid |
| III-69 | CO₂Ph | H | Me | Me | H | H | O | resinoid |
| III-70 | CO₂CH₂Ph | H | Me | Me | H | H | O | 84-88 |
| III-71 | E-7 | H | Me | Me | H | H | O | resinoid |
| III-72 | SO₂CF₃ | H | Me | Me | H | H | O | resinoid |
| III-73 | H | H | Me | Et | H | H | O | resinoid |
| III-74 | COMe | H | Me | Et | H | H | O | resinoid |
| III-75 | COEt | H | Me | Et | H | H | O | resinoid |
| III-76 | COPr-c | H | Me | Et | H | H | O | resinoid |
| III-77 | CO₂Me | H | Me | Et | H | H | O | resinoid |
| III-78 | CO₂Et | H | Me | Et | H | H | O | resinoid |
| III-79 | H | Me | Me | H | H | H | O | resinoid |
| III-80 | CO₂Et | Me | Me | H | H | H | O | resinoid |
| III-81 | CO₂Et | Cl | Cl | H | H | H | O | resinoid |
| III-82 | CO₂Et | Br | Br | H | H | H | O | resinoid |
| III-83 | H | Cl | Me | H | H | H | O | resinoid |
| III-84 | CO₂Et | Cl | Me | H | H | H | O | resinoid |
| III-85 | H | CO₂Et | Me | H | H | H | O | resinoid |
| III-86 | H | CO₂Et | Et | H | H | H | O | resinoid |
| III-87 | CO₂Et | CO₂Et | Et | H | H | H | O | resinoid |
| III-88 | H | H | Me | Me | Me | H | O | resinoid |
| III-89 | CO₂Et | H | Me | Me | Me | H | O | resinoid |
| III-90 | H | H | Me | Me | H | Me | O | resinoid |
| III-91 | CO₂Et | H | Me | Me | H | Me | O | resinoid |
| III-92 | H | H | Me | Me | H | OH | O | 93-95 |
| III-93 | CO₂Et | H | Me | Me | H | OH | O | 95-96 |
| III-94 | H | Me | Me | Me | H | H | O | resinoid |
| III-95 | CO₂Et | Me | Me | Me | H | H | O | resinoid |
| III-96 | H | H | Ph | H | H | H | O | resinoid |
| III-97 | CO₂Et | H | Ph | H | H | H | O | resinoid |
| III-98 | COEt | H | Ph | H | H | H | O | resinoid |
| III-99 | H | H | -(CH₂) ₂- | | H | H | O | resinoid |
| III-100 | COEt | H | -(CH₂)₂- | | H | H | O | 121-122 |
| III-101 | COPr-n | H | -(CH₂)₂- | | H | H | O | 119-120 |
| III-102 | COPr-c | H | -(CH₂)₂- | | H | H | O | 127-128 |
| III-103 | CO₂Et | H | - (CH₂)₂- | | H | H | O | 69-72 |
| III-104 | CO₂Pr-n | H | - (CH₂)₂- | | H | H | O | resinoid |
| III-105 | CO₂Bu-i | H | - (CH₂)₂- | | H | H | O | resinoid |
| III-106 | E-8 | H | -(CH₂)₂- | | H | H | O | resinoid |
| III-107 | H | H | -(CH₂) ₄- | | -H | H | O | resinoid |
| III-108 | COEt | H - | (CH₂)₄- | | H | H | O | 102-104 |
| III-109 | COPr-c | H | -(CH₂) ₄- | | H | H | O | resinoid |
| III-110 | CO₂Et | H | - (CH₂)₄- | | H | H | O | resinoid |
| III-111 | CO₂Bu-i | H | - (CH₂)₄- | | H | H | O | resinoid |
| III-112 | CO₂Et | -CH₂- | | H | H | H | O | resinoid |
| III-113 | CO₂Et | -O- | | H | H | H | O | resinoid |
| III-114 | H | -O- | | Me | H | H | O | resinoid |
| III-115 | H | H | Me | Me | bond | | O | resinoid |
| III-116 | H | H | H | H | H | | S | 116-118 |
| III-117 | H | H | Me | Me | H | H | S | resinoid |
| III-118 | CO₂Et | H | Me | Me | H | H | S | resinoid |
| III-119 | H | H | -CH₂CCl₂- | | H | H | O | resinoid |
| III-120 | CO₂Et | H | -CH₂CCl₂- | | H | H | O | resinoid |

In Table 12, E-1 to E-11 and "bond" are the same as defined above.

**TABLE13**

| | | | | | | | | |
|---|---|---|---|---|---|---|---|---|
| 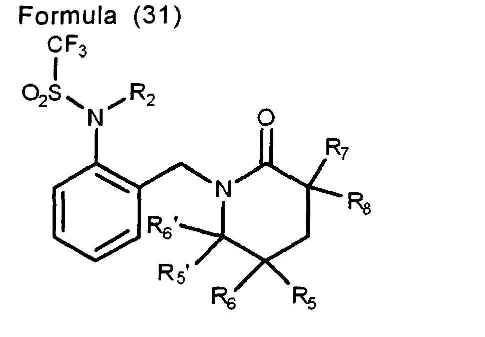 | | | | | | | | |

| No. | R₂ | R₅ | R₆ | R₅' | R₆' | R₇ | R₈ | Physical properties [m.p.(°C)] |
|---|---|---|---|---|---|---|---|---|
| IV-1 | CO₂Et | Me | H | H | H | H | H | resinoid |
| IV-2 | H | Me | Me | H | H | H | H | 64-65 |
| IV-3 | CO₂Et | Me | Me | H | H | H | H | resinoid |
| IV-4 | H | H | H | H | H | Me | H | resinoid |
| IV-5 | CO₂Et | H | H | H | H | Me | H | resinoid |
| IV-6 | H | H | H | H | H | Et | H | resinoid |
| IV-7 | COEt | H | H | H | H | Et | H | resinoid |
| IV-8 | COPr-c | H | H | H | H | Et | H | resinoid |
| IV-9 | CO₂Et | H | H | H | H | Et | H | resinoid |
| IV-10 | CO₂Bu-i | H | H | H | H | Et | H | resinoid |
| IV-11 | H | H | H | H | H | Pr-n | H | resinoid |
| IV-12 | CO₂Et | H | H | H | H | Pr-n | H | resinoid |
| IV-13 | CO₂Et | H | H | H | H | OMe | H | resinoid |
| IV-14 | H | H | H | H | H | SMe | H | resinoid |
| IV-15 | CO₂Et | H | H | H | H | SMe | H | resinoid |
| IV-16 | H | H | H | H | H | SPh | H | resinoid |
| IV-17 | CO₂Et | H | H | H | H | SPh | H | resinoid |
| IV-18 | H | H | H | H | H | Cl | H | resinoid |
| IV-19 | COEt | H | H | H | H | Cl | H | resinoid |
| IV-20 | COPh | H | H | H | H | Cl | H | resinoid |
| IV-21 | CO₂Et | H | H | H | H | Cl | H | resinoid |
| IV-22 | H | H | H | H | H | Br | H | resinoid |
| IV-23 | CO₂Et | H | H | H | H | CN | H | resinoid |
| IV-24 | H | H | H | H | H | Me | Me | 106-108 |
| IV-25 | COEt | H | H | H | H | Me | Me | resinoid |
| IV-26 | CO₂Et | H | H | H | H | Me | Me | resinoid |
| IV-27 | H | H | H | Me | H | H | H | resinoid |
| IV-28 | CO₂Et | H | H | Me | H | H | H | resinoid |
| IV-29 | H | H | H | OH | H | H | H | 102-106 |
| IV-30 | CO₂Et | H | H | OMe | H | H | H | resinoid |
| IV-31 | H | Me | Me | H | H | Me | H | resinoid |
| IV-32 | CO₂Et | Me | Me | H | H | Me | H | resinoid |

**TABLE14**

| | | | | | | | | | |
|---|---|---|---|---|---|---|---|---|---|
| 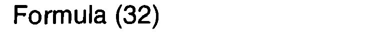 | | | | | | | | | |

| 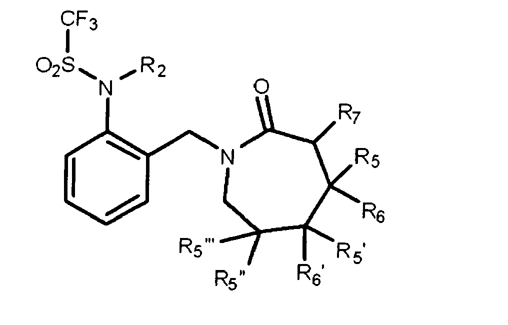 | | | | | | | | | |
|---|---|---|---|---|---|---|---|---|---|
| No. | R₂ | R₅ | R₆ | R₅' | R₆' | R₅" | R₅"' | R₇ | Physical properties [m. p. (°C)] |
| V-1 | H | H | H | H | H | H | H | H | 67-69 |
| V-2 | COMe | H | H | H | H | H | H | H | resinoid |
| V-3 | COEt | H | H | H | H | H | H | H | resinoid |
| V-4 | COPr-n | H | H | H | H | H | H | H | resinoid |
| V-5 | COPr-c | H | H | H | H | H | H | H | resinoid |
| V-6 | E-4 | H | H | H | H | H | H | H | 117-118 |
| V-7 | E-12 | H | H | H | H | H | H | H | 95-97 |
| V-8 | COPh | H | H | H | H | H | H | H | resinoid |
| V-9 | CO₂Me | H | H | H | H | H | H | H | resinoid |
| V-10 | CO₂Et | H | H | H | H | H | H | H | 66-67 |
| V-11 | E-8 | H | H | H | H | H | H | H | 102-103 |
| V-12 | CO₂Bu-i | H | H | H | H | H | H | H | resinoid |
| V-13 | E-3 | H | H | H | H | H | H | H | resinoid |
| V-14 | H | Me | H | H | H | H | H | H | resinoid |
| V-15 | COEt | Me | H | H | H | H | H | H | resinoid |
| V-16 | COPr-c | Me | H | H | H | H | H | H | resinoid |
| V-17 | CO₂Et | Me | H | H | H | H | H | H | resinoid |
| V-18 | H | H | H | Me | H | H | H | H | resinoid |
| V-19 | COMe | H | H | Me | H | H | H | H | 84-87 |
| V-20 | COEt | H | H | Me | H | H | H | H | 93-95 |
| V-21 | COPr-c | H | H | Me | H | H | H | H | resinoid |
| V-22 | CO₂Me | H | H | Me | H | H | H | H | resinoid |
| V-23 | CO₂Et | H | H | Me | H | H | H | H | 66-67 |
| V-24 | CO₂Bu-i | H | H | Me | H | H | H | H | resinoid |
| V-25 | COEt | H | H | Bu-t | H | H | H | H | resinoid |
| V-26 | CO₂Et | H | H | Bu-t | H | H | H | H | resinoid |
| V-27 | CO₂Et | H | H | Ph | H | H | H | H | resinoid |
| V-28 | H | H | H | H | H | Me | H | H | resinoid |
| V-29 | COEt | H | H | H | H | Me | H | H | resinoid |
| V-30 | COPr-c | H | H | H | H | Me | H | H | resinoid |
| V-31 | CO₂Et | H | H | H | H | Me | H | H | resinoid |
| V-32 | H | H | H | Me | Me | H | H | H | 106-108 |
| V-33 | COMe | H | H | Me | Me | H | H | H | 143-145 |
| V-34 | COEt | H | H | Me | Me | H | H | H | 112-113 |
| V-35 | COPr-c | H | H | Me | Me | H | H | H | 131-133 |
| V-36 | CO₂Et | H | H | Me | Me | H | H | H | 87-88 |
| V-37 | COEt | Me | Me | H | H | Me | Me | H | resinoid |
| V-38 | CO₂Et | Me | Me | H | H | Me | Me | H | resinoid |
| V-39 | H | H | H | H | H | H | H | Me | resinoid |
| V-40 | CO₂Et | H | H | H | H | H | H | Me | 71-72.5 |

InTable14,E-3,E-4,E-8andE-12arethesameasdefinedabove.

**TABLE15**

| | | | | | | |
|---|---|---|---|---|---|---|
| 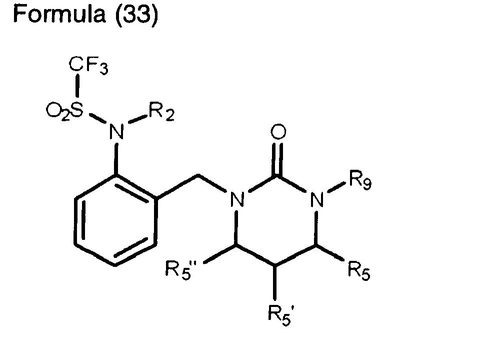 | | | | | | |

| No. | R₂ | R₉ | R₅ | R₅ ' | R₅ " | Physical properties [m.p.(°C)] |
|---|---|---|---|---|---|---|
| VI-1 | H | H | H | H | H | 184-186 |
| VI-2 | CO₂Et | CO₂Et | H | H | H | resinoid |
| VI-3 | H | SO₂CF₃ | H | H | H | resinoid |
| VI-4 | CO₂Et | SO₂CF₃ | H | H | H | 110-111 |
| VI-5 | SO₂CF₃ | SO₂CF₃ | H | H | H | resinoid |
| VI-6 | H | Me | H | H | H | 89-91 |
| VI-7 | CO₂Et | Me | H | H | H | 82-83 |

**TABLE 16**

| | | | | |
|---|---|---|---|---|
| 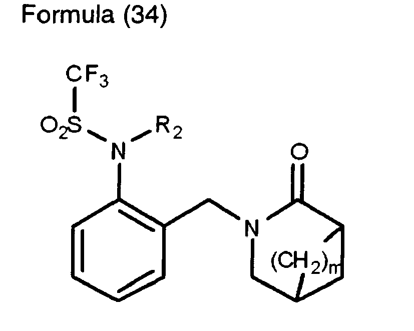 | | | | |

| No. | R₂ | m | Physical properties | [m.p. (°C)] |
|---|---|---|---|---|
| VII-1 | H | 0 | resinoid | |
| VII-2 | COEt | 0 | resinoid | |
| VII-3 | COPr-c | 0 | resinoid | |
| VII-4 | CO₂Et | 0 | resinoid | |
| VII-5 | CO₂Bu-i | 0 | resinoid | |
| VII-6 | H | 2 | resinoid | |
| VII-7 | COEt | 2 | resinoid | |
| VII-8 | CO₂Et | 2 | resinoid | |

**TABLE 17**

| | | | | |
|---|---|---|---|---|
| 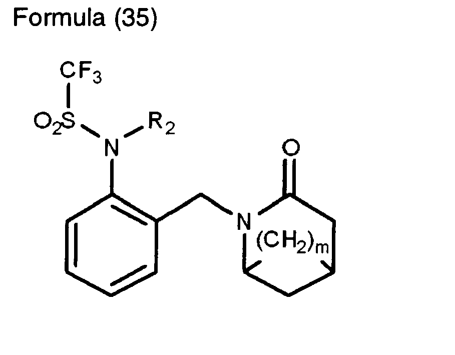 | | | | |

| No. | R₂ | m | Physical properties | [m.p. (°C)] |
|---|---|---|---|---|
| VIII-1 | H | 2 | resinoid | |
| VIII-2 | CO₂Et | 2 | resinoid | |

**TABLE 18**

| | | | |
|---|---|---|---|
| 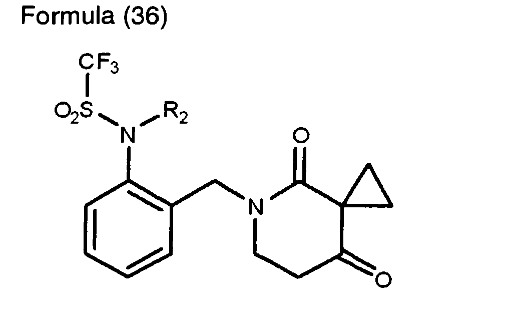 | | | |

| No. | R₂ | Physical properties | [m.p. (°C)] |
|---|---|---|---|
| IX-1 | CO₂Et | resinoid | |

**TABLE 19**

| | | | | | | | |
|---|---|---|---|---|---|---|---|
| 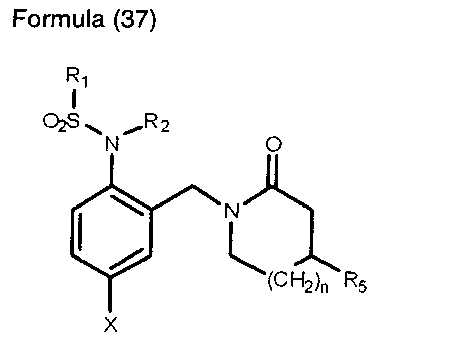 | | | | | | | |

| No. | R₁ | R₂ | R₅ | X | n | Physical properties | [m.p. (°C)] |
|---|---|---|---|---|---|---|---|
| X-1 | CF₃ | H | H | Cl | 1 | resinoid | |
| X-2 | CF₃ | H | Me | F | 1 | resinoid | |
| X-3 | CF₃ | CO₂Et | Me | F | 1 | resinoid | |
| X-4 | CHF₂ | H | H | H | 2 | resinoid | |
| X-5 | CCl₃ | H | Me | H | 1 | resinoid | |
| X-6 | CFCl₂ | H | Me | H | 1 | resinoid | |
| X-7 | CFCl₂ | CO₂Et | Me | H | 1 | resinoid | |

**TABLE 20**

| | | | | | | | |
|---|---|---|---|---|---|---|---|
| 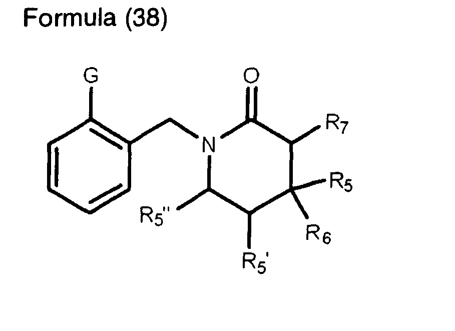 | | | | | | | |

| No. | G | R₅ | R₆ | R₅ ' | R₅ '' | R₇ | Physical properties [m.p. (°C)] |
|---|---|---|---|---|---|---|---|
| XI-1 | NO₂ | Me | H | H | H | H | 92-93 |
| XI-2 | NH₂ | Me | H | H | H | H | resinoid |
| XI-3 | NH₂ | Pr-n | H | H | H | H | resinoid |
| XI-4 | NH₂ | Pr-i | H | H | H | H | resinoid |
| XI-5 | NH₂ | Pr-c | H | H | H | H | resinoid |
| XI-6 | NH₂ | OMe | H | H | H | H | 59-61 |
| XI-7 | NO₂ | Ph | H | H | H | H | 140-141 |
| XI-8 | NH₂ | Ph | H | H | H | H | resinoid |
| XI-9 | NO₂ | Me | Me | H | H | H | 94-96 |
| XI-10 | NH₂ | Me | Me | H | H | H | resinoid |
| XI-11 | NO₂ | Me | Me | Me | H | H | resinoid |
| XI-12 | NH₂ | Me | Me | Me | H | H | resinoid |
| XI-13 | NO₂ | Me | Me | H | H | Me | resinoid |
| XI-14 | NH₂ | Me | Me | H | H | Me | resinoid |
| XI-15 | NO₂ | - (CH₂)₄- | | H | H | H | resinoid |
| XI-16 | NH₂ | -(CH₂)₄- | | H | H | H | resinoid |
| XI-17 | NO₂ | H | H | H | OMe | H | resinoid |
| XI-18 | NH₂ | H | H | H | OMe | H | resinoid |
| XI-19 | NO₂ | Me | Me | | bond | H | 80-82 |
| XI-20 | NH₂ | Me | H | H | bond | H | 148-150 |

In Table 20, "bond" is the same as defined above.

**TABLE 21**

| | | | | | | | |
|---|---|---|---|---|---|---|---|
| 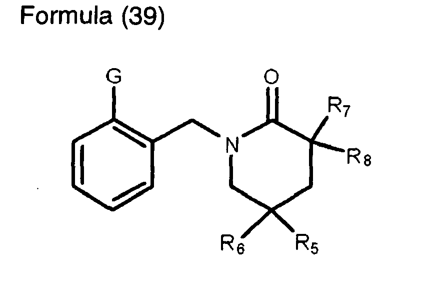 | | | | | | | |

| No. | G | R₅ | R₆ | R₇ | R₈ | Physical properties | [m.p. (°C)] |
|---|---|---|---|---|---|---|---|
| XII-1 | NO₂ | Me | Me | H | H | resinoid | |
| XII-2 | NH₂ | Me | Me | H | H | resinoid | |
| XII-3 | NO₂ | H | H | Me | Me | resinoid | |
| XII-4 | NH₂ | H | H | Me | Me | resinoid | |

**TABLE 22**

| | | | | | | | |
|---|---|---|---|---|---|---|---|
| 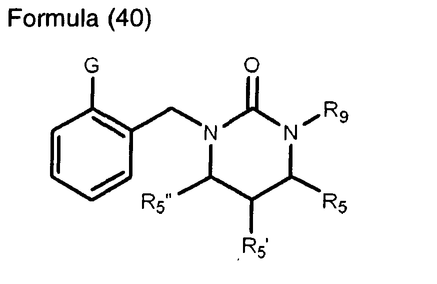 | | | | | | | |

| No. | G | R₉ | R₅ | R₅' | R₅'' | Physical properties | [m.p. (°C)] |
|---|---|---|---|---|---|---|---|
| XIII-1 | NO₂ | H | H | H | H | 223-224 | |
| XIII-2 | NH₂ | H | H | H | H | 194-195 | |

**TABLE 23**

| | | | | |
|---|---|---|---|---|
| 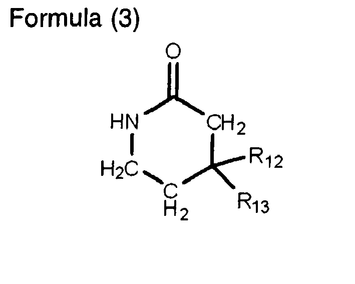 | | | | |

| N o. | R₁₂ | R₁₃ | Physical properties | [m.p.(°C)] |
|---|---|---|---|---|
| XIV-1 | -(CH₂)₂- | | 94-95 | |
| XIV-2 | -CH₂CCl₂- | | 185-186 | |

**TABLE 24**

| No. Proton nuclear magnetic resonance chemical shift (in CDCl₃ ): δ (ppm) |
|---|
| I-5 : 7.35-7.55 (m, 3H), 7. 21 (d, J=7. 5Hz, 1H), 4. 58 (d, J=15. 2Hz, 1H), 4. 31 (d, J=15. 2Hz, 1H) , 3. 95-4. 15 (m, 2H) , 2. 90-3.05(m, 2H) , 1. 85-2. 05 (m, 1H), 1. 31 (d, J=4. 7Hz, 6H) , 0. 88 (d, J=6. 9Hz, 6H) |
| II-1 : 10. 40 (brs, 1H), 7. 58 (d, J=7. 2Hz, 1H), 7. 36 (dt, J=7. 2,1. 8Hz, 1H), 7. 15-7. 30 (m, 2H) , 4. 40(s, 2H), 3.55(t, J=7.2Hz, 2H), 2.42(d, J=7.2Hz, 2H), 2.09(t, J=7.2Hz, 2H) |
| II-2 : 7. 47 (dt, J=7. 5, 1. 2Hz, 1H), 7.30-7.45 (m, 2H), 7. 22 (d, J=7.5Hz, 1H), 4. 53 (s, 2H), 4.2 5-4. 45 (m, 2H) , 3. 25 (t, J=7. 5Hz, 2H) , 2. 45 (dt, J=8. 4, 3. 6Hz, 2H), 1. 90-2. 15 (m, 2H), 1. 32 (t, J=7. 5Hz, 3H) |
| II-3 : 7. 40-7. 50 (m, 1H), 7.30-7.40(m,2H), 7.15-7.25(m, 1H), 4. 53(s, 2H), 4. 00-4. 20 (m, 2H ), 3.20-3.35 (m, 2H), 2. 35-2. 65 (m, 2H), 1. 85-2. 15 (m, 2H), 1. 20-1. 30 (m, 1H), 0. 88 (ddd, J=6. 6,3.6, 1. 8Hz, 6H) |
| III-2 : 7.50(dt, J=1.2, 7. 5Hz, 1H), 7. 40 (dt, J=1. 2, 7. 5Hz, 1H), 7. 32 (dd, J=1. 2, 7. 5Hz, 1H), 7. 21 (d, J=7. 5Hz, 1H), 5. 07 (d, J=15. 6Hz, 1H), 4. 21 (d, J=15. 6Hz, 1H), 3. 30-3. 35 (m, 2H) , 2. 45 -2. 55 (m, 2H), 2. 42 (dq, J=7.5, 18. 3Hz, 1H), 2. 36 (dq, J=7.5, 18. 3Hz, 1H), 1. 85-1. 90 (m, 4H), 1 .11(t, J=7.2Hz, 3H) |
| III-4 : 7.51 (t, J=8.8Hz, 1H), 7. 40 (t, J=8.8Hz, 1H), 7. 33 (d, J=8.8Hz, 1H), 7. 22 (d, J=8.8Hz, 1H), 5.07(d, J=15. 9Hz, 1H), 4. 27 (d, J=15. 9Hz, 1H), 3. 25-3. 35 (m, 2H), 2. 45-2. 60 (m, 3H), 1. 7 0-1.90 (m, 4H), 1.21 (d, J=6.6Hz, 3H), 1. 08 (d, J=6.6Hz, 3H) |
| III-7 : 7. 52 (t, J=6.8Hz, 1H), 7.35-7. 50 (m, 2H), 7.20 (d, J=8.5Hz, 1H), 6. 63 (d, J=16. 5Hz, 1H ), 6.13(dd, J=16.5, 9.1Hz, 1H), 5. 89 (d, J=9.1Hz, 1H), 4. 99 (d, J=15. 7Hz, 1H), 4. 31 (d, J=15. 7 Hz, 1H), 3. 25-3. 40 (m, 2H), 2. 40-2. 55 (m, 2H), 1. 70-2. 00 (m, 4H) |
| III-8 : 7.50(t, J=7.0Hz, 1H), 7. 42 (t, J=7.0Hz 1H), 7. 25-7. 35 (m, 2H), 5. 05 (d, J=15. 9Hz, 1H ), 4.31 (d, J=15. 9Hz, 1H), 4. 12 (q, J=5. 8Hz, 2H), 3. 25-3. 40 (m, 2H), 2. 55-2. 75 (m, 4H), 2.40-2 .50 (m, 2H), 1. 75-1. 90 (m, 4H), 1. 24(t, J=7.1Hz, 3H) |
| III-10: 7.45 (t, J=7.5Hz, 1H), 7.30-7.40 (m, 2H), 7.20 (d, J=8.5Hz, 1H), 5. 05-5. 20 (m, 1H), 4 .72 (d, J=16.4Hz, 1H), 4. 68 (d, J=16.4Hz, 1H), 3. 10-3. 25 (m, 2H), 2. 40-2. 60 (m, 2H) , 1. 70-1. 9 0(m, 4H), 1. 25-1. 40 (m, 6H) |
| III-11: 7. 40-7. 50 (m, 1H), 7. 25-7. 40 (m, 2H), 7. 21 (d, J=7.8Hz, 1H), 4. 75 (d, J=15. 3Hz, 1H), 4. 68(d, J=15.3Hz, 1H), 3. 95-4. 20 (m, 2H), 3.10-3.30(m, 2H), 2.40-2.60(m, 2H), 1. 70-2.05(m , 5H), 0. 87 (d, J=7. 8Hz, 6H) |
| III-12 : 7. 45 (t, J=7. 8Hz, 1H), 7. 30-7. 45 (m, 2H), 7. 21 (d, J=7. 8Hz, 1H), 4. 75 (d, J=15. 6Hz, 1 H) , 4. 67 (d, J=15. 6Hz, 1H), 4. 07 (d, J=10. 2Hz, 1H), 3. 87 (d, J=10. 2Hz, 1H), 3.10-3.25 (m, 2H) , 2. 45-2. 65 (m, 2H) , 1. 70-1. 95 (m, 4H) , 0. 85 (s, 9H) |
| III-13 : 7. 46 (t, J=7. 2Hz, 1H), 7. 30-7. 45 (m, 2H), 7. 21 (d, J=7.2Hz, 1H), 5. 80-5. 95 (m, 1H), 5 .20-5.35(m, 2H), 4.73(d, J=15.6Hz, 2H), 4.71(d, J=15.6Hz, 2H), 3.10-3.30(m, 2H), 2.40-2. 6 5(m, 2H), 1.70-1.95 (m, 4H) |
| III-14 : 7. 35-7. 45 (m, 1H), 7. 30-7. 35 (m, 2H), 7. 25-7. 30 (m, 1H), 5.13 (d, J=10. 5Hz, 1H), 5. 0 3 (d, J=15. 3Hz, 1H), 4. 93 (d, J=10. 5Hz, 1H), 4. 49 (d, J=15. 3Hz, 1H), 3.45 (s, 3H), 3. 25-3. 30 (m , 2H), 2. 45-2. 55 (m, 2H), 1. 75-1. 90 (m, 4H) |
| III-15 : 7. 30-7. 45 (m, 9H), 5. 19 (d, J=10. 5Hz, 1H), 5. 05 (d, J=10. 5Hz, 1H), 4. 95 (d, J=15. 6Hz , 1H), 4. 70 (d, J=12. 0Hz, 1H), 4. 62 (d, J=12. 0Hz, 1H), 4. 59 (d, J=15. 6Hz, 1H), 3. 15-3. 20 (m, 2H ), 2. 45-2. 55 (m, 2H), 1. 70-1. 90 (m, 4H) |
| III-16 : 7. 40-7. 50 (m, 1H), 7. 20-7. 40 (m, 3H), 5. 85 (d, J=11.4Hz, 1H), 5. 77 (d, J=11. 4Hz, 1H) |
| , 5. 05 (d, J=15. 9Hz, 1H), 4. 45 (d, J=15. 9Hz, 1H), 3. 20-3. 40 (m, 2H) , 2. 40-2. 60 (m, 2H) , 2. 40 (q ,J=8. 1Hz, 2H), 1.70-1.95(m,4H), 1. 14 (t, J=8. 1Hz, 3H) |
| III-18: 7.20-7.50 (m, 6H), 7. 05-7. 20 (m, 3H), 5. 11 (d, J=13. 8Hz, 1H), 4. 62 (d, J=13. 8Hz, 1H) ,4.44 (d, J=16. 2Hz, 1H), 4. 07(d, J=16. 2Hz, 1H), 2. 55-2. 80 (m, 2H), 2. 35-2. 55 (m, 2H), 1. 70-1 .90(m, 2H), 1. 55-1. 70 (m, 2H) |
| III-19: 11. 9(brs, 1H), 7. 61 (d, J=8. 2Hz, 1H), 7. 37 (t, J=8. 2Hz, 1H), 7. 28(d, J=8. 2Hz, 1H), 7 . 19(t, J=8.2Hz, 1H), 4.56(d, J=14.6Hz, 1H), 4.40(d, J=14. 6Hz, 1H), 3. 35-3. 55(m, 2H), 2.45-2.60(m, 1H), 1.80-2.05(m, 3H), 1.35-1.55(m, 1H), 0.99(d, J=6.3Hz, 3H) |
| III-20* : 7. 45-7. 55 (m, 1H), 7. 40 (t, J=7. 8Hz, 1H), 7.25-7.35(m, 2H), 5. 24 (d, J=15. 9Hz, 0.5 H), 5.04(d, J=15.6Hz,0. 5H), 4. 45 (d, J=15. 6Hz, 0. 5H), 4. 23(d, J=15.9Hz, 0.5H), 3.30-3.40( ( m, 2H), 2.55-2.65 (m, 1H), 2.00-2.20(m, 2H), 1. 85-1. 95 (m, 1H), 1. 45-1. 55 (m, 2H) , 1. 20-1. 35 (m, 1H), 1. 10-1. 20 (m, 1H), 1. 06 (d, J=6. 0Hz, 3H), 0.95-1. 05 (m, 1H), 0. 85-0. 95 (m, 1H) |
| III-23: 7. 40-7. 50 (m, 1H), 7. 30-7. 40(m, 2H), 7. 21(d, J=8. 1Hz, 1H), 4. 87 (t, J=15. 3Hz, 1H), , 4.54(t, J=16.5Hz, 1H), 4.00-4.15(m, 2H), 3.15-3.25(m, 2H), 2.55-2.65(m, 1H), 1.90-2.20(m , 3H), 1. 75-1. 90 (m, 1H), 1. 40-1. 55 (m, 1H), 1.03(t, J=6. 3Hz, 3H), 0. 85-0. 90 (m, 6H) |
| III-24: 7. 35-7. 50 (m, 3H), 7.21 (d, J=7. 8Hz, 1H), 4. 86 (t, J=15. 3Hz, 1H), 4. 25-4. 60 (m, 3H), 3. 10-3. 25 (m, 2H), 2. 55-2. 70 (m, 1H), 2. 00-2. 20 (m, 1H), 1. 65-1. 95 (m, 2H), 1. 10-1. 55 (m, 6H) , 0. 85-0. 95 (m, 3H) |
| III-25 : 11. 90 (brs, 1H), 7. 62 (d, J=7. 2Hz, 1H), 7. 37 (dt, J=1. 8, 7. 2Hz, 1H), 7. 25-7. 30 (m, 1H ), 7. 18(dt, J=1. 2, 7. 5Hz, 1H), 4.54(d, J=14.7Hz, 1H), 4. 40 (d, J=14. 7Hz, 1H), 3. 35-3. 55 (m, 2 H), 2. 57 (ddd, J=2. 1, 5.1,18. 0Hz, 1H), 1. 85-2. 00 (m, 2H), 1. 70-1. 85 (m, 1H), 1. 50-1. 60 (m, 2H ), 1. 35-1. 50 (m, 1H), 1. 20-1. 35 (m, 2H), 0. 89 (t, J=6. 9Hz, 3H) |
| III-26 : 7.49(t, J=7. 5Hz, 1H), 7. 39 (t, J=7. 8Hz, 1H), 7. 25-7. 35 (m, 1H), 7. 20 (d, J=7. 8Hz, 1H ), 5.06(dd, J=15.6, 46. 8Hz, 1H), 4. 21 (dd, J=15. 6, 60. 3Hz, 1H), 3. 33 (dt, J=3. 9, 7. 5Hz, 2H) , 2 . 55-2. 65 (m, 1H), 2.25-2.50 (m, 2H), 2.00-2. 15 (m, 1H), 1. 80-2. 00 (m, 2H), 1. 50-1. 60 (m, 1H), 1. 25-1. 40 (m, 4H), 1. 11(dt, J=2.4, 7. 5Hz, 3H) , 0. 92 (t, J=6. 6Hz, 3H) |
| III-27: 7. 45-7. 55 (m, 1H), 7. 35-7. 45 (m, 1H), 7. 25-7. 35 (m, 2H), 5. 14 (dd, J=15. 6, 51. 9Hz, 1 H), 4.32(dd, J=15.6, 60. 0Hz, 1H), 3. 25-3. 35(m, 2H), 2.55-2.65 (m, 1H), 2.05-2.15 (m, 1H), 1. 85-2. 00 (m, 2H), 1. 25-1. 40 (m, 6H), 0. 95-1. 15 (m, 4H), 0. 93 (t, J=6. 3Hz, 3H) |
| III-30: 11. 97 (brs, 1H), 7. 55-7. 65(m, 1H), 7. 15-7. 45 (m, 3H), 4. 57 (d, J=14. 7Hz, 1H), 4. 38 ( d, J=14.7Hz, 1H), 3.30-3.60(m, 2H), 2.45-2.65(m, 1H), 1.85-2.15(m, 2H), 1.30-1.60(m, 3H), 0. 80-0. 95 (m, 6H) |
| III-32 : 7. 20-7. 55 (m, 4H), 4. 90-5. 20 (m, 1H), 4. 05-4. 40 (m, 1H), 3. 25-3. 40 (m, 2H), 2. 05-2. 65 (m, 4H), 1. 85-2. 00 (m, 1H), 1. 40-1. 70 (m, 3H), 1. 05-1. 15 (m, 3H), 0. 90 (d, J=7. 6Hz, 6H) |
| III-33 : 7. 20-7. 55 (m, 4H), 5. 00-5. 30 (m, 1H), 4. 15-4. 50 (m, 1H), 3. 25-3. 40(m,2H),2. 50-2. 65 (m, 1H), 2. 10-2. 30 (m, 1H), 1. 85-2. 00 (m, 1H), 0. 80-1. 70 (m, 14H) |
| III-34: 11. 95 (brs, 1H), 7. 55-7. 65 (m, 1H), 7.15-7.45 (m, 3H), 4. 51 (d, J=14. 7Hz, 1H), 4. 43 ( d, J=14.7Hz, 1H), 3.30-3.60(m, 2H), 2.50-2.70(m, 1H), 2.15-2.30(m, 1H), 1.90-2.05(m, 1H), 1. 50-1. 70 (m, 1H), 0. 95-1. 15 (m, 1H), 0.35-0.65(m,3H), 0. 05-0. 20 (m, 2H) |
| III-35 : 7. 15-7. 55 (m, 4H), 4.95-5. 20 (m, 1H), 4. 10-4. 35 (m, 1H), 3. 20-3. 45 (m, 2H) , 2. 20-2. 75 (m, 4H) , 1. 90-2. 10 (m, 1H), 1. 55-1.80(m, 1H), 1. 05-1. 20(m, 4H),0. 40-0. 70 (m, 3H), 0. 05-0 .25(m, 2H) |
| III-36 : 7. 25-7. 55 (m, 4H), 5. 05-5. 25 (m, 1H), 4. 15-4. 50 (m, 1H), 3. 20-3. 45 (m, 2H), 2. 55-2. 75 (m, 1H), 2. 20-2. 40 (m, 1H), 1. 90-2. 10 (m, 1H), 0. 80-1. 85 (m, 7H), 0. 35-0. 75 (m, 3H), 0. 05-0 . 25 (m, 2H) |
| III-37: 7.15-7.55(m, 4H), 4.75-4.90 (m, 1H), 4. 50-4. 65 (m, 1H), 4. 25-4. 50 (m, 2H), 3. 15-3. 20(m, 2H), 2.55-2.75 (m, 1H), 2. 20-2. 40 (m, 1H), 1. 85-2. 05 (m, 1H), 1.50-1. 75 (m, 1H), 1. 25-1 |
| . 40 (m, 3H) , 1. 05-1. 25 (m, 1H), 0.40-0.70(m, 3H), 0.05-0.25(m, 2H) |
| III-39 : 11. 75 (brs, 1H), 7. 61(dd, J=0. 9, 8. 1Hz, 1H), 7. 37 (dt, J=1. 8, 8. 1Hz, 1H), 7. 25-7. 30 (m, 1H), 7.19(dt, J=0. 9, 7. 5Hz, 1H), 4.65(d, J=14.4Hz, 1H), 4. 31 (d, J=14. 4Hz, 1H), 3. 55-3. 7 0 (m, 2H), 3. 35-3. 45 (m, 1H), 3. 33 (s, 3H), 2. 59 (d, J=4. 5Hz, 2H), 1. 85-2. 05 (m, 2H) |
| III-40 : 7. 46 (t, J=7. 5Hz, 1H), 7. 30-7. 40 (m, 2H), 7. 21(d,J=8. 1Hz, 1H), 4. 80 (t, J=15. 0Hz, 1 H), 4.55-4.65(m, 1H), 4. 30-4. 45 (m, 2H), 3. 65-3. 75 (m, 1H), 3. 37 (s, 3H), 3.30-3.40(m, 1H), 3 .05-3.15(m, 1H), 2.73(dd, J=4.2, 17.1Hz, 1H), 2.55-2.70(m, 1H), 1.90-2.00(m, 2H), 1.32(dt , J=0. 9, 7.2Hz, 3H) |
| III-41 : 11. 85 (brs, 1H), 7. 62 (dd, J=1. 2, 8. 1Hz, 1H), 7. 38 (dt, J=1. 8, 7. 5Hz, 1H), 7. 25-7. 30 (m, 1H), 7. 19(dt, J=1. 2, 7. 5Hz, 1H), 4. 67 (d, J=14. 7Hz, 1H), 4. 30 (d, J=14. 7Hz, 1H), 3. 70-3. 8 0(m, 1H), 3. 35-3. 65(m, 4H), 2. 50-2. 65 (m, 2H), 1. 90-2. 00 (m, 2H), 1. 17 (t, J=7. 2Hz, 3H) |
| III-42: 7. 45-7. 50 (m, 1H), 7. 25-7. 40(m, 2H), 7. 20(d, J=6. 9Hz, 1H), 5. 00-5. 10(m, 1H), 4. 22 (t, J=16. 5Hz, 1H), 3.80-3.90(m, 1H), 3.45-3.60(m, 3H), 3.20-3.30 (m, 1H), 2.60-2.65(m, 2H) , 2.25-2.50(m, 2H), 1. 95-2. 05(m, 2H), 1. 20-1. 25 (m, 3H), 1. 11 (t, J=7. 2Hz, 3H) |
| III-43: 7. 40-7. 50 (m, 1H), 7. 30-7. 40 (m, 2H), 7. 20 (d, J=8. 1Hz, 1H), 4. 60-4. 75 (m, 2H) , 4. 25 -4. 45 (m, 2H), 3. 75-3. 85 (m, 1H), 3. 45-3. 60 (m, 2H), 3. 30-3. 40 (m, 1H), 3. 05-3. 15 (m, 1H), 2. 5 5-2.75(m, 2H), 1. 85-2. 00(m, 2H), 1. 32(t, J=7. 2Hz, 3H), 1.21(t, J=7. 2Hz, 3H) |
| III-44 : 7.61(d, J=8. 1Hz, 1H), 7. 38 (dt, J=2. 1, 8. 1Hz, 1H), 7. 25-7. 30 (m, 1H), 7. 15-7. 25 (m, 1H), 4. 55 (d, J=15. 0Hz, 1H), 4. 43 (d, J=14. 4Hz, 1H), 3. 55-3. 70 (m, 1H), 3. 40-3. 50 (m, 1H), 2. 9 5-3.25(m, 1H), 2.75-2.90(m, 1H), 2.40-2.50(m, 1H), 2.10-2.20(m, 1H), 2. 10 (s, 3H), 1. 75-1. 95(m,1H) |
| III-52: 7. 45-7. 55 (m, 1H), 7. 25-7. 40 (m, 2H), 7. 20 (d, J=6. 9Hz, 1H), 5. 11 (d, J=15. 8Hz, 1H), , 4.22(d, J=15.8Hz, 1H), 3.33(t, J=6.3Hz, 2H), 2.30(s, 2H), 2.25-2.40(m, 2H), 1. 55-1. 70 (m, 5 H), 1. 20-1. 35 (m, 1H), 1. 05(s, 6H), 0. 85 (t, J=7. 2Hz, 3H) |
| III-53: 7.49(dt, J=1. 2, 7. 5Hz, 1H), 7.30-7.40(m, 2H), 7.14(d, J=7. 5Hz, 1H), 5.19(d, J=15. 9Hz, 1H), 4.12(d, J=15. 9Hz, 1H), 3. 31 (t, J=6. 3Hz, 2H), 3. 00-3. 15 (m, 1H), 2. 40-2. 55 (m, 1H), 2. 29 (s, 2H), 2. 15-2. 30 (m, 1H), 1. 90-2. 05 (m, 1H), 1. 70-1. 90 (m, 3H), 1. 66 (t, J=6. 3Hz, 2H) , 1 .05(d, J=1.2Hz, 6H) |
| III-54: 7.50(t, J=7.5Hz, 1H), 7.40(t, J=7.5Hz, 1H), 7.34(d, J=7.5Hz, 1H), 7.20(d, J=7.5Hz , 1H), 5.08(d, J=15.6Hz, 1H), 4.24(d, J=15.6Hz, 1H), 3.32(t, J=6.3Hz, 2H), 2.20-2.40(m, 4H) , 1.50-1. 75 (m, 4H), 1. 15-1. 35 (m, 4H) , 1. 05 (s, 6H), 0. 85 (t, J=6. 6Hz, 3H) |
| III-56: 8.10-8.25 (m, 1H), 7. 45-7. 60 (m, 2H), 7. 30-7. 45 (m, 2H), 7. 20-7. 30 (m, 2H), 7.10-7. 20(m, 2H), 5.20(d, J=16. 5Hz, 1H), 4.38(d, J=16.5Hz, 1H), 3.32(t, J=6. 6Hz, 2H), 2.31(s,2H), 1. 67 (t, J=6. 6Hz, 2H), 1. 06 (s, 6H) |
| III-57: 7.45(t, J=7.5Hz, 1H), 7.25-7.40(m, 2H), 7.20(d, J=8.4Hz, 1H), 4.79(d, J=15.6Hz, 1 H), 4.59(d, J=15. 3Hz, 1H), 3.89(s, 3H), 3. 19(t, J=6. 3Hz, 2H), 2. 30 (s, 2H), 1. 55-1. 65 (m, 2H) , 1. 02 (s, 6H) |
| III-61: 7.44(d, J=7.5Hz, 1H), 7.30-7.40(m, 2H), 7.22(t, J=7.5Hz, 1H), 4.83(d, J=15.6Hz, 1 H), 4. 60 (d, J=15. 6Hz, 1H), 4. 06 (d, J=10. 2Hz, 1H), 3. 86 (d, J=10. 2Hz, 1H), 3. 19 (t, J=6. 6Hz, 2 H), 2. 30 (s, 2H), 1. 50-1. 70 (m, 2H), 1.03(s, 6H), 0. 85 (s, 9H) |
| III-62: 7. 46 (t, J=7. 8Hz, 1H), 7. 30-7. 40 (m, 2H), 7. 21 (d, J=7. 8Hz, 1H), 4. 78 (d, J=15. 6Hz, 1 H), 4.66(d, J=15.6Hz, 1H), 4.00-4. 20(m, 2H), 3.20(t, J=6.3Hz, 2H), 2.30(s, 2H), 1. 85-2. 05 ( m, 1H), 1. 50-1. 70 (m, 2H), 1. 02 (s, 6H), 0. 87 (d, J=6. 9Hz, 6H) |
| III-63 : 7. 40-7. 50 (m, 1H), 7. 30-7. 40 (m, 2H), 7. 20 (d, J=8. 1Hz, 1H), 4. 77 (d, J=15. 3Hz, 1H), , 4.64(d, J=15.6Hz.1H), 4.28(t, J=6.9Hz, 2H), 3.19(t, J=6.6Hz, 2H), 2.30(s, 2H), 1.55-1.70( m, 3H), 1. 20-1. 30 (m, 7H), 1. 02 (s, 6H), 0. 86 (t, J=6. 9Hz, 3H) |
| III-64: 7. 47 (d, J=7. 5Hz, 1H), 7. 30-7. 40 (m, 2H), 7. 23 (t, J=7. 5Hz, 1H), 4. 71 (s, 2H), 4. 45-4 |
| .60(m,2H),3.65-3.75(m,2H),3. 21(t, J=6.0Hz, 2H), 2.33(d, J=16.2Hz, 1H), 2.26(d, J=16.2H z, 1H), 1. 50-1. 70 (m, 2H), 1. 02 (d, J=2. 1Hz, 6H) |
| III-65 : 7. 46 (d, J=7. 5Hz, 1H), 7. 30-7. 40 (m, 2H), 7. 15-7. 25 (m, 1H), 6. 28 (t, J=5. 2Hz, 1H), 4 . 79 (d, J=15. 3Hz, 1H), 4.61(d, J=15.3Hz, 1H), 3. 22 (d, J=6. 3Hz, 1H), 3. 17 (d, J=6. 3Hz, 1H), 2. 25-2.35(m, 2H), 2. 05-2. 25(m, 1H), 1. 45-1. 70 (m, 2H), 1. 02 (d, J=3. 6Hz, 6H), 0. 75-1. 00 (m, 6H |
| )III-66: 7. 46 (d, J=7. 5Hz, 1H), 7. 30-7. 40 (m, 2H), 7. 15-7. 25 (m, 1H), 6. 26 (quint, J=6. 0Hz, 1H), 4. 79 (d, J=15. 3Hz, 1H), 4. 60 (d, J=15. 3Hz, 1H), 3. 21 (d, J=6.6Hz, 1H), 3. 17(d, J=6. 6Hz, 1 H), 2.30(s, 2H), 1. 80 (dd, J=6. 0, 4. 5Hz, 3H), 1. 50-1. 70 (m, 2H), 1. 02 (d, J=3. 3Hz, 6H) |
| III-68: 7.40-7. 50 (m, 1H), 7. 30-7. 40 (m, 2H), 7. 22 (d, J=8. 1Hz, 1H), 4. 71 (dd, J=15. 6, 21. 0H z, 2H), 4. 35-4. 50 (m, 2H), 3. 58(dt, J=0.9, 4. 5Hz, 2H), 3. 30(s, 3H), 3. 19 (t, J=6. 3Hz, 2H), 2. 3 0(s, 2H), 1. 55-1. 70 (m, 2H), 1.03(s, 6H) |
| III-69 : 7. 20-7. 55 (m, 9H), 4. 91 (d, J=15. 6Hz, 1H), 4. 73 (d, J=15. 0Hz, 1H), 3. 22 (t, J=6. 6Hz, 2H), 2. 32(dd, J=16.8, 23.4Hz, 2H)1.50-1. 70(m, 2H), 1.02(d, J=4.5Hz, 6H) |
| III-71: 7.65-7.95(m, 4H), 7. 30-7. 60 (m, 7H), 4. 97 (d, J=15. 6Hz, 1H), 4.76(d, J=15. 6Hz, 1H) , 3. 20 (t, J=6. 3Hz, 2H), 2. 35 (d, J=17. 4Hz, 1H), 2. 27 (d, J=17. 4Hz, 1H), 1. 40-1. 70 (m, 2H) , 0,9 7(d, J=6.3Hz, 6H) |
| III-72 : 7. 55 (d, J=6. 9Hz, 1H), 7. 25-7.45 (m, 3H), 4. 81 (s, 2H), 3. 23 (t, J=6. 3Hz, 2H), 2. 35 (s , 2H), 1.55-1.70(m, 2H), 1.06(s, 6H) |
| III-73 : 12.0(brs, 1H), 7.62(d, J=8. 1Hz, 1H), 7. 38 (t, J=8. 1Hz, 1H), 7. 15-7. 35 (m, 2H) , 4. 51 (d, J=14. 4Hz, 1H), 4. 45 (d, J=14. 7Hz, 1H), 3. 46 (t, J=6. 3Hz, 2H), 2. 21 (s, 2H), 1. 50-1. 70 (m, 2 H), 1.28(q, J=7. 6Hz, 2H), 0.89(s, 3H), 0.84(t, J=7.5Hz, 3H) |
| III-74 : 7.45-7.55 (m, 1H), 7. 30-7. 45 (m, 2H), 7. 21 (d, J=7. 5Hz, 1H), 5. 00 (dd, J=15. 6, 18. 9H z, 1H), 4. 28(dd, J=15. 9, 21. 0Hz, 1H), 3.20-3. 40(m, 2H), 2. 15-2. 35 (m, 5H), 1. 55-1. 80 (m, 2H) , 1.37(q, J=7. 5Hz, 2H), 0.99(s, 3H), 0. 89 (dt, J=1. 8, 7. 8Hz, 3H) |
| III-75 : 7. 50 (d, J=7. 8Hz, 1H), 7. 25-7. 45 (m, 2H), 7. 21 (d, J=7. 8Hz, 1H), 5.11 (t, J=13. 2Hz, 1 H), 4. 19 (t, J=13. 2Hz, 1H), 3. 25-3. 45 (m, 2H), 2. 20-2. 50 (m, 4H), 1. 55-1. 80 (m, 2H), 1. 37 (q, J =7.5Hz, 2H), 1. 11 (t, J=7. 5Hz, 3H), 0. 99 (s, 3H), 0. 87 (t, J=7. 5Hz, 3H) |
| III-76 : 7.49(dt, J=1.5, 7. 5Hz, 1H), 7. 30-7. 45 (m, 3H), 5. 18 (dd, J=10. 2, 16. 2Hz, 1H), 4. 30 ( dd, J=11.4, 16. 2Hz, 1H), 3. 25-3. 35 (m, 2H), 2. 25-2. 30 (m, 2H), 1. 60-1. 75 (m, 1H), 1. 50-1. 55 ( m, 4H), 1. 38 (q, J=7. 8Hz, 2H), 1. 25-1. 35 (m, 1H), 1. 10-1. 20 (m, 1H), 0. 99 (s, 3H), 0. 85-0. 95 (m , 3H) |
| III-77 : 7. 46(t, J=7. 8Hz, 1H), 7. 25-7. 50(m, 2H), 7. 21 (d, J=7. 8Hz, 1H), 5. 71 (dd, J=15. 2, 18 .2Hz, 1H), 4. 59 (dd, J=15. 2, 18. 2Hz, 1H), 3. 90 (s, 3H) , 3.10-3.25 (m, 2H) , 2. 20-2. 35 (m, 2H), 1 .50-1. 70 (m, 2H), 1. 35 (q, J=7. 4Hz, 2H), 0. 96(s, 3H), 0.84(t, J=7.4Hz, 3H) |
| III-78: 7. 46 (t, J=7. 5Hz, 1H), 7. 30-7. 40 (m, 2H), 7.21 (d, J=7. 8Hz, 1H), 4. 55-4. 85 (m, 2H) , 4 . 25-4. 45 (m, 2H), 3. 10-3. 25 (m, 2H), 2. 20-2. 35 (m, 2H), 1. 45-1. 70 (m, 2H), 1. 20-1. 40 (m, 5H), 0. 96 (s, 3H), 0.87(t, J=7. 5Hz, 3H) |
| III-79: 11. 94 (brs, 1H), 7. 55-7. 65(m, 1H), 7. 15-7. 40 (m, 3H), 4. 56 (d, J=14. 4Hz, 1H), 4. 35 ( d, J=14. 4Hz, 1H), 3. 35-3. 50 (m, 2H), 1. 80-2. 05 (m, 2H), 1. 45-1. 65 (m, 2H), 1. 20-1. 30 (m, 3H), 1. 01(t,J=6. 0Hz,3H) |
| III-80: 7. 15-7. 50 (m, 4H), 4. 25-5. 05 (m, 4H), 3.10-3.25 (m, 2H), 2. 00-2. 15 (m, 1H), 1. 50-1. 90 (m, 3H), 1. 25-1. 35 (m, 6H), 1. 05-1. 10 (m, 3H) |
| III-81 : 7. 45-7. 55 (m, 1H), 7. 35-7. 45 (m, 2H), 7. 20-7. 30 (m, 1H), 4. 93 (dd, J=15. 3, 31. 8Hz, 1 H), 4. 45-4.65(m, 3H), 4. 30-4.45(m, 2H), 3. 55-3. 65(m, 1H), 3.20-3. 30 (m, 1H), 2. 65-2. 85 (m, 1H), 2. 00-2.10 (m, 1H), 1. 33 (t, J=7. 2Hz, 3H) |
| III-82* : 7. 35-7. 55 (m, 3H), 7. 22 (d, J=7. 8Hz, 1H), 5. 07 (d, J=15. 9Hz, 0. 5H) , 4. 80-4. 90 (m, 1 . 5H), 4. 65-4. 75 (m, 1H), 4. 62 (d, J=15. 6Hz, 0. 5H), 4. 30-4. 45 (m, 2. 5H), 3.65-3.75 (m, 1H), 3. |
| 25-3.40 (m, 1H), 2.80-2. 95 (m, 1H), 2.00-2.15 (m, 1H), 1.33 (dt, J=1. 5, 7. 2Hz, 3H) |
| III-83* : 11. 14 (brs, 1H), 7. 61 (d, J=8. 4Hz, 1H), 7. 35-7. 45 (m, 1H), 7. 15-7. 30 (m, 2H), 4. 55-4. 65 (m, 1H), 4. 35-4. 45 (m, 1.5H), 4. 04 (d, J=7.5Hz, 0.5H), 3. 40-3. 55 (m, 2H), 1. 95-2. 25 (m, 2 H), 1. 55-1. 75 (m, 1H), 1. 12 (dd, J=3.3, 6.6Hz, 3H) |
| III-84* : 7. 45-7. 50 (m, 1H), 7.30-7.40 (m, 2H), 7. 21 (d, J=7.8Hz, 1H), 4. 88 (dd, J=6.0, 15. 6H z, 0.5H), 4. 25-4. 75 (m, 4H), 4. 05-4. 20 (m, 0.5H), 3. 15-3. 30(m, 2H), 2.20-2. 40 (m, 1H), 1.90-2. 20 (m, 1H), 1. 55-1. 65 (m, 1H), 1.25-1.35 (m, 3H), 1.10-1.20 (m, 3H) |
| III-85 : 11. 41 (brs, 1H), 7.61 (d, J=8. 3Hz, 1H), 7. 39 (t, J=8. 3Hz, 1H), 7. 15-7. 30 (m, 2H), 4. 5 1 (d, J=10. 7Hz, 2H), 4.05-4.30 (m, 2H), 3. 35-3. 55 (m, 2H), 1. 85-2.30 (m, 2H) , 1. 45-1. 75 (m, 2H ), 1. 15-1. 30 (m, 3H), 1. 02 (dd, J=2.5, 6. 9Hz, 3H) |
| III-86 : 11.25 (brs, 1H), 7. 55-7. 65 (m, 1H), 7.10-7.40 (m, 3H), 4.49 (q, J=7. 8Hz, 2H), 4.19 (q , J=7.8Hz, 2H), 4.12 (q, J=7.8Hz, 2H) , 3.40-3. 55 (m, 2H), 3.12 (d, J=9.0Hz, 1H), 1. 95-2. 15 (m, 1H), 1. 35-1. 65 (m, 2H), 1. 20-1. 30 (m, 3H), 0.92 (t, J=7.8Hz, 3H) |
| III-87 : 7. 10-7. 60 (m, 4H), 4.15-4.90 (m, 8H), 3. 10-3. 40 (m, 3H), 0.85-2.30 (m, 12H) |
| III-88 : 11. 90 (brs, 1H), 7. 60-7. 65 (m, 1H), 7.15-7. 40 (m, 3H), 4. 60 (d, J=14. 7Hz, 1H), 4. 31 ( d, J=14. 7Hz, 1H), 3.35-3.40 (m, 1H), 3.00-3.10 (m, 1H), 2. 05-2. 35 (m, 2H), 1. 70-1. 85 (m, 1H), 0. 90-1. 00 (m, 6H), 0. 77 (s, 3H) |
| III-89 : 7.15-7.50 (m, 4H), 4.25-5.10 (m, 4H), 3.00-3.15 (m, 1H), 2.75-2.90 (m, 1H), 2. 30-2. 40 (m, 2H), 1.75-1.85 (m, 1H), 1. 25-1. 35 (m, 3H), 0. 95-1. 05 (m, 3H), 0.80-0.90 (m, 6H) |
| III-90 : 11. 59 (brs, 1H), 7. 55-7.60 (m, 1H), 7.15-7.40 (m, 3H), 4. 96 (d, J=15.0Hz, 1H), 4. 27 ( d, J=15. 0Hz, 1H), 3. 60-3. 75 (m, 1H), 2. 20-2. 25 (m, 2H), 1. 70-1.80 (m, 1H), 1. 40-1. 50 (m, 4H), 0. 99 (s, 3H), 0.69 (s, 3H) |
| III-91 : 7.15-7.45 (m, 4H), 4.25-5.00 (m, 4H), 3. 50-3. 60 (m, 1H), 2.30-2.40 (m, 2H), 1. 70-1. 75 (m, 1H), 1. 45-1. 55 (m, 1H), 1.00-1.40 (m, 12H) |
| III-94 : 11. 92 (brs, 1H), 7. 60-7. 65 (m, 1H), 7. 30-7. 40 (m, 1H), 7. 15-7. 30 (m, 2H), 4. 54 (d, J= 14. 4Hz, 1H), 4.37 (d, J=14.4Hz, 1H), 3.35-3.45 (m, 2H), 2.16 (q, J=7. 2Hz, 1H), 1. 55-1. 75 (m, 2 H), 1. 16 (q, J=7. 2Hz, 3H), 0.96 (s, 3H), 0.83 (s, 3H) |
| III-95 : 7. 15-7. 50 (m, 4H), 4. 97 (d, J=15. 7Hz, 1H), 4.55-4. 75 (m, 1H), 4. 25-4. 45 (m, 2H), 3.1 0-3. 20 (m, 2H), 2.25-2. 30 (m, 1H), 1. 60-1. 70 (m, 2H), 1. 20-1. 35 (m, 6H), 0. 04 (s, 3H), 0.92 (s, 3H) |
| III-96 : 11. 80 (brs, 1H), 7. 64 (d, J=8. 1Hz, 1H), 7. 10-7. 45 (m, 8H), 4.60 (d, J=14. 4Hz, 1H), 4. 46 (d, J=14.4Hz, 1H), 3.55 (dd, J=4.2, 7.8Hz, 2H), 3.00-3.15 (m, 1H), 2.70-2.85 (m, 1H), 2.45-2. 60 (m, 1H), 2. 05-2. 25 (m, 1H), 1. 85-2. 05 (m, 1H) |
| III-97* : 7. 40-7. 50 (m, 1H), 7. 25-7.40 (m, 4H), 7. 15-7.25 (m, 4H), 4.95 (d, J=15. 6Hz, 0. 5H), 4. 83 (d, J=15. 6Hz, 0. 5H), 4.66 (d, J=15. 6Hz, 0.5H), 4. 55 (d, J=15. 6Hz, 0. 5H) , 4. 25-4. 50 (m, 2 H), 3.00-3.40 (m, 3H), 2.75-2.95 (m, 1H), 2. 50-2. 75 (m, 1H), 1. 80-2. 20 (m, 2H), 1. 32 (dt, J=2. 1, 7. 2Hz, 3H) |
| III-98* : 7. 45-7. 60 (m, 1H), 7. 30-7.45 (m, 4H), 7.15-7.30 (m, 4H), 5.15 (d, J=15.6Hz, 0. 5H), 5. 00(d,J=15. 6Hz, 0. 5H), 4.42 (d, J=15. 6Hz, 0. 5H), 4.22 (d, J=15. 6Hz, 0. 5H), 3. 25-3. 55 (m, 2 H), 3.10-3.25 (m, 1H), 2.75-2. 90 (m, 1H), 2. 55-2. 70 (m, 1H), 2. 25-2. 55 (m, 2H) , 1. 95-2. 25 (m, 2H), 1.13 (dt, J=3.0, 6.9Hz, 3H) |
| III-99 : 11. 70 (bs, 1H), 7. 60-7. 65 (m, 1H), 7.15-7.45 (m, 3H), 4.52 (s, 2H), 3. 52 (t, J=6. 0Hz, 2H), 2. 33 (s, 2H), 1. 63 (t, J=6. 0Hz, 2H), 0.48 (s, 4H) |
| III-104 : 7. 46 (dt, J=1. 5, 7. 5Hz, 1H), 7. 30-7.40 (m, 2H), 7.21 (d, J=7. 5Hz, 1H), 4. 78 (d, J=15 .6Hz, 1H), 4. 69 (d, J=15. 6Hz, 1H), 4. 26 (t, J=6.3Hz, 2H), 3. 25 (t, J=6. 3Hz, 2H), 2. 44 (d, J=18. 0Hz, 1H), 2. 36 (d, J=18. 0Hz, 1H), 1. 50-1. 75 (m, 4H), 0.89 (t, J=7.5Hz, 3H), 0.40-0.50 (m, 4H) |
| III-105 : 7. 45-7. 50 (m, 1H), 7. 30-7. 40 (m, 2H), 7. 20-7. 25 (m, 1H), 4. 80 (d, J=15. 6Hz, 1H), 4. |
| 69 (d, J=15. 6Hz, 1H), 4.00-4.15 (m, 2H), 3.26 (t, J=6. 0Hz, 2H), 2.44 (d, J=18. 0Hz, 1H), 2.36 (d , J=18. 0Hz, 1H), 1. 90-2. 00 (m, 1H), 1. 55-1. 70 (m, 2H), 0.87 (d, J=6.6Hz, 6H), 0.40-0.45 (m, 4H ) |
| III-106: 7.48 (dt, J=1.2, 7.8Hz, 1H), 7. 30-7. 40 (m, 2H), 7. 20-7. 25 (m, 1H), 4.83 (d, J=15.6H z, 1H), 4.67 (d, J=15.6Hz, 1H), 4.45-4.60 (m, 2H), 3.70 (t, J=5. 7Hz, 2H), 3.27 (t, J=5. 7Hz, 2H) , 2.47 (d, J=18.0Hz, 1H), 2.32 (d, J=18. 0Hz, 1H), 1. 60-1. 75 (m, 1H), 1. 50-1. 60 (m, 1H), 0.40-0 .45 (m, 4H) |
| III-107: 11. 91 (brs, 1H), 7.62(d, J=8.1Hz, 1H), 7. 38 (t, J=8.4Hz, 1H), 7. 25-7. 30 (m, 1H), 7. 19 (t, J=7. 2Hz, 1H), 4.48 (s, 2H), 3.47 (t, J=6. 3Hz, 2H), 2.31 (s, 2H), 1. 60-1.75 (m, 6H) , 1. 40-1.45 (m, 4H) |
| III-109 : 7.49 (dt, J=1.5, 7.5Hz, 1H), 7.40 (dt, J=1.8, 8.1Hz, 1H), 7.25-7.35 (m, 2H) , 5.19 (d , J=15. 9Hz, 1H), 4. 29 (d, J=15. 9Hz, 1H), 3. 30-3. 40 (m, 2H), 2. 39 (s, 2H), 1. 65-1. 80 (m, 6H), 1. 45-1. 55 (m, 5H), 1. 25-1. 35 (m, 1H), 1. 10-1. 20 (m, 1H), 0.95-1. 05 (m, 1H), 0.85-0. 95 (m, 1H) |
| III-110: 7.45 (dt, J=1.2, 7.8Hz, 1H), 7.30-7.40 (m, 2H), 7.20 (d, J=8. 1Hz, 1H), 4.78 (d, J=15 . 3Hz, 1H), 4.60 (d, J=15. 6Hz, 1H), 4.25-4.45 (m, 2H), 3.19 (t, J=6.6Hz, 2H), 2.40 (s, 2H), 1. 60 -1. 80 (m, 6H), 1. 45-1. 50 (m, 4H), 1. 32 (t, J=7.2Hz, 3H) |
| III-111: 7.46 (dt, J=1.5, 7.8Hz, 1H), 7. 30-7.40 (m, 2H), 7.21 (d, J=7. 8Hz, 1H), 4.81 (d, J=15 . 3Hz, 1H), 4. 60 (d, J=15. 6Hz, 1H), 4. 00-4. 15 (m, 2H), 3.19 (t, J=6. 3Hz, 2H), 2. 40 (s, 2H), 1. 90 -2. 00 (m, 1H), 1.60-1. 75 (m, 6H), 1. 45-1. 50 (m, 4H), 0.87 (dd, J=1. 5, 6. 9Hz, 6H) |
| III-112* : 7.40-7.50 (m, 1H), 7. 20-7. 40 (m, 2H), 7.10-7.20 (m, 1H), 4.96 (d, J=15. 8Hz, 0. 5H) , 4.71 (d, J=14. 7Hz, 0.5H), 4.58 (d, J=14. 7Hz, 0.5H), 4. 20-4. 45 (m, 2.5H), 2.90-3. 10 (m, 2H) , 1. 90-2.05 (m, 2H), 1. 80-1. 90 (m, 1H), 1. 60-1. 70 (m, 1H), 1. 25-1. 35 (m, 3H), 1. 15-1. 25 (m, 1H) , 0.95-1.10 (m, 1H) |
| III-113 : 7.15-7.50 (m, 4H), 4.82 (d, J=15.6Hz, 1H), 4.50 (d, J=15.6Hz, 1H), 4.25-4.45 (m, 2H ), 3.50-3.70(m, 2H), 3.25-3.45 (m, 1H), 2.85-2.95 (m, 1H), 2.20-2.35 (m, 1H), 1. 95-2.15 (m, 1 H), 1. 25-1.40 (m, 3H) |
| III-114 : 7.55-7.65 (m, 1H), 7.15-7.45 (m, 3H), 4.60 (d, J=14. 7Hz, 1H), 4.27 (d, J=14. 7Hz, 1H ), 3.45-3.60 (m, 1H), 3.35 (s, 1H), 3.15-3.30 (m, 1H), 1. 95-2. 20 (m, 2H), 1. 47 (s, 3H) |
| III-115 : 7. 57 (d, J=8. 1Hz, 1H), 7.37 (t, J=8.1Hz, 1H), 7.20-7.35 (m, 2H), 6.13 (d, J=7.5Hz, 1 H), 5. 15 (d, J=7.5Hz, 1H), 4. 58 (s, 2H), 2. 38 (s, 2H), 0.97 (s, 6H) |
| III-117 : 10.54 (brs, 1H), 7.60 (d, J=8. 4Hz, 1H), 7.35-7.45 (m, 2H), 7.20-7.35 (m, 1H), 5.26 ( s, 2H), 3.60 (t, J=6.6Hz, 2H), 2.84 (s, 2H), 1.69 (t, J=6.6Hz, 2H), 0.97 (s, 6H) |
| III-118 : 7.48 (t, J=7.2Hz, 1H), 7.30-7.45 (m, 2H), 7.24 (d, J=7.2Hz, 1H), 5.72 (d, J=15.0Hz, 1H), 5.16 (d, J=15.0Hz, 1H), 4.25-4.50 (m, 2H), 3.38 (t, J=6.3Hz, 2H), 2.94 (s, 2H), 1.50-1.80 (m, 2H), 1. 26 (t, J=7. 2Hz, 3H), 1. 02 (s, 6H) |
| III-119 : 11. 50 (brs, 1H), 7. 66 (dd, J=0. 9, 7.8Hz, 1H), 7.41 (dt, J=1.5, 7. 8Hz, 1H), 7.25-7.3 5 (m, 1H), 7.20-7.25 (m, 1H), 4. 68 (d, J=14. 4Hz, 1H), 4. 43 (d, J=14. 4Hz, 1H), 3. 60-3.75 (m, 1H) , 3.50-3.60 (m, 1H), 2.87 (d, J=18.6Hz, 1H), 2.52 (d, J=18. 6Hz, 1H), 2.05-2.15 (m, 1H), 1.90-2 .00 (m, 1H), 1.43 (d, J=7.2Hz, 1H), 1.40 (d, J=7.2Hz, 1H) |
| III-120: 7.49 (tt, J=1.5, 7. 5Hz, 1H), 7. 30-7. 45 (m, 2H), 7.20-7.25 (m, 1H), 4.7 (dd, J=15.6 , 15.9Hz, 1H), 4.65 (dd, J=15.6, 24.0Hz, 1H), 4.30-4.45 (m, 2H), 3.35-3.45 (m, 1H), 3.20-3.30 (m, 1H), 2.92 (dd, J=17.7, 27.0Hz, 1H), 2.60 (d, J=1 7.7, 27.0Hz, 1H), 2.10-2.20 (m, 1H), 1.85-1. 95 (m, 1H), 1. 40-1. 45 (m, 2H), 1. 33 (dt, J=1.2, 7. 2Hz, 3H) |
| IV-1 : 7.40-7.50 (m, 1H), 7.30-7.40 (m, 2H), 7.15-7.25 (m, 1H), 4.87 (d, J=15. 3Hz, 1H), 4.45-4. 55 (m, 1H), 4. 25-4. 45 (m, 2H), 3. 10-3. 20 (m, 1H), 2. 75-2. 90 (m, 1H), 2. 40-2. 60 (m, 2H), 1. 80 -2.05 (m, 2H), 1.40-1.55 (m, 1H), 1. 32 (dd, J=2. 4, 7. 2Hz, 3H), 0. 96 (dd, J=3. 3, 6. 6Hz, 3H) |
| IV-3 : 7.40-7.50 (m, 1H), 7.30-7.40 (m, 2H), 7.20 (d, J=7. 8Hz, 1H), 4.80 (d, J=15.6Hz, 1H), 4. |
| 54 (d, J=15. 6Hz, 1H), 4.25-4.45 (m, 2H), 2.91 (d, J=14.4Hz, 1H), 2. 88 (d, J=14. 4Hz, 1H), 2. 53 ( t, J=6.3Hz, 2H), 1. 64 (dt, J=3.0, 6.3Hz, 2H), 1. 31 (t, J=7.2Hz, 3H) 0.99 (d, J=3. 0Hz, 6H) |
| IV-4 : 11. 95 (brs, 1H), 7.62 (d, J=8.4Hz, 1H), 7.10-7.45 (m, 3H), 4. 52 (d, J=12. 3Hz, 1H) , 4. 40 (d, J=12. 3Hz, 1H), 3.35-3.50 (m, 2H), 2. 35-2. 60 (m, 1H), 1. 40-2. 00 (m, 4H), 1. 23 (d, J=7. 2Hz, 3H) |
| IV-5 : 7.46 (t, J=8.4Hz, 1H), 7.37 (t, J=8.4Hz, 1H), 7.31 (d, J=8.4Hz, 1H), 7.20 (d, J=8.4Hz, 1 H), 4.20-5.00 (m, 4H), 3. 10-3.30 (m, 2H), 2.45-2.65 (m, 1H), 1. 45-2. 10 (m, 4H), 1. 15-1. 45 (m, 6H) |
| IV-6 : 12. 00 (brs, 1H), 7. 05-7. 65 (m, 4H), 4.51 (d, J=14. 6Hz, 1H), 4.41 (d, J=14. 6Hz, 1H), 3. 4 0-3. 45 (m, 2H), 2. 20-2. 30 (m, 1H), 1. 80-2. 05 (m, 3H), 1. 65-1. 80 (m, 1H), 1. 40-1. 60 (m, 2H), 0. 85-0. 95 (m, 3H) |
| IV-7 : 7. 45-7. 50 (m, 1H), 7. 35-7. 40 (m, 1H), 7.20-7.30 (m, 2H), 5.00-5.25 (m, 1H), 4.00-4.25 (m, 1H), 3. 30-3. 35 (m, 2H), 2. 20-2. 45 (m, 3H), 1. 55-2. 05 (m, 6H), 1.05-1. 15 (m, 3H), 0.95-1. 00 (m, 3H) |
| IV-8 : 7. 45-7. 55 (m, 1H), 7. 35-7. 40 (m, 1H), 7. 25-7. 35 (m, 2H), 5.10-5. 30 (m, 1H), 4. 15-4. 40 (m, 1H), 3. 25-3. 40 (m, 2H), 2. 30-2. 40 (m, 1H), 0.85-2.10 (m, 14H) |
| IV-9 : 7.15-7.45 (m, 4H), 4.30-5.00 (m, 4H), 3.15-3.20 (m, 2H), 2.30-2. 45 (m, 1H) , 1. 55-2. 05 (m, 6H), 1. 25-1. 35 (m, 3H), 0.95-1.05 (m, 3H) |
| IV-10 : 7. 40-7. 50 (m, 1H), 7. 25-7. 40 (m, 2H), 7. 15-7. 20 (m, 1H), 4. 35-5. 00 (m, 2H), 3. 95-4.1 5 (m, 2H), 3.15-3. 20 (m, 2H), 2. 30-2. 40 (m, 1H), 1. 50-2. 10 (m, 7H), 0.95-1.00 (m, 3H), 0.80-0. 90 (m, 6H) |
| IV-11 : 12.01 (brs, 1H), 7.60-7.65 (m, 1H), 7.35-7.40 (m, 1H), 7.25-7.30 (m, 1H), 7.15-7.20 ( m, 1H), 4.46 (dd, J=14. 7, 32. 4Hz, 2H), 3. 40-3. 45 (m, 2H), 2.30-2.40 (m, 1H), 1.80-2.00 (m, 3H) , 1.65-1.80 (m, 1H), 1. 25-1.55 (m, 4H), 0.90 (t, J=7.2Hz, 3H) |
| IV-12* : 7. 40-7. 50 (m, 1H), 7. 25-7. 40 (m, 2H), 7. 19 (d, J=8.1Hz, 1H), 4. 95 (d, J=15. 6Hz, 0.5H ), 4.68 (dd, J=15. 6, 18. 0Hz, 1H), 4. 25-4. 45 (m, 2. 5H), 3. 15-3. 20 (m, 2H) 2. 35-2. 50 (m, 1H), 1 . 90-2.05 (m, 2H), 1. 80-1. 90 (m, 1H), 1. 65-1. 80 (m, 1H), 1. 35-1. 65 (m, 4H), 1. 20-1. 35 (m, 3H), 0.96(t,J=7.2Hz,3H) |
| IV-13 : 7. 46 (dt, J=0.9, 7. 5Hz, 1H), 7. 30-7. 40 (m, 2H), 7. 20 (d, J=7.2Hz, 1H), 4. 50-4. 80 (m, 2 H), 4.30-4.45 (m, 2H), 3.75-3.85 (m, 1H), 3. 62 (s, 3H), 3. 10-3. 20 (m, 2H), 1. 90-2. 00 (m, 3H), 1 .70-1.80 (m, 1H), 1. 31 (t, J=7. 2Hz, 3H) |
| IV-14 : 11.57 (brs, 1H), 7. 61 (t, J=9. 0Hz, 1H), 7. 35-7. 40 (m, 1H), 7. 15-7. 25 (m, 2H), 4. 53 (d, J=14. 1Hz, 1H), 4. 44 (d, J=14.1Hz, 1H), 3. 40-3. 50 (m, 2H), 3. 35-3. 40 (m, 1H), 2. 34 (s, 3H), 1. 9 0-2. 15 (m, 3H), 1. 75-1. 85 (m, 1H) |
| IV-15 : 7. 45-7. 50 (m, 1H), 7. 35-7. 40 (m, 2H), 7. 20 (d, J=8.4Hz, 1H), 4. 91 (d, J=16. 2Hz, 1H), 4 .47 (d, J=16.2Hz, 1H), 4.30-4.40 (m, 2H), 3.45 (t, J=4.8Hz, 1H), 3.15-3.25 (m, 2H), 2.39 (s, 3H ), 2.20-2.25 (m, 1H), 1. 95-2. 20 (m, 2H), 1. 70-1. 80 (m, 1H), 1. 31 (t, J=6.9Hz, 3H) |
| IV-16 : 11. 50 (brs, 1H), 7. 10-7. 80 (m, 9H), 4. 40-4. 70 (m, 2H), 3. 75-3. 95 (m, 1H), 3. 30-3. 65 ( m, 2H), 1. 70-2. 30 (m, 4H) |
| IV-17 : 7. 55-7. 65 (m, 3H), 7. 40-7. 55 (m, 2H), 7. 25-7. 40 (m, 3H), 7. 15-7. 25 (m, 1H), 4.85 (d, J =15. 6Hz, 1H), 4. 58 (d, J=15. 6Hz, 1H), 4. 25-4. 50 (m, 2H), 3. 95-4. 10 (m, 1H), 3. 20 (d, J=6.0Hz, 2H), 1. 95-2. 30 (m, 2H), 1.70-1.90 (m, 2H), 1.30 (dt, J=0.9, 7. 2Hz, 3H) |
| IV-18 : 11. 12 (brs, 1H), 7. 15-7. 65 (m, 4H), 4. 40-4. 85 (m, 3H), 3. 40-3. 60 (m, 2H), 1. 75-2. 30 ( m,4H) |
| IV-19 : 7. 45-7. 60 (m, 1H), 7.30-7.45 (m, 2H), 7.15-7.25 (m, 1H), 5. 06 (d, J=16. 2Hz, 1H), 4. 45 -4.55 (m, 1H), 4.15-4.30 (m, 1H), 3. 30-3. 45 (m, 2H), 2.20-2.50 (m, 5H), 1. 80-1. 95 (m, 1H), 1. 0 8 (t, J=7. 2Hz, 3H) |
| IV-20 : 7.50-7.60 (m, 2H), 7. 35-7. 45 (m, 2H), 7.20-7.35 (m, 3H), 7.10-7.20 (m, 2H), 5.20 (d, J =16. 2Hz, 1H), 4.50-4.65 (m, 1H), 4.34 (d, J=16.2Hz, 1H), 3.30-3.45 (m, 2H), 2.20-2.50 (m, 3H) , 1. 75-1. 93 (m, 1H) |
| IV-21 : 7. 20-7. 50 (m, 4H), 4.25-4.80 (m, 5H), 3.20-3.30 (m, 2H), 2.10-2.40 (m, 3H) , 1.75-1.8 5 (m, 1H), 1. 25-1. 35 (m, 3H) |
| IV-22 : 11.12 (brs, 1H), 7.62 (d, J=8.0Hz, 1H), 7.39 (t, J=8.0Hz, 1H), 7.15-7.30 (m, 2H), 4.64 (d, J=14. 8Hz, 1H), 4.55-4.60 (m, 1H), 4. 38 (d, J=14. 8Hz, 1H), 3.40-3.60 (m, 2H), 2. 10-2. 40 (m , 3H), 1. 80-1. 95 (m, 1H) |
| IV-23 : 7.48 (dd, J=1.8, 7.5Hz, 1H), 7. 43 (dt, J=1.8, 7.8Hz, 1H), 7. 30-7. 40 (m, 1H), 7. 20-7. 2 5 (m, 1H), 5. 06 (d, J=15.3Hz, 1H), 4. 35-4. 50 (m, 3H), 3. 71 (dd, J=5.7, 8. 4Hz, 1H), 3. 20 (t, J=5. 7Hz, 2H), 2. 10-2.30 (m, 2H), 1. 80-2. 00 (m, 2H), 1. 34 (t, J=7. 2Hz, 3H) |
| IV-25 : 7.45-7.55 (m, 1H), 7. 35-7. 45 (m, 1H), 7.15-7.30 (m, 2H), 5. 14 (d, J=16. 2Hz, 1H), 4.07 (d, J=16. 2Hz, 1H), 3.34 (t, J=6.0Hz, 2H), 2.15-2.40 (m, 2H), 1.75-2.00 (m, 4H), 1. 30 (s, 3H), 1 .27 (s, 3H), 1. 10 (t, J=7.2Hz, 3H) |
| IV-26 : 7. 40-7.50 (m, 1H), 7.30-7.40 (m, 1H), 7.15-7.30 (m, 2H), 4.89 (d, J=15.9Hz, 1H), 4.25 -4. 45 (m, 3H), 3. 20 (t, J=6.0Hz, 2H), 1. 70-1. 90 (m, 4H), 1. 25-1. 35 (m, 9H) |
| IV-27 : 7.59 (d, J=8.4Hz, 1H), 7.37 (t, J=8. 4Hz, 1H), 7.15-7.35 (m, 2H), 4.97 (d, J=14.7Hz, 1H ), 4.04 (d, J=14.7Hz, 1H), 3. 70-3. 90 (m, 1H), 2.30-2.60 (m, 2H), 1. 60-2.00 (m, 4H), 1. 37 (d, J= 8.1Hz, 3H) |
| IV-28 : 7.44 (t, J=8.4Hz, 1H), 7.10-7.40 (m, 3H), 5. 10-5. 35 (m, 1H), 4.10-4.50 (m, 3H), 3.35-3. 60 (m, 1H), 2. 40-2. 60 (m, 2H), 1. 55-2. 10 (m, 4H), 1. 10-1. 40 (m, 6H) |
| IV-30* : 7.30-7.45 (m, 3H), 7.20-7.25 (m, 1H), 5.40 (dd, J=15.3, 26. 7Hz, 1H), 4. 74 (t, J=15. 0 Hz, 0.5H), 4.52 (t, J=15.0Hz, 0.5H), 4.10-4.45 (m, 3H), 3.27 (d, J=18. 3Hz, 3H), 2. 30-2. 60 (m, 2H), 1. 85-2. 10 (m, 2H), 1. 65-1. 75 (m, 2H), 1. 32 (dt, J=7.2, 12.9Hz, 3H) |
| IV-31 : 11.88 (brs, 1H), 7.15-7.65 (m, 4H), 4.40-4.50 (m, 2H), 3.00-3.25 (m, 2H), 2.40-2.50 ( m, 1H), 1. 60-1. 70 (m, 1H), 1. 20-1. 40 (m, 4H), 0.99 (s, 3H), 0.93 (s, 3H) |
| IV-32 : 7.15-7.50 (m, 4H), 4.20-5.10 (m, 4H), 3.00-3.10 (m, 1H), 2.75-2.85 (m, 1H), 2.55-2.6 0 (m, 1H), 1.65-1.75 (m, 1H) 1.40-1.50 (m, 1H), 1. 25-1. 35 (m, 6H), 1. 03 (s, 3H), 0.95 (s, 3H) |
| V-2 : 7.15-7.50 (m, 4H), 5.1 (d, J=15.6Hz, 1H), 4.29 (d, J=15.6Hz, 1H), 3.30-3.50 (m, 2H), 2. 55-2.70 (m, 2H), 2.23 (s, 3H), 1.65-1.85 (m, 6H) |
| V-3 : 7.15-7.50 (m, 4H), 5.08 (d, J=15.9Hz, 1H), 4. 22 (d, J=15. 9Hz, 1H), 3, 30-3.55 (m, 2H), 2. 55-2. 70 (m, 2H), 2. 20-2. 50 (m, 2H), 1. 65-1. 85 (m, 6H), 1. 05-1. 15 (m, 3H) |
| V-4 : 7, 15-7.50 (m, 4H), 4.73 (d, J=15. 9Hz, 1H), 4.63 (d, J=15.9Hz, 1H), 4.25 (t, J=6.3Hz, 2H) , 3.25-3.30 (m, 2H), 2. 60-2.65 (m, 2H), 1. 55-1.75 (m, 8H), 0. 89 (t, J=7.8Hz, 3H) |
| V-5 : 7.25-7.50 (m, 4H), 5.12 (d, J=16.2Hz, 1H), 4.36 (d, J=16.2Hz, 1H), 3.30-3.55 (m, 2H), 2. 60-2.70 (m, 2H), 1. 65-1. 85 (m, 6H), 0.85-1. 55 (m, 5H) |
| V-8 : 7.15-7.60 (m, 9H), 5.12 (d, J=16.2Hz, 1H), 4.47 (d, J=16.2Hz, 1H), 3.30-3.35 (m, 2H), 2. 60-2.70 (m, 2H), 1. 65-1. 85 (m, 6H) |
| V-9 : 7.15-7.50 (m, 4H), 4.74 (d, J=15.6Hz, 1H), 4.60 (d, J=15.6Hz, 1H), 3.89 (s, 3H), 3.25-3. 30 (m, 2H), 2.60-2.70 (m, 2H), 1. 70-1. 75 (m, 6H) |
| V-12 : 7. 48 (t, J=8.0Hz, 1H), 7.30-7.40 (m, 2H), 7.19 (d, J=8.0Hz, 1H), 4.74 (d, J=15.7Hz, 1H) , 4.64 (d, J=15. 7Hz, 1H), 3.95-4.15 (m, 2H), 3.20-3.30 (m, 2H), 2.55-2.70 (m, 2H), 1.80-2.00 ( m, 1H), 1.65-1.80 (m, 4H), 1. 50-1. 60 (m, 2H), 0.86 (d, J=5. 0Hz, 6H) |
| V-13 : 7. 15-7. 50 (m, 4H), 5. 80-5. 95 (m, 1H), 5. 25-5. 35 (m, 2H), 4. 65-4. 75 (m, 4H), 3. 25-3. 30 (m, 2H) , 2. 60-2. 65 (m, 2H), 1. 55-1. 75 (m, 6H) |
| V-14 (mixed with V-28 in a ratio of 3:2):11.70 (brs, 1H), 7. 61 (d, J=8.1Hz, 1H), 7. 25-7. 40 (m, 2H), 7.1 5-7.25 (m, 1H), 4.40-4.55 (m, 2H), 3.40-3.55 (m, 1.2H), 3.25-3.40 (m, 0.8H), 2.40-2.60 (m, 2H |
| ), 1. 50-1. 90 (m, 3H), 1. 25-1. 45 (m, 2H), 0. 97 (d, J=6.3Hz, 1.8H), 0.92 (d, J=6.9Hz, 1. 2H) |
| V-15* : 7. 45-7. 50 (m, 1H), 7. 30-7. 45 (m, 2H) , 7. 19 (d, J=7.8Hz, 1H), 5. 20 (d, J=15. 9Hz, 0.6H) , 4.91 (d, J=15. 9Hz, 0.4H), 4.38 (d, J=16.2Hz, 0.4H), 4.05-4. 15 (m, 0.6H), 3.25-3.60 (m, 2H) , 2.15-2.65 (m, 4H), 1. 75-2.00 (m, 3H), 1. 50-1. 70 (m, 1H), 1.25-1.50 (m, 1H), 1.00-1.15 (m, 6H) |
| V-16* : 7. 25-7. 50 (m, 4H), 5. 26 (d, J=15. 9Hz, 0.6H), 4.94 (d, J=15.9Hz, 0.4H), 4. 52 (d, J=15. 9Hz, 0.4H), 4. 24 (d, J=16. 5Hz, 0.6H), 3. 40-3. 60 (m, 1H), 3. 25-3. 35 (m, 1H), 2. 45-2. 65 (m, 2H) , 1.75-1.95 (m, 3H), 1. 55-1. 65 (m, 1H), 1. 40-1. 55 (m, 1H), 1.25-1.45 (m, 2H), 1.10-1.20 (m, 1H ), 0.85-1.10 (m, 5H) |
| V-17 : 7. 40-7. 50 (m, 1H), 7. 30-7. 40 (m, 2H), 7. 19 (d, J=7.8Hz, 1H), 4. 91 (dd, J=16. 2, 26.4Hz, 1H), 4. 25-4. 50 (m, 3H), 3. 30-3. 40 (m, 1H), 3. 10-3. 20 (m, 1H), 2. 50-2. 65 (m, 2H), 1. 80-1. 95 (m , 2H), 1. 65-1. 80 (m, 1H), 1. 45-1. 65 (m, 1H), 1. 25-1. 35 (m, 4H), 1.04 (dd, J=2.7, 6.6Hz, 3H) |
| V-18 : 11.68 (brs, 1H), 7. 62 (d, J=7. 5Hz, 1H), 7. 30-7. 40 (m, 1H), 7. 25-7. 30 (m, 1H), 7. 10-7.2 0 (m, 1H), 4.57 (d, J=15.0Hz, 1H), 4.37 (d, J=15.0Hz, 1H), 3. 45-3. 55 (m, 2H), 2. 45-2. 65 (m, 2H) , 1.75-1.85 (m, 2H), 1. 65-1. 70 (m, 1H), 1. 00-1. 30 (m, 2H), 0.93 (d, J=6.3Hz, 3H) |
| V-21* : 7.45-7.55 (m, 1H), 7.35-7.45 (m, 1H), 7. 25-7. 35 (m, 2H), 5. 32 (d, J=16. 2Hz, 0.6H), 4. 84 (d, J=15. 6Hz, 0.4H), 4. 61 (d, J=15.6Hz, 0.4H), 4. 19 (d, J=16. 2Hz, 0.6H), 3. 45-3. 60 (m, 1H) , 3.20-3.35 (m, 1H), 2. 55-2. 75 (m, 2H), 1. 60-1. 90 (m, 3H), 1. 40-1. 55 (m, 1H), 1. 05-1. 40 (m, 4H ), 0.85-1. 05 (m, 5H) |
| V-22* : 7.40-7.50 (m, 1H), 7.30-7.40 (m, 2H), 7.19 (d, J=7.5Hz, 1H), 5.08 (d, J=16. 2Hz, 0.5H) , 4.93 (d, J=15. 6Hz, 0.5H), 4.40 (d, J=15. 3Hz, 0.5H), 4.27 (d, J=15.9Hz, 0.5H), 3. 90 (d, J=6. 6 Hz, 3H), 3.35-3.45 (m, 1H), 3.10-3.20 (m, 1H), 2.60-2.70 (m, 2H), 1.80-1.90 (m, 1H), 1.65-1.8 0 (m, 2H), 1. 55-1. 60 (m, 1H), 1. 10-1. 40 (m, 1H), 0.95-1. 00 (m, 3H) |
| V-24* : 7. 40-7. 50 (m, 1H), 7. 30-7. 40 (m, 2H), 7. 19 (d, J=7. 8Hz, 1H), 5.08 (d, J=16. 2Hz, 0.5H) , 4.95 (d, J=15. 6Hz, 0. 5H), 4. 41 (d, J=15. 3Hz, 0. 5H), 4.31 (d, J=15. 6Hz, 0. 5H), 4. 00-4. 15 (m, 2H), 3. 35-3. 45 (m, 1H), 3. 15-3. 25 (m, 1H), 2. 60-2. 70 (m, 2H), 1. 85-2. 00 (m, 2H), 1.55-1.80 (m , 2H), 1. 25-1. 35 (m, 1H), 1. 10-1. 20 (m, 1H), 0.90-1.00 (m, 3H), 0.80-0.90 (m, 6H) |
| V-25 : 7. 15-7. 55 (m, 4H), 5. 24 (d, J=16. 0Hz, 1H), 4. 07 (d, J=16. 0Hz, 1H), 3. 25-3. 65 (m, 2H), 1 . 85-2. 75 (m, 6H), 1. 20-1. 40 (m, 3H), 1. 10-1. 20 (m, 3H), 0. 89 (s, 9H) |
| V-26 : 7. 15-7.50 (m, 4H), 4.90-5.10 (m, 1H), 4.25-4. 45 (m, 3H), 3.15-3.45 (m, 2H), 2.50-2.75 (m, 2H), 1.80-2.10 (m, 2H), 1.10-1.40 (m, 6H), 0.86 (s, 9H) |
| V-27 : 7. 10-7. 50 (m, 9H), 4.11 (t, J=15. 0Hz, 1H), 4. 25-4. 50 (m, 3H), 3. 20-3. 65 (m, 2H), 2. 75-2.90 (m, 3H), 1.55-2.15 (m, 4H), 1. 25-1. 40 (m, 3H) |
| V-28 :shown together with V-14 |
| V-29* : 7. 45-7. 50 (m, 1H), 7. 30-7.40 (m, 2H), 7. 15-7. 20 (m, 1H), 5. 21 (d, J=16.2Hz, 0.6H), 4. 97 (d, J=16. 2Hz, 0.4H), 4. 33 (d, J=15. 9Hz, 0.4H), 4. 10 (d, J=16.2Hz, 0.6H), 3. 20-3. 40 (m, 1. 5 H), 3. 06 (d, J=15. 6Hz, 0.5H), 2. 15-2. 50 (m, 4H), 1.80-2. 00 (m, 2H), 1. 55-1. 80 (m, 2H), 1. 20-1 .45 (m, 1H), 1. 05-1. 15 (m, 3H), 0.90 (dd, J=1.8, 6. 9Hz, 3H) |
| V-30* : 7.45-7.50 m, 1H), 7. 25-7.40 (m, 3H), 5.28 (d, J=16.2Hz, 0.6H), 5.00 (d, J=15. 9Hz, 0. 4H), 4.46 (d, J=15. 9Hz, 0.4H), 4.22 (d, J=16. 5Hz, 0.6H), 3. 00-3. 40 (m, 2H), 2.55-2.70 (m, 2H) , 1.85-2.00 (m, 2H), 1. 55-1. 80 (m, 2H), 1. 40-1. 55 (m, 1H), 1. 25-1. 40 (m, 2H), 1. 05-1. 20 (m, 1H ), 0.95-1. 10 (m, 1H), 0.80-0.95 (m, 4H) |
| V-31* : 7. 40-7. 50 (m, 1H), 7. 30-7. 40 (m, 2H), 7. 19 (d, J=7.8Hz, 1H), 5. 02 (d, J=15. 6Hz, 0.5H) , 4.77 (d, J=15. 3Hz, 0.5H), 4. 57 (d, J=15. 3Hz, 0.5H), 4. 30-4. 40 (m, 2.5H), 3.15-3. 25 (m, 1H), 3. 01 (t, J=15. 3Hz, 1H), 2. 55-2. 70 (m, 2H), 1. 85-1. 95 (m, 2H), 1. 55-1. 70 (m, 2H), 1. 20-1. 35 (m , 4H), 0.85 (dd, J=2.7, 6.9Hz, 3H) |
| V-37 : 7. 15-7. 55 (m, 4H), 5. 19 (d, J=15.0Hz, 1H), 4. 22 (d, J=15.0Hz, 1H), 3. 41 (d, J=15.0Hz, 1 H), 2. 99 (d, J=12.0Hz, 1H), 2. 71 (d, J=12.0Hz, 1H), 2. 20-2. 45 (m, 3H), 1. 35-1. 50 (m, 2H), 1. 10 |
| -1. 15 (m, 9H), 1.03 (s, 3H), 0.91 (s, 3H) |
| V-38 : 7.15-7.50 (m, 4H), 4.91 (d, J=15.0Hz, 1H), 4. 57 (d, J=15.0Hz, 1H), 4.30-4.45 (m, 2H), 3 .12 (d, J=15.0Hz, 1H), 2. 98 (d, J=15.0Hz, 1H), 2. 63 (d, J=9.0Hz, 1H), 2. 52 (d, J=9.0Hz, 1H), 1. 40 (s, 2H), 1.31 (t, J=7.2Hz, 3H), 1. 09 (s, 3H), 1. 06 (s, 3H), 1.01 (s, 3H), 0. 93 (s, 3H) |
| V-39 : 11.61 (brs, 1H), 7. 50-7. 65 (m, 1H), 7.10-7.50 (m, 3H), 4. 56 (d, J=14.8Hz, 1H), 4. 43 (d, J=14.8Hz, 1H), 3.35-3.65 (m, 2H), 2.50-2.70 (m, 1H), 1. 70-1. 90 (m, 2H), 1. 45-1. 70 (m, 3H), 1. 25-1. 40 (m, 1H), 1. 15 (d, J=6.9Hz, 3H) |
| VI-2 : 7. 30-7. 55 (m, 3H), 7. 15-7. 25 (m, 1H), 4. 84 (d, J=15. 9Hz, 1H), 4. 57 (d, J=15. 9Hz, 1H), 4 .25-4.45 (m, 4H), 3.75-3.90 (m, 2H), 3.21 (t, J=6.0Hz, 2H), 1.98 (t, J=6.0Hz, 2H), 1. 36 (t, J=6 .9Hz, 3H), 1. 30 (t, J=6.9Hz, 3H) |
| VI-3 : 7. 61 (d, J=7.8Hz, 1H), 7. 35-7. 45 (m, 1H), 7. 20-7. 25 (m, 2H), 4. 55 (s, 2H), 3. 90 (t, J=6. 0Hz, 2H), 3.30 (t, J=6.0Hz, 2H), 2.13 (quin, J=6.0Hz, 2H) |
| VI-5 : 7. 60-7. 65 (m, 1H), 7.40-7.50 (m, 1H), 7. 35-7. 40 (m, 2H), 4. 82 (s, 2H), 4. 00 (t, J=6.0Hz , 2H), 3.52 (t, J=6.0Hz, 2H), 2.13 (quin, J=6.0Hz, 2H) |
| VII-1 : 11. 31 (brs, 1H), 7. 55-7. 60 (m, 1H), 7. 30-7. 40 (m, 1H), 7. 15-7. 25 (m, 2H), 4.56 (d, J=1 5. 0Hz, 1H), 3. 96 (d, J=15. 0Hz, 1H), 3. 64 (dd, J=5.7, 10.2Hz, 1H), 3. 40-3. 45 (m, 1H), 1. 85-2. 00 (m, 2H) 1.10-1.20 (m, 1H), 0.60-0. 65 (m, 1H) |
| VII-2 : 7.45-7.55 (m, 1H), 7.35-7.45 (m, 1H), 7.15-7.25 (m, 2H), 4.74 (dd, J=8.4, 15.9Hz, 1H) , 4.10 (dd, J=15. 9, 20. 7Hz, 1H), 3. 45-3. 55 (m, 1H), 3. 20-3. 30 (m, 1H), 2. 25-2. 45 (m, 2H), 1. 95 -2.05 (m, 1H), 1. 85-1. 95 (m, 1H), 1.15-1. 20 (m, 1H), 1. 11 (dt, J=2. 7, 7.5Hz, 3H), 0.65-0.75 (m , 1H) |
| VII-3 : 7. 45-7. 55 (m, 1H), 7. 35-7.45 (m, 2H), 7. 25-7. 30 (m, 1H), 4. 76 (dd, J=10. 5, 15.9Hz, 1H ), 4.10-4.30 (m, 1H), 3.45-3.55 (m, 1H), 3.20-3.30 (m, 1H), 1.95-2.05 (m, 1H), 1. 85-1. 95 (m, 1 H), 1. 40-1. 50 (m, 1H), 1. 25-1. 30 (m, 1H), 1. 15-1.20 (m, 2H), 0.85-1.05 (m, 2H), 0. 65-0. 75 (m, 1H) |
| VII-4 : 7. 40-7. 50 (m, 1H) , 7. 25-7. 40 (m, 2H), 7. 18 (d, J=7. 8Hz, 1H), 4.20-4.60 (m, 4H), 3.35-3. 45 (m, 1H), 3.16 (d, J=10.8Hz, 1H), 1. 95-2. 05 (m, 1H), 1. 80-1. 90 (m, 1H), 1. 32 (dt, J=2.4, 7. 2Hz, 3H), 1. 10-1. 20 (m, 1H), 0.60-0.70 (m, 1H) |
| VII-5 : 7. 40-7. 50 (m, 1H), 7. 30-7. 40 (m, 2H), 7. 18 (t, J=7.5Hz, 1H), 4. 50-4. 60 (m, 1H), 4.20-4. 35 (m, 1H), 4. 00-4. 15 (m, 2H), 3. 35-3. 45 (m, 1H), 3. 15-3. 20 (m, 1H), 1. 80-2. 05 (m, 3H), 1.10 -1. 20 (m, 1H), 0.87 (dd, J=1. 8, 6.6Hz, 6H), 0. 60-0. 75 (m, 1H) |
| VII-6(mixed with V-28 in a ratio of 1:2):11. 79 (brs, 1H), 7.63 (d, J=8. 7Hz, 1H), 7. 30-7. 40 (m, 1.5H ), 7.15-7.25 (m, 1.5H), 4. 68 (d, J=14. 4Hz, 0.7H), 4. 30-4. 45 (m, 0.3H), 4.19 (d, J=14. 4Hz, 0.7 H), 3. 85-3.90 (m, 0.7H), 3. 45-3.55 (m, 0.3H), 3.13 (d, J=11.4Hz, 0.3H), 2. 80-2. 85 (m, 0.3H), 2. 50-2. 65 (m, 2H), 2. 35 (s, 0.4H), 2. 29 (s, 0.3H), 1.80-2. 00 (m, 3H), 1.70-1. 75 (m, 0.7H), 1. 4 0-1. 70 (m, 2. 3H) |
| VII-7 : 7. 45-7. 55 (m, 1H), 7.35-7.40 (m, 1H), 7. 20-7. 30 (m, 2H), 5. 06 (t, J=15. 9Hz, 1H), 4.01 (dd, J=16. 2, 27. 9Hz, 1H), 3. 35-3. 45 (m, 1H), 2. 95-3. 05 (m, 1H), 2. 85 (s, 1H), 2. 56 (s, 1H), 2.2 0-2.45 (m, 2H), 1.90-2.10 (m, 4H), 1.60-1.75 (m, 2H), 1. 10 (t, J=7. 2Hz, 3H) |
| VII-8* : 7. 45 (t, J=7.8Hz, 1H), 7. 34 (t, J=7.5Hz, 1H), 7. 15-7. 25 (m, 2H), 4. 86 (d, J=15. 9Hz, 0 .5H), 4.68 (d, J=15.9Hz, 0.5H), 4.25-4.50 (m, 3H), 3.23 (dd, J=4. 2, 11.4Hz, 1H), 2.80-2.90 (m , 2H), 2.48 (s, 1H), 1.90-2.10 (m, 4H), 1. 50-1.70 (m, 2H), 1. 25-1. 35 (m, 3H) |
| VIII-1 : shown together with VII-6 |
| VIII-2* : 7. 45-7. 50 (m, 1H), 7.35-7.40 (m, 2H), 7.21 (d, J=8.1Hz, 1H), 5.29 (t, J=15. 6Hz, 1H) , 4.25-4.45 (m, 2H), 3. 96 (dd, J=7.2, 15.9Hz, 1H), 3. 50-3. 60 (m, 1H), 2. 65-2. 75 (m, 1H), 2.50-2. 60 (m, 1H), 2. 30-2. 40 (m, 1H), 1. 50-2. 05 (m, 6H), 1. 31 (dt, J=7.2, 10.5Hz, 3H) |
| IX-1 : 7.30-7.45 (m, 3H), 7.20 (d, J=8.1Hz, 1H), 4. 72 (d, J=15. 6Hz, 1H), 4. 63 (d, J=15. 6Hz, 1H |
| ), 4.60 (t,J=9.3Hz, 2H), 4.30-4. 45 (m,2H), 3. 36 (t,J=7.2Hz, 2H), 2.90-3.00 (m,2H), 2. 40-2. 50 (m, 2H) , 1. 32 (t, J=7. 2, 3H) |
| X-1 : 7. 56 (d, J=8. 7Hz, 1H) , 7. 34 (d, J=8. 7Hz, 1H), 7. 25 (s, 1H), 4. 44 (s, 2H) , 3. 46(t, J=5. 4Hz , 2H) , 2. 44 (t, J=6. 6Hz, 2H) , 1. 70-1. 90 (m, 4H) |
| X-2 : 11. 75 (brs, 1H), 7. 50-7. 65 (m, 1H), 6.90-7. 15(m,2H), 4.50 (d, J=14.3Hz, 1H), 4.38(d,J =14. 3Hz, 1H), 3. 35-3. 60 (m, 2H) , 2. 45-2. 60 (m, 1H), 1. 80-2. 10 (m, 3H) , 1. 35-1. 55 (m, 1H), 1. 00 (d, J=6. 3Hz, 3H) |
| X-3 : 7. 15-7. 25(m, 1H), 6.95-7. 10(m, 2H), 4.75-4. 90 (m, 1H), 4. 25-4. 60 (m, 3H), 3. 15-3. 30( m, 2H) , 2. 50-2. 70 (m, 1H), 1. 90-2. 20 (m, 2H) , 1. 70-1. 90 (m, 1H), 1. 40-1. 55 (m, 1H), 1. 20-1. 40 (m, 3H) , 1.04(d, J=6.5Hz, 3H) |
| X-4 : 11. 13 (brs, 1H), 7. 60-7. 70 (m, 1H), 7. 20-7. 40 (m, 2H) , 7.10-7. 20 (m, 1H), 6. 28 (d, JHF=5 3. 7Hz, 1H), 4.52(s, 2H), 3.40-3.55(m, 2H), 2.50-2.65(m, 2H), 1.45-1.80(m, 6H) |
| X-5 : 9. 78 (brs, 1H), 7. 15-7. 40 (m, 3H) , 7. 02 (t, J=8. 2Hz, 1H), 4. 25-4. 90 (m, 2H) , 3. 25-3. 45 ( m, 2H) , 2. 45-2. 60 (m, 1H), 1. 75-2. 05 (m, 3H) , 1. 30-1. 50 (m, 1H), 0. 98 (d, J=6. 3Hz, 3H) |
| X-6 : 11. 84 (brs, 1H), 7. 73 (d, J=8. 0Hz, 1H), 7. 38 (t, J=8. 0Hz, 1H), 7. 25 (d, J=7. 7Hz, 1H), 7. 1 5 (t, J=7. 7Hz, 1H), 4. 61 (d, J=14. 3Hz, 1H), 4. 45 (d, J=14. 3Hz, 1H), 3. 35-3. 55 (m, 2H) , 2. 45-2. 60 (m, 1H), 1. 80-2.05 (m, 3H), 1. 35-1. 50 (m, 1H), 0. 99 (d, J=6. 3Hz, 3H) |
| X-7* : 7.40-7.50 (m, 1H) , 7.25-7.40(m, 3H) , 4.96(d, J=15.0Hz, 0.5H) , 4.79(d, J=15.0Hz, 0. 5 H) , 4.62(d, J=15.0Hz, 0. 5H) , 4.47(d, J=15.0Hz, 0. 5H), 4.20-4.40(m,2H), 3. 10-3. 25(m, 2H) , 2. 50-2. 70 (m, 1H), 1. 90-2. 20 (m, 2H) , 1. 70-1. 90 (m, 1H), 1. 35-1. 55 (m, 1H), 1. 20-1. 35 (m, 3H) , 1.03(d, J=6.3Hz, 3H) |
| XI-2 : 7. 12 (t, J=8. 0Hz, 1H), 7. 02 (d, J=8. 0Hz, 1H), 6. 55-6. 70 (m, 2H) , 4. 68 (brs, 2H) , 4. 63 (d , J=15. 6Hz, 1H), 4. 36 (d, J=15. 6Hz, 1H), 3. 05-3. 30 (m, 2H) , 2. 40-2. 60 (m, 1H), 1. 70-2. 05 (m, 3 H) , 1. 25-1. 45 (m, 1H), 0. 95 (d, J=8. 0Hz, 3H) |
| XI-3 : 7. 10 (t, J=7. 8Hz, 1H), 7. 02 (d, J=7. 8Hz, 1H), 6. 55-6. 70 (m, 2H) , 4. 63 (brs, 2H) , 4. 61 (d , J=14. 1Hz, 1H), 4. 38 (d, J=14. 1Hz, 1H), 3. 05-3. 35 (m, 2H) , 2. 45-2. 60 (m, 1H), 1. 70-2. 05 (m, 3 H) , 1. 20-1.45(m, 5H), 0. 89 (t, J=6. 6Hz, 3H) |
| XI-4 : 6. 95-7. 15(m,2H),6. 55-6. 70(m, 2H) , 4. 65 (brs, 2H), 4. 62 (d, J=14. 4Hz, 1H), 4. 38 (d, J =14. 4Hz, 1H), 3. 05-3. 40 (m, 2H) , 2. 40-2. 60 (m, 1H), 2. 00-2. 20 (m, 1H), 1. 70-1. 95 (m, 1H), 1. 2 0-1.65 (m, 3H), 0.88(d, J=6. 6Hz, 6H) |
| XI-5 : 6. 95-7. 20 (m, 2H) , 6. 55-6. 75 (m, 2H), 4. 60 (brs, 2H), 4. 58 (d, J=14. 4Hz, 1H), 4.41 (d, J =14. 7Hz, 1H) , 3. 05-3. 45 (m, 2H) , 2. 45-2. 70 (m, 1H), 2. 10-2. 35 (m, 1H), 1. 80-2. 00 (m, 1H), 1. 4 0-1. 80 (m, 1H), 0. 95-1. 15 (m, 1H), 0. 35-0. 70 (m, 3H) , 0. 00-0. 25 (m, 2H) |
| XI-8 : 7.00-7.40 (m, 7H), 6.60-6. 70 (m, 2H) , 4. 67 (brs, 2H), 4.66 (d, J=14. 4Hz, 1H) , 4. 47 (d, J =14. 4Hz, 1H), 3. 20-3. 45 (m, 2H) , 3.00-3.15 (m, 1H), 2. 70-2. 95 (m, 1H), 2. 45-2. 60 (m, 1H), 2. 0 0-2. 15 (m, 1H), 1. 80-2. 00 (m, 1H) |
| XI-10 : 7. 11 (t, J=7. 8Hz, 1H), 7. 03 (d, J=7. 8Hz, 1H) , 6. 55-6. 70 (m, 2H) , 4. 53 (s, 2H) , 3. 21 (t, J=6.6Hz, 2H) , 2.20 (s, 2H) , 1.56 (t, J=6. 6Hz, 2H) , 0.97 (s, 6H) |
| XI-11 : 8. 03 (d, J=8. 0Hz, 1H), 7. 60 (t, J=8. 0Hz, 1H), 7. 30-7. 50 (m, 2H) , 5. 09 (d, J=16. 8Hz, 1H ) , 4.80 (d, J=16. 8Hz, 1H), 3. 10-3. 20 (m, 1H), 2. 90-3. 00 (m, 1H), 2. 33 (d, J=6. 2Hz, 2H) , 1. 75-1 . 90 (m, 1H), 1. 00 (s, 3H) , 0. 85-0. 95 (m, 6H) |
| XI-12 : 7. 10 (t, J=8. 0Hz, 1H), 7. 02 (d, J=8. 0Hz 1H), 6. 55-6. 70 (m, 2H) , 4. 62 (d, J=14. 4Hz, 1H ) , 4.60 (brs, 2H) , 4. 37 (d, J=14. 4Hz, 1H), 3. 10-3. 20 (m, 1H), 2. 70-2. 85 (m, 1H), 2. 22 (d, J=6. 9 Hz, 2H) , 1. 60-1. 80 (m, 1H), 0.75-1. 05 (m, 9H) |
| XI-13 : 7. 95-8. 05 (m, 1H), 7. 55-7. 65 (m, 1H), 7. 30-7. 45 (m, 2H) , 5. 00 (d, J=16. 8Hz, 1H) , 4. 83 (d, J=16. 8Hz, 1H) , 3. 15-3. 35 (m, 2H) , 2. 26 (q, J=7. 2Hz, 1H), 1. 65-1. 75 (m, 2H) , 1. 23 (d, J=7. 2 Hz, 3H) , 1.06 (s, 3H) , 0.94 (s, 3H) |
| XI-14 : 7. 05-7. 15 (m, 1H) , 6. 95-7. 05 (m, 1H) , 6. 55-6. 65 (m, 2H) , 4. 59 (d, J=14. 4Hz, 1H), 4. 56 (brs, 2H) , 4. 42 (d, J=14. 4Hz, 1H) , 3. 15-3. 20 (m, 2H) , 2.16 (q, J=7. 2Hz, 1H) , 1. 50-1. 70 (m, 2H) , 1. 18 (d, J=7. 2Hz, 3H), 0. 97 (s, 3H), 0. 86 (s, 3H) |
| XI-15 : 8. 03 (d, J=8. 4Hz, 1H) , 7. 60 (t, J=7. 2Hz, 1H) , 7. 43 (t, J=7. 5Hz, 1H) , 7. 34 (d, J=8. 1Hz, 1H), 4. 96 (s, 2H) , 3. 30 (t, J=6. 0Hz, 2H), 2. 41 (s, 2H) , 1. 65-1. 80 (m, 6H) , 1. 50-1. 55 (m, 4H) |
| XI-16 : 7. 11 (dt, J=0.9, 7. 2Hz, 1H) , 7.03 (dd, J=1. 5, 7. 8Hz, 1H), 6.60-6.65 (m, 2H), 4.56 (brs ,2H), 4.52(s,2H), 3.21(t,J=6.3Hz, 2H), 2.30(s, 2H), 1.55-1.70(m, 6H), 1.40-1.45(m, 4H) |
| XI-17 : 7. 99(d, J=8. 0Hz, 1H) , 7. 57 (t, J=8. 0Hz, 1H) , 7. 35-7. 50 (m,2H), 5. 18 (d, J=16. 7Hz, 1H ), 4.79(d, J=16. 7Hz, 1H), 4.40-4. 50 (m, 1H), 3.27 (s, 3H), 2.30-2.65(m, 2H), 2.00-2. 20 (m, 2H ), 1. 65-1. 80 (m, 2H) |
| XI-18 : 7.11 (t, J=7. 8Hz, 1H), 7. 03 (d, J=7. 8Hz, 1H), 6. 60-6.70(m, 2H), 5.33(d, J=14. 6Hz, 1H ), 4.52 (brs, 2H) , 4. 40-4. 45 (m, 1H), 3. 95 (d, J=14. 6Hz, 1H), 3. 35 (s, 3H) , 2. 45-2. 60 (m, 1H), 2 . 25-2. 40 (m, 1H), 1. 85-2. 10 (m, 2H) , 1. 45-1. 70 (m, 2H) |
| XII-1 : 8. 00-8. 05 (m, 1H), 7. 55-7. 65 (m, 1H), 7. 30-7. 50 (m, 2H) , 4. 93 (s, 2H), 2. 99 (s, 2H) , 2. 54 (t, J=6. 9Hz, 2H) , 1. 68 (t, J=6. 9Hz, 2H) , 1. 04 (s, 6H) |
| XII-2 : 7.10 (dt, J=1.8, 7. 5Hz, 1H), 7. 00 (dd, J=1. 8, 7. 5Hz, 1H), 6. 60-6. 65 (m, 2H) , 4. 58 (brs , 2H), 4. 50 (s, 2H) , 2. 91 (s, 2H) , 2. 44 (t, J=7. 5Hz, 2H) , 1. 56 (t, J=7. 5Hz, 2H) , 0. 93 (s, 6H) |
| XII-3 : 7.95-8.05 (m, 1H), 7. 55-7. 65 (m, 1H), 7. 35-7. 45 (m, 1H), 7. 25-7. 30 (m, 1H), 4. 89 (s, 2 H), 3.28 (t, J=6. 0Hz, 2H), 1. 80-1. 95 (m, 2H) , 1. 75-1. 80 (m, 2H) , 1. 29 (s, 6H) |
| XII -4 : 7.05-7. 15 (m, 1H), 6. 95-7. 05 (m, 1H), 6. 55-6. 65 (m, 2H) , 4. 61 (brs, 2H) , 4. 49 (s, 2H) , 3. 21 (t, J=6. 0Hz, 2H), 1. 70-1. 85 (m, 2H) , 1. 60-1. 70 (m, 2H) , 1. 23 (s, 6H) |

The proton nuclear magnetic resonance chemical shifts in Table 24 were measured by using Me4Si (tetramethylsilane) as the standard at 300 MHz.

Diastereomer mixtures are marked with *.

Now, the usefulness of the compounds of the present invention as herbicides will be described in detail with reference to the following Test Examples.

### [TEST EXAMPLE 1] Test on the herbicide effects in preemergence treatment on weeds under submerged conditions

Alluvial soil was put in 1/30000-are styrol cups and admixed with water so that it was submerged at a depth of 4 cm. In each cup, seeds of barnyeardgrass, bulrush and ducksalad were sown together, and rice seedlings at the 2.5-leaf stage were transplanted. On the same day as the seeding, emulsifiable concentrates containing compounds of the present invention formulated in accordance with Formulation Example 2 were applied to the water surfaces at predetermined doses after diluted with water. The cups were placed in a green house at 25 to 30°C to grow the plants. Three weeks after the application, the herbicidal effects against various plants were determined on the following evaluation scale. The results are shown in Tables 25 and 26.

### Evaluation scale

5 mortality of 90% or above (nearly complete death)
4 mortality of at least 70% and less than 90%
3 mortality of at least 40% and less than 70%
2 mortality of at least 20% and less than 40%
1 mortality of at least 5% and less than 20%
0 mortality of less than 5% (almost ineffective)

### [TEST EXAMPLE 2] Test on the herbicidal effects on weeds during the vegeration period under submerged conditions

Alluvial soil was put in 1/30000-are styrol cups and admixed with water so that it was submerged at a depth of 4 cm. In each cup, seeds of barnyeardgrass, bulrush and ducksalad were sown together, and the cups were placed in a greenhouse at 25 to 30°C to grow the plants. When the barnyardgrass, bulrush and ducksalad reached the 1- or 2-leaf stage, emulsifiable concentrates containing compounds of the present invention formulated in accordance with Formulation Example 2 were applied to the water surfaces at predetermined doses after diluted with water. Three weeks after the application, the herbicidal effects against various plants were determined on the same evaluation scale as in Test Example 1. The results are shown in Table 27.

### [TEST EXAMPLE 3] Test on the herbicidal effects in soil treatment

Sterilized diluvial soil was put in plastic boxes with a length of 21 cm, a width of 13 cm and a depth of 7 cm, and seeds of barnyardgrass, large crabgrass, giant foxtail, wild oat, blackgrass, velvetleaf, common ragweed, redroot pigweed, common lambsquater, posumbu knotweed, common chickweed corn, soybean, rice, wheat and sugar beet were sown in spots and covered with an about 1.5 cm-thick soil layer. Emulsifiable concentrates containing compounds of the present invention formulated in accordance with Formulation Example 2 were uniformly applied to the soil surfaces at a predetermined doses through a small sprayer after dilute with water. Three weeks after the application, the herbicidal effects against various plants were determined on the same evaluation scale as in Test Example 1. The results are shown in Table 28.

### [TEST EXAMPLE 4] Test on the herbicidal effect in foliage treatment

Sterilized diluvial soil was put in plastic boxes with a length of 21 cm, a width of 13 cm and a depth of 7 cm, and seeds of barnyardgrass, large crabgrass, giant foxtail, wild oat, blackgrass, velvetleaf, common ragweed, redroot pigweed, common lambsquater, posumbu knotweed, common chickweed, corn, soybean, rice, wheat and sugar beet were sown in spots and covered with an about 1.5 cm-thick soil layer. The boxes were placed in a greenhouse at 25 to 30°C to grow the plants. After 14 days, emulsifiable concentrates containing compounds of the present invention formulated in accordance with Formulation Example 2 were uniformly applied to the foliage at predetermined doses through a small sprayer after diluted with water. Three weeks after the application, the herbicidal effects against various plants were determined on the same evaluation scale as in Test Example 1. The results are shown in Table 29.

The symbols in Tables 25 to 29 have the following meanings.

A: barnyardgrass, B: bulrush, C: ducksalad, D: large crabgrass, E: giant foxtail, F: wild oat, G: water foxtail, H: Italian ryegrass, I: velvetleaf, J: common ragweed, K: redroot pigweed, L: common lambsquater, M: posumbu knotweed, N: common chickweed, O: beadstraw, a: transplanted rice, b: corn, c: soybean, d: direct-seeded rice, e: wheat, f: sugar beet.

The doses (g/a) represents the amounts in g per 1 are (a) applied after adjustment of concentration adjustment.

**TABLE 25**

| Compound No. | Dose (g/a) | A | B | C |
|---|---|---|---|---|
| I-1 | 2. 52 | 5 | 5 | 5 |
| I-2 | 2. 52 | 5 | 5 | 5 |
| I-3 | 2. 52 | 5 | 5 | 5 |
| I-4 | 2. 52 | 5 | 5 | 5 |
| I-5 | 2. 52 | 5 | 5 | 5 |
| II-1 | 2. 52 | 5 | 5 | 5 |
| II-2 | 2. 52 | 5 | 5 | 5 |
| II-3 | 2. 52 | 5 | 5 | 5 |
| III-1 | 2. 52 | 5 | 5 | 5 |
| III-2 | 2. 52 | 5 | 5 | 5 |
| III-3 | 2. 52 | 5 | 5 | 5 |
| III-4 | 3. 00 | 5 | 5 | 5 |
| III-5 | 2. 52 | 5 | 5 | 5 |
| III-6 | 2. 52 | 5 | 5 | 5 |
| III-7 | 1. 52 | 5 | 5 | 5 |
| III-8 | 2. 52 | 5 | 5 | 5 |
| III-9 | 2. 52 | 5 | 5 | 5 |
| III-10 | 2. 52 | 5 | 5 | 5 |
| III-11 | 2. 52 | 5 | 5 | 5 |
| III-12 | 2. 52 | 5 | 5 | 5 |
| III-13 | 2. 52 | 5 | 5 | 5 |
| III-14 | 2. 52 | 5 | 5 | 0 |
| III-15 | 2. 52 | 5 | 5 | 3 |
| III-16 | 2. 52 | 5 | 5 | 5 |
| III-18 | 2. 52 | 5 | 5 | 5 |
| III-19 | 2. 52 | 5 | 5 | 5 |
| III-20 | 2. 52 | 5 | 5 | 5 |
| III-21 | 2. 52 | 5 | 5 | 5 |
| III-22 | 2. 52 | 5 | 5 | 5 |
| III-23 | 2. 52 | 5 | 5 | 5 |
| III-25 | 2. 52 | 5 | 5 | 5 |
| III-26 | 2. 52 | 5 | 5 | 5 |
| III-27 | 2. 52 | 5 | 5 | 5 |
| III-28 | 2. 52 | 5 | 5 | 5 |
| III-29 | 2. 52 | 5 | 5 | 5 |
| III-30 | 2. 52 | 5 | 5 | 5 |
| III-31 | 2. 52 | 5 | 5 | 5 |
| III-32 | 2. 52 | 5 | 5 | 5 |
| III-33 | 2. 52 | 5 | 5 | 5 |
| III-34 | 2. 52 | 5 | 5 | 5 |
| III-35 | 2. 52 | 5 | 5 | 5 |
| III-36 | 2. 52 | 5 | 5 | 5 |
| III-37 | 2. 52 | 5 | 5 | 5 |
| III-38 | 2. 52 | 5 | 5 | 5 |
| III-39 | 2. 52 | 5 | 5 | 5 |
| III-40 | 2. 52 | 5 | 5 | 5 |
| III-41 | 2. 52 | 5 | 5 | 5 |
| III-42 | 2. 52 | 5 | 5 | 5 |
| III-43 | 2. 52 | 5 | 5 | 5 |
| III-44 | 2. 52 | 5 | 5 | 5 |
| III-45 | 2. 52 | 5 | 5 | 5 |
| III-46 | 2. 52 | 5 | 5 | 5 |
| III-47 | 2. 52 | 5 | 5 | 5 |
| III-48 | 2. 52 | 5 | 5 | 5 |
| III-49 | 2. 52 | 5 | 5 | 5 |
| III-50 | 2. 52 | 5 | 5 | 5 |
| III-51 | 2. 52 | 5 | 5 | 5 |
| III-52 | 2. 52 | 5 | 5 | 5 |
| III-53 | 2. 52 | 5 | 5 | 5 |
| III-54 | 2. 52 | 5 | 5 | 5 |
| III-55 | 2. 52 | 5 | 5 | 5 |
| III-56 | 2. 52 | 5 | 5 | 5 |
| III-57 | 2. 52 | 5 | 5 | 5 |
| III-58 | 2. 52 | 5 | 5 | 5 |
| III-59 | 2. 52 | 5 | 5 | 5 |
| III-60 | 2. 52 | 5 | 5 | 5 |
| III-61 | 2. 52 | 5 | 5 | 5 |
| III-62 | 2. 52 | 5 | 5 | 5 |
| III-63 | 2. 52 | 5 | 5 | 5 |
| III-64 | 2. 52 | 5 | 5 | 5 |
| III-65 | 2. 52 | 5 | 5 | 5 |
| III-66 | 2. 52 | 5 | 5 | 5 |
| III-67 | 2. 52 | 5 | 5 | 5 |
| III-68 | 2. 52 | 5 | 5 | 5 |
| III-69 | 2. 52 | 5 | 5 | 5 |
| III-70 | 2. 52 | 3 | 5 | 5 |
| III-71 | 2. 52 | 5 | 5 | 5 |
| III-72 | 2. 52 | 5 | 5 | 5 |
| III-73 | 2. 52 | 5 | 5 | 5 |
| III-74 | 2. 52 | 5 | 5 | 5 |
| III-75 | 2. 52 | 5 | 5 | 5 |
| III-76 | 2. 52 | 5 | 5 | 5 |
| III-77 | 2. 52 | 5 | 5 | 5 |
| III-78 | 2. 52 | 5 | 5 | 5 |
| III-79 | 1. 04 | 5 | 5 | 5 |
| III-80 | 2. 52 | 5 | 5 | 5 |
| III-81 | 2. 52 | 5 | 5 | 5 |
| III-82 | 2. 52 | 5 | 5 | 5 |
| III-83 | 6. 30 | 5 | 5 | 5 |
| III-84 | 2. 52 | 5 | 5 | 5 |
| III-85 | 2. 52 | 5 | 5 | 5 |
| III-86 | 2. 52 | 5 | 5 | 5 |
| III-87 | 2. 52 | 5 | 5 | 5 |
| III-88 | 2. 52 | 5 | 5 | 5 |
| III-89 | 2. 52 | 5 | 5 | 5 |
| III-90 | 1. 28 | 5 | 5 | 5 |
| III-91 | 2. 52 | 5 | 5 | 5 |
| III-92 | 2. 52 | 2 | 5 | 3 |
| III-93 | 2. 52 | 0 | 5 | 2 |
| III-94 | 2. 52 | 5 | 5 | 5 |
| III-95 | 2. 52 | 5 | 5 | 5 |
| III-96 | 2. 52 | 5 | 5 | 5 |
| III-97 | 2. 52 | 5 | 5 | 5 |
| III-98 | 2. 52 | 5 | 5 | 5 |
| III-99 | 2. 17 | 5 | 5 | 5 |
| III-100 | 2. 52 | 5 | 5 | 5 |
| III-101 | 2. 52 | 5 | 5 | 5 |
| III-102 | 2. 52 | 5 | 5 | 5 |
| III-103 | 2. 52 | 5 | 5 | 5 |
| III-104 | 2. 52 | 5 | 5 | 5 |
| III-105 | 2. 52 | 5 | 5 | 5 |
| III-106 | 2. 52 | 5 | 5 | 5 |
| III-107 | 2. 52 | 5 | 5 | 5 |
| III-108 | 2. 52 | 5 | 5 | 5 |
| III-109 | 2. 52 | 5 | 5 | 5 |
| III-110 | 2. 52 | 5 | 5 | 5 |
| III-111 | 2. 52 | 5 | 5 | 5 |
| III-112 | 2. 52 | 5 | 5 | 5 |
| III-113 | 2. 52 | 5 | 5 | 5 |
| III-114 | 0. 64 | 5 | 5 | 5 |
| IV-1 | 2. 52 | 5 | 5 | 5 |
| IV-2 | 2. 52 | 5 | 5 | 5 |
| IV-3 | 2. 52 | 5 | 5 | 5 |
| IV-4 | 2. 52 | 5 | 5 | 5 |
| IV-5 | 2. 52 | 5 | 5 | 5 |
| IV-6 | 2. 52 | 5 | 5 | 5 |
| IV-7 | 2. 52 | 5 | 5 | 5 |
| IV-8 | 2. 52 | 5 | 5 | 5 |
| IV-9 | 2. 52 | 5 | 5 | 5 |
| IV-10 | 2. 52 | 5 | 5 | 5 |
| IV-11 | 2. 52 | 5 | 5 | 5 |
| IV-12 | 2. 52 | 5 | 5 | 5 |
| IV-13 | 2. 52 | 5 | 5 | 5 |
| IV-14 | 1. 79 | 5 | 5 | 5 |
| IV-15 | 2. 52 | 5 | 5 | 5 |
| IV-16 | 2. 52 | 5 | 5 | 5 |
| IV-17 | 2. 52 | 5 | 5 | 5 |
| IV-18 | 2. 52 | 5 | 5 | 5 |
| IV-19 | 2. 52 | 5 | 5 | 5 |
| IV-20 | 2. 52 | 5 | 5 | 5 |
| IV-21 | 2. 52 | 5 | 5 | 5 |
| IV-23 | 2. 52 | 5 | 5 | 5 |
| IV-24 | 2. 52 | 5 | 5 | 5 |
| IV-25 | 2. 52 | 5 | 5 | 5 |
| IV-26 | 2. 52 | 5 | 5 | 5 |
| IV-27 | 2. 52 | 5 | 5 | 5 |
| IV-28 | 2. 52 | 5 | 5 | 5 |
| IV-29 | 2. 52 | 0 | 4 | 0 |
| IV-30 | 2. 52 | 5 | 5 | 4 |
| IV-31 | 2. 52 | 5 | 5 | 5 |
| IV-32 | 2. 52 | 5 | 5 | 5 |
| IV-33 | 2. 52 | 5 | 5 | 5 |
| IV-34 | 2. 52 | 5 | 5 | 5 |
| IV-35 | 2. 52 | 5 | 5 | 0 |
| V-1 | 2. 52 | 5 | 5 | 5 |
| V-2 | 2. 52 | 5 | 5 | 5 |
| V-3 | 2. 52 | 5 | 5 | 5 |
| V-4 | 2. 52 | 5 | 5 | 5 |
| V-5 | 2. 52 | 5 | 5 | 5 |
| V-6 | 2. 52 | 5 | 5 | 5 |
| V-8 | 2. 52 | 5 | 5 | 5 |
| V-9 | 2. 52 | 5 | 5 | 5 |
| V-10 | 2. 52 | 5 | 5 | 5 |
| V-12 | 2. 52 | 5 | 5 | 5 |
| V-13 | 2. 52 | 5 | 5 | 5 |
| V-14 | 2. 52 | 5 | 5 | 5 |
| V-15 | 2. 52 | 5 | 5 | 5 |
| V-16 | 2. 52 | 5 | 5 | 5 |
| V-17 | 2. 52 | 5 | 5 | 5 |
| V-18 | 2. 52 | 5 | 5 | 5 |
| V-19 | 2. 52 | 5 | 5 | 5 |
| V-20 | 2. 52 | 5 | 5 | 5 |
| V-21 | 2. 52 | 5 | 5 | 5 |
| V-22 | 2. 52 | 5 | 5 | 5 |
| V-23 | 2. 52 | 5 | 5 | 5 |
| V-24 | 2. 52 | 5 | 5 | 5 |
| V-25 | 2. 52 | 5 | 5 | 5 |
| V-26 | 2. 52 | 5 | 5 | 5 |
| V-27 | 2. 52 | 5 | 5 | 5 |
| V-29 | 2. 52 | 5 | 5 | 5 |
| V-30 | 2. 52 | 5 | 5 | 5 |
| V-31 | 2. 52 | 5 | 5 | 5 |
| V-32 | 2. 52 | 5 | 5 | 5 |
| V-33 | 2. 52 | 5 | 5 | 5 |
| V-34 | 2. 52 | 5 | 5 | 5 |
| V-35 | 2. 52 | 5 | 5 | 5 |
| V-36 | 2. 52 | 5 | 5 | 5 |
| V-37 | 2. 52 | 5 | 5 | 3 |
| V-38 | 2. 52 | 5 | 5 | 0 |
| V-39 | 2. 52 | 5 | 5 | 0 |
| V-40 | 2. 52 | 5 | 5 | 0 |
| VI-1 | 2. 52 | 5 | 5 | 5 |
| VI-2 | 2. 52 | 5 | 5 | 5 |
| VI-3 | 2. 52 | 5 | 5 | 5 |
| VI-4 | 2. 52 | 5 | 5 | 5 |
| VI-5 | 2. 52 | 5 | 5 | 5 |
| VI-6 | 2. 52 | 5 | 5 | 5 |
| VI-7 | 2. 52 | 5 | 5 | 5 |
| VII-1 | 2. 52 | 5 | 5 | 5 |
| VII-2 | 2. 52 | 5 | 5 | 5 |
| VII-3 | 2. 52 | 5 | 5 | 5 |
| VII-4 | 2. 52 | 5 | 5 | 5 |
| VII-5 | 2. 52 | 5 | 5 | 5 |
| VII-6 | 2. 52 | 5 | 5 | 5 |
| VII-7 | 1. 00 | 5 | 5 | 5 |
| VII-8 | 2. 52 | 5 | 5 | 5 |
| VIII-2 | 2. 52 | 5 | 5 | 5 |
| VIII-3 | 2. 52 | 5 | 5 | 5 |
| IX-1 | 2. 52 | 5 | 5 | 5 |
| X-1 | 2. 52 | 5 | 5 | 5 |
| X-2 | 2. 52 | 5 | 5 | 5 |
| X-3 | 2. 52 | 5 | 5 | 5 |
| X-4 | 2. 52 | 5 | 5 | 4 |
| X-5 | 2. 52 | 5 | 3 | 4 |
| X-6 | 2. 52 | 5 | 5 | 5 |
| X-7 | 2. 52 | 5 | 5 | 5 |

**TABLE 26**

| Compound No. | Dose (g/a) | a |
|---|---|---|
| I-1 | 2. 52 | 0 |
| I-2 | 2. 52 | 0 |
| I-3 | 2. 52 | 0 |
| I-4 | 2. 52 | 0 |
| I-5 | 2. 52 | 0 |
| II-1 | 2. 52 | 0 |
| II-2 | 2. 52 | 0 |
| II-3 | 2. 52 | 0 |
| III-1 | 0. 64 | 3 |
| III-2 | 0. 64 | 3 |
| III-3 | 0. 64 | 2 |
| III-4 | 0. 75 | 1 |
| III-5 | 0. 64 | 3 |
| III-6 | 0. 64 | 0 |
| III-7 | 0. 40 | 0 |
| III-8 | 0. 64 | 2 |
| III-9 | 0. 64 | 0 |
| III-10 | 0. 64 | 0 |
| III-11 | 0. 64 | 0 |
| III-12 | 0. 64 | 0 |
| III-13 | 0. 64 | 0 |
| III-14 | 0. 64 | 0 |
| III-15 | 0. 64 | 0 |
| III-16 | 0. 64 | 2 |
| III-17 | 0. 64 | 0 |
| III-18 | 0. 64 | 0 |
| III-19 | 0. 64 | 0 |
| III-20 | 0. 64 | 0 |
| III-21 | 2. 52 | 0 |
| III-22 | 0. 64 | 0 |
| III-23 | 0. 64 | 0 |
| III-25 | 2. 52 | 0 |
| III-26 | 2. 52 | 0 |
| III-27 | 2. 52 | 0 |
| III-28 | 2. 52 | 0 |
| III-29 | 2. 52 | 0 |
| III-30 | 2. 52 | 0 |
| III-31 | 2. 52 | 0 |
| III-32 | 2. 52 | 3 |
| III-33 | 2. 52 | 2 |
| III-34 | 2. 52 | 1 |
| III-35 | 2. 52 | 4 |
| III-36 | 2. 52 | 0 |
| III-37 | 2. 52 | 0 |
| III-38 | 2. 52 | 0 |
| III-39 | 2. 52 | 5 |
| III-40 | 2. 52 | 2 |
| III-41 | 2. 52 | 2 |
| III-42 | 2. 52 | 2 |
| III-43 | 2. 52 | 0 |
| III-44 | 2. 52 | 0 |
| III-45 | 2. 52 | 0 |
| III-46 | 0. 64 | 0 |
| III-47 | 2. 52 | 0 |
| III-48 | 0. 64 | 0 |
| III-49 | 2. 52 | 0 |
| III-50 | 0. 64 | 0 |
| III-51 | 0. 64 | 0 |
| III-52 | 2. 52 | 0 |
| III-53 | 2. 52 | 0 |
| III-54 | 0. 64 | 0 |
| III-55 | 2. 52 | 0 |
| III-56 | 2. 52 | 0 |
| III-57 | 2. 52 | 0 |
| III-58 | 0. 64 | 0 |
| III-59 | 2. 52 | 0 |
| III-60 | 2. 52 | 0 |
| III-61 | 2. 52 | 0 |
| III-62 | 0. 64 | 0 |
| III-63 | 2. 52 | 0 |
| III-64 | 2. 52 | 0 |
| III-65 | 2. 52 | 0 |
| III-66 | 2. 52 | 0 |
| III-67 | 0. 64 | 0 |
| III-68 | 2. 52 | 0 |
| III-69 | 2. 52 | 0 |
| III-70 | 2. 52 | 0 |
| III-71 | 2. 52 | 0 |
| III-72 | 0. 64 | 0 |
| III-73 | 0. 64 | 0 |
| III-74 | 2. 52 | 0 |
| III-75 | 0. 64 | 0 |
| III-76 | 2. 52 | 0 |
| III-77 | 2. 52 | 0 |
| III-78 | 0. 64 | 0 |
| III-79 | 1. 04 | 0 |
| III-80 | 2. 52 | 0 |
| III-81 | 2. 52 | 0 |
| III-82 | 2. 52 | 0 |
| III-83 | 6. 30 | 0 |
| III-84 | 2. 52 | 0 |
| III-85 | 2. 52 | 0 |
| III-86 | 2. 52 | 0 |
| III-87 | 2. 52 | 0 |
| III-88 | 2. 52 | 0 |
| III-89 | 2. 52 | 0 |
| III-90 | 1. 28 | 0 |
| III-91 | 2. 52 | 0 |
| III-92 | 2. 52 | 0 |
| III-93 | 2. 52 | 0 |
| III-95 | 2. 52 | 0 |
| III-96 | 0. 64 | 0 |
| III-97 | 0. 64 | 0 |
| III-98 | 0. 64 | 0 |
| III-99 | 2. 17 | 0 |
| III-100 | 2. 52 | 0 |
| III-101 | 2. 52 | 1 |
| III-102 | 2. 52 | 0 |
| III-103 | 2. 52 | 0 |
| III-104 | 2. 52 | 0 |
| III-105 | 2. 52 | 0 |
| III-106 | 2. 52 | 0 |
| III-107 | 0. 64 | 0 |
| III-108 | 0. 64 | 0 |
| III-109 | 0. 64 | 0 |
| III-110 | 0. 64 | 0 |
| III-111 | 0. 64 | 0 |
| III-112 | 2. 52 | 2 |
| III-113 | 2. 52 | 4 |
| III-114 | 0. 64 | 0 |
| IV-1 | 0. 64 | 0 |
| IV-2 | 0. 64 | 0 |
| IV-3 | 0. 64 | 0 |
| IV-4 | 0. 64 | 0 |
| IV-5 | 0. 64 | 0 |
| IV-6 | 0. 64 | 0 |
| IV-7 | 2. 52 | 3 |
| IV-8 | 2. 52 | 2 |
| IV-9 | 0. 64 | 0 |
| IV-10 | 2. 52 | 0 |
| IV-11 | 2. 52 | 0 |
| IV-12 | 2. 52 | 0 |
| IV-13 | 2. 52 | 5 |
| IV-14 | 1.79 | 0 |
| IV-15 | 2. 52 | 0 |
| IV-16 | 2. 52 | 0 |
| IV-17 | 2. 52 | 0 |
| IV-18 | 2. 52 | 0 |
| IV-19 | 2. 52 | 0 |
| IV-20 | 2. 52 | 0 |
| IV-21 | 2. 52 | 0 |
| IV-23 | 2. 52 | 0 |
| IV-24 | 0. 64 | 0 |
| VI-25 | 0. 64 | 0 |
| VI-26 | 0. 64 | 0 |
| IV-27 | 0. 64 | 0 |
| IV-28 | 0. 64 | 1 |
| IV-29 | 2. 52 | 0 |
| IV-30 | 2. 52 | 0 |
| IV-31 | 2. 52 | 0 |
| IV-32 | 2. 52 | 0 |
| IV-33 | 2. 52 | 0 |
| IV-34 | 2. 52 | 0 |
| IV-35 | 2. 52 | 0 |
| V-1 | 0. 64 | 0 |
| V-2 | 2. 52 | 0 |
| V-4 | 2. 52 | 0 |
| V-5 | 0. 64 | 0 |
| V-8 | 2. 52 | 0 |
| V-9 | 0. 64 | 0 |
| V-10 | 2. 52 | 0 |
| V-12 | 0. 64 | 0 |
| V-13 | 2. 52 | 0 |
| V-14 | 2. 52 | 0 |
| V-15 | 2. 52 | 1 |
| V-16 | 2. 52 | 0 |
| V-17 | 2. 52 | 1 |
| V-18 | 0. 64 | 0 |
| V-19 | 0. 64 | 0 |
| V-20 | 0. 64 | 0 |
| V-21 | 0. 64 | 0 |
| V-22 | 0. 64 | 0 |
| V-23 | 0. 64 | 0 |
| V-24 | 0. 64 | 0 |
| V-25 | 2. 52 | 0 |
| V-26 | 2. 52 | 0 |
| V-27 | 2. 52 | 0 |
| V-29 | 2. 52 | 0 |
| V-30 | 2. 52 | 0 |
| V-31 | 2. 52 | 0 |
| V-32 | 0. 64 | 0 |
| V-33 | 0. 64 | 0 |
| V-34 | 0. 64 | 0 |
| V-35 | 0. 64 | 0 |
| V-36 | 0. 64 | 0 |
| V-37 | 2. 52 | 0 |
| V-38 | 2. 52 | 0 |
| V-39 | 2. 52 | 0 |
| V-40 | 2. 52 | 0 |
| VI-1 | 0. 64 | 3 |
| VI-2 | 0. 64 | 0 |
| VI-3 | 0. 64 | 0 |
| VI-4 | 0. 64 | 0 |
| VI-5 | 0. 64 | 0 |
| VI-6 | 0. 64 | 0 |
| VI-7 | 0. 64 | 0 |
| VII-1 | 2. 52 | 0 |
| VII-2 | 2. 52 | 0 |
| VII-3 | 2. 52 | 0 |
| VII-4 | 2. 52 | 0 |
| VII-5 | 2. 52 | 0 |
| VII-6 | 2. 52 | 2 |
| VII-7 | 1. 00 | 0 |
| VII-8 | 2. 52 | 0 |
| VIII-2 | 2. 52 | 2 |
| VIII-3 | 2. 52 | 0 |
| IX-1 | 2. 52 | 0 |
| X-1 | 2. 52 | 5 |
| X-2 | 2. 52 | 3 |
| X-3 | 2. 52 | 4 |
| X-4 | 2. 52 | 0 |
| X-5 | 2. 52 | 0 |
| X-6 | 2. 52 | 2 |
| X-7 | 2. 52 | 0 |

**TABLE 27**

| Compound No. | Dose (g/a) | A | B | C |
|---|---|---|---|---|
| I-1 | 2. 52 | 4 | 4 | 4 |
| I-2 | 2. 52 | 4 | 5 | 5 |
| I-3 | 2. 52 | 5 | 5 | 5 |
| I-4 | 2. 52 | 5 | 5 | 5 |
| I-5 | 2. 52 | 5 | 5 | 5 |
| II-1 | 2. 52 | 5 | 5 | 5 |
| II-2 | 2. 52 | 5 | 5 | 5 |
| II-3 | 2. 52 | 5 | 5 | 5 |
| III-1 | 2. 52 | 5 | 5 | 5 |
| III-2 | 2. 52 | 5 | 5 | 5 |
| III-3 | 2. 52 | 5 | 5 | 5 |
| III-4 | 1. 50 | 5 | 5 | 5 |
| III-5 | 2. 52 | 5 | 5 | 5 |
| III-6 | 2. 52 | 5 | 5 | 5 |
| III-7 | 1. 52 | 5 | 5 | 5 |
| III-8 | 2. 52 | 5 | 5 | 5 |
| III-9 | 2. 52 | 5 | 5 | 5 |
| III-10 | 2. 52 | 5 | 5 | 5 |
| III-11 | 2. 52 | 5 | 5 | 5 |
| III-12 | 2. 52 | 5 | 5 | 5 |
| III-13 | 2. 52 | 5 | 5 | 5 |
| III-14 | 2. 52 | 4 | 5 | 3 |
| III-15 | 2. 52 | 4 | 4 | 0 |
| III-16 | 2. 52 | 5 | 5 | 5 |
| III-17 | 2. 52 | 4 | 5 | 0 |
| III-18 | 2. 52 | 5 | 5 | 5 |
| III-19 | 2. 52 | 5 | 5 | 5 |
| III-20 | 2. 52 | 5 | 4 | 5 |
| III-21 | 2. 52 | 5 | 5 | 5 |
| III-22 | 2. 52 | 5 | 5 | 5 |
| III-23 | 2. 52 | 5 | 5 | 5 |
| III-25 | 2. 52 | 5 | 4 | 5 |
| III-26 | 2. 52 | 5 | 5 | 5 |
| III-27 | 2. 52 | 4 | 5 | 5 |
| III-28 | 2. 52 | 4 | 5 | 4 |
| III-29 | 2. 52 | 4 | 5 | 4 |
| III-30 | 2. 52 | 5 | 5 | 5 |
| III-31 | 2. 52 | 5 | 5 | 5 |
| III-32 | 2. 52 | 5 | 5 | 5 |
| III-33 | 2. 52 | 5 | 5 | 4 |
| III-34 | 2. 52 | 5 | 5 | 5 |
| III-35 | 2. 52 | 5 | 5 | 4 |
| III-36 | 2. 52 | 5 | 5 | 5 |
| III-37 | 2. 52 | 5 | 5 | 5 |
| III-38 | 2. 52 | 5 | 5 | 5 |
| III-39 | 2. 52 | 5 | 5 | 5 |
| III-40 | 2. 52 | 5 | 5 | 5 |
| III-41 | 2. 52 | 5 | 5 | 5 |
| III-42 | 2. 52 | 5 | 5 | 5 |
| III-43 | 2. 52 | 5 | 5 | 5 |
| III-44 | 2. 52 | 4 | 5 | 5 |
| III-45 | 2. 52 | 3 | 5 | 4 |
| III-46 | 2. 52 | 5 | 5 | 5 |
| III-47 | 2. 52 | 5 | 5 | 5 |
| III-48 | 2. 52 | 5 | 5 | 5 |
| III-49 | 2. 52 | 5 | 5 | 5 |
| III-50 | 2. 52 | 5 | 5 | 5 |
| III-51 | 2. 52 | 5 | 5 | 5 |
| III-52 | 2. 52 | 5 | 5 | 5 |
| III-53 | 2. 52 | 5 | 5 | 4 |
| III-54 | 2. 52 | 5 | 5 | 5 |
| III-55 | 2. 52 | 5 | 5 | 4 |
| III-56 | 2. 52 | 5 | 5 | 5 |
| III-57 | 2. 52 | 5 | 5 | 5 |
| III-58 | 2. 52 | 5 | 5 | 5 |
| III-59 | 2. 52 | 5 | 5 | 5 |
| III-60 | 2. 52 | 5 | 5 | 5 |
| III-61 | 2. 52 | 5 | 5 | 5 |
| III-62 | 2. 52 | 5 | 5 | 5 |
| III-63 | 2. 52 | 5 | 5 | 5 |
| III-64 | 2. 52 | 5 | 5 | 5 |
| III-65 | 2. 52 | 5 | 5 | 5 |
| III-66 | 2. 52 | 5 | 5 | 5 |
| III-67 | 2. 52 | 5 | 5 | 5 |
| III-68 | 2. 52 | 5 | 5 | 5 |
| III-69 | 2. 52 | 5 | 5 | 5 |
| III-70 | 2. 52 | 0 | 5 | 4 |
| III-71 | 2. 52 | 5 | 5 | 4 |
| III-72 | 2. 52 | 5 | 5 | 5 |
| III-73 | 2. 52 | 5 | 5 | 5 |
| III-74 | 2. 52 | 5 | 5 | 5 |
| III-75 | 2. 52 | 5 | 5 | 5 |
| III-76 | 2. 52 | 5 | 5 | 5 |
| III-77 | 2. 52 | 5 | 5 | 5 |
| III-78 | 2. 52 | 5 | 5 | 5 |
| III-79 | 1. 04 | 5 | 5 | 5 |
| III-80 | 2. 52 | 5 | 5 | 5 |
| III-81 | 2. 52 | 5 | 4 | 4 |
| III-82 | 2. 52 | 5 | 5 | 4 |
| III-83 | 6. 30 | 5 | 5 | 5 |
| III-84 | 2. 52 | 5 | 5 | 5 |
| III-85 | 2. 52 | 5 | 4 | 5 |
| III-86 | 2. 52 | 5 | 5 | 5 |
| III-87 | 2. 52 | 3 | 5 | 4 |
| III-88 | 2. 52 | 5 | 5 | 5 |
| III-89 | 2. 52 | 5 | 5 | 5 |
| III-90 | 1. 28 | 4 | 5 | 5 |
| III-91 | 2. 52 | 5 | 5 | 5 |
| III-92 | 2. 52 | 5 | 5 | 5 |
| III-93 | 2. 52 | 4 | 5 | 4 |
| III-94 | 2. 52 | 5 | 5 | 5 |
| III-95 | 2. 52 | 5 | 5 | 5 |
| III-96 | 2. 52 | 5 | 5 | 5 |
| III-97 | 2. 52 | 5 | 5 | 5 |
| III-98 | 2. 52 | 5 | 5 | 5 |
| III-99 | 2. 17 | 5 | 5 | 5 |
| III-100 | 2. 52 | 5 | 5 | 5 |
| III-101 | 2. 52 | 5 | 5 | 5 |
| III-102 | 2. 52 | 5 | 5 | 5 |
| III-103 | 2. 52 | 5 | 5 | 4 |
| III-104 | 2. 52 | 5 | 5 | 5 |
| III-105 | 2. 52 | 5 | 5 | 5 |
| III-106 | 2. 52 | 5 | 5 | 5 |
| III-107 | 2. 52 | 4 | 4 | 5 |
| III-108 | 2. 52 | 5 | 5 | 5 |
| III-109 | 2. 52 | 5 | 5 | 5 |
| III-110 | 2. 52 | 5 | 5 | 5 |
| III-111 | 2. 52 | 5 | 5 | 5 |
| III-112 | 2. 52 | 5 | 5 | 5 |
| III-113 | 2. 52 | 5 | 5 | 5 |
| III-114 | 0. 64 | 3 | 5 | 5 |
| IV-1 | 2. 52 | 5 | 5 | 5 |
| IV-2 | 2. 52 | 5 | 5 | 5 |
| IV-3 | 2. 52 | 5 | 5 | 5 |
| IV-4 | 2. 52 | 5 | 5 | 5 |
| IV-5 | 2. 52 | 5 | 5 | 5 |
| IV-6 | 2. 52 | 5 | 5 | 5 |
| IV-7 | 2. 52 | 5 | 4 | 5 |
| IV-8 | 2. 52 | 5 | 5 | 5 |
| IV-9 | 2. 52 | 5 | 5 | 5 |
| IV-10 | 2. 52 | 5 | 5 | 5 |
| IV-11 | 2. 52 | 5 | 5 | 5 |
| IV-12 | 2. 52 | 4 | 5 | 4 |
| IV-13 | 2. 52 | 5 | 5 | 5 |
| IV-14 | 1. 79 | 3 | 4 | 4 |
| IV-15 | 2. 52 | 5 | 5 | 4 |
| IV-16 | 2. 52 | 5 | 5 | 5 |
| IV-17 | 2. 52 | 5 | 5 | 5 |
| IV-18 | 2. 52 | 5 | 5 | 5 |
| IV-19 | 2. 52 | 5 | 5 | 5 |
| IV-20 | 2. 52 | 5 | 5 | 5 |
| IV-21 | 2. 52 | 4 | 5 | 4 |
| IV-23 | 2. 52 | 5 | 5 | 5 |
| IV-24 | 2. 52 | 5 | 5 | 5 |
| IV-25 | 2. 52 | 5 | 5 | 5 |
| IV-26 | 2. 52 | 5 | 5 | 5 |
| IV-27 | 2. 52 | 5 | 5 | 5 |
| IV-28 | 2. 52 | 5 | 5 | 5 |
| IV-29 | 2. 52 | 0 | 5 | 3 |
| IV-30 | 2. 52 | 0 | 5 | 3 |
| IV-31 | 2. 52 | 5 | 5 | 5 |
| IV-32 | 2. 52 | 5 | 5 | 5 |
| IV-33 | 2. 52 | 4 | 5 | 3 |
| IV-34 | 2. 52 | 5 | 5 | 0 |
| IV-35 | 2. 52 | 0 | 5 | 0 |
| V-1 | 2. 52 | 5 | 5 | 5 |
| V-2 | 2. 52 | 5 | 5 | 5 |
| V-3 | 2. 52 | 5 | 5 | 5 |
| V-4 | 2. 52 | 5 | 5 | 5 |
| V-5 | 2. 52 | 5 | 5 | 5 |
| V-6 | 2. 52 | 5 | 5 | 4 |
| V-8 | 2. 52 | 5 | 5 | 4 |
| V-9 | 2. 52 | 5 | 5 | 5 |
| V-10 | 2. 52 | 5 | 5 | 5 |
| V-12 | 2. 52 | 5 | 5 | 5 |
| V-13 | 2. 52 | 5 | 5 | 5 |
| V-14 | 2. 52 | 5 | 5 | 5 |
| V-15 | 2. 52 | 5 | 5 | 4 |
| V-16 | 2. 52 | 5 | 5 | 4 |
| V-17 | 2. 52 | 5 | 5 | 4 |
| V-18 | 2. 52 | 5 | 5 | 5 |
| V-19 | 2. 52 | 5 | 5 | 5 |
| V-20 | 2. 52 | 5 | 5 | 5 |
| V-21 | 2. 52 | 5 | 5 | 5 |
| V-22 | 2. 52 | 5 | 5 | 5 |
| V-23 | 2. 52 | 5 | 5 | 5 |
| V-24 | 2. 52 | 5 | 5 | 5 |
| V-25 | 2. 52 | 5 | 5 | 5 |
| V-26 | 2. 52 | 0 | 5 | 5 |
| V-27 | 2. 52 | 0 | 4 | 4 |
| V-29 | 2. 52 | 5 | 5 | 4 |
| V-30 | 2. 52 | 5 | 5 | 4 |
| V-31 | 2. 52 | 5 | 5 | 4 |
| V-32 | 2. 52 | 5 | 5 | 5 |
| V-33 | 2. 52 | 5 | 5 | 5 |
| V-34 | 2. 52 | 5 | 5 | 5 |
| V-35 | 2. 52 | 5 | 5 | 5 |
| V-36 | 2. 52 | 5 | 5 | 5 |
| V-37 | 2. 52 | 0 | 5 | 0 |
| V-38 | 2. 52 | 0 | 0 | 0 |
| V-39 | 2. 52 | 4 | 5 | 4 |
| V-40 | 2. 52 | 4 | 5 | 3 |
| VI-1 | 2. 52 | 5 | 5 | 5 |
| VI-2 | 2. 52 | 4 | 5 | 5 |
| VI-3 | 2. 52 | 0 | 5 | 3 |
| VI-4 | 2. 52 | 4 | 5 | 4 |
| VI-5 | 2. 52 | 0 | 5 | 3 |
| VI-6 | 2. 52 | 5 | 5 | 5 |
| VI-7 | 2. 52 | 5 | 5 | 5 |
| VII-1 | 2. 52 | 4 | 4 | 4 |
| VII-2 | 2. 52 | 5 | 5 | 5 |
| VII-3 | 2. 52 | 5 | 5 | 5 |
| VII-4 | 2. 52 | 5 | 5 | 5 |
| VII-5 | 2. 52 | 5 | 5 | 5 |
| VII-6 | 2. 52 | 5 | 4 | 4 |
| VII-7 | 1. 00 | 5 | 5 | 5 |
| VII-8 | 2. 52 | 5 | 5 | 5 |
| VIII-2 | 2. 52 | 5 | 4 | 5 |
| VIII-3 | 2. 52 | 5 | 5 | 5 |
| IX-1 | 2. 52 | 5 | 5 | 4 |
| X-1 | 2. 52 | 5 | 5 | 5 |
| X-2 | 2. 52 | 5 | 5 | 5 |
| X-3 | 2. 52 | 5 | 5 | 5 |
| X-4 | 2. 52 | 5 | 5 | 4 |
| X-5 | 2. 52 | 5 | 5 | 4 |
| X-6 | 2. 52 | 5 | 5 | 4 |
| X-7 | 2. 52 | 5 | 4 | 4 |

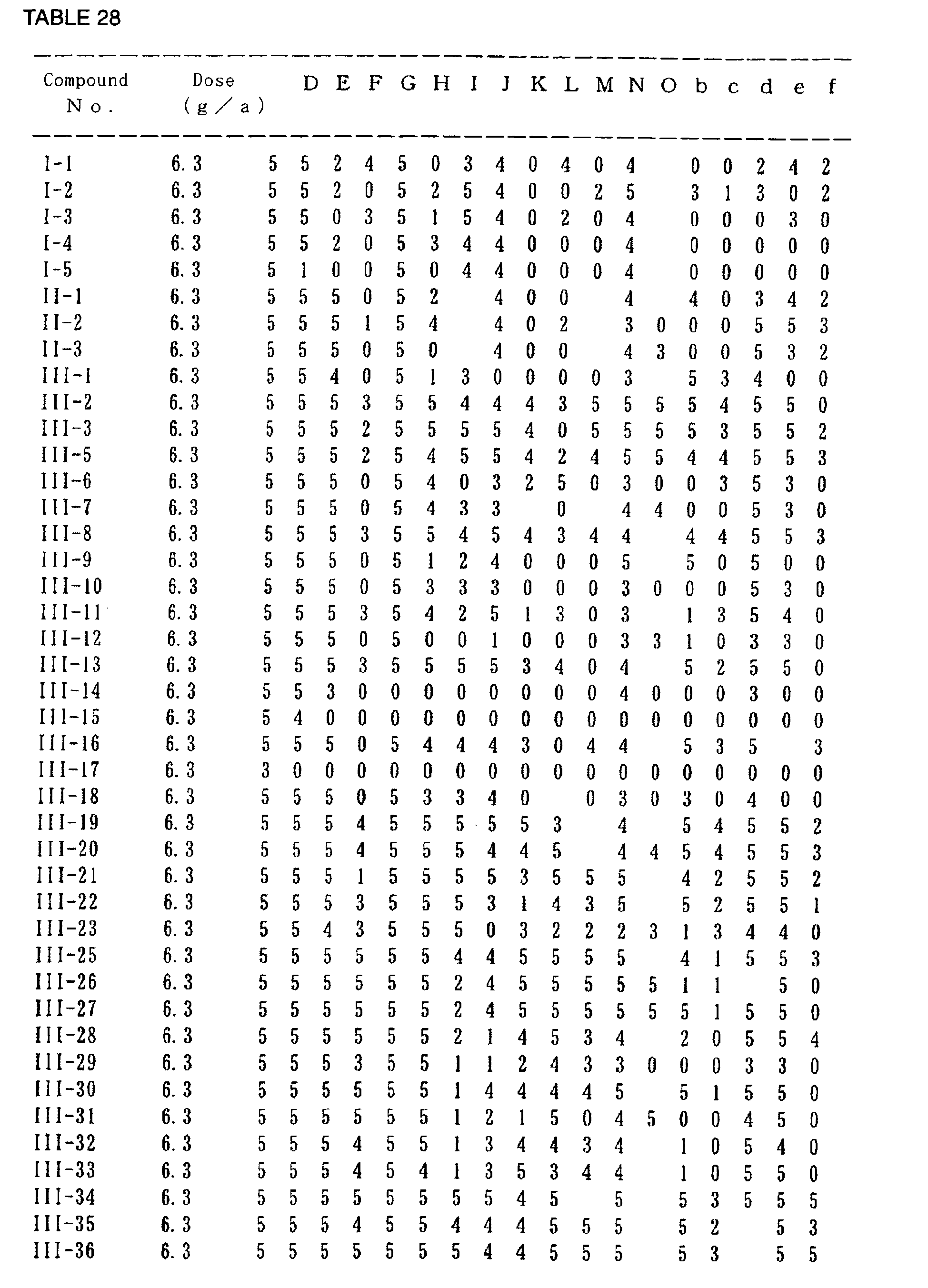

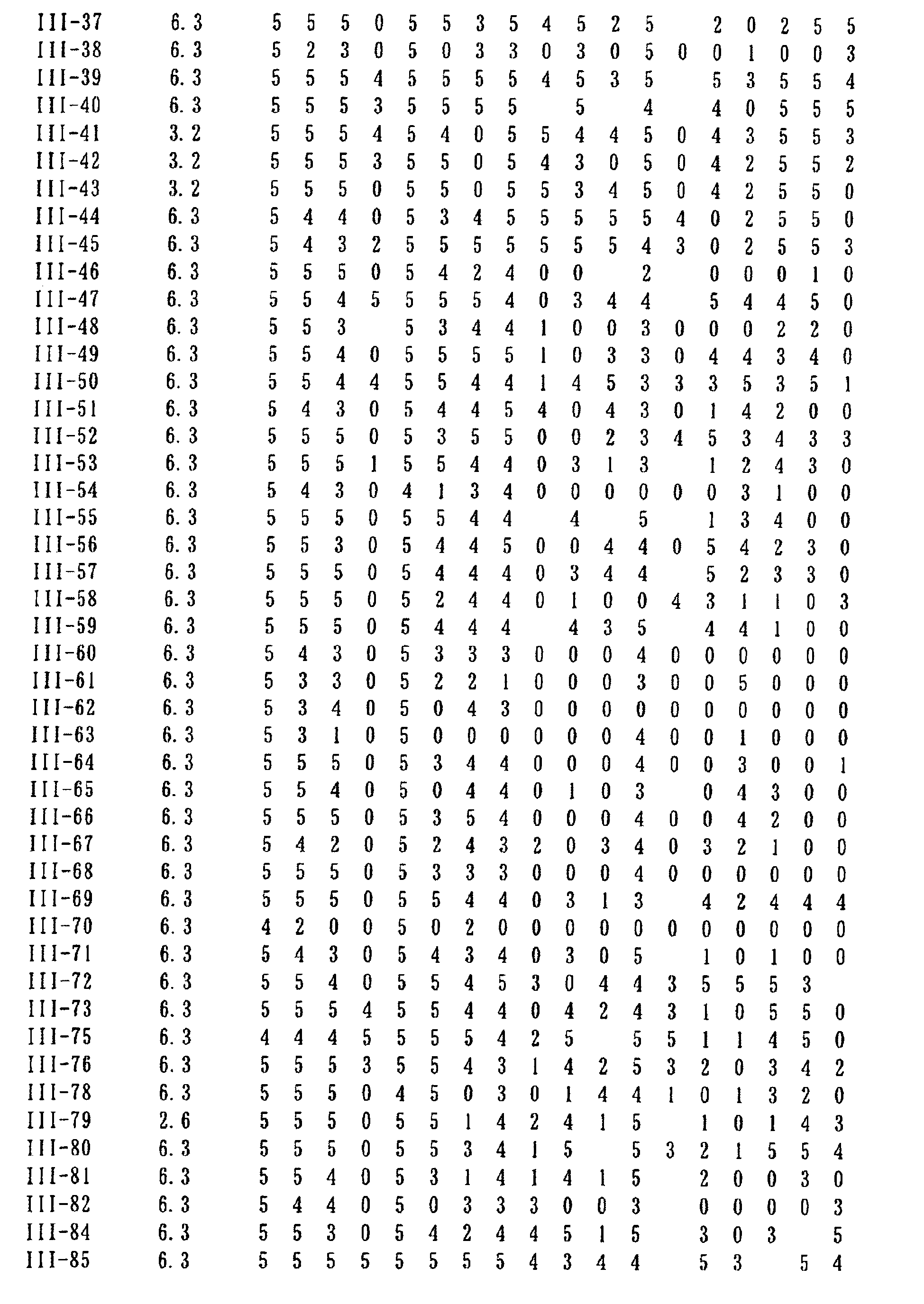

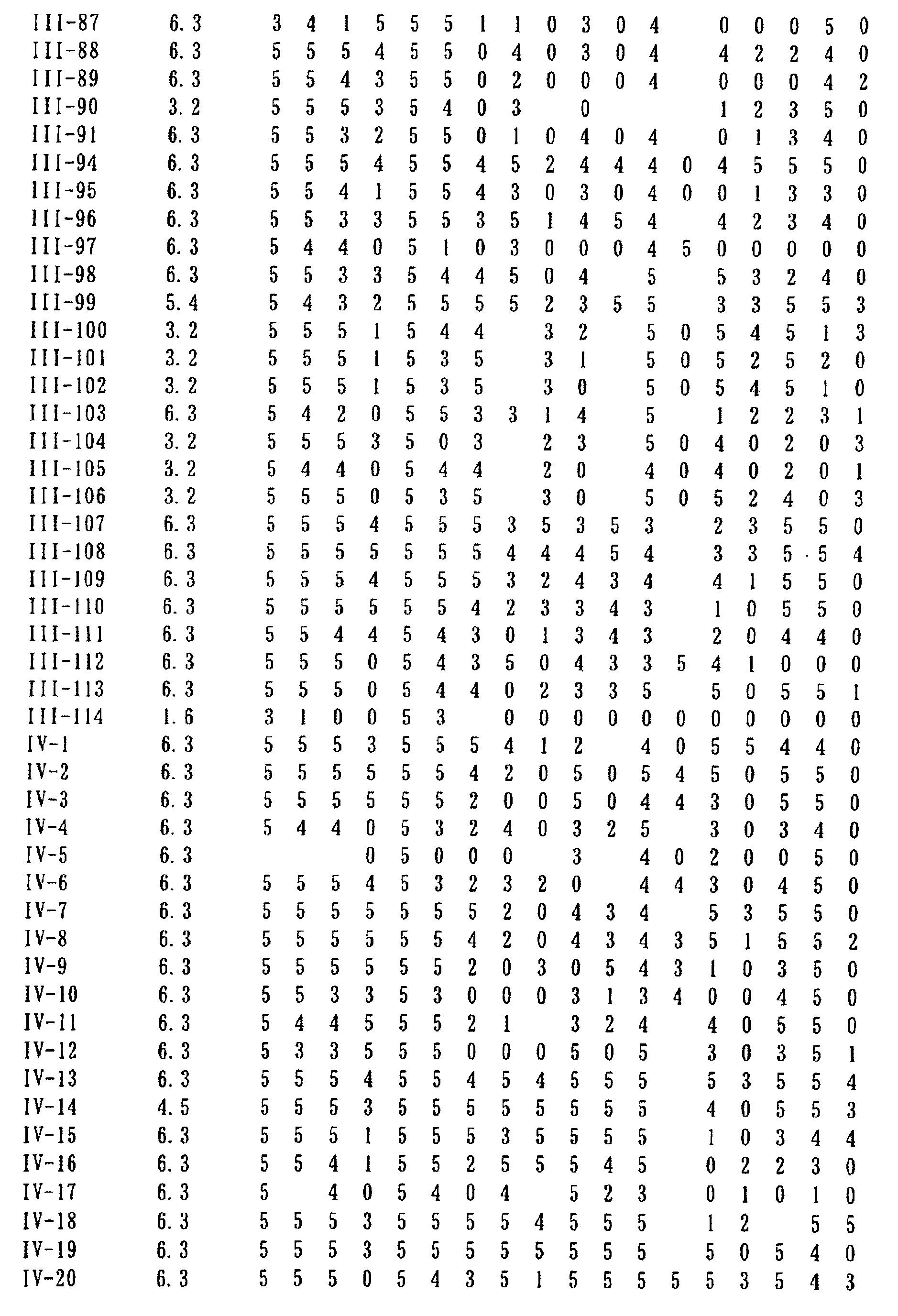

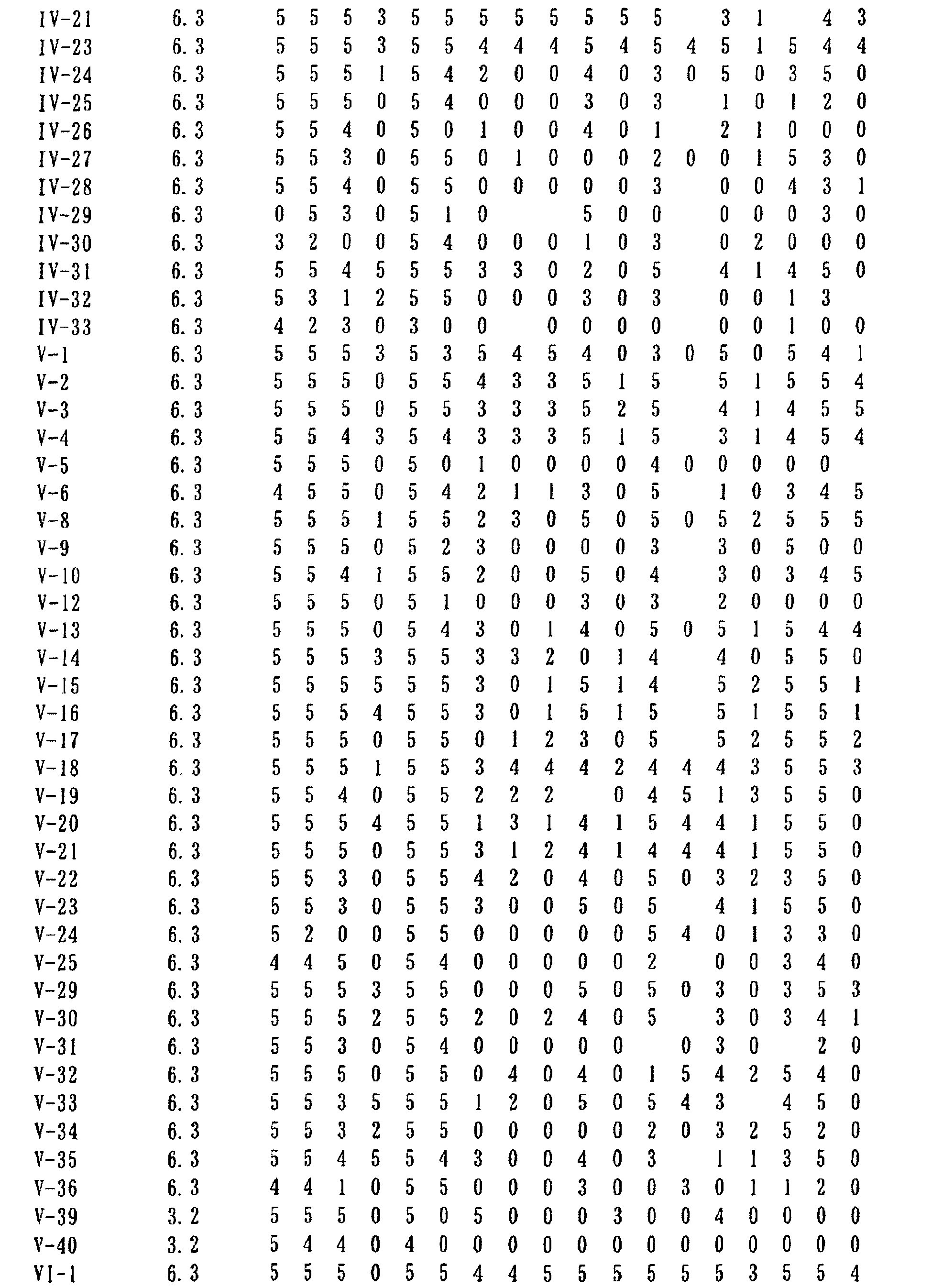

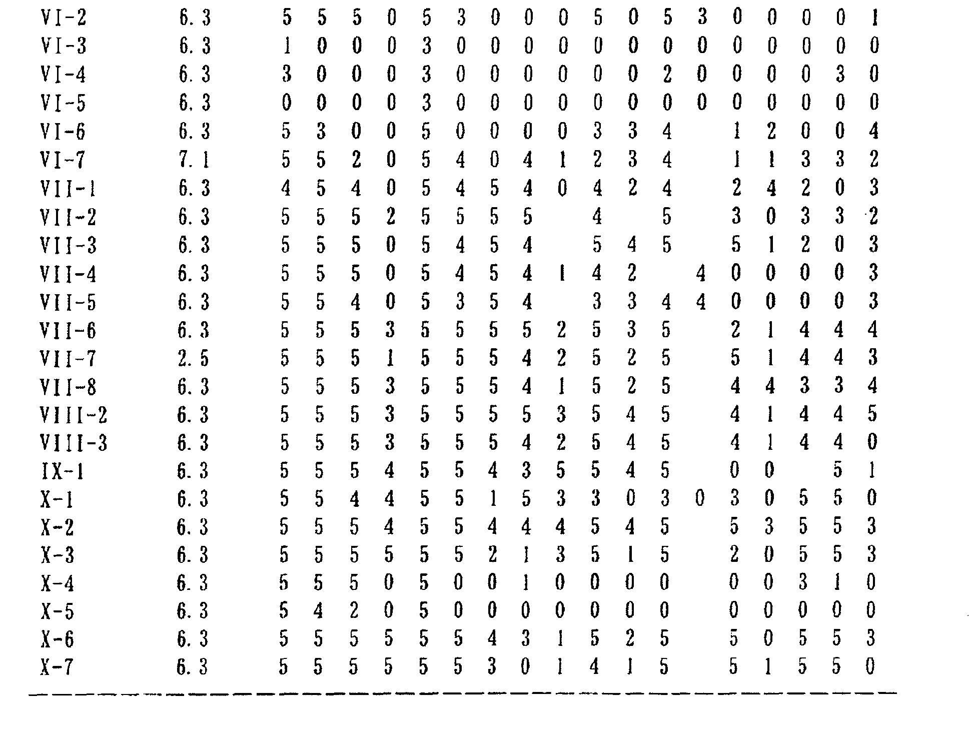

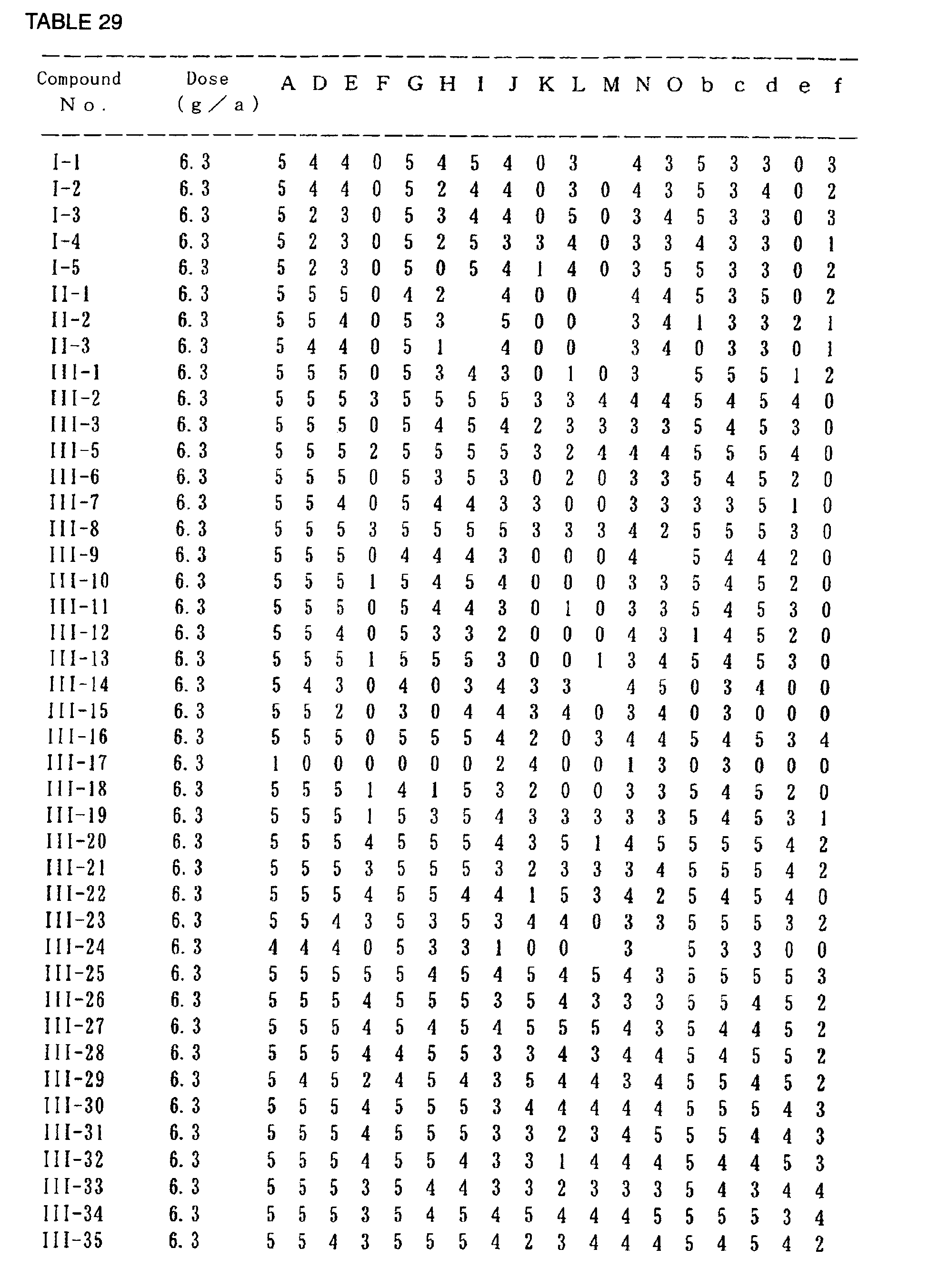

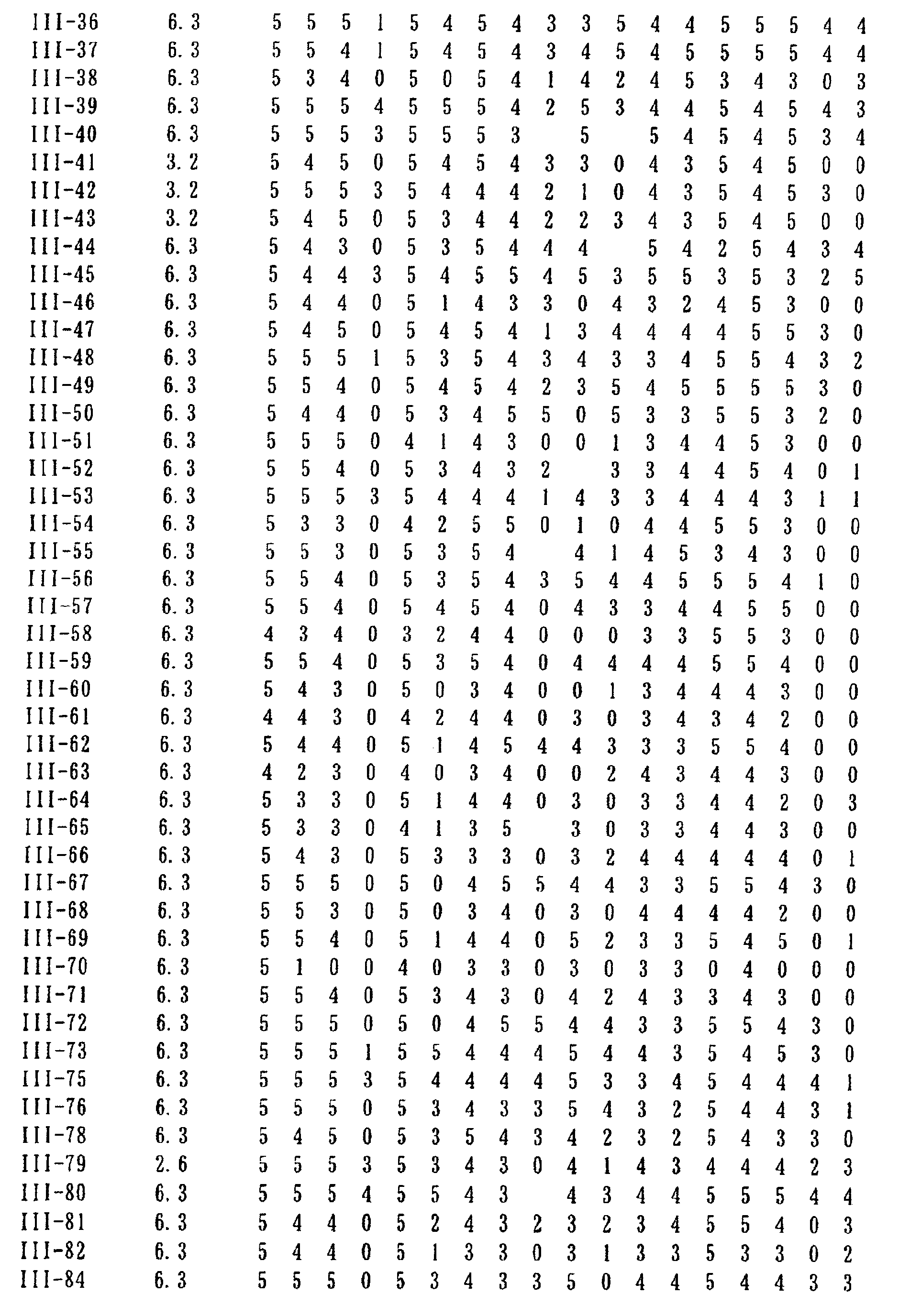

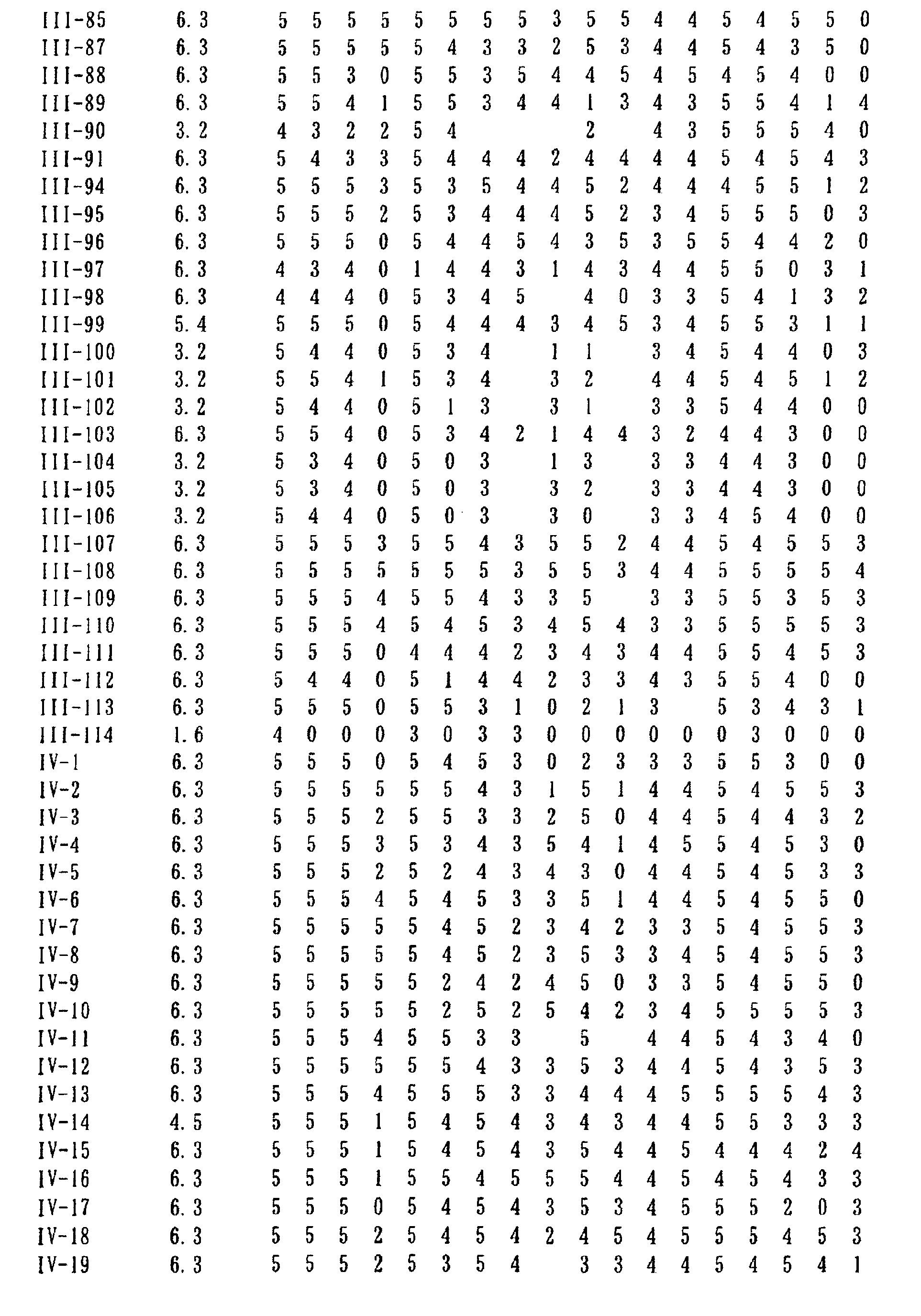

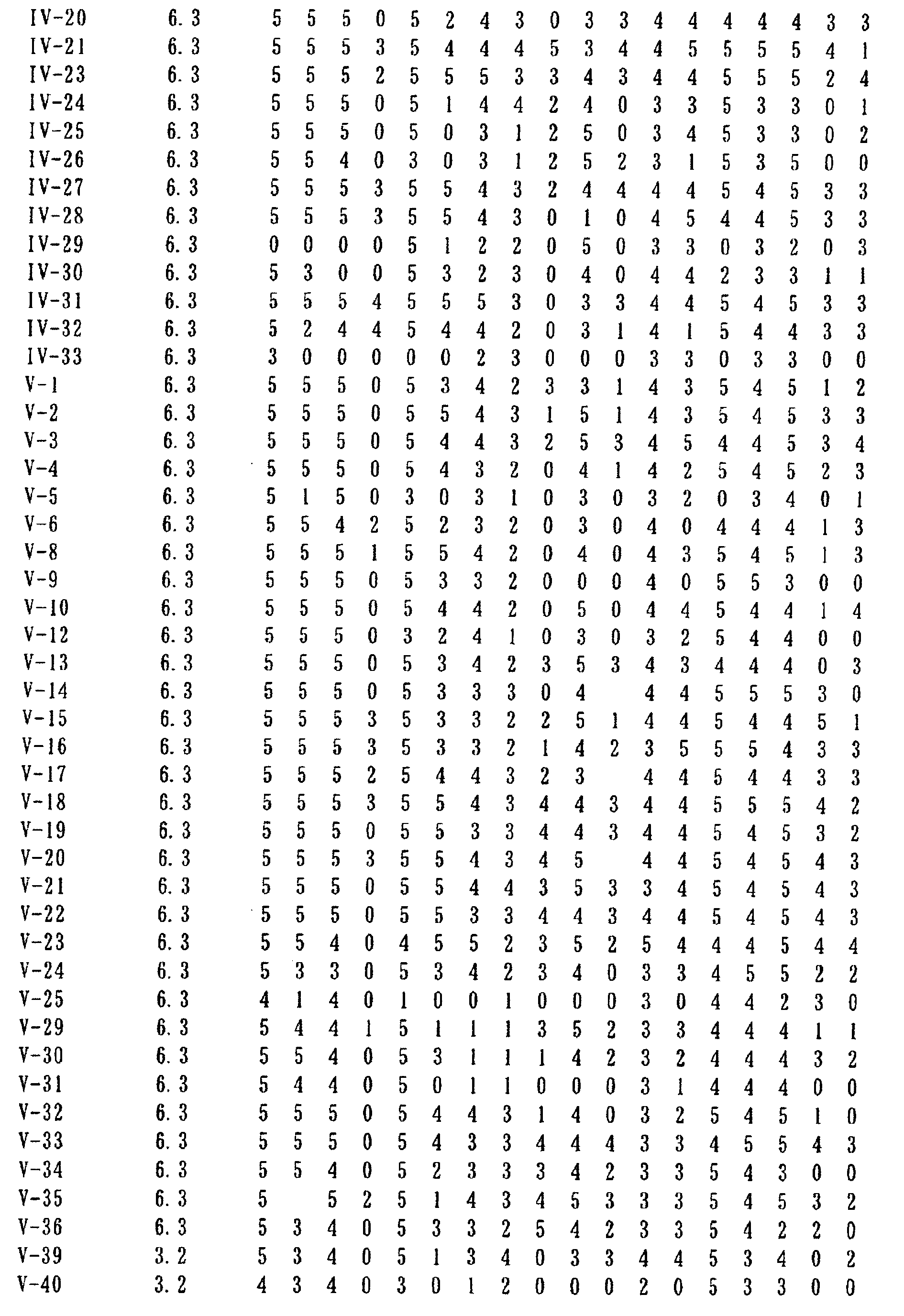

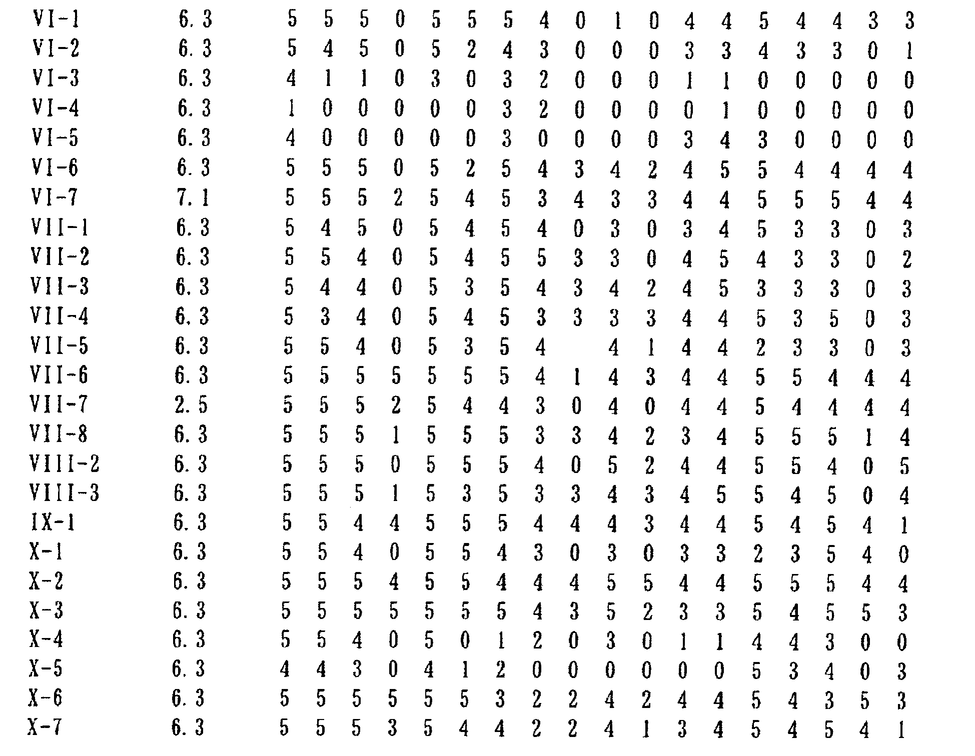

Although the compounds of the present invention are novel, the following comparative compounds a to h are known to have analogous structures.

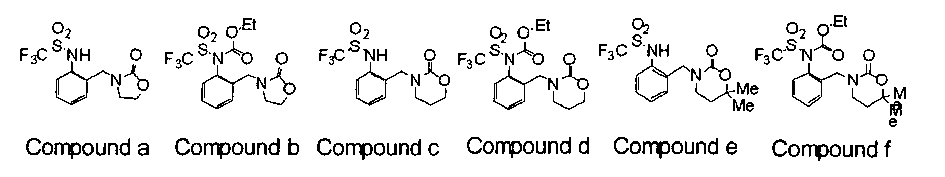

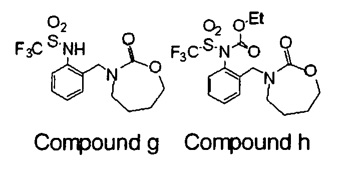

Comparative Compounds a and b are disclosed in Patent Document 2, Comparative Compounds c to h are disclosed in Patent Document 3. the present inventors discovered that the compounds of the present invention are clearly superior to the comparative compounds in herbicidal effect. To demonstrate this, in the following Comparative Example, their herbicidal effects against barnyardgrass, a major paddy field weed, are compared.

### [COMPARATIVE EXAMPLE] Test on the herbicidal effects on barnyardgrass during the vegeration period under submerged conditions

Alluvial soil was put in 1/30000-are styrol cups and admixed with water so that it was submerged at a depth of 4 cm. In each cup, seeds of barnyeardgrass were sown, and the cups were placed in a greenhouse at 25 to 30°C to grow the plants. When the barnyardgrass reached the 2- or 2.5-leaf stage, emulsifiable concentrates containing compounds of the present invention and the comparative compounds formulated in accordance with Formulation Example 2 were applied to the water surfaces at predetermined doses after diluted with water. Three weeks after the application, the herbicidal effects against various plants were determined on the same evaluation scale as in Test Example 1. The results are shown in Table 30.

The symbol A in the table means barnyardgrass.

**TABLE 30**

| Compound No. | Dose (g/a) | A |
|---|---|---|
| a | 0. 16 | 0 |
| a | 0. 64 | 0 |
| a | 2. 52 | 4 |
| II- 1 | 0. 16 | 2 |
| II- 1 | 0. 64 | 5 |
| II- 1 | 2. 52 | 5 |
| b | 0. 16 | 0 |
| b | 0. 64 | 0 |
| b | 2. 52 | 3 |
| II-2 | 0. 16 | 0 |
| II-2 | 0. 64 | 5 |
| II-2 | 2. 52 | 5 |
| c | 0. 16 | 0 |
| c | 0. 64 | 2 |
| c | 2. 52 | 5 |
| III-1 | 0. 16 | 3 |
| III-1 | 0. 64 | 5 |
| III-1 | 2. 52 | 5 |
| d | 0. 16 | 0 |
| d | 0. 64 | 2 |
| d | 2. 52 | 5 |
| III-9 | 0. 16 | 4 |
| III-9 | 0. 64 | 5 |
| III-9 | 2. 52 | 5 |
| e | 0. 16 | 0 |
| e | 0. 64 | 3 |
| e | 2. 52 | 5 |
| III-46 | 0. 16 | 0 |
| III-46 | 0. 64 | 4 |
| III-46 | 2. 52 | 5 |
| f | 0. 16 | 0 |
| f | 0. 64 | 0 |
| f | 2. 52 | 5 |
| III-58 | 0. 16 | 0 |
| III-58 | 0. 64 | 5 |
| III-58 | 2. 52 | 5 |
| g | 0. 16 | 0 |
| g | 0. 64 | 0 |
| g | 2. 52 | 5 |
| V-1 | 0. 16 | 0 |
| V-1 | 0. 64 | 5 |
| V-1 | 2. 52 | 5 |
| h | 0. 16 | 0 |
| h | 0. 64 | 1 |
| h | 2. 52 | 5 |
| V-10 | 0. 16 | 0 |
| V-10 | 0. 64 | 5 |
| V-10 | 2. 52 | 5 |

### INDUSTRIAL APPLICABILITY

The haloalkylsulfonanilide derivatives and their agrochemically acceptable salts of the present invention are useful as selective herbicides for rice, corn, wheat, sugar beet and soybean.

The entire disclosures of Japanese Patent Application No. 2008-225137 filed on September 2, 2008, Japanese Patent Application No. 2008-260652 filed on October 7, 2008, Japanese Patent Application No. 2009-031818 filed on February 13, 2009, Japanese Patent Application No. 2009-066161 filed on March 18, 2009, Japanese Patent Application No. 2009-066163 filed on March 18, 2009 and Japanese Patent Application No. 2009-149403 filed on June 24, 2009 including specifications, claims and summaries are incorporated herein by reference in their entireties.

## Claims

1. A haloalkylsulfonanilide derivative represented by the formula (1) or a agrochemically acceptable salt thereof:
the formula (1): 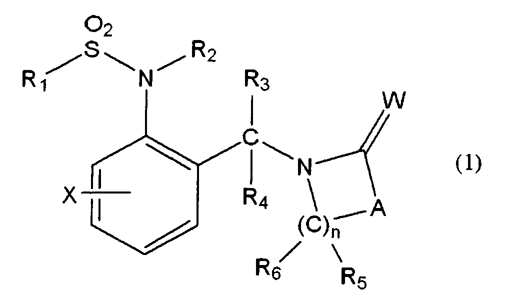 wherein A is C(R₇)(R₈)- or -N(R₉)-,
W is an oxygen atom or a sulfur atom,
n is an integer of from 1 to 4,
R₁ is halo C₁- C₆ alkyl,
R₂ is a hydrogen atom, C₁-C₆ alkyl, halo C₁-C₆ alkyl, C₂-C₆ alkenyl, halo C₂-C₆ alkenyl, C₂-C₆ alkynyl, halo C₂-C₆ alkynyl, C₁-C₆ alkoxy C₁-C₆ alkyl, halo C₁-C₆ alkoxy C₁-C₆ alkyl, C₁-C₆ alkoxy C₁-C₆ alkoxy C₁-C₆ alkyl, tri C₁-C₆ alkylsilyl C₁-C₆ alkoxy C₁-C₆ alkyl, phenyl C₁-C₆ alkyl, substituted phenyl C₁-C₆ alkyl having at least one substituent selected from Y, C₁-C₁₂ alkylcarbonyl, halo C₁-C₁₂ alkylcarbonyl, cyclo C₃-C₆ alkylcarbonyl, cyclo C₃-C₆ alkyl C₁-C₆ alkylcarbonyl, C₂-C₆ alkenylcarbonyl, phenylcarbonyl, substituted phenylcarbonyl having at least one substituent selected from Y, heterocyclic carbonyl, substituted heterocyclic carbonyl having at least one substituent selected from Y, C₁-C₁₂ alkoxycarbonyl, halo C₁-C₁₂ alkoxycarbonyl, C₁-C₆ alkoxy C₁-C₆ alkoxycarbonyl, C₁-C₆ alkylthio C₁-C₆ alkoxycarbonyl, C₁-C₆ alkylsulfinyl C₁-C₆ alkoxycarbonyl, C₁-C₆ alkylsulfonyl C₁-C₆ alkoxycarbonyl, C₂-C₆ alkenyloxycarbonyl, halo C₂-C₆ alkenyloxycarbonyl, C₂-C₆ alkynyloxycarbonyl, phenoxycarbonyl, substituted phenoxycarbonyl having at least one substituent selected from Y, phenyl C₁-C₆ alkoxycarbonyl, substituted phenyl C₁-C₆ alkoxycarbonyl having at least one substituent selected from Y, phenoxy C₁-C₆ alkylcarbonyl, substituted phenoxy C₁-C₆ alkylcarbonyl having at least one substituent selected from Y, mono(C₁-C₆ alkyl)aminocarbonyl, mono(halo C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, C₁-C₆ alkylthiocarbonyl, halo C₁-C₆ alkylthiocarbonyl, C₁-C₆ alkylsulfonyl, halo C₁-C₆ alkylsulfonyl, phenylsulfonyl, substituted phenylsulfonyl having at least one substituent selected from Y, C₁-C₆ alkylthio C₁-C₆ alkyl, halo C₁-C₆ alkylthio C₁-C₆ alkyl, phenyl C₁-C₆ alkoxy C₁-C₆ alkyl, substituted phenyl C₁-C₆ alkoxy C₁-C₆ alkyl having at least one substituent selected from Y, phenylthio C₁-C₆ alkyl, substituted phenylthio C₁-C₆ alkyl having at least one substituent selected from Y, phenyl C₁-C₆ alkylthio C₁-C₆ alkyl, substituted phenyl C₁-C₆ alkylthio C₁-C₆ alkyl having at least one substituent selected from Y, phenylsulfonyl C₁-C₆ alkyl, substituted phenylsulfonyl C₁-C₆ alkyl having at least one substituent selected from Y, phenyl C₁-C₆ alkylsulfonyl C₁-C₆ alkyl, substituted phenyl C₁-C₆ alkylsulfonyl C₁- C₆ alkyl having at least one substituent selected from Y, C₁-C₆ alkylcarbonyloxy C₁-C₆ alkyl, phenylcarbonyloxy C₁-C₆ alkyl, substituted phenylcarbonyloxy C₁ - C₆ alkyl having at least one substituent selected from Y, phenylcarbonyl C₁ - C₆ alkyl, substituted phenylcarbonyl C₁ - C₆ alkyl having at least one substituent selected from Y, C₁ - C₆ alkoxycarbonyloxy C₁ - C₆ alkyl, phenylcarbonyloxy C₁ - C₆ alkoxy C₁ - C₆ alkyl, substituted phenylcarbonyloxy C₁ - C₆ alkoxy C₁ - C₆ alkyl having at least one substituent selected from Y, mono C₁ - C₆ alkylaminocarbonyloxy C₁ - C₆ alkyl, symmetric or asymmetric di(C₁ - C₆ alkyl)aminocarbonyloxy C₁ - C₆ alkyl, phenylaminocarbonyloxy C₁ - C₆ alkyl, substituted phenylaminocarbonyloxy C₁ - C₆ alkyl having at least one substituent selected from Y, C₁ - C₆ alkyl(phenyl)aminocarbonyloxy C₁ - C₆ alkyl, substituted C₁ - C₆ alkyl(phenyl)aminocarbonyloxy C₁ - C₆ alkyl having at least one substituent selected from Y, C₁ - C6 alkylcarbonyl C₁ - C₆ alkyl, C₁ - C₆ alkoxycarbonyl C₁ - C₆ alkyl, halo C₁ - C₆ alkylthio, symmetric or asymmetric di(C₁ - C₆ alkyl)aminothio or cyano,
each of R₃ and R₄ is independently a hydrogen atom, C₁ - C₆ alkyl, halo C₁ - C₆ alkyl, cyclo C₃ - C₆ alkyl, halocyclo C₃ - C₆ alkyl, C₁ - C₆ alkoxy, halo C₁ - C₆ alkoxy, halogen or cyano, or R₃ and R₄ may form a 3- to 7-membered ring together with each other,
each of R₅, R₆, R₇ and R₈ is independently a hydrogen atom, a halogen, C₁ - C6 alkyl, halo C₁ - C₆ alkyl, C₂ - C₆ alkenyl, C₂ - C₆ alkynyl, cyclo C₃ - C₆ alkyl, halocyclo C₃ - C₆ alkyl, C₁ - C₆ alkoxy C₁ - C₆ alkyl, halo C₁ - C₆ alkoxy C₁ - C₆ alkyl, hydroxy C₁ - C₆ alkyl, C₁ - C₆ alkylthio C₁ - C₆ alkyl, halo C₁ - C₆ alkylthio C₁ - C₆ alkyl, mono(C₁ - C₆ alkyl)amino C₁ - C₆ alkyl, symmetric or asymmetric di(C₁ - C₆ alkyl)amino C₁ - C₆ alkyl, phenyl, substituted phenyl having at least one substituent selected from Y, heterocyclyl, substituted heterocyclyl having at least one substituent selected from Y, phenyl C₁ - C₆ alkyl, substituted phenyl C₁ - C₆ alkyl having at least one substituent selected from Y, phenoxy C₁ - C₆ alkyl, substituted phenoxy C₁ - C₆ alkyl having at least one substituent selected from Y, C₁ - C₆ alkylcarbonyl, halo C₁ - C₆ alkylcarbonyl, phenylcarbonyl, substituted phenylcarbonyl having at least one substituent selected from Y, C₁ - C₆ alkoxycarbonyl, halo C₁ - C₆ alkoxycarbonyl, carboxyl, mono(C₁ - C₆ alkyl)aminocarbonyl, symmetric or asymmetric di(C₁ - C₆ alkyl)aminocarbonyl, phenylaminocarbonyl, substituted phenylaminocarbonyl having at least one substituent selected from Y on the ring, phenyl C₁ - C₆ alkylaminocarbonyl, substituted phenyl C₁ - C₆ alkylaminocarbonyl having at least one substituent selected from Y on the ring, C₁ - C₆ alkoxy, halo C₁ - C₆ alkoxy, phenoxy, substituted phenoxy having at least one substituent selected from Y on the ring, C₁ - C₆ alkylthio, halo C₁ - C₆ alkylthio, phenylthio, substituted phenylthio having at least one substituent selected from Y on the ring, C₁ - C₆ alkylsulfonyl, halo C₁ - C₆ alkylsulfonyl, mono(C₁ - C₆ alkyl)amino, di(C₁ - C₆ alkyl)amino, C₁ - C₆ alkylcarbonyloxy, hydroxy, amino, cyano or nitro,
R₉ is a hydrogen atom, C₁ - C₆ alkyl, halo C₁ - C₆ alkyl, cyclo C₃ - C₆ alkyl, halocyclo C₃ - C₆ alkyl, C₂ - C₆ alkenyl, halo C₂ - C₆ alkenyl, C₂ - C₆ alkynyl, C₁ - C₆ alkoxy C₁ - C₆ alkyl, halo C₁ - C₆ alkoxy C₁ - C₆ alkyl, hydroxy C₁ - C₆ alkyl, cyclo C₃ - C₆ alkyl C₁ - C₆ alkyl, phenyl C₁ - C₆ alkyl, substituted phenyl C₁ - C₆ alkyl having at least one substituent selected from Y on the ring, phenyl, substituted phenyl having at least one substituent selected from Y on the ring, phenoxy C₁ - C₆ alkyl, substituted phenoxy C₁ - C₆ alkyl having at least one substituent selected from Y on the ring, C₁ - C₆ alkylcarbonyl C₁ - C₆ alkyl, halo C₁ - C₆ alkylcarbonyl C₁ - C₆ alkyl, C₁ - C₆ alkoxycarbonyl C₁ - C₆ alkyl, halo C₁ - C₆ alkoxycarbonyl C₁ - C₆ alkyl, C₁ - C₆ alkylcarbonyl, halo C₁ - C₆ alkylcarbonyl, cyclo C₃ - C₆ alkylcarbonyl, cyclo C₃ - C₆ alkyl C₁ - C₆ alkylcarbonyl, C₂ - C₆ alkenylcarbonyl, phenylcarbonyl, substituted phenylcarbonyl having at least one substituent selected from Y on the ring, heterocyclic carbonyl, substituted heterocyclic carbonyl having at least one substituent selected from Y, C₁ - C₆ alkoxycarbonyl, halo C₁ - C₆ alkoxycarbonyl, phenoxycarbonyl, substituted phenoxycarbonyl having at least one substituent selected from Y, aminocarbonyl, mono(C₁ - C₆ alkyl)aminocarbonyl, monohalo(C₁ - C₆ alkyl)aminocarbonyl, symmetric or asymmetric di(C₁ - C₆ alkyl)aminocarbonyl, symmetric or asymmetric halo di(C₁ - C₆ alkyl)aminocarbonyl, C₁ - C₆ alkylthiocarbonyl, halo C₁ - C₆ alkylthiocarbonyl, C₁ - C₆ alkylsulfonyl, halo C₁ - C₆ alkylsulfonyl, phenylsulfonyl, substituted phenylsulfonyl having at least one substituent selected from Y, C₁ - C₆ alkylthio C₁ - C₆ alkyl, halo C₁ - C₆ alkylthio C₁ - C₆ alkyl, phenylthio C₁ - C₆ alkyl, substituted phenylthio C₁ - C₆ alkyl having at least one substituent selected from Y, C₁ - C₆ alkylsulfinyl C₁ - C₆ alkyl, halo C₁ - C₆ alkylsulfinyl C₁ - C₆ alkyl, phenylsulfinyl C₁ - C₆ alkyl, substituted phenylsulfinyl C₁ - C₆ alkyl having at least one substituent selected from Y, C₁ - C₆ alkylsulfonyl C₁ - C₆ alkyl, halo C₁ - C₆ alkylsulfonyl C₁ - C₆ alkyl, phenylsulfonyl C₁ - C₆ alkyl, substituted phenylsulfonyl C₁ - C₆ alkyl having at least one substituent selected from Y, cyano, amino or hydroxy, or
R₅, R₆, R₇ or R₈ may form, together with R₅, R₆, R₇ or R₈ on the same carbon, an optionally substituted 3- to 7-membered ring which may contain one or two hetero atoms selected from oxygen atoms, sulfur atoms and nitrogen atoms (the nitrogen atoms may be substituted with C₁ - C₆ alkyl, C₂ - C₆ alkenyl, C₂ - C₆ alkynyl or cyclo C₃ - C₆ alkyl), an olefin or carbonyl, or
R₅, R₆, R₇ or R₈ may form, together with R₅, R₆, R₇ or R₈ on a different carbon, an optionally substituted 3- to 8-membered ring which may contain one or two hetero atoms selected from oxygen atoms, sulfur atoms and nitrogen atoms (the nitrogen atoms may be substituted with C₁ - C₆ alkyl, C₂ - C₆ alkenyl, C₂ - C₆ alkynyl or cyclo C₃ - C₆ alkyl), or
R₅ or R₆ may form a bond together with R₅ or R₆ on an adjacent carbon,
when n is from 2 to 4, R₅ or R₆ may be the same as or different from R₅ or R₆ on an adjacent carbon,
X is halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, phenyl, substituted phenyl having at least one substituent selected from Y on the ring, heterocyclyl, substituted heterocyclyl having at least one substituent selected from Y on the ring, phenoxy, substituted phenoxy having at least one substituent selected from Y on the ring, phenylthio, substituted phenylthio having at least one substituent selected from Y on the ring, phenylsulfinyl, substituted phenylsulfinyl having at least one substituent selected from Y on the ring, phenylsulfonyl, substituted phenylsulfonyl having at least one substituent selected from Y on the ring, C₁ - C₆ alkylcarbonyl, halo C₁ - C₆ alkylcarbonyl, phenylcarbonyl, substituted phenylcarbonyl having at least one substituent selected from Y on the ring, C₁ - C₆ alkoxycarbonyl, halo C₁ - C₆ alkoxycarbonyl, carboxyl, mono(C₁ - C₆ alkyl)aminocarbonyl, monohalo(C₁ - C₆ alkyl)aminocarbonyl, symmetric or asymmetric di(C₁ - C₆ alkyl)aminocarbonyl, symmetric or asymmetric halo di(C₁ - C₆ alkyl)aminocarbonyl, phenylaminocarbonyl, substituted phenylaminocarbonyl having at least one substituent selected from Y on the ring, phenyl C₁ - C₆ alkylaminocarbonyl, substituted phenyl C₁ - C₆ alkylaminocarbonyl having at least one substituent selected from Y on the ring, hydroxy, amino, cyano or nitro,
Y is a halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, phenyl, substituted phenyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, heterocyclyl, substituted heterocyclyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenoxy, substituted phenoxy having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenylthio, substituted phenylthio having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenylsulfinyl, substituted phenylsulfinyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C6 alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenylsulfonyl, substituted phenylsulfonyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, C₁ - C₆ alkylcarbonyl, halo C₁ - C₆ alkylcarbonyl, phenylcarbonyl, substituted phenylcarbonyl having at least one substituent selected from the group consisting of halogen, C₁ - C₆ alkyl, C₂ - C₆ alkenyl, C₂ - C₆ alkynyl, cycloC₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, C₁ - C₆ alkoxycarbonyl, carboxyl, mono(C₁ - C₆ alkyl)aminocarbonyl, symmetric or asymmetric di(C₁ - C₆ alkyl)aminocarbonyl, phenylaminocarbonyl, substituted phenylaminocarbonyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ -C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, phenyl C₁ - C₆ alkylaminocarbonyl, substituted phenyl C₁ - C₆ alkylaminocarbonyl having at least one substituent selected from the group consisting of halogen, C₁ - C₆ alkyl, C₂ - C₆ alkenyl, C₂ - C₆ alkynyl, cyclo C₃ - C₆ alkyl, halo C₁ - C₆ alkyl, halocyclo C₃ - C₆ alkyl, C₁ - C₆ alkoxy, halo C₁ - C₆ alkoxy, C₁ - C₆ alkylthio, halo C₁ - C₆ alkylthio, C₁ - C₆ alkylsulfinyl, halo C₁ - C₆ alkylsulfinyl, C₁ - C₆ alkylsulfonyl, halo C₁ - C₆ alkylsulfonyl, C₁ - C₆ alkylcarbonyl, halo C₁ - C₆ alkylcarbonyl, C₁ - C₆ alkoxycarbonyl, carboxyl, mono(C₁ - C₆ alkyl)aminocarbonyl, symmetric or asymmetric di(C₁ - C₆ alkyl)aminocarbonyl, hydroxy, amino, cyano and nitro, hydroxy, amino, cyano or nitro, or
Y may be C₁ - C₄ alkylene, halo C₁ - C₄ alkylene, C₂ - C₄ alkenylene or halo C₂ - C₄ alkenylene which may contain one or two hetero atoms selected from oxygen atoms, sulfur atoms and nitrogen atoms (the nitrogen atoms may be substituted with C₁ - C₆ alkyl, C₂ - C₆ alkenyl, C₂ - C₆ alkynyl or cyclo C₃ - C₆ alkyl) and form a 5- or 6-membered ring together with an adjacent carbon or nitrogen atom on a benzene ring or a heterocyclyl, and
the heteroxyclyl is thienyl, furyl, pyrrolyl, oxazolyl, isoxazolyl, isoxazolinyl, thiazolyl, isothiazolyl, pyrazolyl, imidazolyl, 1,3,4-oxadiazolyl, 1,2,4-oxadiazolyl, 1,3,4-thiadiazolyl, 1,2,4-thiadiazolyl, 1,2,4-triazolyl, 1,2,3-thiadiazolyl, 1,2,3-triazolyl, 1,2,3,4-tetrazolyl, pyridyl, pyrimidinyl, pyrazinyl, pyridazinyl, 1,3,5-triazinyl, 1,2,4-triazinyl, benzothienyl, benzofuryl, indolyl, benzothiazolyl, benzoimidazolyl, benzoisoxazolyl, benzoisothiazolyl, indazolyl, benzoxazolyl, quinolyl, isoquinolyl, quinoxalinyl, phthalazinyl, cinnolinyl or quinazolinyl,
provided that in a group having two or more substituents, the substituents may be the same or different.

2. The haloalkylsulfonanilide derivative according to Claim 1 wherein n is 3 or 4, or an agrochemically acceptable salt thereof.

3. The haloalkylsulfonanilide derivative according to Claim 1 wherein A is -C(R₇)(R₈)-, or an agrochemically acceptable salt thereof.

4. The haloalkylsulfonanilide derivative according to Claim 3 wherein n is 1, or an agrochemically acceptable salt thereof.

5. The haloalkylsulfonanilide derivative according to Claim 3 wherein n is 2, or an agrochemically acceptable salt thereof.

6. The haloalkylsulfonanilide derivative according to Claim 3 wherein n is 3, or an agrochemically acceptable salt thereof.

7. The haloalkylsulfonanilide derivative according to Claim 3 wherein n is 4, or an agrochemically acceptable salt thereof.

8. The haloalkylsulfonanilide derivative according to any one of Claims 1 to 7 wherein R₃ and R₄ are hydrogen atoms, or an agrochemically acceptable salt thereof.

9. An agrochemical containing the haloalkylsulfonanilide derivative as defined in any one of Claims 1 to 8 or an agrochemically acceptable salt thereof, as an active ingredient.

10. A herbicide containing the haloalkylsulfonanilide derivative as defined in any one of Claims 1 to 8 or an agrochemically acceptable salt thereof, as an active ingredient.

11. An intermediate represented by the formula (2):
the formula (2): 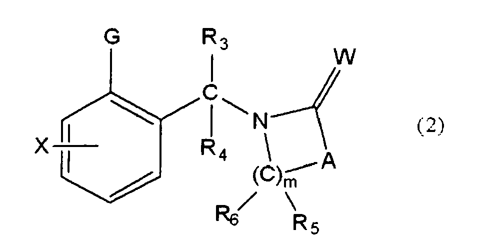 wherein A, W, R₃, R₄, R₅, R₆ and X are the same as defined above,
m is an integer of 3 or 4, and
G is amino or nitro,
provided that wherein A is -C(R₇)(R₈)-, at least one of R₅, R₆, R₇ and R₈ is not a hydrogen atom.

12. An intermediate represented by the formula (3):
the formula (3): 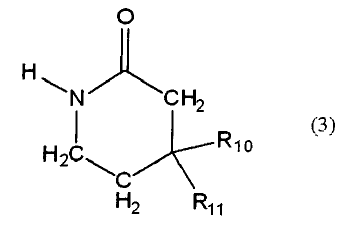 wherein R₁₀ and R₁₁ are C₁ - C₆ alkylene and form, together with each other, a 3-to 7-membered ring which may contain one or two hetero atoms selected from oxygen atoms, sulfur atoms and nitrogen atoms (the nitrogen atoms may be substituted with C₁ - C₆ alkyl, C₂ - C₆ alkenyl, C₂ - C₆ alkynyl or cyclo C₃ - C₆ alkyl).
